# EUROPEAN PATENT SPECIFICATION

(11) **EP 1 981 881 B1**
(45) Date of publication and mention of the grant of the patent: **07.12.2011**
(21) Application number: 07705700.8
(22) Date of filing: 25.01.2007
(51) Int. Cl.: C07D 405/06, C07D 405/12, C07D 471/04, C07D 513/04, A61K 31/435, A61K 31/54, A61P 31/00

(54) **TETRAHYDROPYRANE ANTIBIOTICS**
TETRAHYDROPYRAN-ANTIBIOTIKA
ANTIBIOTIQUES DE TYPE TÉTRAHYDROPYRANNE

(30) Priority: 26.01.2006 WO PCT/IB2006/050288; 03.03.2006 WO PCT/IB2006/050675
(43) Date of publication of application: 22.10.2008
(73) Proprietor: Actelion Pharmaceuticals Ltd., 4123 Allschwil (CH)
(72) Inventor: HUBSCHWERLEN, Christian, F-68480 Durmenach (FR); SURIVET, Jean-Philippe, F-68680 Kembs (FR); ZUMBRUNN ACKLIN, Cornelia, CH-4058 Basel (CH)
(74) Representative: Ruhlmann, Eric
(86) International application number: PCT/IB2007/050252
(87) International publication number: WO 2007/086016

(56) References cited:
- WO-A-03/064421
- WO-A-2004/035569
- DE-A1- 10 256 405

## Description

The present invention concerns novel antibiotics, a pharmaceutical antibacterial composition containing them and the use of these compounds in the manufacture of a medicament for the treatment of infections (e.g. bacterial infections). These compounds are useful antimicrobial agents effective against a variety of human and veterinary pathogens including among others Gram positive and Gram negative aerobic and anaerobic bacteria and mycobacteria.

The intensive use of antibiotics has exerted a selective evolutionary pressure on micro-organisms to produce genetically based resistance mechanisms. Modem medicine and socio-economic behaviour exacerbates the problem of resistance development by creating slow growth situations for pathogenic microbes, e.g. in artificial joints, and by supporting long-term host reservoirs, e.g. in immuno-compromised patients.

In hospital settings, an increasing number of strains of *Staphylococcus aureus, Streptococcus pneumoniae, Enterococcus spp.,* and *Pseudomonas aeruginosa,* major sources of infections, are becoming multi-drug resistant and therefore difficult if not impossible to treat:
- *S. aureus* is resistant to ß-lactam and quinolone antibiotics and now even to vancomycin;
- *S. pneumoniae* is becoming resistant to penicillin and quinolone antibiotics and even to new macrolides;
- *Enteroccocci* are quinolone and vancomycin resistant and ß-lactam antibiotics are inefficacious against these strains;
- *Enterobacteriacea* are cephalosporin and quinolone resistant;
- *P. aeruginosa* are ß-lactam and quinolone resistant.

Further new emerging organisms like *Acinetobacter spp.* or *C*. *difficile,* which have been selected during therapy with the currently used antibiotics, are becoming a real problem in hospital settings.

In addition, microorganisms that are causing persistent infections are increasingly being recognized as causative agents or cofactors of severe chronic diseases like peptic ulcers or heart diseases.

A new type of quinoline or naphthyridine derivatives having antibacterial activity and therefore useful for treating infections in mammals, particularly in humans, has been reported.

WO 99/37635, WO 00/21948, WO 00/21952, WO 00/43383, WO 03/101138, WO 01/25227, WO 02/40474 and WO 2004/011454 disclose quinoline, naphthyridine and quinazoline derivatives containing a 4-methylpiperidinyl spacer.

WO 00/78748, WO 02/50040 and WO 02/50061 disclose quinoline and naphthyridine derivatives containing a piperazinyl spacer.

WO 01/07432, WO 01/07433, WO 02/08224, WO 02/056882, WO 03/064421, WO 03/064431, WO 2004/002490 and WO 2004/058144 disclose quinoline, quinoxaline and naphthyridine derivatives containing a 4-aminopiperidinyl spacer. In particular, WO 03/064421 discloses quinoline derivatives and azaisosteres thereof wherein a 4-aminopiperidinyl group is linked through a 2 to 3 atom bridge to position 4 of the quinoline or azaisostere core.

WO 2004/035569 discloses quinoline and naphthyridine derivatives containing a 3-aminomethylpiperidinyl spacer.

WO 2004/002992, WO 03/087098, WO 2004/014361 and WO 2004/035569 disclose quinoline, quinoxaline and naphthyridine derivatives containing a 4-aminocyclohexyl spacer. In particular, WO 2004/035569 discloses quinoline derivatives and azaisosteres thereof wherein a 1-piperidinyl, 4-piperidinyl group or 1-piperazinyl group is linked through a 2 to 5 atom bridge to position 4 of the quinoline or azaisostere core.

Besides, PCT application No. PCT/EP2005/010154 (published as WO 2006/032466) discloses antibacterial compounds that are structurally similar to those of the instant invention except however the fact that their tetrahydropyrane spacer bears different substituents.

It has now been found that certain novel bicyclic derivatives are useful antimicrobial agents and effective against a variety of multi-drug resistant bacteria. Thus, the present invention relates to novel bicyclic derivatives of the formula 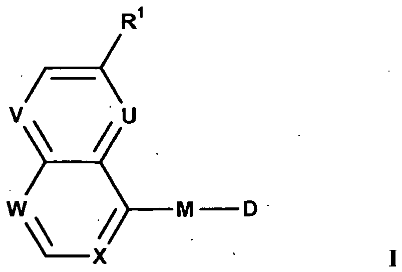 wherein
R¹ represents alkyl, alkoxy, haloalkoxy, halogen or cyano;
one or two ofU, V, W and X represent(s) N and the remaining each represent CH or, in the case of X, may also represent CR^{a};
R^{a} represents halogen;
M is 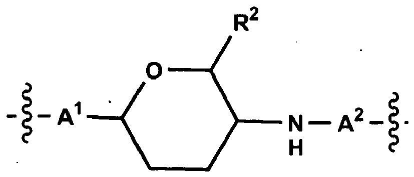 wherein
A¹ represents NHCO, CH₂CH₂, CH=CH, CH(OH)CH₂, CH₂CH(OH), CH(OH)CH(OH) or OCH₂;
A² represents CH₂, CO, CH₂CH=CH or COCH=CH;
R² represents carboxy, carbamoyl, hydroxycarbamoyl, cyano or -CH₂R³,
R³ representing hydroxycarbamoyl, (C₂-C₄)alkynylmethoxycarbamoyl, (C₁-C₃)dialkylcarbamoyl, cycloalkylcarbamoyl, [(C₂-C₅)hydroxyalkyl]carbamoyl, (arylalkyl)carbamoyl, cyano, (C₁-C₅)alkylaminomethyl, (C₁-C₅)dialkylaminomethyl, (C₁-C₃)alkoxyimino, hydroxyimino, 5-membered heteroaryl, or heteroarylalkyl wherein the heteroaryl is a 5-membered heteroaryl and the alkyl is a (C₁-C₄)alkyl,
or R³ representing a radical -NR⁴R⁵ wherein R⁴ and R⁵, taken together with the nitrogen atom that carries them, form a 5- or 6-membered heterocyclic ring, the members being used to complete said heterocyclic ring being chosen independently from -CH₂-, -O- and -S-, it being understood that only one member selected from -O- and -S- may be present in said heterocyclic ring,
or also R³ representing a radical -CH(OH)R⁶,
R⁶ representing hydroxymethyl, (C₁-C₃)alkylaminomethyl, (C₁-C₃)dialkylaminomethyl, phenylaminomethyl wherein the phenyl group may be substituted once by a member of the group consisting of halogen, alkoxy and carboxy, heteroarylaminomethyl wherein the heteroaryl is a 5-membered heteroaryl, benzylaminomethyl wherein the phenyl of the benzyl group may be substituted once by a member of the group consisting of halogen, alkoxy and carboxy, or a radical -CH₂-NR⁷R⁸ wherein R⁷ and R⁸, taken together with the nitrogen atom that carries them, form a 5- or 6-membered heterocyclic ring, the members being used to complete said heterocyclic ring being chosen independently from -CH₂-, -O- and -S-, it being understood that only one member selected from -O- and -S- may be present in said heterocyclic ring,
or furthermore R³ representing a radical -CO-NR^{x}R^{y} wherein the group -NR^{x}R^{y} is selected from pyrrolidin-1-yl, piperidin-1-yl, morpholin-4-yl, thiomorpholin-4-yl, (3*R*)-3-hydroxypyrrolidin-1-yl, (3*S*)-3-hydroxy-pyrrolidin-1-yl and 4-hydroxy-piperidin-1-yl;
D represents aryl or heteroaryl;
and to salts of compounds of formula I.

The following paragraphs provide definitions of the various chemical moieties for the compounds according to the invention and are intended to apply uniformly throughout the specification and claims, unless an otherwise expressly set out definition provides a broader or narrower definition:
❖ The term "alkyl", used alone or in combination, refers to a straight or branched chain alkyl group, containing from one to ten, preferably one to six, and in particular one to four carbon atoms. Representative examples of alkyl groups include, but are not limited to, methyl, ethyl, propyl, *iso*-propyl, *n*-butyl, *iso*-butyl, *sec*-butyl, *tert*-butyl, *n*-pentyl, *iso*-pentyl, *n*-hexyl, 2,2-dimethylbutyl, *n*-heptyl or *n*-octyl. The term "(C₁-Cₓ)alkyl" (x being an integer) refers to a straight or branched chain alkyl group containing 1 to x carbon atoms.
❖ The term "alkoxy", used alone or in combination, refers to a straight or branched chain alkoxy group, containing from one to ten, preferably one to six, and in particular one to four carbon atoms. Representative examples of alkoxy groups include, but are not limited to, methoxy, ethoxy, propoxy, *iso*-propoxy, *n*-butoxy, *iso*-butoxy, *sec*-butoxy, *tert*-butoxy or *n*-hexyloxy. The term "(C₁-Cₓ)alkoxy" refers to a straight or branched chain alkoxy group containing 1 to x carbon atoms.
❖ The term "haloalkoxy" refers to a straight or branched chain alkoxy group, containing from one to six and preferably one to four carbon atoms, in which at least one hydrogen atom (and possibly all) has been replaced by a halogen atom. Representative examples of haloalkoxy groups include, but are not limited to, trifluoromethoxy or difluoromethoxy. The term "(C₁-Cₓ)haloalkoxy" (x being an integer) refers to a straight of branched chain haloalkoxy group containing 1 to x carbon atoms.
❖ The term "halogen" refers to fluorine, chlorine, bromine or iodine, preferably to fluorine or chlorine.
❖ The term "alkynyl", used alone or in combination, refers to a straight or branched hydrocarbon chain of 2 to 6 (and preferably 2 to 4) carbon atoms with at least one carbon-carbon triple bond. Representative examples of alkynyl groups include, but are not limited to, prop-2-ynyl and but-3-ynyl. The term "(C₂-Cₓ)alkynyl" (x being an integer) refers to a straight or branched chain alkynyl group containing 2 to x carbon atoms.
❖ The term "cycloalkyl", used alone or in combination, refers to a saturated cyclic hydrocarbon moiety of 3 to 5 carbon atoms. Representative examples of cycloalkyl groups include, but are not limited to, cyclopropyl.and cyclopentyl.
❖ The term "hydroxyalkyl", used alone or in combination, refers to an alkyl group wherein one of the hydrogen atoms has been replaced by a hydroxy group. Representative examples of hydroxyalkyl groups include, but are not limited to, hydroxymethyl and 2-hydroxy-ethyl. The term "(Cₓ-C_{y})hydroxyalkyl" (x and y being integers) refers to a hydroxyalkyl group containing x to y carbon atoms.
❖ The term "aryl", used alone or in combination, refers to an aromatic cyclic group with one, two or three rings, having five to 14 carbon ring atoms and preferably from five or six to ten carbon ring atoms, for example phenyl or naphthyl groups. Any aryl group as defined herein may be substituted with one, two or more substituents, each of which is independently selected from the group consisting of halogen, alkyl, alkoxy, trifluoromethyl and trifluoromethoxy. Specific examples of aryl are phenyl, naphtyl, 4-fluoro-phenyl, 4-chloro-phenyl, 4-methoxy-phenyl, 4-methyl-phenyl, 4-trifluoromethyl-phenyl, 4-trifluoromethoxy-phenyl, 2,4-difluoro-phenyl, 2,4-dichloro-phenyl, 2,4-dimethoxy-phenyl, 2,4-dimethyl-phenyl, 2,5-difluorophenyl, 2,4-ditrifluoromethyl-phenyl and 2,4-ditrifluoromethoxy-phenyl.
❖ The term "heteroaryl", used alone or in combination, refers to an aryl group as defined herein where one, two or more (preferably one to four, and notably one or two) ring carbon atoms are replaced by an oxygen, nitrogen or sulphur atom, for example pyridyl, imidazolyl, pyrazolyl, quinolinyl, isoquinolinyl, naphthyridinyl, quinazolinyl, quinoxazinyl, pyrrolyl, thienyl, oxazolyl, isoxazolyl, thiazolyl, isothiazolyl, 1,2,3-triazolyl, 1,2,4-triazolyl, oxadiazolyl, thiadiazolyl, indolyl, indazolyl, tetrazolyl, pyrazinyl, pyrimidinyl and pyridazinyl groups. The term "heteroaryl" also covers bicyclic structures selected from the group consisting of benzo[1,3]dioxol-5-yl, 2,3-dihydro-benzo[1,4]dioxin-6-yl, 2,3-dihydro-[1,4]dioxino[2,3-*b*]pyridin-6-yl, 2,3-dihydro-[1,4]dioxino[2,3-*c*]pyridin-7-yl, 3-oxo-3,4-dihydro-2*H*-benzo[1,4]thiazin-6-yl, 3-oxo-3,4-dihydro-2*H*-benzo[1,4]oxazin-6-yl, 3-oxo-3,4-dihydro-2*H*-pyrido[3,2-*b*][1,4]thiazin-6-yl, 3-oxo-3,4-dihydro-2*H*-pyrido[3,2-b][1,4]oxazin-6-yl, 2-oxo-2,3-dihydro-1*H*-pyrido[3,4-*b*][1,4]oxazine-7-yl, 2-oxo-3,4-dihydro-1*H*-quinolin-7-yl, benzo[1,2,5]thiadiazol-5-yl, chroman-7-yl and benzofuran-3-yl. Any heteroaryl group as defined herein may be substituted with one, two or more substituents on its aromatic ring(s), said substituents being from the group consisting of halogen, alkyl and alkoxy; preferably, any heteroaryl group as defined herein may be substituted with one halogen substituent. Hence, exemples of heteroaryl groups include, but are not limited to, benzo[1,3]dioxol-5-yl, 2,3-dihydro-benzo[1,4]dioxin-6-yl, 2,3-dihydro-[1,4]dioxino[2,3-*b*]pyridin-6-yl, 2,3-dihydro-[1,4]dioxino[2,3-*c*]pyridin-7-yl, 3-oxo-3,4-dihydro-2*H*-benzo[1,4]thiazin-6-yl, 7-fluoro-3-oxo-3,4-dihydro-2*H*-benzo[1,4]thiazin-6-yl, 3-oxo-3,4-dihydro-2*H*-benzo[1,4]oxazin-6-yl, 3-oxo-3,4-dihydro-2H-pyrido[3,2-*b*][1,4]thiazin-6-yl, 3-oxo-3,4-dihydro-2*H*-pyrido[3,2-*b*][1,4]oxazin-6-yl, 2-oxo-2,3-dihydro-1*H*-pyrido[3,4-*b*][1,4]oxazine-7-yl, 2-oxo-3,4-dihydro-1*H*-quinolin-7-yl, benzo[1,2,5]thiadiazol-5-yl, thiophen-2-yl, thiazol-2-yl, 2,2-dimethyl-chroman-7-yl and benzofuran-3-yl.
❖ The term "(C₂-C₄)alkynylmethoxycarbamoyl" refers to a carbamoyl group wherein one of the two hydrogen atoms attached to the nitrogen has been replaced by a (C₂-C₄)alkynylmethoxy group, i.e. a methoxy group wherein one of the hydrogen atoms has been replaced by a (C₂-C₄)alkynyl group. Representative examples of (C₂-C₄)alkynylmethoxycarbamoyl groups include, but are not limited to, prop-2-ynyloxycarbamoyl and but-3-ynyloxycarbamoyl.
❖ The term "(C₁-C₅)alkylcarbamoyl" refers to a carbamoyl group wherein one of the two hydrogen atoms attached to the nitrogen has been replaced by a (C₁-C₅)alkyl group. Representative examples of (C₁-C₅)alkylcarbamoyl groups include, but are not limited to, methylcarbamoyl, ethylcarbamoyl and *iso*-propylcarbamoyl.
❖ The term "(C₁-C₃)dialkylcarbamoyl" refers to a carbamoyl group wherein each of the hydrogen atoms attached to the nitrogen has been replaced by a (C₁-C₃)alkyl group, whereby the two (C₁-C₃)alkyl groups can be identical or different. Representative examples of (C₁-C₃)dialkylcarbamoyl groups include, but are not limited to, dimethylcarbamoyl and diethylcarbamoyl.
❖ The term "cycloalkylcarbamoyl" refers to a carbamoyl group wherein one of the two hydrogen atoms attached to the nitrogen has been replaced by a cycloalkyl group. Representative examples of cycloalkylcarbamoyl groups include, but are not limited to, cyclopropylcarbamoyl and cyclobutylcarbamoyl.
❖ The term "[(C₂-C₅)hydroxyalkyl]carbamoyl" refers to a carbamoyl group wherein one of the two hydrogen atoms attached to the nitrogen has been replaced by a (C₂-C₅)hydroxyalkyl group. Representative examples of [(C₂-C₅)hydroxyalkyl]carbamoyl groups include, but are not limited to, (2-hydroxy-ethyl)carbamoyl and (3-hydroxy-propyl)carbamoyl.
❖ The term "(C₁-C₃)alkoxyimino" refers to a hydroxyimino group wherein the hydrogen atom attached to the oxygen has been replaced by a (C₁-C₃)alkyl group.
❖ The term "(arylalkyl)carbamoyl" refers to a carbamoyl group wherein one of the two hydrogen atoms attached to the nitrogen has been replaced by an arylalkyl group. Representative examples of (arylalkyl)carbamoyl include, but are not limited to, benzylcarbamoyl, (2-phenylethyl)carbamoyl and (3-phenylpropyl)carbamoyl.
❖ The term "5-membered heteroaryl" refers to an unsubstituted heteroaryl of 5 ring members having from 1 to 4 heteroatoms selected from nitrogen, oxygen and sulphur. Representative examples of 5-membered heteroaryl include, but are not limited to, furan-2-yl, thien-2-yl, imidazol-2-yl, thiazol-2-yl, oxazol-2-yl, isoxazol-3-yl, 1,2,3-triazol-1-yl, 1,2,3,4-tetrazol-1-yl, 1,2,3,4-tetrazol-2-yl and 1,2,3,4-tetrazol-5-yl.
❖ The term "arylalkyl" (or "aralkyl"), as used herein, alone or in any combination, refers to an aryl group appended to the parent molecular moiety through an alkyl group wherein however the aryl group may be unsubstituted or substituted with 1 to 3 substituents selected independently from the group consisting of halogen, alkyl, alkoxy, trifluoromethyl and trifluoromethoxy. Representative examples of arylalkyl include, but are not limited to, benzyl, 2-phenylethyl, 3-phenylpropyl and 2-naphth-2-ylethyl. Preferred aralkyl groups are the phenylalkyl groups, especially benzyl.
❖ The term "phenylalkyl", as used herein, alone or in any combination, refers to an unsubstituted phenyl group appended to the parent molecular moiety through an alkyl group. Representative examples of phenylalkyl include, but are not limited to, benzyl, 2-phenylethyl and 3-phenylpropyl.
❖ The term "(C₁-Cₓ)alkylaminomethyl" (x being an integer) refers to an aminomethyl group wherein one of the two hydrogen atoms attached to the nitrogen has been replaced by a (C₁-Cₓ)alkyl group.
❖ The term "(C₁-Cₓ)dialkylaminomethyl" (x being an integer) refers to an aminomethyl group wherein both hydrogen atoms attached to the nitrogen have been replaced by (C₁-Cₓ)alkyl groups which may be the same or different.
❖ When in the formula 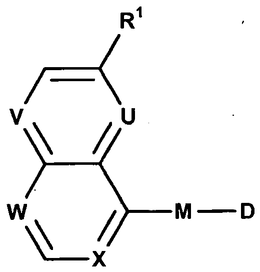 M represents the radical 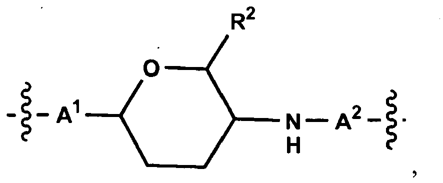 this means specifically that the radical A¹ is attached to the 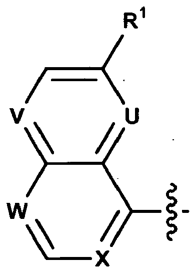 group whereas the radical A² is attached to the D group.
   This is applicable *mutatis mutandis* to all radicals that make M radicals (i.e. A¹ and A²). As a further example, in the substructure M, if it is stated that A² represents COCH=CH, it is thereby meant that the CO part of the COCH=CH radical is attached to the nitrogen and that the =CH part of the COCH=CH radical is attached to the D group. In other words, the left part of a radical is always attached to the right part of the radical that is next to the left.

Besides, the term "room temperature" as used herein refers to a temperature of 25°C.

Unless used regarding temperatures, the term "about" placed before a numerical value "X" refers in the current application to an interval extending from X minus 10% of X to X plus 10% of X, and preferably to an interval extending from X minus 5% of X to X plus 5% of X. In the particular case of temperatures, the term "about" placed before a temperature "Y" refers in the current application to an interval extending from the temperature Y minus 10 °C to Y plus 10 °C, and preferably to an interval extending from Y minus 5 °C to Y plus 5 °C.

A particular embodiment of this invention relates to compounds of formula Iₚ 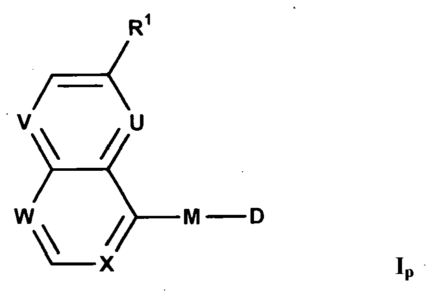 wherein
R¹ represents alkyl, alkoxy, haloalkoxy, halogen or cyano;
one or two of U, V, W and X represent(s) N and the remaining represent each independently CH or CR^{a};
R^{a} represents halogen;
M is 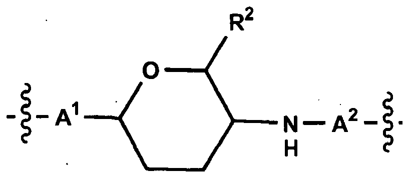 wherein
A¹ represents NHCO, CH₂CH₂, CH=CH, CH(OH)CH₂, CH₂CH(OH), CH(OH)CH(OH) or OCH₂;
A² represents CH₂, CO, CH₂CH=CH or COCH=CH;
R² represents carboxy, carbamoyl, hydroxycarbamoyl, (C₁-C₅)alkylcarbamoyl, cyano or CH₂R³,
R³ representing hydroxycarbamoyl, (C₁-C₅)alkylcarbamoyl, (arylalkyl)carbamoyl, cyano, (C₁-C₅)alkylaminomethyl, (C₁-C₅)dialkylaminomethyl, (C₁-C₃)alkoxyimino, hydroxyimino, 5-membered heteroaryl, or heteroarylalkyl wherein the heteroaryl is a 5-membered heteroaryl and the alkyl is a (C₁-C₄)alkyl,
or R³ representing a radical -NR⁴R⁵ wherein R⁴ and R⁵, taken together with the nitrogen atom that carries them, form a 5- or 6-membered heterocyclic ring, the members being used to complete said heterocyclic ring being chosen independently from -CH₂-, -O- and -S-, it being understood that only one member selected from -O- and -S- may be present in said heterocyclic ring,
or also R³ representing a radical CH(OH)R⁶,
R⁶ representing hydroxymethyl, (C₁-C₃)alkylaminomethyl, (C₁-C₃)dialkylaminomethyl, phenylaminomethyl wherein the phenyl group may be substituted once by a member of the group consisting of halogen, alkoxy and carboxy, heteroarylaminomethyl wherein the heteroaryl is a 5-membered heteroaryl, benzylaminomethyl wherein the phenyl of the benzyl group may be substituted once by a member of the group consisting of halogen, alkoxy and carboxy, or a radical -CH₂-NR⁷R⁸ wherein R⁷ and R⁸, taken together with the nitrogen atom that carries them, form a 5- or 6-membered heterocyclic ring, the members being used to complete said heterocyclic ring being chosen independently from -CH₂-, -O- and -S-, it being understood that only one member selected from -O- and -S- may be present in said heterocyclic ring;
D represents aryl or heteroaryl;
and to salts of compounds of formula I.

In particular, the invention relates to compounds of formula I that are also compounds of formula I_{CE} 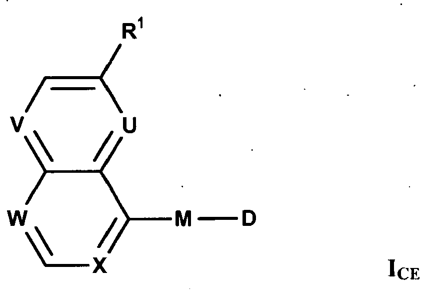 wherein
R¹ represents alkoxy or halogen (preferably (C₁-C₂)alkoxy or fluorine and notably methoxy);
one or two of U, V, W and X represent(s) N and the remaining represent CH, or, in the case of X, may also represent CR^{a};
R^{a} represents halogen (notably fluorine);
D represents phenyl substituted once or twice (and preferably twice) by halogen substituents or the heteroaryl group 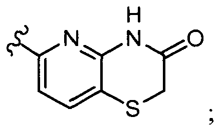 Mis 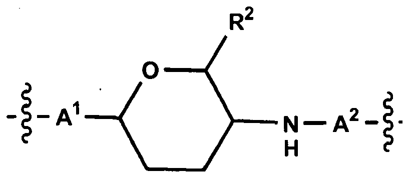 wherein
A¹ represents NHCO, CH₂CH₂, CH=CH, CH₂CH(OH), CH(OH)CH(OH) or OCH₂;
A² represents CH₂, CO, CH₂CH=CH or COCH=CH;
R² represents -CH₂R³,
R³ representing hydroxycarbamoyl, (C₂-C₄)alkynylmethoxycarbamoyl, dimethylcarbamoyl, cycloalkylcarbamoyl, [(C₂-C₅)hydroxyalkyl]carbamoyl, cyano, -CH(OH)R⁶ or -CO-NR^{x}R^{y},
R⁶ representing hydroxymethyl, and
the group -NR^{x}R^{y} is selected from morpholin-4-yl, thiomorpholin-4-yl and 4-hydroxypiperidin-1-yl (and notably from morpholin-4-yl and 4-hydroxy-piperidin-1-yl);
and to salts of compounds of formula I_{CE}.

More particularly, the invention relates to compounds of formula I that are also compounds of formula I_{CEP} 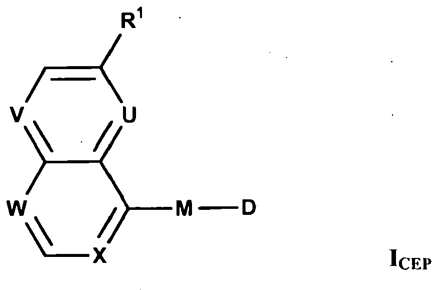 wherein
R¹ represents alkoxy (preferably (C₁-C₂)alkoxy and notably methoxy);
one or two of U, V, W and X represent(s) N and the remaining represent CH, or, in the case of X, may also represent CR^{a};
R^{a} represents halogen (notably fluorine);
D represents phenyl substituted once or twice by halogen substituents or the heteroaryl group 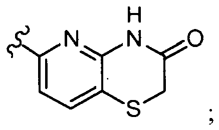 M is 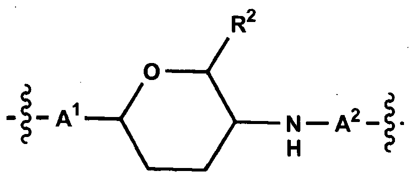 wherein
A¹ represents CH₂CH₂, CH=CH, CH₂CH(OH) or OCH₂;
A² represents CH₂, CO, CH₂CH=CH or COCH=CH;
R² represents CH₂R³,
R³ representing hydroxycarbamoyl, cyano, CH(OH)R⁶, and
R⁶ representing hydroxymethyl;
and to salts of compounds of formula I_{CEP}.

Preferably, the compounds of formula I_{CE} or I_{CEP} will be such that D represents 2,5-difluorophenyl or the heteroaryl group

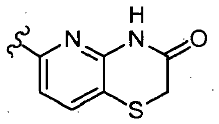

Preferred compounds of formula I are also those wherein at least one of the following characteristics is present:
❖ R¹ represents (C₁-C₃)alkyl, (C₁-C₃)alkoxy, (C₁-C₂)haloalkoxy, halogen or cyano;
❖ one or two of U, V, W and X represent(s) N, and the remaining represent CH or CR^{a}, R^{a} representing fluorine;
❖ A¹ represents NHCO, CH₂CH₂, CH=CH, CH(OH)CH₂, CH₂CH(OH) or OCH₂, or also, provided U is N, CH(OH)CH(OH);
❖ A² represents CH₂ or CO, or also, provided D is a non-annelated aryl or heteroaryl group, CH₂CH=CH or COCH=CH;
❖ R² represents carboxy, carbamoyl, hydroxycarbamoyl, cyano, -CH₂R³ (and in particular -CH₂R³),
   R³ being selected from the group consisting of hydroxycarbamoyl, (C₂-C₄)alkynylmethoxycarbamoyl, cycloalkylcarbamoyl, [(C₂-C₅)hydroxyalkyl]carbamoyl, cyano, -CH(OH)R⁶ and -CO-NR^{x}R^{y}, R⁶ being hydroxymethyl, and
   the group -NR^{x}R^{y} is selected from pyrrolidin-1-yl, piperidin-1-yl, morpholin-4-yl, thiomorpholin-4-yl, (3*R*)-3-hydroxy-pyrrolidin-1-yl, (3*S*)-3-hydroxy-pyrrolidin-1-yl and 4-hydroxy-piperidin-1-yl;
❖ D represents phenyl optionally substituted one to three times with substituents each selected independently from the group consisting of alkyl, alkoxy and halogen, or D represents a five or six-membered monocyclic heteroaryl or also D is a bicyclic heteroaryl of the formula 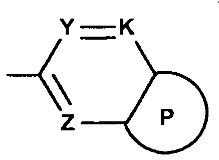 wherein
   K, Y and Z are each independently N or CR^{α},
   R^{α} is hydrogen or halogen, and
   P is a ring selected from the group consisting of the following rings: 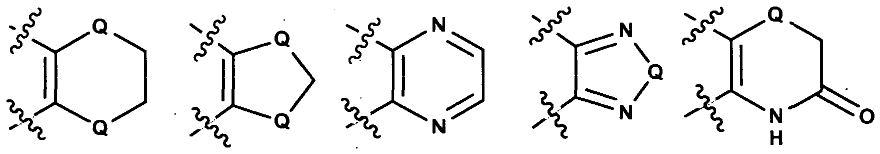 in which Q is O or S.

Preferred compounds of formula I_{P} are also those wherein at least one of the following characteristics is present:
❖ R¹ represents (C₁-C₃)alkyl, (C₁-C₃)alkoxy, (C₁-C₂)haloalkoxy, halogen or cyano;
❖ one or two of U, V, W and X represent(s) N, and the remaining represent CH or CR^{a}, R^{a} representing fluorine;
❖ A¹ represents NHCO, CH₂CH₂, CH=CH, CH(OH)CH₂, CH₂CH(OH) or OCH₂, or also, provided U is N, CH(OH)CH(OH);
❖ A² represents CH₂ or CO, or also, provided D is a non-annelated aryl or heteroaryl group, CH₂CH=CH or COCH=CH;
❖ R² represents carboxy, carbamoyl, hydroxycarbamoyl, (C₁-C₅)alkylcarbamoyl, cyano, CH₂R³,
   R³ being selected from the group consisting of hydroxycarbamoyl, (C₁-C₅)alkylcarbamoyl, (phenylalkyl)carbamoyl, cyano, imidazol-2-yl, 1,3-oxazol-2-yl, 1,3,4-oxadiazol-2-yl, 1,2,4-oxadiazol-5-yl, thiazol-2-yl, thiazol-4-yl, thiazol-5-yl, isoxazol-3-yl, furan-2-yl, thiophen-2-yl, 1,2,3-triazol-1-yl, 1,2,3-triazol-5-yl, 1,2,3,4-tetrazol-5-yl, 1,2,3,4-tetrazol-2-yl, 1,2,3,4-tetrazol-1-yl, imidazol-2-ylmethyl, 1,3-oxazol-2-ylmethyl, 1,3,4-oxadiazol-2-ylmethyl, 1,2,4-oxadiazol-5-ylmethyl, thiazol-2-ylmethyl, thiazol-4-ylmethyl, thiazol-5-ylmethyl, isoxazol-3-ylmethyl, furan-2-ylmethyl, thiophen-2-ylmethyl, 1,2,3-triazol-1-ylmethyl, 1,2,3-triazol-5-ylmethyl, 1,2,3,4-tetrazol-2-ylmethyl, 1,2,3,4-tetrazol-5-yl-methyl, 1,2,3,4-tetrazol-1-ylmethyl, (C₁-C₅)alkylaminomethyl, (C₁-C₅)dialkylaminomethyl, (C₁-C₃)alkoxyimino, hydroxyimino and CH(OH)R⁶, and
   R⁶ being hydroxymethyl, (C₁-C₃)alkylaminomethyl or (C₁-C₃)dialkylaminomethyl;
❖ D represents phenyl optionally substituted one to three times with substituents each selected independently from the group consisting of alkyl, alkoxy and halogen, or D represents a five or six-membered monocyclic heteroaryl or also D is a bicyclic heteroaryl of the general formula 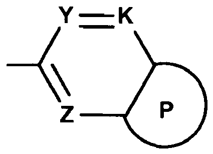 wherein
   K, Y and Z are each independently N or CR^{α},
   R^{α} is hydrogen or halogen, and
   P is a ring selected from the group consisting of the following rings: 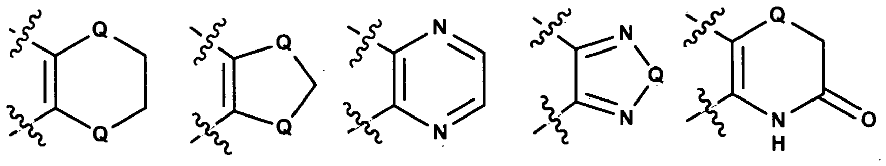 in which Q is O or S.

More preferred compounds of formula I are those wherein at least one of the following further characteristics is present:
❖ R¹ represents methyl, methoxy, trifluoromethoxy, halogen or cyano;
❖ one or two of U, V, W and X represent(s) N, and the remaining represent CH or, in the case of X, may also represent CR^{a}, R^{a} representing fluorine;
❖ A¹ represents NHCO, CH₂CH₂, CH=CH, CH(OH)CH₂ or CH₂CH(OH), or also, provided U is N, CH(OH)CH(OH);
❖ A² represents CH₂ or CO, or also, provided D is a non-annelated aryl or heteroaryl group, CH₂CH=CH;
❖ R² represents -CH₂R³,
   R³ being selected from the group consisting of hydroxycarbamoyl, prop-2-ynyloxycarbamoyl, cyclopropylcarbamoyl, (2-hydroxy-ethyl)carbamoyl, cyano, -CH(OH)R⁶ and -CO-NR^{x}R^{y},
   R⁶ being hydroxymethyl, and
   the group -NR^{x}R^{y} is selected from pyrrolidin-1-yl, piperidin-1-yl, morpholin-4-yl, thiomorpholin-4-yl, (3*R*)-3-hydroxy-pyrrolidin-1-yl, (3*S*)-3-hydroxy-pyrrolidin-1-yl and 4-hydroxy-piperidin-1-yl (and in particular from morpholin-4-yl and 4-hydroxypiperidin-1-yl);
❖ D represents phenyl optionally substituted one to three times with substituents each selected independently from the group consisting of alkyl, alkoxy and halogen, or D is a bicyclic heteroaryl of the formula 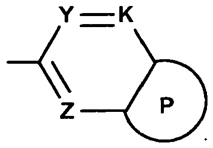 wherein
   K, Y and Z are each independently N or CR⁵,
   R⁵ is hydrogen or halogen, and
   P is a ring selected from the group consisting of the following rings: 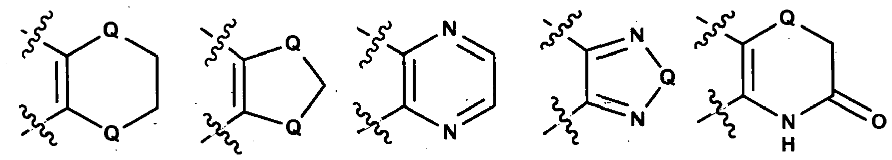 in which Q is O or S.

More preferred compounds of formula I_{P} are those wherein at least one of the following further characteristics is present:
❖ R¹ represents methyl, methoxy, trifluoromethoxy, halogen or cyano;
❖ one or two of U, V, W and X represent(s) N, and the remaining represent CH or, in the case of X, may also represent CR^{a}, R^{a} representing fluorine;
❖ A¹ represents NHCO, CH₂CH₂, CH=CH, CH(OH)CH₂ or CH₂CH(OH), or also, provided U is N, CH(OH)CH(OH);
❖ A² represents CH₂ or CO, or also, provided D is a non-annelated aryl or heteroaryl group, CH₂CH=CH;
❖ R² represents carboxy, carbamoyl, hydroxycarbamoyl, methylcarbamoyl, cyano or CH₂R³,
   R³ being selected from the group consisting of hydroxycarbamoyl, methylcarbamoyl, benzylcarbamoyl, cyano, 1,2,3-triazol-1-yl, 1,2,3,4-tetrazol-1-yl, 1,2,3,4-tetrazol-2-yl or 1,2,3,4-tetrazol-5-yl, 1,2,3-triazol-1-ylmethyl, 1,2,3,4-tetrazol-2-ylmethyl, 1,2,3,4-tetrazol-1-ylmethyl, methylaminomethyl, dimethylaminomethyl, methoxyimino, hydroxyimino and CH(OH)R⁶, and
   R⁶ being hydroxymethyl;
❖ D represents phenyl optionally substituted one to three times with substituents each selected independently from the group consisting of alkyl, alkoxy and halogen, or D is a bicyclic heteroaryl of the general formula 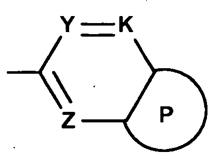 wherein
   K, Y and Z are each independently N or CR⁵,
   R⁵ is hydrogen or halogen, and
   P is a ring selected from the group consisting of the following rings: 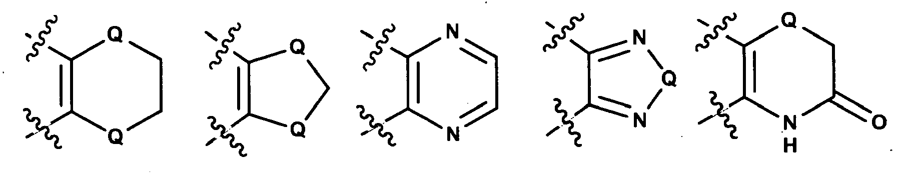 in which Q is O or S.

Particularly preferred compounds of formula I are those wherein at least one of the following further characteristics is present:
❖ R¹ represents methoxy or fluorine (and in particular methoxy);
❖ one or two ofU, V, W and X represent(s) N, and the remaining represent CH;
❖ A¹ represents CH₂CH₂ or CH₂CH(OH), or also, provided U is N, CH(OH)CH(OH);
❖ A² represents CH₂, or also, provided D is a non-annelated aryl or heteroaryl group, CH₂CH=CH;
❖ R² represents -CH₂R³,
   R³ being selected from the group consisting of prop-2-ynyloxycarbamoyl, cyclopropylcarbamoyl, (2-hydroxy-ethyl)carbamoyl, cyano and -CH(OH)R⁶, and
   R⁶ being hydroxymethyl;
❖ D represents phenyl optionally substituted one to three times (preferably one to two times and in particular two times) with substituents each selected independently from the group consisting of halogen atoms (fluorine atoms being preferred), or D is a bicyclic heteroaryl of the formula 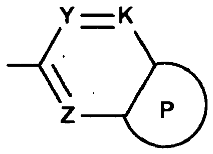 wherein
   K, Y and Z are each independently N or CR⁵,
   R⁵ is hydrogen or fluorine (and notably hydrogen), and
   P is a ring selected from the group consisting of the following rings: 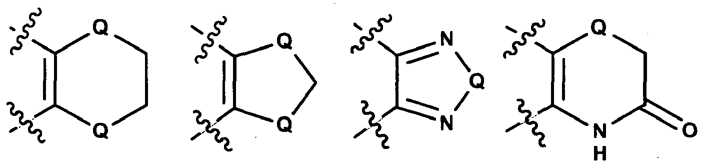 in which Q is O or S.

Particularly preferred compounds of formula I_{P} are those wherein at least one of the following further characteristics is present:
❖ R¹ represents methoxy or trifluoromethoxy (and in particular methoxy);
❖ one or two of U, V, W and X represent(s) N, and the remaining represent CH;
❖ A¹ represents CH₂CH₂, CH(OH)CH₂ or CH₂CH(OH), or also, provided U is N, CH(OH)CH(OH);
❖ A² represents CH₂, or also, provided D is a non-annelated aryl or heteroaryl group, CH₂CH=CH;
❖ R² represents CH₂R³,
   R³ being selected from the group consisting of hydroxycarbamoyl, cyano and CH(OH)R⁶, and
   R⁶ being hydroxymethyl;
❖ D represents phenyl optionally substituted one to three times with substituents each selected independently from the group consisting of halogen atoms, or D is a bicyclic heteroaryl of the general formula 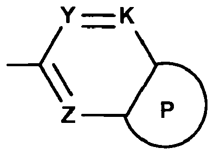 wherein
   K, Y and Z are each independently N or CR⁵,
   R⁵ is hydrogen or fluorine (and notably hydrogen), and
   P is a ring selected from the group consisting of the following rings: 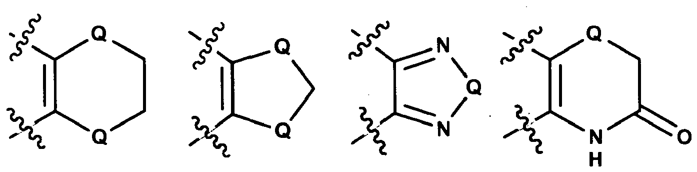 in which Q is O or S.

Preferred combinations for the symbols U, V, W and X in the compounds of formula I are evident from the following particular structures: 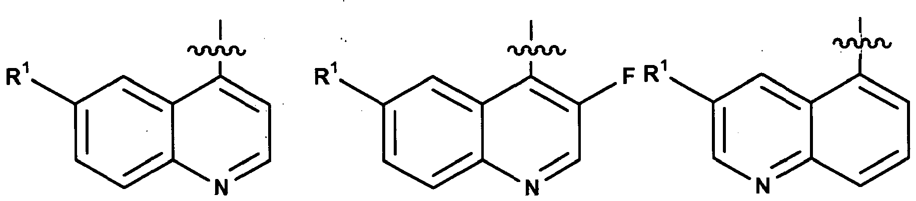 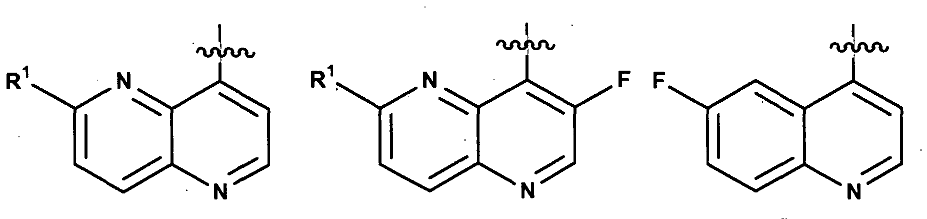 wherein R¹ represents alkoxy (and preferably methoxy).

Preferred combinations for the symbols U, V, W and X in the compounds of formula I_{P} are evident from the following particular structures: 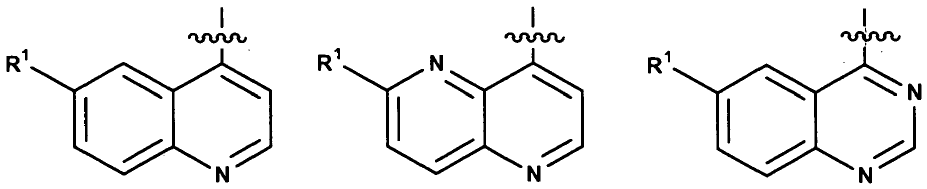 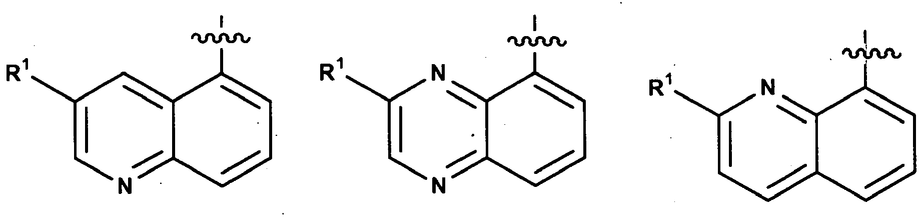 wherein R¹ is as defined in formula I_{P} above, and preferably (C₁-C₃)alkyl, methoxy, ethoxy, trifluoromethyl, trifluoromethoxy or cyano (in particular methyl, methoxy or cyano and notably methoxy).

According to a quite preferred variant of this invention, the compounds of formula I or I_{CE} will be such that U and W each represent N, and V represents CH and X represents CH or CR^{a}, R^{a} being halogen (and preferably fluorine).

According to another quite preferred variant of this invention, the compounds of formula I or I_{CE} will be such that V represents N and U, W and X each represent CH.

According to a further quite preferred variant of this invention, the compounds of formula I or I_{CE} will be such that W represents N, U and V each represent CH and X represents CH or CR^{a}, R^{a} being halogen (and preferably fluorine). According to a subvariant, the compounds of formula I or I_{CE} will be such that W represents N, U and V each represent CH, X represents CH or CR^{a}, R^{a} being halogen (and preferably fluorine) and R¹ represents alkoxy (and preferably methoxy). According to another subvariant, the compounds of formula I or I_{CE} will be such that W represents N, U and V each represent CH, X represents CH and R¹ represents halogen (and preferably fluorine).

In general, compounds of formula I wherein D is either phenyl substituted with 2 halogen atoms (and in particular by two fluorine atoms) or a bicyclic heteroaryl of the formula 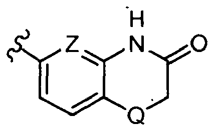 wherein
Q is O or S and
Z is CH or N
will be preferred over other compounds of formula I.

Particularly preferred meanings for D in the compounds of formula I or I_{P} are 2,5-difluorophenyl and 3-oxo-3,4-dihydro-2*H*-pyrido[3,2-*b*][1,4]thiazin-6-yl.

Besides, a preferred stereochemistry for the spacer M of the compounds of formula I or I_{P} is the following:

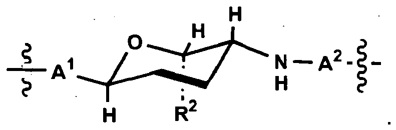

According to a first variant of this invention, the compounds of formula I will be such that D is an aryl group. The compounds according to this variant will be referred to hereafter as "compounds of formula I_{Ar}". In addition, compounds of formula I_{P} wherein D is an aryl group, a particular case of said first variant, will be referred to hereafter as "compounds of formula I_{ArP}".

Preferred compounds of formula I_{Ar} are those wherein at least one of the following characteristics is present:
❖ R¹ represents (C₁-C₃)alkyl, (C₁-C₃)alkoxy, (C₁-C₂)haloalkoxy, halogen or cyano;
❖ one or two ofU, V, W and X represent(s) N, the remaining represent CH or, in the case of X, may also represent CR^{a}, R^{a} representing fluorine;
❖ A¹ represents NHCO, CH₂CH₂, CH=CH, COCH₂, CH(OH)CH₂, CH₂CH(OH) or OCH₂, or also, provided U is N, CH(OH)CH(OH);
❖ A² represents CH₂CH=CH or COCH=CH;
❖ R² represents -CH₂R³,
   R³ being selected from the group consisting of hydroxycarbamoyl, (C₂-C₄)alkynylmethoxycarbamoyl, cycloalkylcarbamoyl, [(C₂-C₅)hydroxyalkyl]carbamoyl, -CH(OH)R⁶ and -CO-NR^{x}R^{y}, R⁶ being hydroxymethyl, and
   the -NR^{x}R^{y} being selected from pyrrolidin-1-yl, piperidin-1-yl, morpholin-4-yl, thiomorpholin-4-yl, (3R)-3-hydroxy-pyrrolidin-1-yl, (3S)-3-hydroxy-pyrrolidin-1-yl and 4-hydroxy-piperidin-1-yl (and in particular from morpholin-4-yl and 4-hydroxypiperidin-1-yl);
❖ D represents phenyl optionally substituted one to three times (preferably one to two times and in particular two times) with substituents each selected independently from the group consisting of halogen atoms (fluorine atoms being preferred).

Preferred compounds of formula I_{ArP} are those wherein at least one of the following characteristics is present:
❖ R¹ represents (C₁-C₃)alkyl, (C₁-C₃)alkoxy, (C₁-C₂)haloalkoxy, halogen or cyano;
❖ one or two of U, V, W and X represent(s) N, the remaining represent CH or CR^{a}, R^{a} representing fluorine;
❖ A¹ represents NHCO, CH₂CH₂, CH=CH, COCH₂, CH(OH)CH₂, CH₂CH(OH) or OCH₂, or also, provided U is N, CH(OH)CH(OH);
❖ A² represents CH₂CH=CH or COCH=CH;
❖ R² represents carboxy, carbamoyl, hydroxycarbamoyl, (C₁-C₅)alkylcarbamoyl, cyano or CH₂R³,
   R³ being selected from the group consisting of hydroxycarbamoyl, (C₁-C₅)alkylcarbamoyl, (phenylalkyl)carbamoyl, cyano, imidazol-2-yl, 1,3-oxazol-2-yl, 1,3,4-oxadiazol-2-yl, 1,2,4-oxadiazol-5-yl, thiazol-2-yl, thiazol-4-yl, thiazol-5-yl, isoxazol-3-yl, furan-2-yl, thiophen-2-yl, 1,2,3-triazol-1-yl, 1,2,3-triazol-5-yl, 1,2,3,4-tetrazol-5-yl, 1,2,3,4-tetrazol-2-yl, 1,2,3,4-tetrazol-1-yl, imidazol-2-ylmethyl, 1,3-oxazol-2-ylmethyl, 1,3,4-oxadiazol-2-ylmethyl, 1,2,4-oxadiazol-5-ylmethyl, thiazol-2-ylmethyl, thiazol-4-ylmethyl, thiazol-5-ylmethyl, isoxazol-3-ylmethyl, furan-2-ylmethyl, thiophen-2-ylmethyl, 1,2,3-triazol-1-ylmethyl, 1,2,3-triazol-5-ylmethyl, 1,2,3,4-tetrazol-2-ylmethyl, 1,2,3,4-tetrazol-5-yl-methyl, 1,2,3,4-tetrazol-1-ylmethyl, (C₁-C₅)alkylaminomethyl, (C₁-C₅)dialkylaminomethyl, (C₁-C₃)alkoxyimino, hydroxyimino and CH(OH)R⁶, and
   R⁶ being hydroxymethyl, (C₁-C₃)alkylaminomethyl or (C₁-C₃)dialkylaminomethyl;
❖ D represents phenyl optionally substituted one to three times with substituents each selected independently from the group consisting of halogen atoms.

More preferred compounds of formula I_{Ar} are those wherein at least one of the following characteristics is present:
❖ R¹ represents methoxy or fluorine (and notably methoxy);
❖ one or two of U, V, W and X represent(s) N and the remaining represent CH, or, in the case of X, may also represent CR^{a}, R^{a} representing fluorine;
❖ A¹ represents NHCO, CH₂CH₂, CH=CH or CH₂CH(OH), or also; provided U is N, CH(OH)CH(OH) (notably CH₂CH₂, CH=CH or CH₂CH(OH), and in particular CH₂CH₂);
❖ A² represents CH₂CH=CH or COCH=CH (and notably CH₂CH=CH);
❖ R² represents -CH₂R³,
   R³ being selected from the group consisting of hydroxycarbamoyl, cyclopropylcarbamoyl, (2-hydroxy-ethyl)carbamoyl, cyano and -CH(OH)R⁶, and R⁶ being hydroxymethyl;
❖ D represents phenyl or 2,5-difluorophenyl (and notably 2,5-difluorophenyl).

More preferred compounds of formula I_{ArP} are those wherein at least one of the following characteristics is present:
❖ R¹ represents methoxy or trifluoromethoxy (and notably methoxy);
❖ one or two of U, V, W and X represent(s) N and the remaining represent CH, or, in the case of X, may also represent CR^{a}, R^{a} representing fluorine;
❖ A¹ represents NHCO, CH₂CH₂, CH=CH, CH(OH)CH₂ or CH₂CH(OH), or also, provided U is N, CH(OH)CH(OH) (notably CH₂CH₂, CH=CH or CH₂CH(OH), and in particular CH₂CH₂);
❖ A² represents CH₂CH=CH or COCH=CH (and notably CH₂CH=CH);
❖ R² represents CH₂R³,
   R³ being selected from the group consisting of hydroxycarbamoyl, cyano and CH(OH)R⁶, and
   R⁶ being hydroxymethyl;
❖ D represents phenyl or 2,5-difluorophenyl (and notably 2,5-difluorophenyl).

According to a second variant of this invention, the compounds of formula I will be such that D is a heteroaryl group. The compounds according to this variant will be referred to hereafter as "compounds of formula I_{Het}". In addition, compounds of formula I_{P} wherein D is a heteroaryl group, a particular case of said second variant, will be referred to hereafter as "compounds of formula I_{HetP}".

Preferred compounds of formula I_{Het} are those wherein at least one of the following characteristics is present:
❖ R¹ represents (C₁-C₃)alkyl, (C₁-C₃)alkoxy, (C₁-C₂)haloalkoxy, halogen or cyano;
❖ one or two of U, V, W and X represent(s) N, the remaining represent CH or, in the case of X, may also represent CR^{a}, R^{a} representing fluorine;
❖ A¹ represents NHCO, CH₂CH₂, CH=CH, COCH₂, CH(OH)CH₂, CH₂CH(OH) or OCH₂, or also, provided U is N, CH(OH)CH(OH);
❖ A² represents CH₂ or CO;
❖ R² represents -CH₂R³,
   R³ being selected from the group consisting of hydroxycarbamoyl, [(C₂-C₅)hydroxyalkyl]carbamoyl and -CH(OH)R⁶, and
   R⁶ being hydroxymethyl;
❖ D is a bicyclic heteroaryl of the general formula 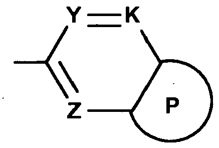 wherein
   K, Y and Z are each independently N or CR⁵,
   R⁵ is hydrogen or halogen, and
   P is a ring selected from the group consisting of the following rings: 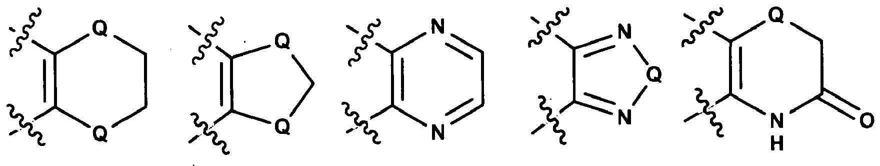 in which Q is O or S.

Preferred compounds of formula I_{HetP} are those wherein at least one of the following characteristics is present:
❖ R¹ represents (C₁-C₃)alkyl, (C₁-C₃)alkoxy, (C₁-C₂)haloalkoxy, halogen or cyano;
❖ one or two of U, V, W and X represent(s) N, the remaining represent CH or CR^{a}, R^{a} representing fluorine;
❖ A¹ represents NHCO, CH₂CH₂, CH=CH, COCH₂, CH(OH)CH₂, CH₂CH(OH) or OCH₂, or also, provided U is N, CH(OH)CH(OH);
❖ A² represents CH₂ or CO;
❖ R² represents carboxy, carbamoyl, hydroxycarbamoyl, (C₁-C₅)alkylcarbamoyl, cyano, CH₂R³ or CH(OH)R⁶,
   R³ being selected from the group consisting of hydroxycarbamoyl, (C₁-C₅)alkylcarbamoyl, (phenylalkyl)carbamoyl, cyano, imidazol-2-yl, 1,3-oxazol-2-yl, 1,3,4-oxadiazol-2-yl, 1,2,4-oxadiazol-5-yl, thiazol-2-yl, thiazol-4-yl, thiazol-5-yl, isoxazol-3-yl, furan-2-yl, thiophen-2-yl, 1,2,3-triazol-1-yl, 1,2,3-triazol-5-yl, 1,2,3,4-tetrazol-5-yl, 1,2,3,4-tetrazol-2-yl, 1,2,3,4-tetrazol-1-yl, imidazol-2-ylmethyl, 1,3-oxazol-2-ylmethyl, 1,3,4-oxadiazol-2-ylmethyl, 1,2,4-oxadiazol-5-ylmethyl, thiazol-2-ylmethyl, thiazol-4-ylmethyl, thiazol-5-ylmethyl, isoxazol-3-ylmethyl, furan-2-ylmethyl, thiophen-2-ylmethyl, 1,2,3-triazol-1-ylmethyl, 1,2,3-triazol-5-ylmethyl, 1,2,3,4-tetrazol-2-ylmethyl, 1,2,3,4-tetrazol-5-yl-methyl, 1,2,3,4-tetrazol-1-ylmethyl, (C₁-C₅)alkylaminomethyl, (C₁-C₅)dialkylaminomethyl, (C₁-C₃)alkoxyimino and hydroxyimino, and
   R⁶ being hydroxymethyl, (C₁-C₃)alkylaminomethyl or (C₁-C₃)dialkylaminomethyl;
❖ D is a bicyclic heteroaryl of the general formula 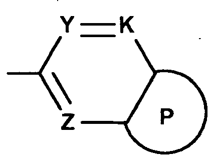 wherein
   K, Y and Z are each independently N or CR⁵,
   R⁵ is hydrogen or halogen, and
   P is a ring selected from the group consisting of the following rings: 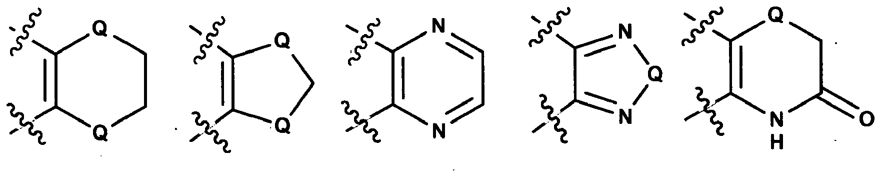 in which Q is O or S.

More preferred compounds of formula I_{Het} are those wherein at least one of the following characteristics is present:
❖ R¹ represents methoxy or fluorine;
❖ one or two of U, V, W and X represent(s) N and the remaining represent CH, or, in the case of X, may also represent CR^{a}, R^{a} representing fluorine;
❖ A¹ represents NHCO, CH₂CH₂, CH=CH or CH₂CH(OH), or also, provided U is N, CH(OH)CH(OH);
❖ A² represents CH₂ or CO;
❖ R² represents -CH₂R³,
   R³ being selected from the group consisting of hydroxycarbamoyl, (2-hydroxy-ethyl)carbamoyl, cyano and -CH(OH)R⁶, and
   R⁶ being hydroxymethyl;
❖ D is selected from the group consisting of 2,3-dihydro-benzo[1,4]dioxin-6-yl, benzo[1,3]dioxol-5-yl, 4*H*-benzo[1,4]oxazin-3-one-6-yl, 4*H*-benzo[1,4]thiazin-3-one-6-yl, 7-fluoro-4*H*-benzo[1,4]thiazin-3-one-6-yl, 2,3-dihydro[1,4]dioxino[2,3-*c*]pyridin-7-yl, 2,3-dihydro[1,4]dioxino[2,3-*b*]pyridin-6-yl, 3-oxo-3,4-dihydro-2*H*-pyrido[3,2-*b*][1,4]thiazin-6-yl, 2-oxo-1*H*-pyrido[2,3-*b*][1,4]thiazin-7-yl and benzo[1,2,5]thiadiazol-5-yl.

More preferred compounds of formula I_{HetP} are those wherein at least one of the following characteristics is present:
❖ R¹ represents methoxy or trifluoromethoxy;
❖ one or two of U, V, W and X represent(s) N and the remaining represent CH, or, in the case of X, may also represent CR^{a}, R^{a} representing fluorine;
❖ A¹ represents NHCO, CH₂CH₂, CH=CH, CH(OH)CH₂ or CH₂CH(OH), or also, provided U is N, CH(OH)CH(OH);
❖ A² represents CH₂ or CO;
❖ R² represents carboxy, carbamoyl, hydroxycarbamoyl, methylcarbamoyl, cyano, CH₂R³,
   R³ being selected from the group consisting of hydroxycarbamoyl, methylcarbamoyl, benzylcarbamoyl, cyano, 1,2,3-triazol-1-yl, 1,2,3,4-tetrazol-1-yl, 1,2,3,4-tetrazol-2-yl or 1,2,3,4-tetrazol-5-yl, 1,2,3-triazol-1-ylmethyl, 1,2,3,4-tetrazol-2-ylmethyl, 1,2,3,4-tetrazol-1-ylmethyl, methylaminomethyl, dimethylaminomethyl, methoxyimino hydroxyimino and CH(OH)R⁶, and
   R⁶ being hydroxymethyl;
❖ D is selected from the group consisting of 2,3-dihydro-benzo[1,4]dioxin-6-yl, benzo[1,3]dioxol-5-yl, 4*H*-benzo[1,4]oxazin-3-one-6-yl, 4*H*-benzo[1,4]thiazin-3-one-6-yl, 7-fluoro-4*H*-benzo[1,4]thiazin-3-one-6-yl, 2,3-dihydro[1,4]dioxino[2,3-c]pyridin-7-yl, 2,3-dihydro[1,4]dioxino[2,3-*b*]pyridin-6-yl, 3-oxo-3,4-dihydro-2*H*-pyrido[3,2-*b*][1,4]thiazin-6-yl, 2-oxo-1*H*-pyrido[2,3-*b*][1,4]thiazin-7-yl and benzo[ 1,2,5]thiadiazol-5-yl.

Even more preferred compounds of formula I_{Het} are those wherein at least one of the following characteristics is present:
❖ R¹ represents methoxy;
❖ one or two of U, V, W and X represent(s) N and the remaining represent CH;
❖ A¹ represents CH₂CH₂, CH=CH or CH₂CH(OH) (notably CH₂CH₂);
❖ A represents CH₂;
❖ R² represents -CH₂R³,
   R³ being selected from the group consisting of (2-hydroxy-ethyl)carbamoyl and - CH(OH)R⁶ (and preferably R³ being -CH(OH)R⁶), and
   R⁶ being hydroxymethyl;
❖ D is selected from the group consisting of 4*H*-benzo[1,4]thiazin-3-one-6-yl, 7-fluoro-4*H*-benzo[1,4]thiazin-3-one-6-yl and 3-oxo-3,4-dihydro-2*H*-pyrido[3,2-*b*][1,4]thiazin-6-yl (notably D being 4*H*-benzo[1,4]thiazin-3-one-6-yl or 3-oxo-3,4-dihydro-2*H*-pyrido[3,2-*b*][1,4]thiazin-6-yl and in particular D *b*eing 3-oxo-3,4-dihydro-2*H*-pyrido[3,2-*b*][1,4]thiazin-6-yl).

Even more preferred compounds of formula I_{HetP} are those wherein at least one of the following characteristics is present:
❖ R¹ represents methoxy;
❖ one or two of U, V, W and X represent(s) N and the remaining represent CH;
❖ A¹ represents CH₂CH₂, CH=CH or CH₂CH(OH) (notably CH₂CH₂);
❖ A² represents CH₂;
❖ R² represents CH₂R³,
   R³ being selected from the group consisting of hydroxycarbamoyl, cyano and CH(OH)R⁶, and
   R⁶ being hydroxymethyl;
❖ D is selected from the group consisting of 4*H*-benzo[1,4]thiazin-3-one-6-yl, 7-fluoro-4*H*-benzo[1,4]thiazin-3-one-6-yl and 3-oxo-3,4-dihydro-2*H*-pyrido[3,2-*b*][1,4]thiazin-6-yl (notably D being 3-oxo-3,4-dihydro-2*H*-pyrido[3,2-*b*][1,4]thiazin-6-yl).

Compounds of formula I or I_{P} carrying a double bond in A¹ or A² are present as *Z*/*E* (*cis*/*trans*) isomer mixtures or as *Z* (*cis*) or *E* (*trans*) isomers. Preferred are the *E* (*trans*) isomers.

Particularly preferred compounds of formula I are the following:
- (*E*)-3-(2,5-difluoro-phenyl)-*N*-{(2*R*,3*R*,6*R*)-2-((2*R*)-2,3-dihydroxy-propyl)-6-[2-(3-methoxy-quinolin-5-yl)-ethyl]-tetrahydro-pyran-3-yl}-acrylamide;
- (*E*)*-*3-(2,5-difluoro-phenyl)-*N-*{(2*R*,3*R*,6*R*)*-*2-((2*S*)-2,3-dihydroxy-propyl)-6-[2-(3-methoxy-quinolin-5-yl)-ethyl]-tetrahydro-pyran-3-yl}-acrylamide;
- 3-oxo-3,4-dihydro-2*H*-pyrido[3,2-*b*][1,4]thiazine-6-carboxylic acid {(2*R*,3*R*,6*R*)-2-((2*R*)-2,3-dihydroxy-propyl)-6-[2-(3-methoxy-quinolin-5-yl)-ethyl]-tetrahydro-pyran-3-yl -amide;
- 3-oxo-3,4-dihydro-2*H*-pyrido[3,2-*b*][1,4]thiazine-6-carboxylic acid {(2*R*,3*R*,6*R*)-2-((2*S*)-2,3-dihydroxy-propyl)-6-[2-(3-methoxy-quinolin-5-yl)-ethyl]-tetrahydro-pyran-3-yl}-amide;
- 6-({(2*R*,3*R*,6*R*)-2-((2*R*)-2,3-dihydroxy-propyl)-6-[2-(3-methoxy-quinolin-5-yl)-ethyl]-tetrahydro-pyran-3-ylamino}-methyl)-4*H*-pyrido[3,2-*b*][1,4]thiazin-3-one;
- 6-({(2*R*,3*R*,6*R*)-2-((2*S*)-2,3-dihydroxy-propyl)-6-[2-(3-methoxy-quinolin-5-yl)-ethyl]-tetrahydro-pyran-3-ylamino}-methyl)-4*H*-pyrido[3,2-*b*][1,4]thiazin-3-one;
- 6-({(2*R*,3*R*,6*R*)-2-((2*R*)-2,3-dihydroxy-propyl)-6-[2-(6-methoxy-[1,5]naphthyridin-4-yl)-ethyl]-tetrahydro-pyran-3-ylamino}-methyl)-4*H*-pyrido[3,2-*b*][1,4]thiazin-3-one;
- 6-({(2*R*,3*R*,6*R*)-2-((2*S*)-2,3-dihydroxy-propyl)-6-[2-(6-methoxy-[1,5]naphthyridin-4-yl)-ethyl]-tetrahydro-pyran-3-ylamino}-methyl)-4*H*-pyrido[3,2-*b*][1,4]thiazin-3-one;
- (2*R*)-3-{(2*R*,3*R*,6*R*)-3-[(*E*)-3-(2,5-difluoro-phenyl)-allylamino]-6-[2-(6-methoxy-[1,5]naphthyridin-4-yl)-ethyl]-tetrahydro-pyran-2-yl}-propane-1,2-diol;
- (2*S*)-3-{(2*R*,3*R*,6*R*)-3-[(*E*)-3-(2,5-difluoro-phenyl)-allylamino]-6-[2-(6-methoxy-[1,5]naphthyridin-4-yl)-ethyl]-tetrahydro-pyran-2-yl}-propane-1,2-diol;
- 2-{(2*R*,3*R*,6*R*)-3-[(*E*)-3-(2,5-difluoro-phenyl)-allylamino]-6-[2-(6-methoxy-[1,5]naphthyridin-4-yl)-ethyl]-tetrahydro-pyran-2-yl}-*N*-hydroxy-acetamide;
- (*E*)-3-(2,5-difluoro-phenyl)-*N*-{(2*R*,3*R*,6*R*)-2-((2*R*)-2,3-dihydroxy-propyl)-6-[2-(6-methoxy-[1,5]naphthyridin-4-yl)-ethyl]-tetrahydro-pyran-3-yl}-acrylamide;
- (*E*)-3-(2,5-difluoro-phenyl)-*N*-{(2*R*,3*R*,6*R*)-2-((2*S*)-2,3-dihydroxy-propyl)-6-[2-(6-methoxy-[1,5]naphthyridin-4-yl)-ethyl]-tetrahydro-pyran-3-yl}-acrylamide;
- (2*R*)-3-{(2*R*,3*R*,6*S*)-3-[(*E*)-3-(2,5-difluoro-phenyl)-allylamino]-6-[(*S*)-1-hydroxy-2-(6-methoxy-[1,5]naphthyridin-4-yl)-ethyl]-tetrahydro-pyran-2-yl}-propane-1,2-diol;
- (2*S*)-3-{(2*R*,3*R*,6*S*)-3-[(*E*)-3-(2,5-difluoro-phenyl)-allylamino]-6-[(*S*)-1-hydroxy-2-(6-methoxy-[1,5]naphthyridin-4-yl)-ethyl]-tetrahydro-pyran-2-yl}-propane-1,2-diol;
- (2*R*)-3-{(2*R*,3*R*,6*R*)-3-[(*E*)-3-(2,5-difluoro-phenyl)-allylamino]-6-[2-(6-methoxy-quinolin-4-yl)-ethyl]-tetrahydro-pyran-2-yl}-propane-1,2-diol;
- (2*S*)-3-{(2*R*,3*R*,6*R*)-3-[(*E*)-3-(2,5-difluoro-phenyl)-allylamino]-6-[2-(6-methoxy-quinolin-4-yl)-ethyl]-tetrahydro-pyran-2-yl}-propane-1,2-diol;
- {(2*R*,3*R*,6*R*)-3-[(*E*)-3-(2,5-difluoro-phenyl)-allylamino]-6-[2-(6-methoxy-quinolin-4-yl)-ethyl]-tetrahydro-pyran-2-yl}-acetonitrile;
- (*E*)-3-(2,5-difluoro-phenyl)-*N*-{(2*R*,3*R*,6*S*)-2-((2*R*)-2,3-dihydroxy-propyl)-6-[(*E*)-2-(3-fluoro-6-methoxy-[1,5]naphthyridin-4-yl)-vinyl]-tetrahydro-pyran-3-yl} acrylamide;
- (*E*)-3-(2,5-difluoro-phenyl)-*N*-{(2*R*,3*R*,6*S*)-2-((2*S*)-2,3-dihydroxy-propyl)-6-[(*E*)-2-(3-fluoro-6-methoxy-[1,5]naphthyridin-4-yl)-vinyl]-tetrahydro-pyran-3-yl} acrylamide;
- (2*R*)-3-{(2*R*,3*R*,6*S*)-3-[(*E*)-3-(2,5-difluoro-phenyl)-allylamino]-6-[(*E*)-2-(3-fluoro-6-methoxy-[1,5]naphthyridin-4-yl)-vinyl]-tetrahydro-pyran-2-yl}-propane-1,2-diol;
- (2*S*)-3-{(2*R*,3*R*,6*S*)-3-[(*E*)-3-(2,5-difluoro-phenyl)-allylamino]-6-[(*E*)-2-(3-fluoro-6-methoxy-[1,5]naphthyridin-4-yl)-vinyl]-tetrahydro-pyran-2-yl}-propane-1,2-diol;
- (*E*)-3-(2,5-difluoro-phenyl)-*N*-[(2*R*,3*R*,6*S*)-2-((2*R*)-2,3-dihydroxy-propyl)-6-(6-methoxy-quinolin-4-yloxymethyl)-tetrahydro-pyran-3-yl]-acrylamide;
- (*E*)-3-(2,5-difluoro-phenyl)-*N*-[(2*R*,3*R*,6*S*)-2-((2*S*)-2,3-dihydroxy-propyl)-6-(6-methoxy-quinolin-4-yloxymethyl)-tetrahydro-pyran-3-yl]-acrylamide;
- (2*S*,5*R*,6*R*)-5-[(*E*)-3-(2,5-difluoro-phenyl)-allylamino]-6-[(2*R*)-2,3-dihydroxy-propyl]-tetrahydro-pyran-2-carboxylic acid (6-methoxy-[1,5]naphthyridin-4-yl)-amide;
- (2*S*,5*R*,6*R*)-5-[(*E*)-3-(2,5-difluoro-phenyl)-allylamino]-6-[(2*S*)-2,3-dihydroxy-propyl]-tetrahydro-pyran-2-carboxylic acid (6-methoxy-[1,5]naphthyridin-4-yl)-amide;
- (2*S*,5*R*,6*R*)-6-[(2*R*)-2,3-dihydroxy-propyl]-5-[(3-oxo-3,4-dihydro-2*H*-pyrido[3,2-*b*][1,4]thiazin-6-ylmethyl)-amino]-tetrahydro-pyran-2-carboxylic acid (6-methoxy-[1,5]naphthyridin-4-yl)-amide;
- (2*S*,5*R*,6*R*)-6-[(2*S*)-2,3-dihydroxy-propyl]-5-[(3-oxo-3,4-dihydro-2*H*-pyrido[3,2-*b*][1,4]thiazin-6-ylmethyl)-amino]-tetrahydro-pyran-2-carboxylic acid (6-methoxy-[1,5]naphthyridin-4-yl)-amide;
- (2*S*,5*R*,6*R*)-5-[(*E*)-3-(2,5-difluoro-phenyl)-acryloylamino]-6-[(2*R*)-2,3-dihydroxypropyl]-tetrahydro-pyran-2-carboxylic acid (6-methoxy-[1,5]naphthyridin-4-yl)-amide;
- (2*S*,5*R*,6*R*)-5-[(*E*)-3-(2,5-difluoro-phenyl)-acryloylamino]-6-[(2*S*)-2,3-dihydroxypropyl]-tetrahydro-pyran-2-carboxylic acid (6-methoxy-[1,5]naphthyridin-4-yl)-amide;
- 3-oxo-3,4-dihydro-2*H*-pyrido[3,2-*b*][1,4]thiazine-6-carboxylic acid {(2*R*,3*R*,6*S*)-2-((2*R*)-2,3-dihydroxy-propyl)-6-[(*E*)-2-(6-methoxy-[1,5]naphthyridin-4-yl)-vinyl]- - tetrahydro-pyran-3-yl} -amide;
- 3-oxo-3,4-dihydro-2*H*-pyrido[3,2-*b*][1,4]thiazine-6-carboxylic acid {(2*R*,3*R*,6*S*)-2-((2*S*)-2,3-dihydroxy-propyl)-6-[(*E*)-2-(6-methoxy-[1,5]naphthyridin-4-yl)-vinyl]-tetrahydro-pyran-3-yl}-amides;
- (2*R*)-3-{(2*R*,3*R*,6*S*)-3-[(*E*)-3-(2,5-difluoro-phenyl)-allylamino]-6-[(*E*)-2-(6-methoxy-[1,5]naphthyridin-4-yl)-vinyl]-tetrahydro-pyran-2-yl}-propane-1,2-diol;
- (2*S*)-3-{(2*R*,3*R*,6*S*)-3-[(*E*)-3-(2,5-difluoro-phenyl)-allylamino]-6-[(*E*)-2-(6-methoxy-[1,5]naphthyridin-4-yl)-vinyl]-tetrahydro-pyran-2-yl}-propane-1,2-diol;
- (2*R*)-3-{2*R*,3*R*,6*R*)-3-[(*E*)-3-(2,5-difluoro-phenyl)-allylamino]-6-[2-(6-fluoro-quinolin-4-yl)-ethyl]-tetrahydro-pyran-2-yl}-propane-1,2-diol;
- (2*S*)-3-{2*R*,3*R*,6*R*)-3-[(*E*)-3-(2,5-difluoro-phenyl)-allylamino]-6-[2-(6-fluoro-quinolin-4-yl)-ethyl]-tetrahydro-pyran-2-yl}-propane-1,2-diol;
- 2-{(2*R*,3*R*,6*S*)-3-[(*E*)-3-(2,5-difluoro-phenyl)-allylamino]-6-[(*E*)-2-(6-methoxy-[1,5]naphthyridin-4-yl)-vinyl]-tetrahydro-pyran-2-yl}-*N*-(2-hydroxy-ethyl)-acetamide;
- 2-{(2*R*,3*R*,6*S*)-3-[(*E*)-3-(2,5-difluoro-phenyl)-allylamino]-6-[(1*R*,2*R*)-1,2-dihydroxy-2-(6-methoxy-[1,5]naphthyridin-4-yl)-ethyl]-tetrahydro-pyran-2-yl}-*N*-(2-hydroxy-ethyl)-acetamide;
- 2-{(2*R*,3*R*,6*S*)-3-[(*E*)-3-(2,5-difluoro-phenyl)-allylamino]-6-[(1*S*)-1-hydroxy-2-(6-methoxy-[1,5]naphthyridin-4-yl)-efhyl]-tetrahydro-pyran-2-yl}-*N*-(2-hydroxy-ethyl)-acetamide;
- 2-{(2*R*,3*R*,6*S*)-3-[(*E*)-3-(2,5-difluoro-phenyl)-allylamino]-6-[(1*S*)-1-hydroxy-2-(6-methoxy-[1,5]naphthyridin-4-yl)-ethyl]-tetrahydro-pyran-2-yl}-1-morpholin-4-yl-ethanone;
- *N*-cyclopropyl-2-{(2*R*,3*R,*6*S*)-3-[(*E*)-3-(2,5-difluoro-phenyl)-allylamino]-6-[(1*S*)-1-hydroxy-2-(6-methoxy-[1,5]naphthyridin-4-yl)-ethyl]-tetrahydro-pyran-2-yl}-acetamide;
- 2-{(2*R*,3*R*,6*S*)-3-[(*E*)-3-(2,5-difluoro-phenyl)-allylamino]-6-[(1*S*)-1-hydroxy-2-(6-methoxy-[1,5]naphthyridin-4-yl)-ethyl]-tetrahydro-pyran-2-yl}-*N*,*N*-dimethylacetamide;
- 2-{(2*R*,3*R*,6*S*)-3-[(*E*)-3-(2,5-difluoro-phenyl)-allylamino]-6-[(1*S*)-1-hydroxy-2-(6-methoxy-[1,5]naphthyridin-4-yl)-ethyl]-tetrahydro-pyran-2-yl}-1-(4-hydroxy-piperidin-1-yl)-ethanone;
- 2-{(2*R*,3*R*,6*S*)-3-[(*E*)-3-(2,5-difluoro-phenyl)-allylamino]-6-[(1*S*)-1-hydroxy-2-(6-methoxy-[1,5]naphthyridin-4-yl)-ethyl]-tetrahydro-pyran-2-yl}-*N*-prop-2-ynyloxy-acetamide;
- 2-{(2*R*,3*R*,6*R*)-3-[(*E*)-3-(2,5-difluoro-phenyl)-allylamino]-6-[2-(3-fluoro-6-methoxy-quinolin-4-yl)-ethyl]-tetrahydro-pyran-2-yl}-*N*-(2-hydroxy-ethyl)-acetamide;
- 2-{(2*R*,3*R*,6*S*)-3-[(*E*)-3-(2,5-difluoro-phenyl)-allylamino]-6-[(*E*)-2-(3-fluoro-6-methoxy-quinolin-4-yl)-vinyl]-tetrahydro-pyran-2-yl}-*N*,*N*-dimethyl-acetamide;
- (2*R*,3*R*,6*S*)-6-({2-[(2*R*)-2,3-dihydroxy-propyl]-6-[(*E*)-(6-methoxy-[1,5]naphthyridin-4-yl)-vinyl]-tetrahydro-pyran-3-ylamino}-methyl)-4*H*-pyrido[3,2-*b*][1,4]thiazin-3-one;
- (2*R*,3*R*,6*S*)-6-({2-[(2*S*)-2,3-dihydroxy-propyl]-6-[(*E*)-(6-methoxy-[1,5]naphthyridin-4-yl)-vinyl]-tetrahydro-pyran-3-ylamino}-methyl)-4*H*-pyrido[3,2-*b*][1,4]thiazin-3-one;
- (2*R*,3*R*,6*S*)-6-[(2-hydroxy-ethylcarbamoyl)-methyl]-5-[(3-oxo-3,4-dihydro-2*H*-pyrido[3,2-*b*][1,4]thiazin-6-ylmethyl)-amino]-tetrahydro-pyran-2-carboxylic acid (6-methoxy-[1,5]naphthyridin-4-yl)-amide;
and salts (in particular pharmaceutically acceptable salts) thereof.

Especially preferred are:
- (*E*)-3-(2,5-difluoro-phenyl)-*N*-{(2*R*,3*R*,6*R*)-2-((2*R*)-2,3-dihydroxy-propyl)-6-[2-(3-methoxy-quinolin-5-yl)-ethyl]-tetrahydro-pyran-3-yl}-acrylamide;
- (*E*)-3-(2,5-difluoro-phenyl)-*N*-{(2*R*;3*R*,6*R*)-2-((2*S*)-2,3-dihydroxy-propyl)-6-[2-(3-methoxy-quinolin-5-yl)-ethyl]-tetrahydro-pyran-3-yl}-acrylamide;
- 3-oxo-3,4-dihydro-2*H*-pyrido[3,2-*b*][1,4]thiazine-6-carboxylic acid {(2*R*,3*R*,6*R*)-2-((2*R*)-2,3-dihydroxy-propyl)-6-[2-(3-methoxy-quinolin-5-yl)-ethyl]-tetrahydro-pyran-3-yl -amide;
- 3-oxo-3,4-dihydro-2*H*-pyrido[3,2-*b*][1,4]thiazine-6-carboxylic acid {(2*R*,3*R*,6*R*)-2-((2*S*)-2,3-dihydroxy-propyl)-6-[2-(3-methoxy-quinolin-5-yl)-ethyl]-tetrahydro-pyran-3-yl}-amide;
- 6-({(2*R*,3*R*,6*R*)-2-((2*R*)-2,3-dihydroxy-propyl)-6-[2-(3-methoxy-quinolin-5-yl)-ethyl]-tetrahydro-pyran-3-ylamino}-methyl)-4*H*-pyrido[3,2-*b*][1,4]thiazin-3-one;
- 6-({(2*R*,3*R*,6*R*)-2-((2*S*)-2,3-dihydroxy-propyl)-6-[2-(3-methoxy-quinolin-5-yl)-ethyl]-tetrahydro-pyran-3-ylamino}-methyl)-4*H*-pyrido[3,2-*b*][1,4]thiazin-3-one;
- 6-({(2*R*,3*R*,6*R*)-2-((2*R*)-2,3-dihydroxy-propyl)-6-[2-(6-methoxy-[1,5]naphthyridin-4-yl)-ethyl]-tetrahydro-pyran-3-ylamino}-methyl)-4*H*-pyrido[3,2-*b*][1,4]thiazin-3-one;
- 6-({(2*R*,3*R*,6*R*)-2-((2*S*)-2,3-dihydroxy-propyl)-6-[2-(6-methoxy-[1,5]naphthyridin-4-yl)-ethyl]-tetrahydro-pyran-3-ylamino}-methyl)-4*H*-pyrido[3,2-*b*][1,4]thiazin-3-one;
- (2*R*)-3-{(2*R*,3*R*,6*R*)-3-[(*E*)-3-(2,5-difluoro-phenyl)-allylamino]-6-[2-(6-methoxy-[1,5]naphthyridin-4-yl)-ethyl]-tetrahydro-pyran-2-yl}-propane-1,2-diol;
- (2*S*)-3-{(2*R*,3*R*,6*R*)-3-[(*E*)-3-(2,5-difluoro-phenyl)-allylamino]-6-[2-(6-methoxy-[1,5]naphthyridin-4-yl)-ethyl]-tetrahydro-pyran-2-yl}-propane-1,2-diol;
- 2-{(2*R*,3*R*,6*R*)-3-[(*E*)-3-(2,5-difluoro-phenyl)-allylamino]-6-[2-(6-methoxy-[1,5]naphthyridin-4-yl)-ethyl]-tetrahydro-pyran-2-yl}-*N*-hydroxy-acetamide;
- (*E*)-3-(2,5-difluoro-phenyl)-*N*-{(2*R*,3*R*,6*R*)-2-((2*R*)-2,3-dihydroxy-propyl)-6-[2-(6-methoxy-[1,5]naphthyridin-4-yl)-ethyl]-tetrahydro-pyran-3-yl} -acrylamide;
- (*E*)-3-(2,5-difluoro-phenyl)-*N*-{(2*R*,3*R*,6*R*)-2-((2*S*)-2,3-dihydroxy-propyl)-6-[2-(6-methoxy-[1,5]naphthyridin-4-yl)-ethyl]-tetrahydro-pyran-3-yl} -acrylamide;
- (2*R*)-3-{(2*R*,3*R*,6*S*)-3-[(*E*)-3-(2,5-difluoro-phenyl)-allylamino]-6-[(*S*)-1-hydroxy-2-(6-methoxy-[1,5]naphthyridin-4-yl)-ethyl]-tetrahydro-pyran-2-yl}-propane-1,2-diol;
- (2*S*)-3-{(2*R*,3*R*,6*S*)-3-[(*E*)-3-(2,5-difluoro-phenyl)-allylamino]-6-[(*S*)-1-hydroxy-2-(6-methoxy-[1,5]naphthyridin-4-yl)-ethyl]-tetrahydro-pyran-2-yl}-propane-1,2-diol;
- (2*R*)-3-{(2*R*,3*R*,6*R*)-3-[(*E*)-3-(2,5-difluoro-phenyl)-allylamino]-6-[2-(6-methoxy-quinolin-4-yl)-ethyl]-tetrahydro-pyran-2-yl}-propane-1,2-diol;
- (2*S*)-3-{(2*R*,3*R*,6*R*)-3-[(*E*)-3-(2,5-difluoro-phenyl)-allylamino]-6-[2-(6-methoxy-quinolin-4-yl)-ethyl]-tetrahydro-pyran-2-yl}-propane-1,2-diol; (2*R*,3*R*,6*R*)-3-[(*E*)-3-(2,5-difluoro-phenyl)-allylamino]-6-[2-(6-methoxy-quinolin-4-yl)-ethyl]-tetrahydro-pyran-2-yl -acetonitrile;
- (*E*)-3-(2,5-difluoro-phenyl)-*N*-{(2*R*,3*R*,6*S*)-2-((2R)-2,3-dihydroxy-propyl)-6-[(*E*)-2-(3-fluoro-6-methoxy-[1,5]naphthyridin-4-yl)-vinyl]-tetrahydro-pyran-3-yl} acrylamide;
- (*E*)-3-(2,5-difluoro-phenyl)-*N*-{(2*R*,3*R*,6*S*)-2-((2*S*)-2,3-dihydroxy-propyl)-6-[(*E*)-2-(3-fluoro-6-methoxy-[1,5]naphthyridin-4-yl)-vinyl]-tetrahydro-pyran-3-yl} acrylamide;
- (2*R*)-3-{(2*R*,3*R*,6*S*)-3-[(*E*)-3-(2,5-difluoro-phenyl)-allylamino]-6-[(*E*)-2-(3-fluoro-6-methoxy-[1,5]naphthyridin-4-yl)-vinyl]-tetrahydro-pyran-2-yl}-propane-1,2-diol;
- (2*S*)-3-{(2*R*,3*R*,6*S*)-3-[(*E*)-3-(2,5-difluoro-phenyl)-allylamino]-6-[(*E*)-2-(3-fluoro-6-methoxy-[1,5]naphthyridin-4-yl)-vinyl]-tetrahydro-pyran-2-yl}-propane-1,2-diol;
- (*E*)-3-(2,5-difluoro-phenyl)-*N*-[(2*R*,3*R*,6*S*)-2-((2*R*)-2,3-dihydroxy-propyl)-6-(6-methoxy-quinolin-4-yloxymethyl)-tetrahydro-pyran-3-yl]-acrylamide;
- (*E*)-3-(2,5-difluoro-phenyl)-*N*-[(2*R*,3*R*,6*S*)-2-((2*S*)-2,3-dihydroxy-propyl)-6-(6-methoxy-quinolin-4-yloxymethyl)-tetrahydro-pyran-3-yl]-acrylamide;
- (2*S*,5*R*,6*R*)-5-[(*E*)-3-(2,5-difluoro-phenyl)-allylamino]-6-[(2*R*)-2,3-dihydroxy-propyl]-tetrahydro-pyran-2-carboxylic acid (6-methoxy-[1,5]naphthyridin-4-yl)-amide;
- (2*S*,5*R*,6*R*)-5-[(*E*)-3-(2,5-difluoro-phenyl)-allylamino]-6-[(2*S*)-2,3-dihydroxy-propyl]-tetrahydro-pyran-2-carboxylic acid (6-methoxy-[1,5]naphthyridin-4-yl)-amide;
- (2*S*,5*R*,6*R*)-6-[(2*R*)-2,3-dihydroxy-propyl]-5-[(3-oxo-3,4-dihydro-2*H*-pyrido[3,2-*b*][1,4]thiazin-6-ylmethyl)-amino]-tetrahydro-pyran-2-carboxylic acid (6-methoxy-[1,5]naphthyridin-4-yl)-amide;
- (2*S*,5*R*,6*R*)-6-[(2*S*)-2,3-dihydroxy-propyl]-5-[(3-oxo-3,4-dihydro-2*H*-pyrido[3,2-*b*][1,4]thiazin-6-ylmethyl)-amino]-tetrahydro-pyran-2-carboxylic acid (6-methoxy-[1,5]naphthyridin-4-yl)-amide;
and salts (in particular pharmaceutically acceptable salts) thereof;
and in particular
- (*E*)-3-(2,5-difluoro-phenyl)-*N*-{(2*R*,3*R*,6*R*)-2-((2*R*)-2,3-dihydroxy-propyl)-6-[2-(3-methoxy-quinolin-5-yl)-ethyl]-tetrahydro-pyran-3-yl}-acrylamide;
- (*E*)-3-(2,5-difluoro-phenyl)-*N*-{(2*R*,3*R*,6*R*)-2-((2*S*)-2,3-dihydroxy-propyl)-6-[2-(3-methoxy-quinolin-5-yl)-ethyl]-tetrahydro-pyran-3-yl}-acrylamide;
- 3-oxo-3,4-dihydro-2*H*-pyrido[3,2-*b*][1,4]thiazine-6-carboxylic acid {(2*R*,3*R*,6*R*)-2-((2*R*)-2,3-dihydroxy-propyl)-6-[2-(3-methoxy-quinolin-5-yl)-ethyl]-tetrahydro-pyran-3-yl} -amide;
- 3-oxo-3,4-dihydro-2*H*-pyrido[3,2-*b*][1,4]thiazine-6-carboxylic acid {(2*R*,3*R*,6*R*)-2-((2*S*)-2,3-dihydroxy-propyl)-6-[2-(3-methoxy-quinolin-5-yl)-ethyl]-tetrahydro-pyran-3-yl} -amide;
- 6-({(2R,3*R*,6*R*)-2-((2*R*)-2,3-dihydroxy-propyl)-6-[2-(3-methoxy-quinolin-5-yl)-ethyl]-tetrahydro-pyran-3-ylamino}-methyl)-4*H*-pyrido[3,2-*b*][1,4]thiazin-3-one;
- 6-({(2*R*,3*R*,6*R*)-2-((2*S*)-2,3-dihydroxy-propyl)-6-[2-(3-methoxy-quinolin-5-yl)-ethyl]-tetrahydro-pyran-3-ylamino}-methyl)-4*H*-pyrido[3,2-*b*][1,4]thiazin-3-one;
- 6-({(2*R*,3*R*,6*R*)-2-((*2*R)-2,3-dihydroxy-propyl)-6-[2-(6-methoxy-[1,5]naphthyridin-4-yl)-ethyl]-tetrahydro-pyran-3-ylamino}-methyl)-4*H*-pyrido[3,2-*b*][1,4]thiazin-3-one;
- 6-({(2*R*,3*R*,6*R*)-2-((2*S*)-2,3-dihydroxy-propyl)-6-[2-(6-methoxy-[1,5]naphthyridin-4-yl)-ethyl]-tetrahydro-pyran-3-ylamino}-methyl)-4*H*-pyrido[3,2-*b*][1,4]thiazin-3-one;
- (2*R*)-3-{(2*R*,3*R*,6*R*)-3-[(*E*)-3-(2,5-difluoro-phenyl)-allylamino]-6-[2-(6-methoxy-[1,5]naphthyridin-4-yl)-ethyl]-tetrahydro-pyran-2-yl}-propane-1,2-diol;
- (2*S*)-3-{(2*R*,3*R*,6*R*)-3-[(*E*)-3-(2,5-difluoro-phenyl)-allylamino]-6-[2-(6-methoxy-[1,5]naphthyridin-4-yl)-ethyl]-tetrahydro-pyran-2-yl}-propane-1,2-diol;
- 2-{(2*R*,3*R*,6*R*)-3-[(*E*)-3-(2,5-difluoro-phenyl)-allylamino]-6-[2-(6-methoxy-[1,5]naphthyridin-4-yl)-ethyl]-tetrahydro-pyran-2-yl}-*N*-hydroxy-acetamide;
- (E)-3-(2,5-difluoro-phenyl)-*N*-{(2*R*,3*R*,6*R*)-2-((2*R*)-2,3-dihydroxy-propyl)-6-[2-(6-methoxy-[1,5]naphthyridin-4-yl)-ethyl]-tetrahydro-pyran-3-yl}-acrylamide;
- (*E*)-3-(2,5-difluoro-phenyl)-*N*-{(2*R*,3*R*,6*R*)-2-((2*S*)-2,3-dihydroxy-propyl)-6-[2-(6-methoxy-[1,5]naphthyridin-4-yl)-ethyl]-tetrahydro-pyran-3-yl}-acrylamide;
- (2*R*)-3-{(2*R*,3*R*,6*S*)-3-[(*E*)-3-(2,5-difluoro-phenyl)-allylamino]-6-[(*S*)-1-hydroxy-2-(6-methoxy-[1,5]naphthyridin-4-yl)-ethyl]-tetrahydro-pyran-2-yl}-propane-1,2-diol;
- (2*S*)-3-{(2*R*,3*R*,6*S*)-3-[(*E*)-3-(2,5-difluoro-phenyl)-allylamino]-6-[(*S*)-1-hydroxy-2-(6-methoxy-[1,5]naphthyridin-4-yl)-ethyl]-tetrahydro-pyran-2-yl}-propane-1,2-diol;
- (2*R*)-3-{(2*R*,3*R*,6*R*)-3-[(*E*)-3-(2,5-difluoro-phenyl)-allylamino]-6-[2-(6-methoxy-quinolin-4-yl)-ethyl]-tetrahydro-pyran-2-yl}-propane-1,2-diol;
- (2*S*)-3-{(2*R*,3*R*,6*R*)-3-[(*E*)-3-(2,5-difluoro-phenyl)-allylamino]-6-[2-(6-methoxy-quinolin-4-yl)-ethyl]-tetrahydro-pyran-2-yl}-propane-1,2-diol;
- {(2*R*,3*R*,6*R*)-3-[(*E*)-3-(2,5-difluoro-phenyl)-allylamino]-6-[2-(6-methoxy-quinolin-4-yl)-ethyl]-tetrahydro-pyran-2-yl}-acetonitrile;
- (*E*)-3-(2,5-difluoro-phenyl)-*N*-{(2*R*,3*R*,6*S*)-2-((2*R*)-2,3-dihydroxy-propyl)-6-[(*E*)-2-(3-fluoro-6-methoxy-[1,5]naphthyridin-4-yl)-vinyl]-tetrahydro-pyran-3-yl} acrylamide;
- (*E*)-3-(2,5-difluoro-phenyl)-*N*-{(2*R*,3*R*,6*S*)-2-((2*S*)-2,3-dihydroxy-propyl)-6-[(*E*)-2-(3-fluoro-6-methoxy-[1,5]naphthyridin-4-yl)-vinyl]-tetrahydro-pyran-3-yl} acrylamide;
- (2*R*)-3-{(2*R*,3*R*,6*S*)-3-[(*E*)-3-(2,5-difluoro-phenyl)-allylamino]-6-[(*E*)-2-(3-fluoro-6-methoxy-[1,5]naphthyridin-4-yl)-vinyl]-tetrahydro-pyran-2-yl}-propane-1,2-diol;
- (2*S*)-3-{(2*R*,3*R*,6*S*)-3-[(*E*)-3-(2,5-difluoro-phenyl)-allylamino]-6-[(*E*)-2-(3-fluoro-6-methoxy-[1,5]naphthyridin-4-yl)-vinyl]-tetrahydro-pyran-2-yl}-propane-1,2-diol;
- (*E*)-3-(2,5-difluoro-phenyl)-*N*-[(2*R*,3*R*,6*S*)-2-((2*R*)-2,3-dihydroxy-propyl)-6-(6-methoxy-quinolin-4-yloxymethyl)-tetrahydro-pyran-3-yl]-acrylamide;
- (*E*)-3-(2,5-difluoro-phenyl)-*N*-[(2*R*,3*R*,6*S*)-2-((2*S*)-2,3-dihydroxy-propyl)-6-(6-methoxy-quinolin-4-yloxymethyl)-tetrahydro-pyran-3-yl]-acrylamide;
and salts (in particular pharmaceutically acceptable salts) thereof;

Compounds of formula I are suitable for the use as chemotherapeutic active compounds in human and veterinary medicine and as substances for preserving inorganic and organic materials in particular all types of organic materials for example polymer, lubricants, paints, fibres, leather, paper and wood.

These compounds according to the invention are particularly active against bacteria and bacteria-like organisms. They are therefore particularly suitable in human and veterinary medicine for the prophylaxis and chemotherapy of local and systemic infections caused by these pathogens as well as disorders related to bacterial infections comprising pneumonia, otitis media, sinusitis, bronchitis, tonsillitis, and mastoiditis related to infection by *Streptococcus pneumoniae, Haemophilus influenzae, Moraxella catarrhalis, Staphylococcus aureus, Enterococcus faecalis, E.faecium, E. casseliflavus, S. epidermizidis, S. haemolyticus,* or *Peptostreptococcus spp*.; pharyngitis rheumatic fever, and glomerulonephritis related to infection by *Streptococcus pyogenes,* Groups C and G streptococci, *Coynebacterium diphtheriae,* or *Actinobacillus haemolyticum;* respiratory tract infections related to infection by *Mycoplasma pneumoniae, Legionella pneumophila, Streptococcus pneumoniae, Haemophilus influenzae,* or *Chlamydia pneumoniae;* blood and tissue infections, including endocarditis and osteomyelitis, caused by *S. aureus, S. haemolyticus, E. faecalis, E. faecium, E. durans,* including strains resistant to known antibacterials such as, but not limited to, beta-lactams, vancomycin, aminoglycosides, quinolones, chloramphenicol, tetracyclines and macrolides; uncomplicated skin and soft tissue infections and abscesses, and puerperal fever related to infection by *Staphylococcus aureus,* coagulase-negative staphylococci (i.e., *S. epidermidis, S. haemolyticus,* etc.), *Streptococcus pyogenes, Streptococcus agalactiae,* Streptococcal groups C-F (minute colony streptococci), viridans streptococci, *Corynebacterium minutissimum, Clostridium spp.,* or *Bartonella henselae;* uncomplicated acute urinary tract infections related to infection by *Staphylococcus aureus,* coagulase-negative staphylococcal species, or *Enterococcus spp.;* urethritis and cervicitis; sexually transmitted diseases related to infection by *Chlamydia trachomatis, Haemophilus ducreyi, Treponema pallidum, Ureaplasma urealyticum,* or *Neiserria gonorrheae;* toxin diseases related to infection by S. *aureus* (food poisoning and toxic shock syndrome), or Groups A, B, and C streptococci; ulcers related to infection by *Helicobacter pylori;* systemic febrile syndromes related to infection by *Borrelia recurrentis;* Lyme disease related to infection by *Borrelia burgdorferi;* conjunctivitis, keratitis, and dacrocystitis related to infection by *Chlamydia trachomatis, Neisseria gonorrhoeae, S. aureus, S. pneumoniae, S. pyogenes, H. influenzae,* or *Listeria* spp.; disseminated *Mycobacterium avium* complex (MAC) disease related to infection by *Mycobacterium avium,* or *Mycobacterium intracellulare;* infections caused by *Mycobacterium tuberculosis, M. leprae, M. paratuberculosis, M. kansasii,* or *M. chelonei;* gastroenteritis related to infection by *Campylobacter jejuni*; intestinal protozoa related to infection by *Cryptosporidium* spp.; odontogenic infection related to infection by viridans streptococci; persistent cough related to infection by *Bordetella pertussis*; gas gangrene related to infection by *Clostridium perfringens* or *Bacteroides* spp.; and atherosclerosis or cardiovascular disease related to infection by *Helicobacter pylori* or *Chlamydia pneumoniae.*

Compounds of formula I according to the present invention are further useful for the preparation of a medicament for the treatment of infections that are mediated by bacteria such as *E. coli, Klebsiella pneumoniae* and other Enterobacteriaceae, *Acinetobacter spp*., *Stenothrophomonas itialtophilia, Neisseria meningitidis, Bacillus cereus, Bacillus anthracis, Corynebacterium spp., Propionibacterium acnes* and bacteroide *spp.*

Compounds of formula I according to the present invention are further useful to treat protozoal infections caused by *Plasmodium malaria, Plasmodium falciparum, Toxoplasma gondii, Pneumocystis carinii, Trypanosoma brucei* and *Leishmania spp.*

The present list of pathogens is to be interpreted merely as examples and in no way as limiting.

One aspect of this invention therefore relates to the use of a compound of formula I according to this invention, or of a pharmaceutically acceptable salt thereof, for the manufacture of a medicament for the prevention or treatment of a bacterial infection.

As well as in humans, bacterial infections can also be treated using compounds of formula I (or pharmaceutically acceptable salts thereof) in other species like pigs, ruminants, horses, dogs, cats and poultry.

The present invention also relates to pharmacologically acceptable salts and to compositions and formulations of compounds of formula I.

Any reference to a compound of formula I is to be understood as referring also to the salts (and especially the pharmaceutically acceptable salts) of such compounds, as appropriate and expedient.

Examples of pharmacologically acceptable salts of sufficiently basic compounds of formula I are selected from the group consisting of salts of physiologically acceptable mineral acids like hydrochloric, hydrobromic, hydroiodic, sulfuric, sulfamic, nitric and phosphoric acid; or salts of organic acids like methanesulfonic, ethanedisulfonic, p-toluenesulfonic, lactic, acetic, trifluoroacetic, oxalic, benzoic, citric, succinic, fumaric, maleic, mandelic, cinnamic, pamoic, stearic, glutamic, aspartic and salicylic acid. Further, a sufficiently acidic compound of formula I may form alkali or earth alkaline metal salts, for example sodium, potassium, lithium; calcium or magnesium salts; ammonium salts; or organic base salts, for example methylamine, dimethylamine, trimethylamine, triethylamine, ethylenediamine, ethanolamine, choline hydroxide, meglumin, piperidine, morpholine, tris-(2-hydroxyethyl)amine, lysine or arginine salts. For other examples of pharmaceutically acceptable salts, reference can be made notably to "Salt selection for basic drugs", Int. J. Pharm. (1986), 33, 201-217.

A pharmaceutical composition according to the present invention contains at least one compound of formula I (or a pharmaceutically acceptable salt thereof) as the active agent and optionally carriers and/or diluents and/or adjuvants, and may also contain additional known antibiotics.

As mentioned above, therapeutically useful agents that contain compounds of formula I, their salts and formulations thereof are also comprised in the scope of the present invention. In general, compounds of formula I will be administered by using the known and acceptable modes known in the art, either alone or in combination with any other therapeutic agent. Such therapeutically useful agents can be administered by one of the following routes: oral, e.g. as tablets, dragee, coated tablets, pills, semisolids, soft or hard capsule, for example soft and hard gelatine capsules, aqueous or oily solutions, emulsions, suspensions or syrups, parenteral including intravenous, intramuscular and subcutaneous injection, e.g. as an injectable solution or suspension, rectal as suppositories, by inhalation or insufflation, e.g. as a powder formulation, as microcrystal or as a spray (e.g. liquid aerosol), transdermal, for example via an transdermal delivery system (TDS) such as a plaster containing the active ingredient, topical or intranasal. The substance of the present invention can also be used to impregnate or coated devices that are foreseen for implantation like catheters or artificial joints. The pharmaceutically useful agents may also contain additives for conservation, stabilisation, e.g. UV stabilizers, emulsifiers, sweetener, aromatisers, salts to change the osmotic pressure, buffers, coating additives and antioxidants.

The production of the pharmaceutical compositions can be effected in a manner which will be familiar to any person skilled in the art (see for example Remington, The Science and Practice of Pharmacy, 21st. Edition (2005), Part 5, "Pharmaceutical Manufacturing" [published by Lippincott Williams & Wilkins]) by bringing the described compounds of Formula (I) or their pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.

Besides, any preferences indicated for the compounds of formula I (whether for the compounds themselves, salts thereof, compositions containing the compounds or salts thereof, uses of the compounds or salts thereof, etc.) apply *mutatis mutandis* to compounds of formula I_{P}, compounds of formula I_{CE}, compounds of formula I_{CEP}, compounds of formula I_{Ar}, compounds of formula I_{ArP}, compounds of formula I_{Het} and compounds of formula I_{HetP}.

Moreover, the compounds of formula I may also be used for cleaning purposes, e.g. to remove pathogenic microbes and bacteria from surgical instruments or to make a room or an area aseptic. For such purposes, the compounds of formula I could be contained in a solution or in a spray formulation.

The compounds of formula I can be manufactured in accordance with the present invention using the procedures described hereafter.

### PREPARATION OF COMPOUNDS OF FORMULA I OR I_{P}

### Abbreviations:

The following abbreviations are used throughout the specification and the examples:
- AD-mix α: 1,4-*bis*(dihydroquinine)phthalazine, K₃Fe(CN)₆, K₂CO₃ and K₂O_{S}O₄.2H₂O
- AD-mix β: 1,4-*bis*(dihydroquinidine)phthalazine, K₃Fe(CN)₆, K₂CO₃ and K₂OsO₄.2H₂O
- Alloc: allyloxycarbonyl
- aq.: aqueous
- BINAP: 2,2'-*bis*-(diphenylphosphino)-1,1'-binaphthalin
- Boc: *tert*-butoxycarbonyl
- BOC₂O: di-*tert*-butyl dicarbonate
- Cbz: benzyloxycarbonyl
- dba: dibenzylidene acetone
- DBU: 1,8-diazabicyclo[5.4.0]undec-7-ene
- DBN: 1,5-diazabicyclo[4.3.0]non-ene
- DCC: dicyclohexyl carbodiimide
- 1,2-DCE: 1,2-dichloroethane
- DCM: dichloromethane
- (DHQ)₂PHAL: 1,4-*bis*(dihydroquinine)phthalazine
- DEAD: diethyl azo dicarboxylate
- DIAD: diisopropyl azo dicarboxylate
- DIBAH: diisobutylaluminium hydride
- DIPEA: *N*,*N*-diisopropylethylamine
- 1,2-DME: 1,2-dimethoxyethane
- DMF: *N*,*N*-dimethylformamide
- DMSO: dimethylsulfoxide
- DMPU: 1,3-dimethyl-3,4,5,6-tetrahydro-2(1*H*)-pyrimidone
- EA: ethyl acetate
- EDC: 1-(3-dimethylaminopropyl)-3-ethylcarbodiimide hydrochloride
- ESI: Electron Spray Ionisation
- ether or Et₂O: diethyl ether
- EtOH: ethanol
- h: hour(s)
- HATU: *O*-(7-azabenzotriazol-1-yl)-*N*,*N*,*N*',*N*'-tetramethyluronium hexafluorophosphate
- Hex: hexane
- HOBT: 1-hydroxybenzotriazole hydrate
- HPLC: high performance liquid chromatography
- HV: high vacuum conditions
- LG: leaving group
- LiHMDS: lithium hexamethyldisilazane
- KHMDS: potassium hexamethyldisilazane
- MeOH: methanol
- min: minute(s)
- MCPBA: *meta*-chloroperbenzoic acid
- MeCN: acetonitrile
- MS: mass spectroscopy
- MsCl: methanesulfonyl chloride
- NBS: *N*-bromosuccinimide
- NHS: *N*-hydroxysuccinimide
- *n*-BuLi: *n*-butyl lithium
- NMO: 4-methylmorpholine-*N*-oxide
- org.: organic
- OTf: triflate
- Pd/C: palladium on charcoal
- PPh₃: triphenylphosphine
- PTSA: *para*-toluene sulfonic acid
- Rf: retention factor
- rt: room temperature
- SiO₂: silica gel
- TBAF: tetrabutylammonium fluoride
- TBDMS: *tert*-butyldimethylsilyl
- TEA: triethylamine
- TFA: trifluoroacetic acid
- THF: tetrahydrofuran
- Ts: *para*-toluenesulfonyl
- TsCl: *para*-toluenesulfonyl chloride

### General preparation methods:

The compounds of formula I or I_{P} can be manufactured in accordance with the present invention by
a) reacting a compound of formula II 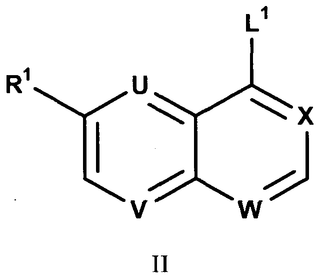 with a compound of formula III

   **A⁰M⁰E** III

   wherein
   R¹, U, V, W, X and D are as in formula I,
   A⁰M⁰ is the spacer M in which A⁰ is the group A¹ that has been functionally modified so as to be able to interact with the reactive group L¹ in order to connect the two moieties of formulae II. and III and yield the compound of formula I,
   M⁰ is the spacer M diminished by A¹,
   E is D or a protecting group (e.g. Cbz, Alloc or Boc), and
   M and A¹ are as in formula I,
   and, in case E is a protecting group, deprotection of the nitrogen protecting group and reaction of the intermediate obtained with a compound of formula IV

   **L²D** IV

   wherein L² is a reactive group yielding the group A² and A¹, A², R¹, R², U, V, W, X and D are as in formula I;
   or
b) reacting a compound of formula V: 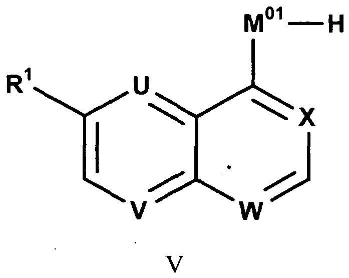 wherein M⁰¹ is 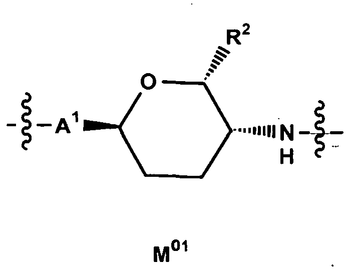 with a compound of formula VI:

   **L³D** VI

   wherein L³ is a reactive group yielding the group A² and A¹, A², R¹, R², U, V, W, X and D are as in formula I,
   and, where required, transforming the group R² (e.g. by oxidation or nucleophilic substitution),
   or also
c) reacting a compound of formula VII 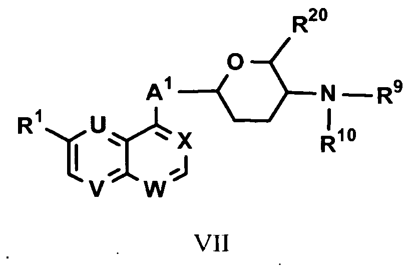 with a compound of formula VIII

   **L⁴R¹¹** VIII

   wherein
   L⁴ is a reactive group connecting the groups R²⁰, functionally modified, and R¹¹ in order to form R²,
   R⁹ is hydrogen or an optional protecting group,
   R¹⁰ is the group D or a protecting group E (e.g. Boc, Alloc or Cbz), and
   A¹, R¹, U, V, W, X and D are as in formula I,
   and where required, deprotecting the nitrogen of the spacer M and, in case R¹⁰ is a protecting group E, reacting the intermediate obtained with a compound of formula IV as described in process alternative a).

The compounds or formula I or I_{P} thus obtained may, if desired, be converted into their pharmaceutically acceptable salts.

Besides, whenever the compounds of formula I or I_{P} are obtained in the form of mixtures of enantiomers, the enantiomers can be separated using methods known to one skilled in the art (e.g. by formation and separation of diastereomeric salts or by chromatography on a chiral stationary phase). Whenever the compounds of formula I or I_{P} are obtained in the form of mixtures of diasteromers they may be separated by an appropriate combination of silica gel chromatography, HPLC and crystallization techniques.

In process alternative a), preferred reactive groups L¹ and A⁰ and resulting connections A¹, as the case may be, are evident from the following Table 1:

**Table 1**

| **L¹** | **A⁰** | **A¹** | **Subsequent transformations** |
|---|---|---|---|
| NH₂ | HOOC | NHCO | |
| OSO₂CF₃or halogen | H₂NCO | NHCO | |
| OH or halogen | HO-CH₂ | OCH₂ | |
| Li or MgBr | HCO-CH₂ | CH(OH)CH₂ | |
| Li or MgBr | CH₃N(OCH₃)COCH₂ | COCH₂ | CH(OH)CH₂ |
| Li or MgBr | CF₃SO₂O-CH₂CH₂ | CH₂CH₂ | |
| Li or MgBr | halogen-CH₂CH₂ | CH₂CH₂ | |
| OSO₂CF₃, Br, I | HC≡C | C≡C | CH=CH, CH₂CH₂, CH(OH)CH(OH), CH(OH)CH₂, CH₂CH(OH) |
| OSO₂CF₃, Br | (HO) ₂B-CH₂CH₂ | CH₂CH₂ | |
| OSO₂CF₃ | Y-HC=CH (Y = H or Bu₃Sn) | CH=CH | CH₂CH₂, CH(OH)CH(OH), CH(OH)CH₂, CH₂CH(OH) |
| CHO | P(Ph)₃=CH | CH=CH | CH₂CH₂, CH(OH)CH(OH), CH(OH)CH₂, CH₂CH(OH) |
| CHO | PO(OR)₂CH₂ (R = alkyl) | CH=CH | CH₂CH₂, CH(OH)CH(OH), CH(OH)CH₂, CH₂CH(OH) |
| CHO | RSO₂CH₂ . (R = aryl, heteroaryl) | CH=CH | CH₂CH₂, CH(OH)CH(OH), CH(OH)CH₂, CH₂CH(OH) |

In process alternative a), b) or c), preferred reactive groups L² or L³ and resulting connections A² are evident from the following Table 2:

**Table 2**

| **L² or L³** | **NH-A²** |
|---|---|
| OHC-CH=CH | NHCH₂CH=CH |
| XCH₂ | NHCH₂ |
| TsOCH₂ | NHCH₂ |
| YCO-[CH=CH]ₙ | NHCO[CH=CH]ₙ |

| | |
|---|---|
| X= halogen; Y = OH or Cl; Ts= tosyl; n = 0 or 1 | |

In process alternative c), preferred reactive groups R²⁰ and L⁴R¹¹ and resulting groups R², as the case may be, are evident from the following Table 3:

**Table 3**

| **R²⁰** | **L⁴R¹¹** | **R²** | **Subsequent transformations** |
|---|---|---|---|
| (CH₂)ₙCHOHCH₂OH | (oxidation) | (CH₂)ₙCHO | |
| (CH₂)ₙCHO | (reduction) | (CH₂)ₙCH₂0H | |
| (CH₂)ₙoxirane | NHR^{b}R^{c} | (CH₂)ₙCHOHCH₂NR^{b}R^{c} | |
| (CH₂)ₙCHO | NHR^{b}R^{c} | (CH₂)ₙCH₂NR^{b}R^{c} | |
| (CH₂)ₙCHO | NH₂OR^{d} | (CH₂)ₙCH=NOR^{d} | |
| (CH₂)ₙCH=NOR^{d} | (-H₂O) | (CH₂)ₙCN | (CH₂)ₙ-[1,2,3,4-tetrazol-5-yl] (CH₂)ₙ-[thiazol-2-yl] |
| (CH₂)ₙCN | (hydrolysis) | (CH₂)ₙCONH₂ | (CH₂)ₙ-[imidazol-2-yl] (CH₂)ₙ-[oxazol-2-yl] |
| (CH₂)ₙCONH₂ | (dehydration) | (CH₂)ₙCN | |
| (CH₂)ₙCHO | (oxidation) | (CH₂)ₙCOOH | (CH₂)ₙCONR^{g}R^{h} (CH₂)ₙCONHOR^{k} (CH₂)ₙCONR^{x}R^{y} |
| (CH₂)ₙCHO | CBr₄ | (CH₂)ₙC≡CH | (CH₂)ₙ-[1,2,3-triazol-4-yl] |
| (CH₂)ₙCH₂OSO₂R^{e} | LiC≡ CH | (CH₂)ₙCH₂C≡CH | (CH₂)ₙ₊₁-[1,2,3-triazol-4-yl] |
| (CH₂)ₙCH₂OSO₂R^{e} | tetrazol | (CH₂)ₙCH₂-[tetrazol-1-yl] | |
| (CH₂)ₙCH₂OSO₂R^{e} | NaN₃ | (CH₂)ₙCH₂N₃ | (CH₂)ₙCH₂-[tetrazol-2-yl] (CH₂)ₙCH₂-[triazol-1-yl] |
| (CH₂)ₙCH₂OSO₂R^{e} | NHR^{b}R^{c} | (CH₂)ₙCH₂NR^{b}R^{c} | |
| (CH₂)ₙCH₂X' | NHR^{b}R^{c} | (CH₂)ₙCH₂NR^{b}R^{c} | |

R^{b}, R^{c} = H, alkyl, aryl or arylalkyl; R^{d} = H, alkyl, aryl or arylalkyl; R^{e} = CH₃, CF₃ or Ts; R⁸ = (C₁-C₅)alkyl, cycloalkyl, [(C₂-C₅)hydroxyalkyl] or arylalkyl; R^{h} represents H, or when R^{g} = (C₁-C₃)alkyl, can also represent (C₁-C₃)alkyl; R^{k} = H or (C₂-C₄)alkynylmethyl; NR^{x}R^{y} = pyrrolidin-1-yl, piperidin-1-yl, morpholin-4-yl, thiomorpholin-4-yl, (3*R*)-3-hydroxy-pyrrolidin-1-yl, (3*S*)-3-hydroxy-pyrrolidin-1-yl or 4-hydroxy-piperidin-1-yl; X' = halogen; n = 0,1.

The required quinoline, [1,5]-naphthyridine, quinazoline and quinoxaline derivatives of formula II can be prepared following literature procedures. For example, 4-hydroxy-[1,5]-naphthyridines (L¹ = OH, U = W = N and V = X= CH) and 4-hydroxy quinolines (L¹=OH, W=N and U = V= X= CH) can be prepared from the corresponding aminopyridines or anilines by reaction with diethyl ethoxymethylene malonate to produce the 4-hydroxycarboxylic acid ester derivative with subsequent hydrolysis to acid, followed by thermal decarboxylation in inert solvents (J.T. Adams, J. Am. Chem. Soc. (1946), 68, 1317). Others routes to such derivatives use the condensation of substituted aminopyridines or anilines with 2,2-dimethyl-[1,3]dioxane-dione and triethylorthoformate followed by heating of the resulting 2,2-dimethyl-5-[(arylamino)methylidene]-1,3-dioxane-4,6-dione intermediate in refluxing diphenyl ether. Quinazolines (L¹ = OH, Cl, NH₂, W = X= N and U = V = CH) may be prepared by standard routes as described by T.A. Williamson in Heterocyclic Compounds (1957), 6, 324. 3-substituted quinoxalin-5-ols (L¹ = OH, U = V = N and X = W= CH) can be prepared as described by Y. Abe et al. in J. Med. Chem. (1998), 41, 4062.

The compounds of formula I can be prepared by different routes as illustrated in Schemes 1-14 below (reference is made to Tables 1 and 2 above).

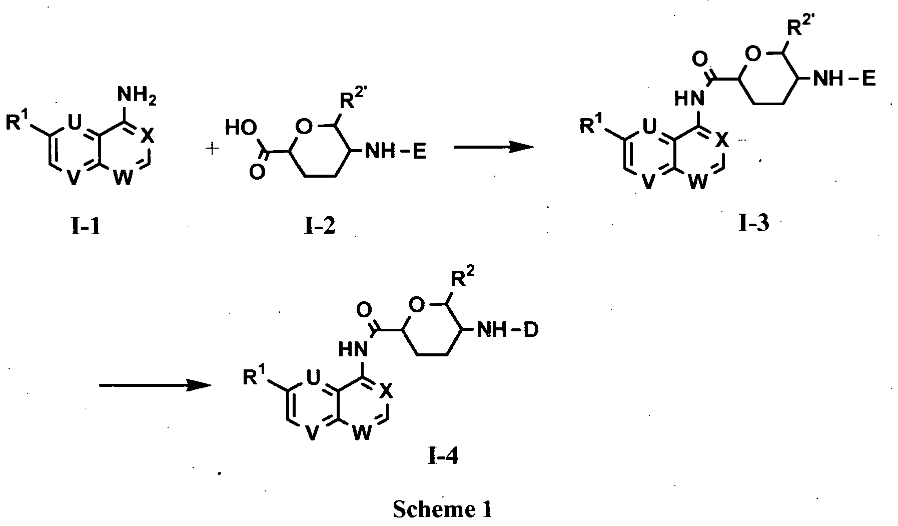

In Scheme 1, E is a protecting group (e.g. Boc, Alloc or Cbz), R² is either R² or a reactive group R²⁰ as defined in Table 3 above, the reactive function of which has been appropriately protected if necessary (see e.g. P.J. Kocienski, 'Protecting Groups', Thieme (1994)) and U, V, W, X, R¹, D and R² are as defined in formula I. Depending on the nature of D, compounds **I-2** wherein E = D can be used in the first step.

Compounds of formula I or I_{P} can for example be obtained from an amine **I-1** and an acid **I-2**. Thus, a 4-hydroxy-[1,5]-naphthyridine, a 4-hydroxyquinazoline, a 5-hydroxy quinoxaline or a 4-hydroxy quinoline derivative can be converted into the corresponding chloro derivative by heating in phosphorous oxychloride between 40°C and 100°C neat or in an inert solvent like 1,2-DCE, or to the corresponding 4-trifluoromethanesulphonyloxy derivative by reaction with trifluoromethanesulphonic anhydride, in the presence of an organic base between -40°C and 80°C in an aprotic solvent like DCM or THF (K. Ritter, Synthesis (1993), 735). 4-amino-[1,5]-naphthyridine, 4-aminoxyquinazoline, 5-amino quinoxaline or 4-amino quinoline derivatives can be obtained by reaction of the corresponding 4-trifluoromethanesulphonyloxy derivatives with ammonia in a solvent like DCM or THF, or with n-propylamine hydrochloride in pyridine between -20°C and 100°C (R. Radinov, Synthesis (1986), 886). 4-aminoxyquinazoline can also be obtained from its 4-chloro analogue by reaction with ammonia under the same conditions.

Carboxylic acids **I-2** may be prepared by Jones' oxidation of the corresponding alcohols using chromium acid and sulphuric acid in water/MeOH between 40°C and 110°C (E. R. H. Jones et al, J. Chem. Soc. (1946), 39). Other oxidising agents may be used for this transformation such as NaIOₐ catalysed by ruthenium trichloride (G. F. Tutwiler et al, J. Med. Chem. (1987), 30, 1094), or KMnO₄ (D. E. Reedich et al, J. Org. Chem. (1985), 50, 3535).

Derivatives **I-3** can be obtained by reacting the 4-amino derivative **I-1** with a carboxylic acid derivative **I-2**, in the presence of an activating agent such as DCC, EDC, HOBT or HATU (G. Benz in Comprehensive Organic Synthesis, B.M. Trost, I. Fleming, Eds; Pergamon Press: New York (1991), vol. 6, p. 381) between -20°C and 60°C in an dry aprotic solvent like DCM, MeCN or DMF. Alternatively, the carboxylic acid can be activated by conversion into its corresponding acid chloride by reaction with oxalyl chloride or thionyl chloride neat or in a solvent like DCM between -20° and 60°C.

When R^{2'} is a reactive group R²⁰ as defined in Table 3 above, the reactive function of which has been appropriately protected if necessary (see e.g. P.J. Kocienski, 'Protecting Groups', Thieme (1994)), a deprotection step is carried out if necessary and the reactive group R²⁰ is converted into the side chain R² using standard reactions known to one skilled in the art.

Removal of the protecting group (E) such as Boc or Cbz on a nitrogen atom in **I-3** wherein R^{2'} = R² can be carried out under standard acidic conditions to give the corresponding free amine. Alternatively the Cbz group can be removed under catalytic hydrogenation over Pd/C. Removal of the allyloxycarbonyl protecting group is achieved in presence of a palladium salt such as palladium acetate or Pd(PPh₃)₄ and an allyl scavenger such as pyrrolidine, tributylstannane or dimedone in a solvent such as THF, acetone or CH₃CN between 0° and 70°C. The use of protecting groups to mask reactive functionality is well-known to those of skill in the art, and other protecting groups are listed in reference book such as P.J. Kocienski, 'Protecting Groups', Thieme (1994). The amine can then be reacted with an (hetero)aryl aldehyde and a suitable reducing agent to provide the homologue **I-4.** The intermediate imine may be formed in a variety of protic or aprotic solvents such as DMF, N,N-dimethylacetamide, 1,2-DCE, MeOH, MeCN, in presence or not of a drying agent such as molecular sieves. The imine can be reduced subsequently or simultaneously with a suitable reagent such a NaBH₄, sodium triacetoxyborohydride or sodium cyanoborohydride (R.O. and M.K. Hutchins Comprehensive Organic Synthesis, B.M. Trost, I. Fleming, Eds; Pergamon Press: New York (1991), vol. 8, p. 25-78). Alternatively, the free amine may also be homologated to give product **I-4** by nucleophilic displacement of a suitable alkyl (hetero)aryl halide, mesylate or tosylate between -20°C and 100°C in a dry aprotic solvent like DCM, MeCN, DMF or THF in presence of a base such as K₂CO₃ or DIPEA.

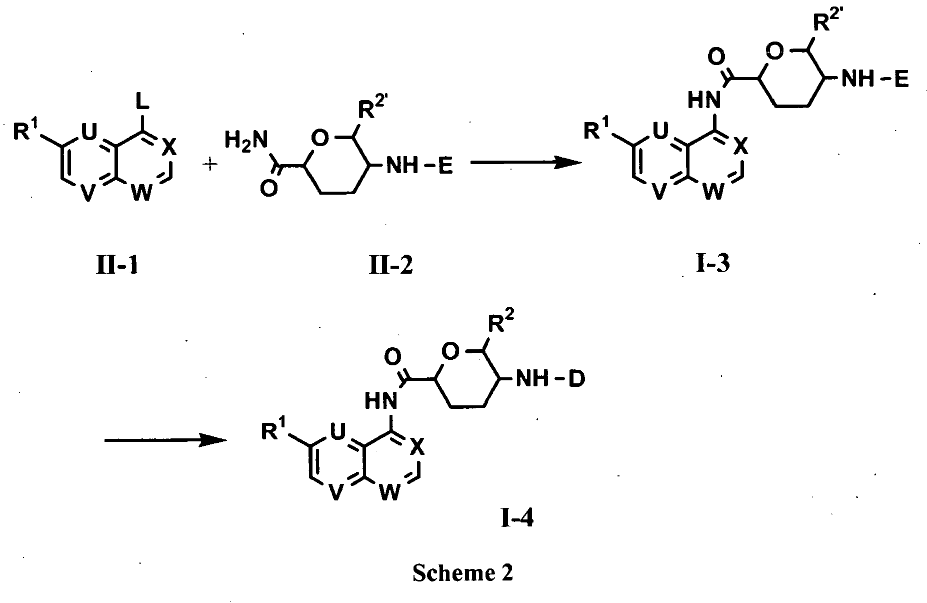

In Scheme 2, E is a protecting group, L is OTf or halogen, R^{2'} is either R² or a reactive group R²⁰ as defined in Table 3 above, the reactive function of which has been appropriately protected if necessary (see e.g. P.J. Kocienski, 'Protecting Groups', Thieme (1994)) and U, V, W, X, R¹, D and R² are as defined in formula I. Depending on the nature of D, compounds **II-2** wherein E = D can be used in the first step.

As illustrated in Scheme 2, the intermediate **I-3** can also be obtained from a 4-trifluoromethanesulfonate derivative **II-1** (L = OSO₂CF₃) and an amide derivative **II-2**. This amide derivative is obtained from a suitable carboxylic acid **I-2**, which is converted into an activated form using, for example, EDC and HOBT, SOCl₂ or NHS and DCC between -20°C and 60°C in a dry aprotic solvent like DCM, EA or THF, and the activated acid is subsequently reacted with aq. ammonium hydroxide or gaseous ammonia to afford amide **II-2** in an appropriate solvent such as THF or DCM between -20°C and 60°C. The amide **II-2** and the 4-trifluoromethanesulphonate **II-1** can be coupled under palladium-catalyzed Buchwald-Hartwig conditions (J. Am. Chem. Soc. (1996), 118, 10333) or copper-catalyzed conditions (J. Am. Chem. Soc. (2002), 124, 7421) to afford the derivative **I-3.** Various palladium sources and ligands may be used, as well as a variety of solvents, including for example dioxane and toluene. Other partners of formula **II-1**, such as the iodo (L = I), bromo (L = Br) or chloro (L = Cl) derivatives may be used in the metal-catalysed coupling reaction. The conversion of the compound **I-3** into the compound **I-4** can then be achieved as explained earlier (see Scheme 2).

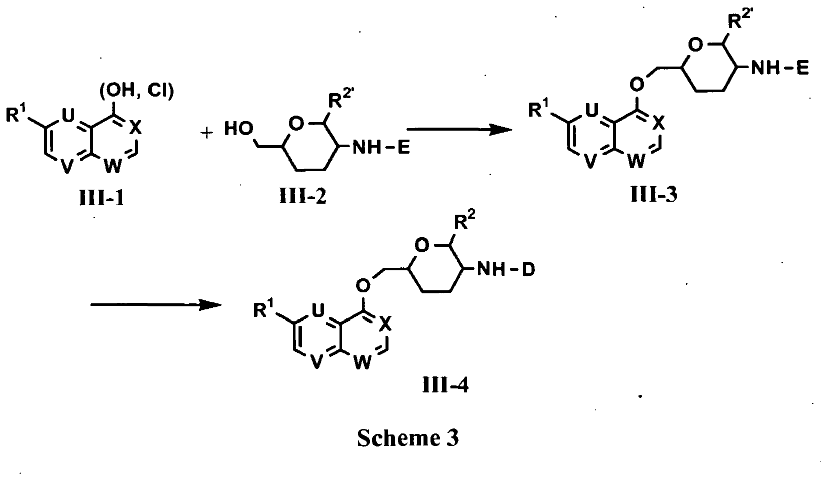

In Scheme 3, E is a protecting group, R^{2'} is either R² or a reactive group R²⁰ as defined in Table 3 above, the reactive function of which has been appropriately protected if necessary (see e.g. P.J. Kocienski, 'Protecting Groups', Thieme (1994)) and U, V, W, X, R¹, D and R² are as defined in formula I. Depending on the nature of D, compounds **III-2** wherein E = D can be used in the first step.

As shown in Scheme 3, certain compounds of formula I or I_{P} can also be obtained by coupling, for example, a substituted 4-hydroxy-quinoline, 8-hydroxy-quinoline, 4-hydroxy-[1,5]-naphthyridine, 4-hydroxy-[1,3]-quinazoline or 5-hydroxyquinoxaline **III-1** and an alcohol derivative **III-2**. The coupling reaction between **III-1** and **III-2** may be achieved under Mitsunobu conditions (as reviewed in O. Mitsunobu, Synthesis (1981), 1). For example, an alcohol **III-2** and a 4-hydroxy derivative **III-1** can be reacted to form ether **III-3** in the presence of DEAD or DIAD and PPh₃. The reaction may be performed in a wide range of solvents such as DMF, THF, DCM and at a wide range of temperature (between -78°C and 50°C). An alternate route to **III-3** may require the activation of the alcohol **III-2** as for example a tosylate, a triflate or a mesylate by treatment with TsCl, trifluoromethanesulphonic anhydride or MsCl respectively in the presence of an organic base such as TEA between -40°C and 60°C in a dry aprotic solvent like DCM, MeCN or THF. Once activated, alcohol **III-2** reacts with the anion of the 4-hydroxy derivative **III-1,** generated with a mineral base such as NaH or K₂CO₃ or an organic base such as LiHMDS, to generate **III-3** between -20°C and 60°C. Alternatively, derivative **III-3** wherein U = V = CH and W = X = N can be obtained by reaction of a 4-halogeno quinazoline derivative with an alcohol derivative in presence of a strong base, e.g. an alkali alkoxide like sodium or potassium methylate, metal hydride like NaH, DBU or DBN between -20°C and 60°C in a dry aprotic solvent like DMF, MeCN or THF. When R^{2'} is a reactive group R²⁰ as defined in Table 3 above, the reactive function of which has been appropriately protected if necessary (see e.g. P.J. Kocienski, 'Protecting Groups', Thieme (1994)), a deprotection step is carried out if necessary and the reactive group R²⁰ is converted into the side chain R² using standard reactions known to one skilled in the art. In the final step, the protecting group E is removed and the free amine is reacted with an alkyl (hetero)aryl halide in presence of a base or with an (hetero)aryl aldehyde in presence of a reducing reagent as previously described to yield compound **III-4**.

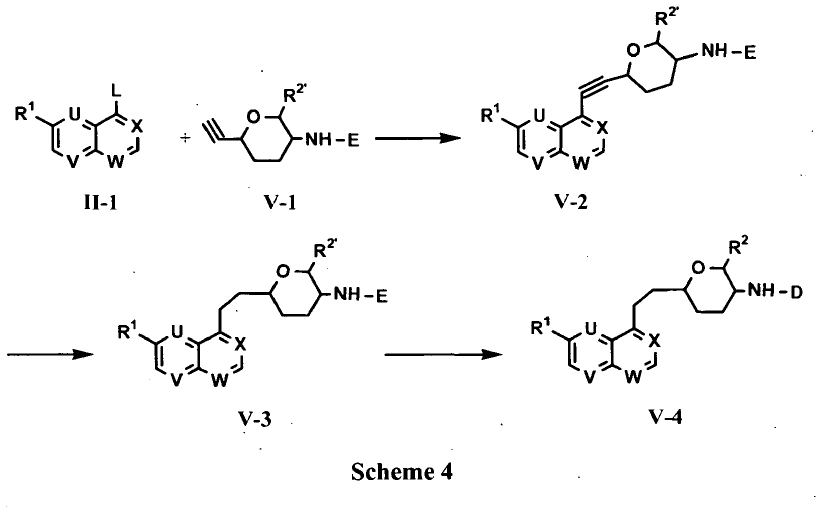

In Scheme 4, L is OSO₂CF₃ or a halogen atom, E is a protecting group, R^{2'} is either R² or a reactive group R²⁰ as defined in Table 3 above, the reactive function of which has been appropriately protected if necessary (see e.g. P.J. Kocienski, 'Protecting Groups'; Thieme (1994)) and U, V, W, X, R¹, D and R² are as defined in formula I. Depending on the nature of D, compounds **V-1** wherein E = D can be used in the first step.

Compounds of formula I or I_{P} can also be obtained from compound **II-1** (Scheme 4) Intermediate **V-2** may be obtained from derivative **II-1** and a terminal alkyne derivative V-1. These alkyne derivatives **V-1** are generally obtained from a suitable alcohol **III-2** (see Scheme 3) which is converted first into an aldehyde using for example the Moffat-Swern (see Synthesis (1981), 165), or the Dess-Martin periodinane (see J. Am. Chem. Soc. (1991), 113, 7277) oxidation protocols. The aldehyde can be converted into the corresponding alkyne using either the Corey-Fuchs protocol (formation of the gem-dibromide then treatment with *n*-BuLi) as described in Tetrahedron Lett. (1972), 3769 or using dimethyl-2-oxopropylphosphonate diazo derivative (so called Ohira's reagent, Synth. Commun. (1989), 19, 561) or dimethyldiazomethylphosphonate as described in Synlett (2003), 59 and Synlett (1996), 521. The alkyne **V-1** and the 4-trifluoromethanesulfonate or bromide **II-1** (L = OSO₃CF₃, Br) are coupled under Sonogashira conditions using catalytic amount of a palladium salt, an organic base such as TEA and a catalytic amount of a copper derivative (usually copper iodide) in a solvent such a DMF between 20°C to 100°C (see Sonogashira, K. in Metal-Catalyzed Reactions, Diedrich, F., Stang, P.J., Eds; Wiley-VCH: New York 1998). Alternatively, for example when U = V = CH and W=X=N, the 4-trifluoromethanesulfonate **II-1** can be replaced by a halogeno (e.g. chloro) derivative **II-1.** The resulting alkyne **V-2** is hydrogenated to the alkane **V-3** using catalytic system such as Pd/C or platinum oxide in a solvent like EtOH or EA in presence of hydrogen. Other methods may also be suitable as reviewed by Siegel, S. et al. in Comprehensive Organic Synthesis, B. M. Trost, I. Fleming, Eds; Pergamon Press: New York (1991), vol. 8, p. 417-488. When R^{2'} is a reactive group R²⁰ as defined in Table 3 above, the reactive function of which has been appropriately protected if necessary (see e.g. P.J. Kocienski, 'Protecting Groups', Thieme (1994)), a deprotection step is carried out if necessary and the reactive group R²⁰ is converted into the side chain R² using standard reactions known to one skilled in the art. The alkane **V-3** wherein R^{2'} = R² is further transformed into the compound **V-4** using procedures previously described.

Alternatively, compounds **V-3** are obtained from the alkenes **VI-3** (described in Scheme 5 hereafter) under similar hydrogenation conditions.

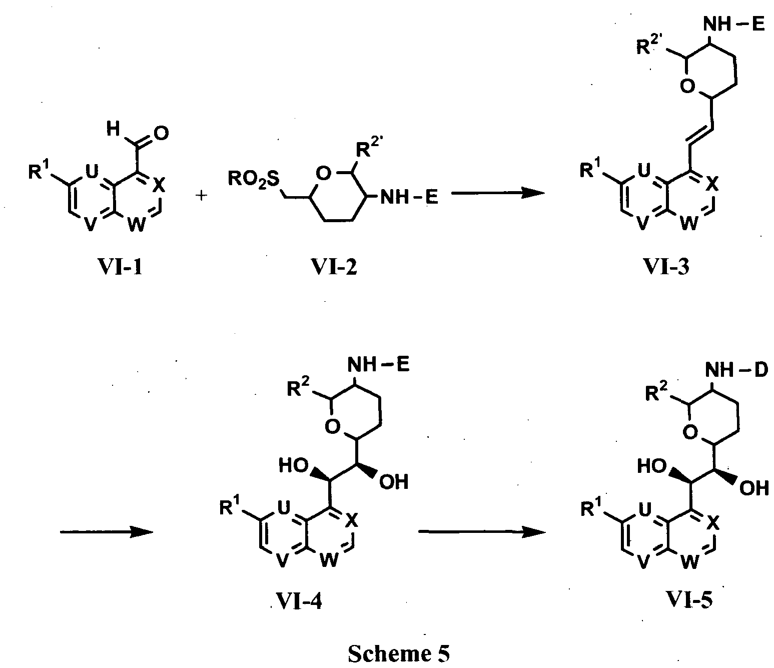

In Scheme 5, E is a protecting group, R may be 1-phenyl-1*H*-tetrazol-5-yl or benzothiazol-2-yl, R^{2'} is either R² or a reactive group R²⁰ as defined in Table 3 above, the reactive function of which has been appropriately protected if necessary (see e.g. P.J. Kocienski, 'Protecting Groups', Thieme (1994)) and U, V, W, X, R¹, D and R² are as defined in formula 1.

Compounds of formula I or I_{P} can also be obtained from compound **VI-1** (Scheme 5). Intermediate **VI-3** may be obtained as an (*E*)-isomer from an aldehyde derivative **VI-1** and a sulfone **VI-2.** The sulfone is generated from the corresponding sulphide *via* an oxidation reaction. A wide range of oxidizing agents may be used to perform such a reaction, such as MCPBA in a solvent such as DCM, oxone^{®} in a solvent such as aq. MeOH (see Tetrahedron Lett. (1981), 22, 1287), or aq. hydrogen peroxide in presence of ammonium heptamolybdate tetrahydrate in EtOH (see J. Org. Chem. (1963), 28, 1140). The sulphide is obtained from a suitable alcohol **III-2** (Scheme 3) *via* a Mitsunobu coupling (as reviewed in O. Mitsunobu, Synthesis (1981), 1) with 1-phenyl-1*H*-tetrazole-5-thiol in the presence of DEAD or DIAD and PPh₃. The reaction may be performed in a wide range of solvents such as DMF, THF or DCM and within a wide range of temperatures (between -78°C and 50°C). An alternate route to form the intermediate sulphide requires the activation of the alcohol **III-2** as for example a tosylate, a triflate or a mesylate by treatment with TsCl, trifluoromethanesulphonic anhydride or MsCl respectively in the presence of an organic base such as TEA between -40°C and 60°C in a dry aprotic solvent like DCM, acetonitrile or THF. Once activated, alcohol **III-2** reacts with NaI or KI in acetone at a temperature ranging between 0°C and 65°C, to form the corresponding iodide. The latter serves as an alkylating agent of the 1-phenyl-1*H*-tetrazole-5-thiol. The alkylation reaction is performed in presence of an inorganic base such as KOH or NaOH in a solvent such as EtOH at a temperature ranging between -20°C and 70°C.

The sulfone **VI-2** and the aldehyde **VI-1** are coupled in presence of a base such as LiHMDS or KHMDS in a solvent such as 1,2-DME, DMF or toluene as reviewed by Blakemore, P.R in J. Chem. Soc., Perkin Trans. 1 (2002), 2563-2585. When R^{2'} is a reactive group R²⁰ as defined in Table 3 above, the reactive function of which has been appropriately protected if necessary (see e.g. P.J. Kocienski, 'Protecting Groups', Thieme (1994)), a deprotection step is carried out if necessary and the reactive group R²⁰ is converted into the side chain R² using standard reactions known to one skilled in the art. The resulting (*E*)-alkene **VI-3** wherein R^{2'} = R² is transformed into the corresponding chiral cis-diol derivative by treatment with AD mixtures (AD-mix α or AD-mix β) in presence of methanesulfonamide in a water/2-methyl-2-propanol mixture as described in Chem. Rev. (1994), 94, 2483. The sense of induction relies on the chiral ligand contained in the mixture, either a dihydroquinine-based ligand in AD-mix α or a dihydroquinidinebased ligand in AD-mix β. The chiral cis-diol **VI-4** is further transformed into the chiral compounds **VI-5** using procedures previously described.

An alternate route to obtain (*E*)-alkene **VI-3** may be to couple a 4-trifluoromethanesulfonate derivative **II-1** (Scheme 2) with an organostannane deriving from a terminal alkyne derivative **V-1** (see Scheme 4). Indeed, the hydrostannation reaction of an alkyne derivative **V-1** using tributyl tin hydride and a catalytic amount of either a palladium salt or a molybdenum complex generates an *E:Z* mixture of the vinylstannane intermediate as described in J. Org. Chem. (1990), 55, 1857. The vinylstannane is reacted with a 4-trifluoromethanesulfonate derivative **II-1** under Stille coupling conditions (as described in J. Am. Chem. Soc. (1987), 109, 5478). Typical reaction conditions involve a palladium salt such as tetrakis(triphenylphosphine) palladium or dichloro bis(triphenylphophine) palladium, LiCl and a radical scavenger such as 2,6-dimethyl-4-methyl phenol in a solvent such as DMF or dioxane at a temperature ranging between 0°C and 100°C, more preferably at a temperature ranging between 20°C and 80°C. As the reaction proceeds normally at a faster rate using (*E*)-vinylstannane, the resulting (*E*)-alkene **VI-3** is usually obtained with a high isomeric purity.

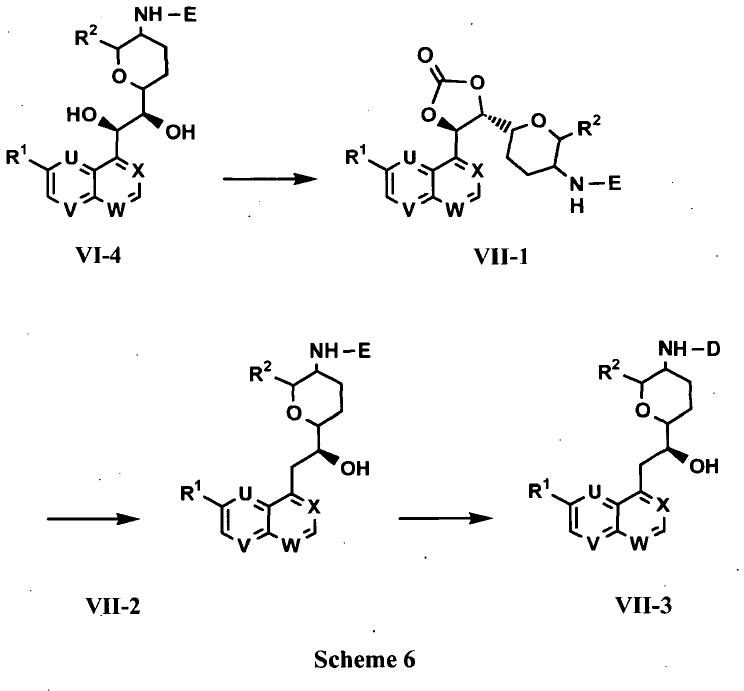

In Scheme 6, E is a protecting group and U, V, W, X, R¹, D and R² are as defined in formula I.

As illustrated in Scheme 6, the previously mentioned chiral cis-diol **VI-4** may be transformed in the corresponding cyclic carbonate **VII-1,** by treatment with either phosgene, diphosgene or triphosgene in presence of an organic base such as TEA or pyridine or carbonyldimidazole in an inert solvent such as DCM or THF at a temperature ranging between -78°C and 50°C, more conveniently at a temperature ranging between 0°C and 20°C. The cyclic carbonate **VII-1** is subsequently transformed to the homobenzylic alcohol **VII-2** by hydrogenolysis using a catalytic system such as Pd/C in presence of hydrogen in a solvent such as EA. The intermediate **VII-2** is further transformed into the compounds **VII-3** using procedures previously described.

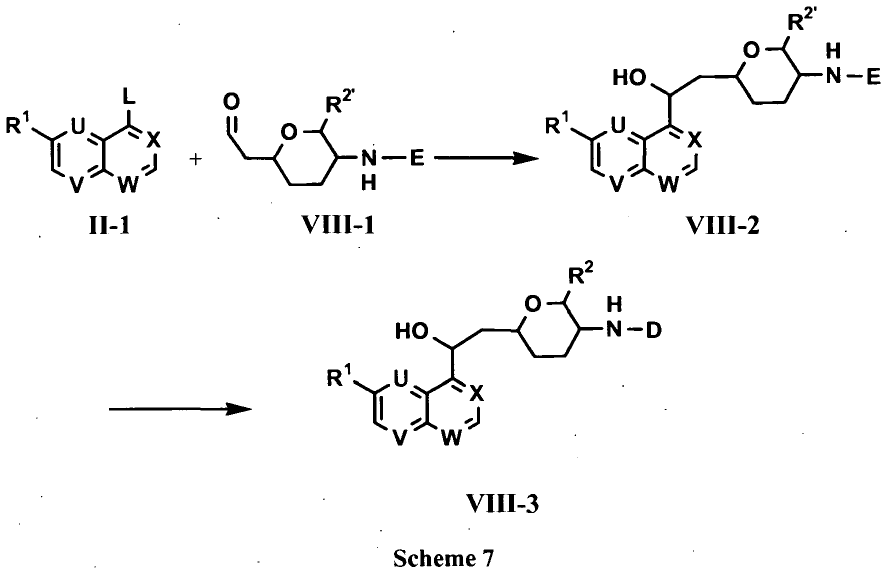

In Scheme 7, E is a protecting group, R^{2'} is either R² or a reactive group R²⁰ as defined in Table 3, the reactive function of which has been appropriately protected if necessary (see e.g. P.J. Kocienski, 'Protecting Groups', Thieme (1994)) and U, V, W, X, R¹, D and R² are as defined in formula I. Depending on the nature of D, compounds **VIII-1** wherein E = D can be used in the first step.

As illustrated in Scheme 7, the benzylic alcohol **VIII-2** may be obtained by addition of an organometallic derivative of aromatic **II-1** (L= Li) onto an aldehyde **VIII-1.** The aldehyde **VIII-1** can be obtained from a suitable alcohol **III-2** by a homologation reaction. Oxidation of the alcohol **III-2** into its corresponding aldehyde may be performed using one of the aforementioned oxidation methods. The resulting aldehyde can further be converted to the corresponding alkene using the phosphorane generated from methyltriphenylphosphonium bromide and a base like *n*-BuLi or potassium *tert*-butoxide in a solvent such as THF at a temperature between -80°C and 0°C (see *Org. Synth. Coll.* (1973), **5,** 751). The terminal alkene is subsequently transformed into the primary alcohol via an hydroboration reaction using either BH₃-dimethylsulfide complex, or 9-borabicyclo[3.3.1]nonane (9-BBN) (for a review see Smith, K.; Pelter, A. G. Comprehensive Organic Synthesis, B.M. Trost, I. Fleming, Eds; Pergamon Press: New York (1991), vol. 8, p. 703-731) followed by oxidative workup with aq. NaOH and 30% H₂O₂ (see also Pelter, A.; Smith, K. G. Comprehensive Organic Synthesis, B.M. Trost, I. Fleming, Eds; Pergamon Press: New York (1991), vol. 7, p. 593-611). The alcohol is finally oxidized to the aldehyde **VIII-1** as already described. Derivative **II-1** (L¹ = Br) can be treated with an alkyllithium such as *n*-BuLi at a temperature ranging between -80°C and -30°C to generate a lithio species that undergoes nucleophilic addition onto the aldehyde **VIII-1** to yield the benzylic alcohol **VIII-2.** When R^{2'} is a reactive group R²⁰ as defined in Table 3 above, the reactive function of which has been appropriately protected if necessary (see e.g. P.J. Kocienski, 'Protecting Groups', Thieme (1994)), a deprotection step is carried out if necessary and the reactive group R²⁰ is converted into the side chain R² using standard reactions known to one skilled in the art. The resulting intermediate **VIII-2** wherein R^{2'} = R² can further be transformed into the compound **VIII-3** using procedures previously described.

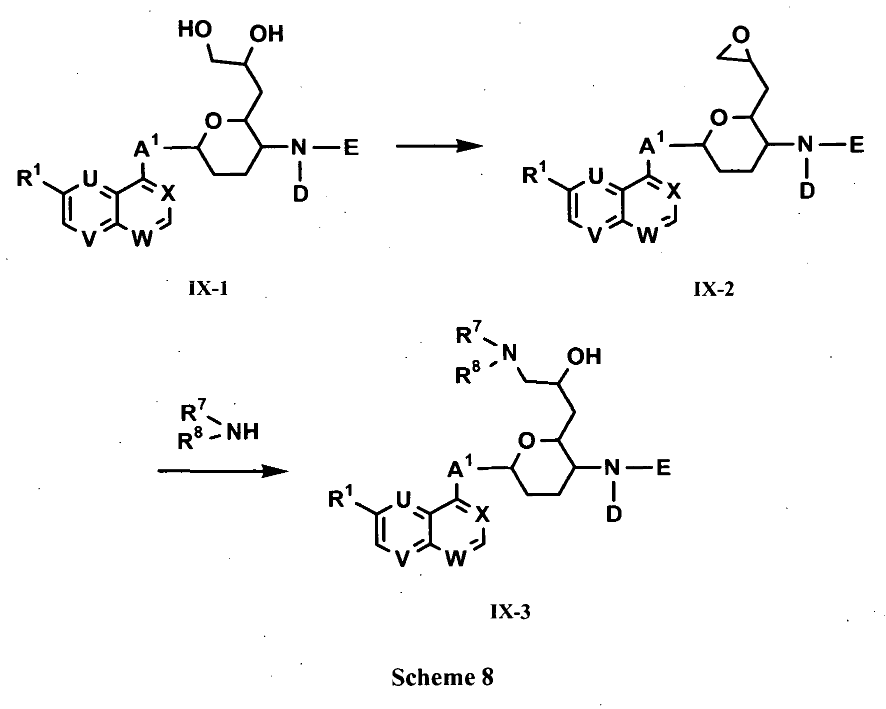

In Scheme 8, E is a protecting group and U, V, W, X, R¹, A¹, D, R⁷ and R⁸ are as defined in formula I.

As illustrated in Scheme 8, the 1,2-diol **IX-1** can be transformed into the corresponding epoxide **IX-2** using several strategies. The primary alcohol is selectively transformed into a leaving group, such as methanesulfonyloxy or *para*-toluenesulfonyloxy, by treatment with MsCl or TsCl in the presence of an organic base such as TEA or pyridine at a temperature ranging between -78°C and 0°C, more preferably at a temperature ranging between -30°C and -10°C. The resulting methane- or toluene-sulfonyloxy derivatives are transformed into the desired epoxides **IX-2** by treatment with an inorganic base such as K₂CO₃ in a solvent such as MeOH or EtOH. Alternatively, the diols are transformed into the corresponding epoxides using the methodology developed by Sharpless and reported in Tetrahedron (1992), 48, 10515.

The reaction between the epoxide **IX-2** and a suitable primary (R⁷ ≠ H), a secondary amine (R⁷ and R⁸ ≠ H) or an aniline may be carried out in the presence of a Lewis acid such as lithium perchlorate or a bismuth salt in an organic solvent such as DMF, EtOH or MeCN at a temperature ranging between 20°C and 100°C, more preferably at a temperature in the vicinity of 80°C, to give amino-alcohol **IX-3**. Removal of the protecting group (E) may be achieved using one of the aforementioned methods to give a compound of formula I or I_{P}.

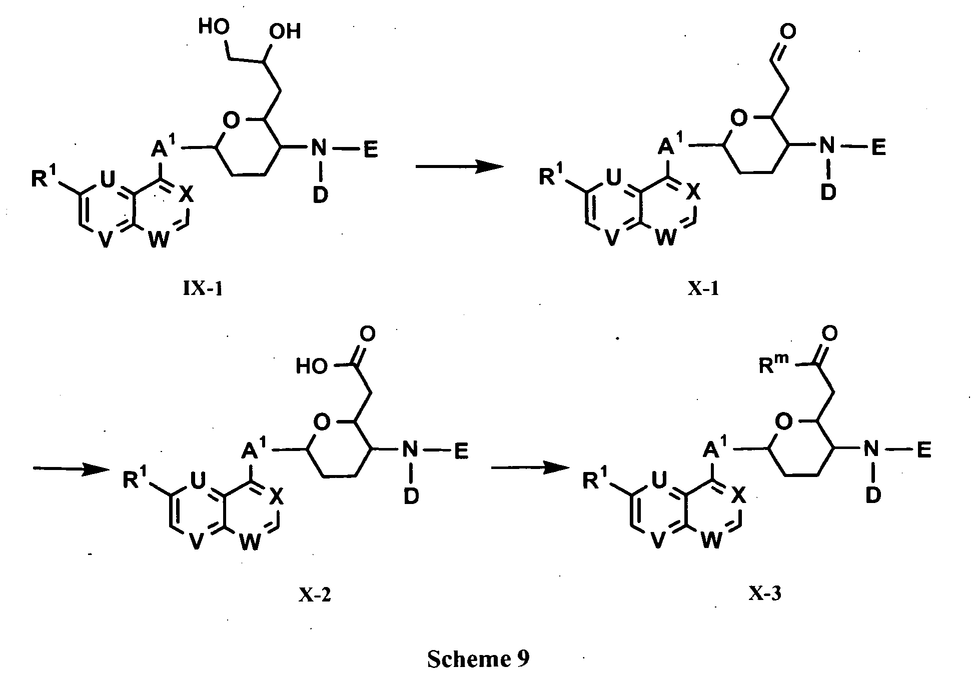

In Scheme 9, E is a protecting group, R^{m} is NR^{g}R^{h}, NHOR^{k} or NR^{x}R^{y} (R^{g}, R^{h}, R^{k} and NR^{x}R^{y} being as defined in Table 3) and U, V, W, X, R¹, A¹ and D are as defined in formula I.

As illustrated in Scheme 9, the 1,2-diol **IX-1** may be transformed into the aldehyde **X-1** by treatment with NaIO₄ in aq. acetone. Further oxidation of the aldehyde to the acid **X-2** is usually carried out using either KMnO₄ in aq. acetone or sodium chlorite in an aq. alcohol solvent such as 2-propanol or 2-methyl-2-propanol. The primary amides **X-3** (R^{m} = NH₂) can be obtained from an activated form of acid **X-2** using one of the aforementioned methods. The acids **X-2** may also be transformed into hydroxamic acids **X-3** (R^{m} = NHOH) or derivatives thereof (R^{m} = NHOR^{k}) by treatment with hydroxylamine or an O-substituted substituted hydroxylamine in presence of a coupling agent such as HATU, EDC or DCC. O-protected hydroxylamine reagents such as O-(tetrahydropyran-2-yl)hydroxylamine, O-benzyl hydroxylamine or O-trimethylsilylhydroxylamine can be used as hydroxylamine surrogates. In any cases, an appropriate deprotection step is required to free the hydroxamic acid. Substituted amides **X-3** (R^{m} ≠ NH₂, R^{m} ≠ NHOH) can also be obtained using the same strategy using a variety of primary or secondary amines or anilines and a coupling reagent. In the particular cases wherein R^{m} is NHR^{g} and R^{g} is [(C₂-C₅)hydroxyalkyl], the hydroxy group of the [(C₂-C₅)hydroxyalkyl] group is protected (for example by a silyl group such as TMS or TBDMS) before the coupling reaction and removed afterwards. Removal of the protecting group (E) can be achieved as usual.

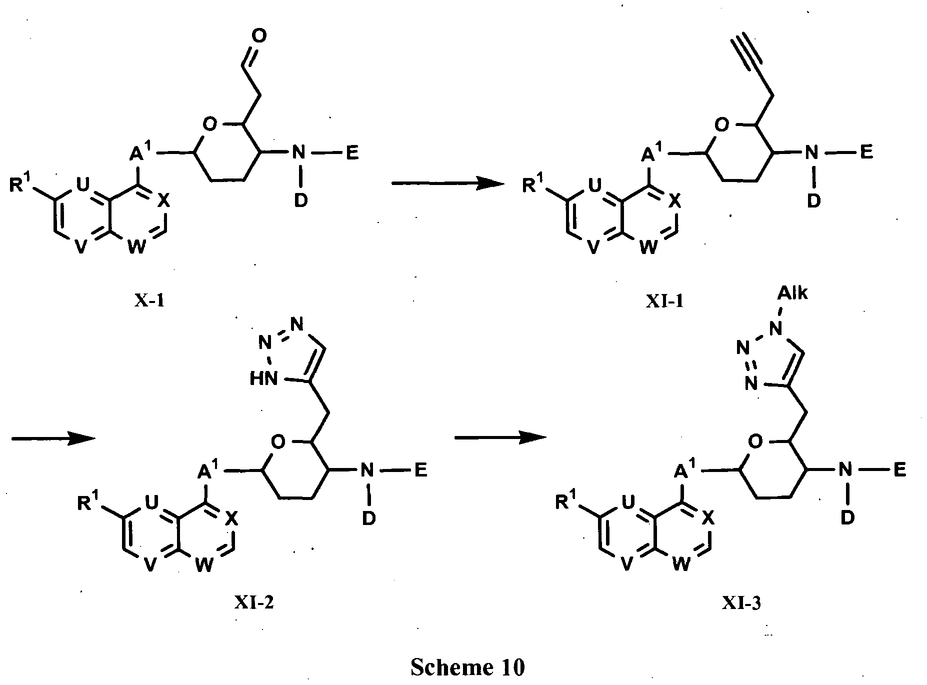

In Scheme 10, E is a protecting group, Alk is alkyl and U, V, W, X, R¹, A¹ and D are as defined in formula I.

As illustrated in Scheme 10, the aldehyde **X-1** may be transformed to the corresponding alkyne **XI-1** using one of the methods reported before. The alkyne leads to the corresponding 1,2,3-triazole **XI-2** via a dipolar cycloaddition using NaN₃ in an aprotic solvent such as acetone at a temperature ranging between 20°C and 60°C. The derivative **XI-2** may be further transformed to **XI-3** by alkylation of the triazole using a suitable alkylating agent Alk-LG (Alk = alkyl and LG = Br, I). In any cases, removal of the protecting group (E) may be achieved using one of the aforementioned methods.

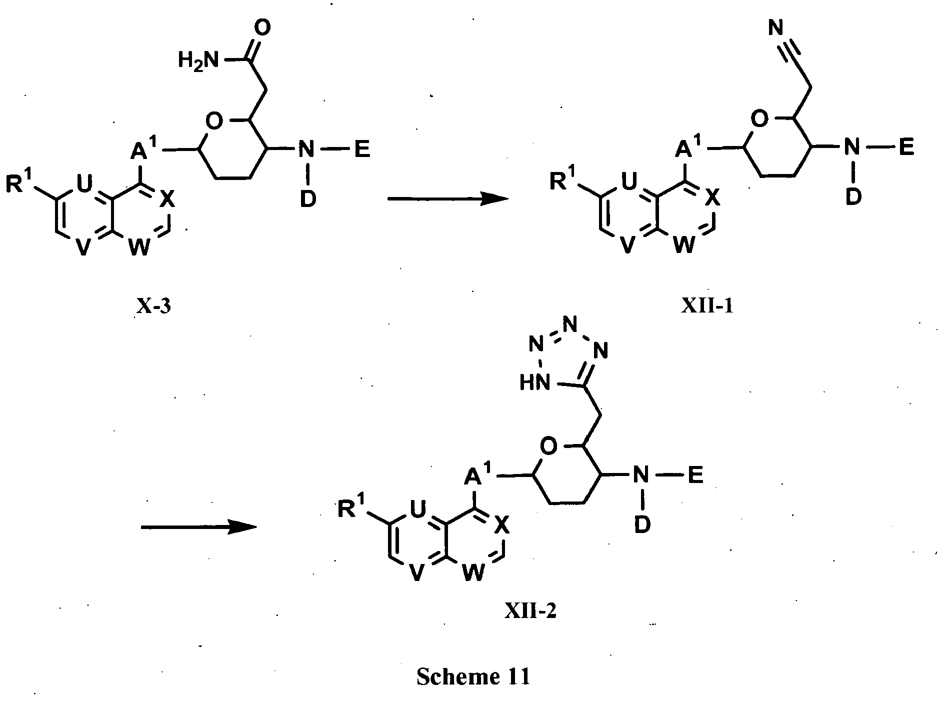

In Scheme 11, E is a protecting group and U, V, W, X, R¹, A¹ and D are as defined in formula I.

As illustrated in Scheme 11, the amide **X-3** (R^{m} = NH₂) can be dehydrated to the corresponding nitrile **XII-1.** A wide range of conditions is known such as treatment of the amide with pivaloyl chloride, or TsCl or acetic anhydride in presence of a base such as pyridine. The nitrile may be then transformed into the 1,2,3,4-tetrazol **XII-2** using NaN₃ in an aprotric solvent such as toluene or DMF at a temperature ranging between 20°C and reflux, in presence of TEA hydrochloride as described in Eur. J. Org. Chem. (2005), 326-333 or NH₄Cl. Removal of the protecting group (E) can be achieved using one of the aforementioned methods.

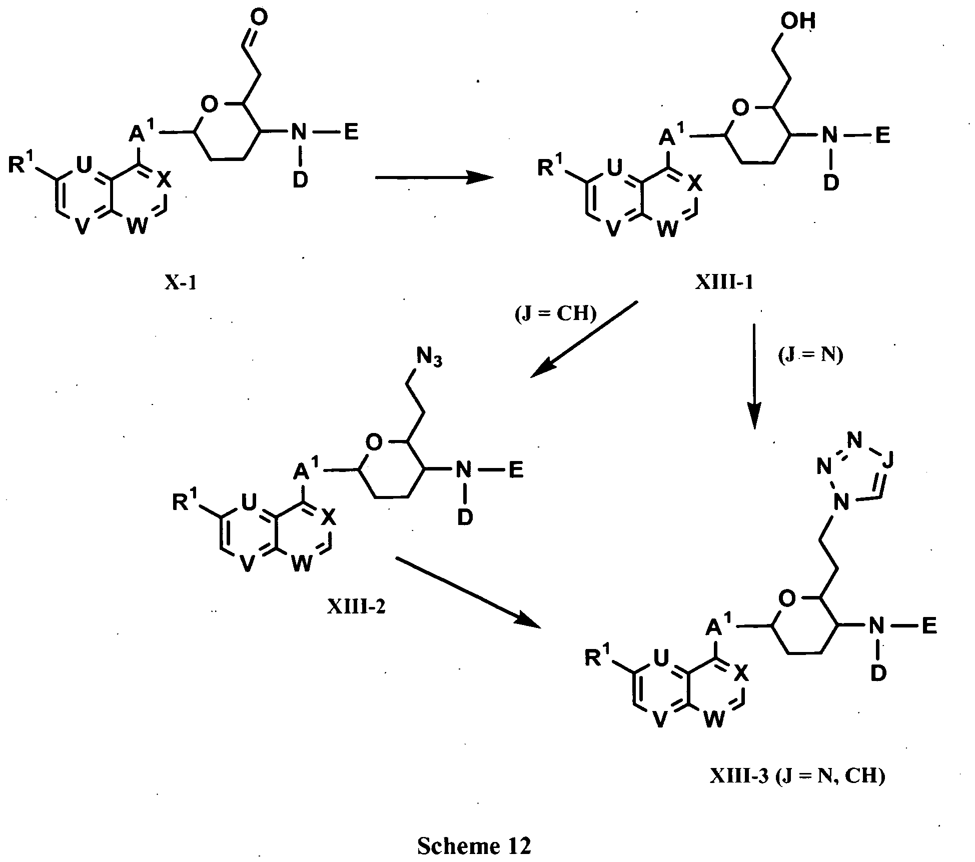

In Scheme 12, E is a protecting group, J is N or CH and U, V, W, X, R¹, A¹ and D are as defined in formula I.

As illustrated in Scheme 12, the aldehydes **X-1** can be reduced to the corresponding alcohols **XIII-1** using a variety of reducing agents as reviewed by Barda, D.A. in Encyclopedia of Reagents for Organic Synthesis, Ed.: Paquette, L.A., Wiley, New-York (1995), p. 616. The alcohol function of **XIII-1** is further transformed into a leaving group, such as methanesulfonyloxy or *para*-toluenesulfonyloxy, by treatment with MsCl or TsCl in the presence of an organic base such as TEA or pyridine at a temperature ranging between -78°C and 0°C, more preferably at a temperature ranging between -30°C and -10°C in an organic solvent such as THF, DMF or DCM. The resulting methane- or toluene-sulfonyloxy derivatives are transformed into the corresponding tetrazoles **XIII-3** (J = N) by a nucleophilic substitution with tetrazole in presence of a base. The resulting methane- or toluene-sulfonyloxy derivatives can also be transformed to azides **XIII-2** by treatment with NaN₃ in a polar solvent such as DMF or DMSO at a temperature ranging between 60 and 80°C. A dipolar cycloaddition between azide **XIII-2** and acetylene or a synthetic surrogate such a 2,5-norbornadiene, in a solvent such as dioxane or toluene in refluxing conditions gives rise to unsubstituted triazoles **XIII-3** (J = CH). Removal of the protecting group (E) can be achieved using one of the aforementioned methods.

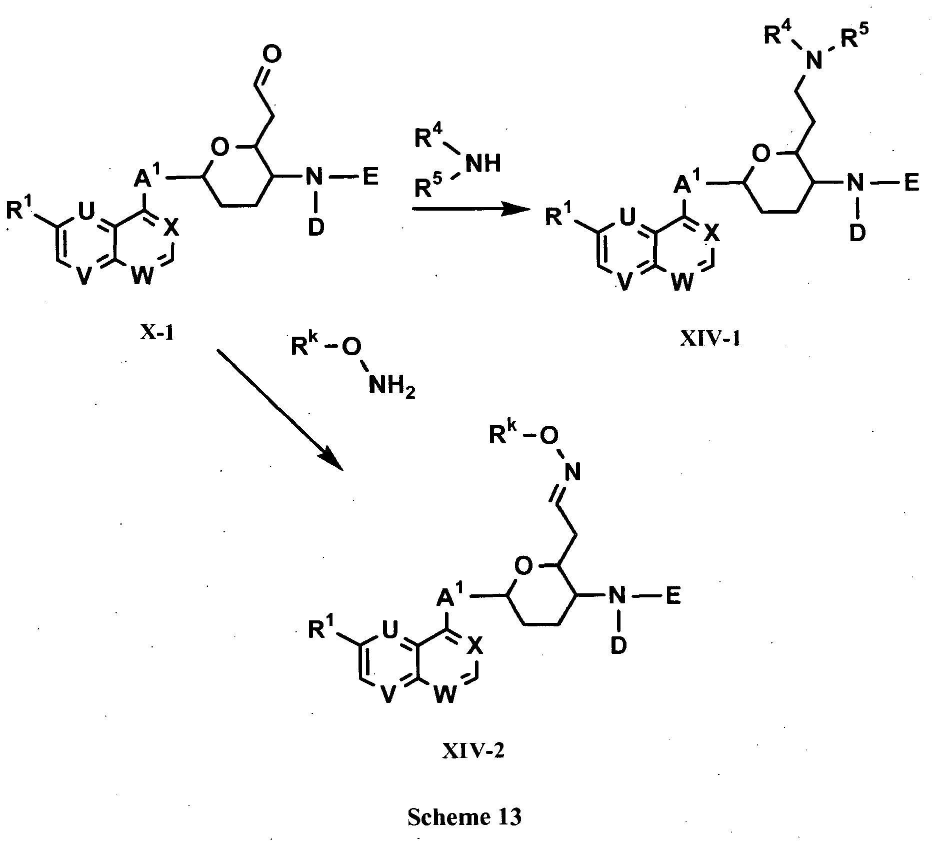

In Scheme 13, E is a protecting group, R^{k} is H or (C₂-C₄)alkynylmethyl, and U, V, W, X, R¹, A¹, D, R⁴ and R⁵ are as defined in formula I.

As illustrated in Scheme 13, the aldedydes **X-1** may also be converted into amines **XIV-1** using amines of formula NHR⁴R⁵ and employing one of the reductive amination methods described above. Alternatively, such amine derivatives **XIV-1** may also be obtained by nucleophilic displacement of the resulting methane- or toluene-sulfonyloxy derivatives of alcohol **XIII-1** with the same reagents. Oxime derivatives **XIV-2** are usually obtained by reacting the aldehydes **X-1** with hydroxylamine (R^{k} = H) or substituted hydroxylamines (R^{k} ≠ H). Removal of the protecting group (E) may be achieved using one of the aforementioned methods.

Aldehydes **VI-1** can be prepared following literature procedures or from the corresponding derivatives **II-1** (L¹ = Br) are after treatment with an alkyllithium such as n-BuLi at a temperature ranging between -80°C and -30°C and subsequent quenching of the lithio species with DMF as described in J. Org. Chem. (1980), 45, 1514. In the particular case wherein U = V = CH, W = N and X = CF, the aldehydes **VI-1** can be prepared by reacting the compounds of formula 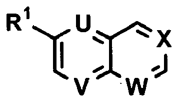 wherein U = V = CH, W = N and X = CF and R¹ is as defined in formula **VI-1** with an alkyllithium such as *n*-BuLi and then quenching the lithio species with DMF. An alternate route to generate aldehyde **VI-1** consists in reacting derivative **II-1** (L = OTf, Br or Cl) with *trans*-phenylvinyl boronic acid under typical Miyaura-Suzuki coupling conditions (see Synth. Commun. (1981), 11, 513) or with vinyl tributylstannane under typical Stille coupling conditions, employing a palladium salt, an inorganic base such as K₂CO₃ or Na₂CO₃, in an aq. solvent such as a dioxane-water mixture at a temperature ranging between 20° and 100°C. The corresponding alkene may be directly transformed into the aldehyde **VI-1** by ozonolysis (O₃ stream then quenching with either dimethylsulfide or PPh₃) or *via* a periodic cleavage of the intermediate diol using NaIO₄ in aq. acetone. The diol is obtained using a catalytic amount of osmium tetroxide in the presence a co-oxidant such as NMO in aq. solvent such as an acetone-water or DCM-water mixture (see Cha, J.K. Chem. Rev. (1995), 95, 1761-1795).

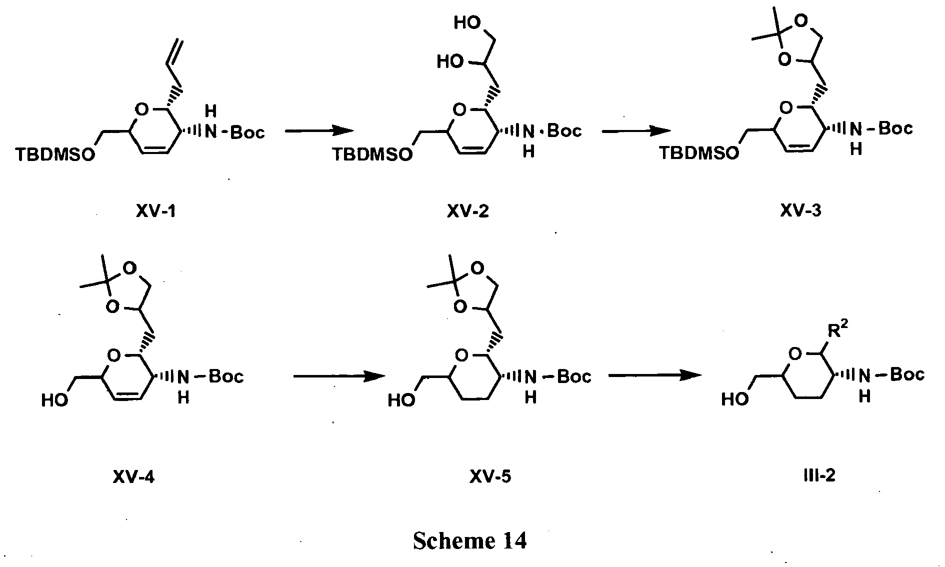

The required tetrahydropyran derivatives **III-2** used to access compounds **I-2, II-2, V-1, VI-2** were obtained from compound **XV-1** (Scheme 14) (see Eur. J. Org. Chem. (2003), 2418-2427). In a series of reactions, compound **XV-1** was dihydroxylated using aforementioned methods to give the diol **XV-2.** The resulting diol is then reacted with acetone, acetonedimethylacetal, or 2-methoxypropene in presence of a catalytic amount of acid such as PTSA in a solvent like DCM or THF to yield a compound of formula **XV-3.** The protecting silyl protecting group of compounds **XV-3,** is removed using methods listed in reference books such as P.J. Kocienski, 'Protecting Groups', Thieme (1994). Finally the double bond is hydrogenated over Pd/C as aforementioned. The compound **XV-5** can then be converted into the compound **III-2** using reactions similar to those described previously or known to one skilled in the art.

### EXAMPLES

All temperatures are stated in °C. All analytical and preparative HPLC investigations on non-chiral phases are performed using RP-C 18 based columns.

### Preparation A: 3-methoxy-quinoline-5-carbaldehyde:

### A.i. 3,5-dibromoquinoline:

To concentrated H₂SO₄ (130 ml) was added dropwise at 0°C, over 80 min, 3-bromoquinoline (50 g) at a rate allowing the internal temperature to be maintained between 0° and 10°C. After the addition was complete, NBS (48 g) was added portionwise and the reaction mixture was stirred at rt overnight. The reaction mixture was poured onto ice (2 1) and the resulting solid was dissolved in DCM (600 ml). The aq. layer was further extracted with DCM (600 ml) and the combined extracts were washed with 1*M* NaOH (300 ml) and concentrated *in vacuo.* The residue was adsorbed on SiO₂ and the resulting dispersal was loaded on the top of a column and eluted with a DCM-Hex (1-1, 31) then DCM (3 1) and finally DCM-ether (1-1, 21). The title compound was recovered from the last fraction after evaporation to yield 40 g of a white solid.
¹H NMR (CDCl₃) δ: 8.94 (d, J = 2.2 Hz, 1H); 8.73 (d, J = 2.2 Hz, 1H); 8.08 (d, J = 8.5 Hz, 1H); 7.88 (d, J = 7.5 Hz, 1H); 7.62 (dd, J = 7.5, 8.5 Hz, 1H).

### A.ii. 5-bromo-3-methoxyquinoline:

To a mixture of sodium methoxide (14.5 g) in DMPU (350 ml) heated at 125°C, was added in one portion intermediate A.i (34.5 g). The reaction was then heated at the same temperature for 1 h. The reaction mixture was then cooled to rt and poured onto ice (300 g). After the ice melt, the solid was filtered off and dried under vacuum. The filtrate was extracted with ether (4 x 150 ml). The combined extracts were washed with brine and dried over Na₂SO₄. After filtration, the solvent was evaporated and the residue purified over SiO₂ (Hex-EA 4-1) to afford a material that was pooled with the solid. The material was dissolved in DCM and dried over Na₂SO₄. After filtration and evaporation, the solid was further dried under HV to afford the title compound (24.5 g) as a beige solid.
¹H NMR (CDCl₃) δ: 8.68 (d, J =2.8 Hz, 1H); 8.03 (d, J = 8.3 Hz, 1H); 7.80 (d, J = 7.5 Hz, 1H); 7.72 (d, J = 2.8 Hz, 1H); 7.42 (dd, J = 7.5, 8.3 Hz, 1H); 4.02 (s, 3H).
MS (ESI, m/z): 239.7 [M+H⁺].

### A. iii. 3-methoxy-quinoline-S-carbaldehyde:

To a solution of intermediate A.ii (10 g) in THF (250 ml) cooled to -78°C, was added *n*-BuLi (22 ml). After 15 min, a solution of DMF (10 ml) in ether (20 ml) was quickly added. The solution was stirred 15 min and EtOH (5 ml), followed by 1*M* NaHSO₄ (40 ml), was added. After warming to rt, the organic layer was diluted with EA (100 ml). The two layers were separated and the aq. layer was extracted once with EA (100 ml). The combined organic layers were washed with brine and concentrated to dryness. The residue was chromatographed (EA-Hex 1-2 then 1-1) to afford the title compound (4.75 g) as a yellowish solid.
¹H NMR (CDCl₃) δ: 10.32 (s, 1H); 9.02 (d, J=2.9Hz, 1H); 8.75 (d, J = 2.9 Hz, 1H); 8.31 (d, J = 8.3 Hz, 1H); 8.02 (d, J = 7.1 Hz, 1H); 7.72 (dd, J = 7.1, 8.3 Hz, 1H); 4.02 (s, 3H).
MS (ESI, m/z): 187.9 [M+H⁺].

### Preparation B: 6-methoxy-[1,5]naphthyridine-4-carbaldehyde:

### B.i. 2-methoxy-8-styryl-[1,5]naphthyridine:

Trifluoromethanesulfonic acid 6-methoxy-[1,5]naphthyridin-4-yl ester (1.5 g, 4.86 mmol), *trans*-phenylvinyl boronic acid (0.8 g, 5.35 mmol) and K₂CO₃ (0.9 g, 6.32 mmol) were introduced in a two-neck flask. The atmosphere was flushed with nitrogen. Dioxane (20 ml) and water (5 ml) were added. The mixture was stirred at rt for 5 min and Pd(PPh₃)₄ (0.28 g, 0.24 mmol) was added. The mixture was heated at reflux for 5 h. After cooling, the reaction mixture was diluted with EA (10 ml) and water (50 ml). The aqueous layer was extracted with EA (2 x 100 ml). The combined extracts were concentrated to dryness. The residue was chromatographed (Hex-EA 1-1) to afford the title alkene (1.26 g, 4.8 mmol) as an oil that crystallized on standing.
¹H NMR (d6-DMSO) δ: 8.77 (d, J = 4.7 Hz, 1H); 8.28 (d, J = 9.0 Hz, 1H); 8.19 (d, J = 16.7 Hz, 1H); 8.01 (d, J = 4.7 Hz, 1H); 7.91 (d, J = 16.7 Hz, 1H); 7.74 (m, 2H); 7.40-7.34 (m, 3H); 7.30 (d, J = 9.0 Hz, 1H); 4.12 (s, 3H).

### B.ii. 1-(6-methoxy-[1,5]naphthyridin-4-yl)-2-phenyl-ethane-1,2-diol:

To a mixture of intermediate B.i (1.26 g, 4.8 mmol) in 2-methyl-2-propanol (24 ml) and water (24 ml) were added methanesulfonamide (0.52 g) and AD mix β^{®} (7 g). The mixture was stirred at rt for 12 h. Sodium bisulfite (7.5 g) was added carefully and stirring was continued 20 min. The two layers were decanted and the aqueous layer was extracted with EA (2 x 100 ml). The combined organic layers were dried over Na₂SO₄, filtered and concentrated to dryness. The residue was triturated in Hex-EA (1-3, 30 ml) and the resulting solid was filtered off and dried *in vacuo* to afford the title diol (1.3 g) as a white solid.
MS (ESI, m/z): 297.1 [M+H⁺].

### B.iii. 6-methoxy-[1,5]naphthyridine-4-carbaldehyde:

To a solution of intermediate B.ii (1.3 g, 4.4 mmol) in acetone (15 ml) was added a solution of NaIOₐ (2.35 g, 10.96 mmol) in water (5 ml). The reaction mixture was stirred at rt for 30 min. The reaction mixture was diluted with THF (100 ml) and the solids were filtered off. The filtrate was concentrated to dryness and the residue was resuspended in water (100 ml), ether (10 ml) and Hex (100 ml). The slurry was stirred at rt for 15 min and filtered. The solids were washed with water and Hex. After drying, the title aldehyde (0.42 g) was recovered as a white solid.
¹H NMR (d6-DMSO) δ: 11.25 (s, 1H); 9.02 (d, J = 4.4 Hz, 1H); 8.42 (d, J = 9.1 Hz, 1H); 7.92 (d, J = 4.4 Hz, 1H); 7.40 (d, J=9.1 Hz, 1H); 4.11 (s, 3H).

### Preparation C: (E)-3-(2,5-difluoro-phenyl)-propenal:

### C.i. (E)-3-(2,5-difluoro-phenyl)-acrylic acid ethyl ester:

To an iced chilled suspension of NaH (1.13 g, 60% in oil dispersion, 28.2 mmol) in THF (32 ml) was added triethylphosphonoacetate (5.6 ml, 28.2 mmol). The reaction mixture was stirred at rt for 20 min. 2,5-difluoro-benzaldehyde (3.34 g, 23.5 mmol) was added drop wise. After 30 min, 10% aq. NaHSO₄ (100 ml) was added and the mixture was diluted with EA (150 ml). The two phases were separated and the aq. layer was extracted twice with EA (2 x 100 ml). The combined org. layers were washed with brine (100 ml), dried over Na₂SO₄, filtered and concentrated to dryness. The residue was chromatographed over SiO₂ (Hex-EA 19-1) to afford the title unsaturated ester (5.0 g, 100%) as colourless oil.
¹H NMR (CDCl₃): 7.76 (dd, J= 1, 16.1 Hz, 1H); 7.26-7.21 (m, 1H); 7.13-7.03 (m, 2H); 16.1Hz, 1H); 4.29 (q, J = 7.1 Hz, 2H); 1.36 (t, J = 7.1 Hz, 3H).

### C.ii. (E)-3-(2,5-difluoro-phenyl)-prop-2-en-1-ol:

To a solution of intermediate C.i (5.0 g, 23.5 mmol) in ether (100 ml), cooled to 0°C, was added a solution of DIBAH (1M in Hex, 60 ml, 60 mmol). The mixture was stirred at the same temperature for 40 min. Water (6 ml) was added and the mixture was stirred 30 min. The solid was filtered off and thoroughly washed with ether. The filtrate was concentrated to dryness to afford the title alcohol (4.0 g, 98% yield) as colourless oil.
¹H NMR (CDCl₃): 7.15 (ddd, J = 3.1, 5.9, 9.0 Hz, 1H); 7.00 (td, J = 4.6, 9.0 Hz, 1H); 6.95-6.87 (m, 1H); 6.75 (dd, J = 1.3, 16.1Hz, 1H); 6.45 (td, J = 5.3, 16.1Hz, 1H); 4.38 (br d, J= 5.3 Hz, 2H); 1.63 (s, 1H).

### C.iii. (E)-3-(2,5-difluoro-phenyl)-propenal

To a solution of intermediate C.ii (1.70 g, 10 mmol) in DCM (20 ml) was added at rt, a solution of Dess-Martin periodinane (15wt% in DCM, 20 ml). The mixture was stirred at rt for 3 h. After concentration to dryness, the residue was chromatographed over SiO₂ (Hex-EA 9-1) to afford the title aldehyde (1.06 g, 63% yield) as a white solid.
¹H NMR (d6-DMSO): 9.74 (d, J = 7.6Hz, 1H); 7.88-7.81 (m, 1H); 7.79 (overlapped dd, J = 1.4, 16.0 Hz, 1H); 7.46-7.37 (m, 2H); 6.67 (dd, J = 7.6, 16.0 Hz, 1H).

### Preparation D: 3-fluoro-6-methoxy-[1,5]naphthyridine-4-carbaldehyde:

### D.i. trans-7-fluoro-2-methoxy-8-styryl-[1,5]naphthyridine:

8-bromo-7-fluoro-2-methoxy-[1,5]naphthyridine (prepared as in WO 2004/058144; 7 g, 27.2 mmol), *trans*-phenylvinyl boronic acid (4.23 g, 1.05 eq) and K₂CO₃ (4.9 g) were introduced in a two-necked flask. The atmosphere was flushed with nitrogen and dioxane (40 ml) and water (10 ml) were added. The mixture was stirred at rt for 5 min and Pd(PPh₃)₄ (1.56 g, 5 mol%) was added. The mixture was heated at reflux overnight. After cooling, the solvent was evaporated *in vacuo* and the residue was extracted with EA (2 x 150 ml). The combined extracts were washed with brine, dried over Na₂SO₄, filtered and concentrated to dryness,The residue was chromatographed (Hept-EA 2-1) to afford the title compound (7.2 g, 94% yield) as a white solid.
MS (ESI, m/z): 281.0 [M+H⁺].

### D. ii. 1-(3-fluoro-6-methoxy-[1,5]naphthyridin-4-yl)-2-phenyl-ethane-1,2-diol:

The title diol (7.6 g, 94% yield) was obtained as a white foam, starting from intermediate D.i (7.2 g, 8.9 mmol) and using the procedure of Example 8, step 8.i. The compound was purified by chromatography using EA as an eluent.
¹H NMR (CDCl₃) δ: 8.42 (d, J = 0.7 Hz, 1H); 8.28 (d, J = 9.1 Hz, 1H); 7.24-7.15 (m, 4H); 7.08 (m, 2H); 6.70 (br s, 1H); 5.28 (br s, 1H); 5.10 (d, J = 7.9 Hz, 1H); 4.11 (s, 3H); 3.85 (br s, 1H).

### D. iii. 3-fluoro-6-methoxy-[1,5]naphthyridine-4-carbaldehyde:

To a solution of intermediate D.ii (7.6g, 23.95mmol) in acetone (150 ml) was added a solution of NaIO₄ (12.8 g) in water (30 ml). The mixture was stirred at rt for 1 h. The solvent was removed in vacuo and the residue was diluted with water (500 ml). The resulting solid was filtered off, thoroughly washed with water, collected and dried under HV to afford the title aldehyde (4.0 g) as a light beige solid.
¹H NMR (d6-DMSO) δ: 11.08 (s, 1H); 9.01 (d, J = 1.3 Hz, 1H); 8.41 (d, J = 9.1 Hz, 1H); 7.37 (d, J = 9.1 Hz, 1H); 4.09 (s, 3H).
MS (ESI, m/z): 206.9 [M+H⁺].

### Example 1: (E)-3-(2,5-difluoro-phenyl)-N-{(2R,3R,6R)-2-((2RS)-2,3-dihydroxy-propyl)-6-[2-(3-methoxy-quinolin-5-yl)-ethyl]-tetrahydro-pyran-3-yl}-acrylamide:

### l.i. [(2R, 3R, 6S)-6-(tert-butyl-dimethyl-silanyloxymethyl)-2-((2RS)-1,3-dihydroxy-propyl)-3,6-dihydro-2H-pyran-3-yl]-carbamic acid tert-butyl ester:

To a mixture of (2*R*,3*R*,6*S*)-[2-allyl-6-(*tert*-butyl-dimethyl-silanyloxymethyl)-3,6-dihydro-2*H*-pyran-3-yl]-carbamic acid *tert*-butyl ester (obtained, as described in Eur. J. Org. Chem. (2003), 2418-2427; 60.7 g; 158.2 mmol) in 2-methyl-2-propanol (750 ml) and water (750 ml) were added potassium ferricyanide (182.4 g, 553.8 mmol), K₂CO₃ (65.8 g, 476.0 mmol), (DHQ)₂PHAL (1.12 g, 1.4 mmol) and potassium osmate dihydrate (0.118 g). The reaction mixture was mechanically stirred at rt for 24 h. Sodium bisulfite (316 g) was added slowly. The two layers were decanted and the aq. layer was extracted once more with EA (500 ml). The combined org. extracts were washed with brine, dried over MgSO₄, filtered and concentrated to dryness. The residue was filtered through a pad of silica (Hex-EA 1-4) to afford the title diol as a yellow oil (61.0 g, 146.0 mmol). The compound was obtained as a 2-1 mixture of epimers.
MS (ESI, m/z): 418.0 [M+H⁺].

### l.ii. [(2R,3R,6S)-2-((4RS)-2,2-dimethyl-[1,3]dioxolan-4-ylmethyl)-6-hydroxymethyl-tetrahydro-pyran-3-yl]-carbamic acid tert-butyl ester:

To a mixture of intermediate l.i (61 g, 146.0 mmol) in THF (710 ml) were added PTSA (1.38 g, 7.3 mmol) and 2,2-dimethoxypropane (54.0 ml, 439.1 mmol). The reaction mixture was stirred at rt for 90 min. TBAF (1*M* in THF, 230 ml) was added. The reaction proceeded for 90 min. The reaction mixture was concentrated to dryness and the residue was chromatographed over SiO₂ (Hex-EA 1-4) to afford the title compound as a thick oil (44.7 g, 130.1 mmol). The compound was obtained as a 2-1 mixture of epimers.
MS (ESI, m/z): 344.2 [M+H⁺].

### l.iii. [(2R, 3R, 6S)-1-((4RS)-1,1-dimethyl-[1,3]dioxolan-4-ylmethyl)-6-hydroxymethyl-tetrahydro-pyran-3-yl]-carbamic acid tert-butyl ester:

To a solution of intermediate l.ii (19.7 g, 57.3 mmol) in EA (230 ml) was added platinum oxide (0.7 g). The resulting suspension was stirred under hydrogen for 5 h. The catalyst was removed by filtration through celite and the filtrate evaporated under reduced pressure. The residue was purified by column chromatography (EA-Hex 4-1 to 1-0) to afford the title compound as a white solid (19.43 g). The compound was obtained as a 2-1 mixture of epimers.
MS (ESI, m/z): 346.1 [M+H⁺].

### l.iv. [(2R, 3R, 6S)-2-((4R)-2,2-dimethyl-[1,3]dioxolan-4-ylmethyl)-6-(1-phenyl-1H-tetrazol-5-ylsulfanylmethyl)-tetrahydro-pyran-3-yl]-carbamic acid tert-butyl ester:

To an ice-chilled solution of intermediate l.iii (19.4 g, 56.1 mmol) in THF (500 ml) were added PPh₃ (29 g), phenyltetrazole thiol (14 g) and, drop wise, DIAD (16.7 ml). The resulting solution was stirred at rt overnight. The reaction mixture was concentrated to dryness and the residue purified by column chromatography (EA-Hex 1-3 to 1-0) to afford the title sulfide as a colourless solid (28.85 g, 99% yield). The compound was obtained as a 2-1 mixture of epimers.
MS (ESI, m/z): 506.0 [M+H⁺].

### l.v. [(2R, 3R, 6S)-2-((4RS)-2,2-dimethyl-[1,3]dioxolan-4-ylmethyl)-6-(1-phenyl-1H-tetrazole-5-sulfonylmethyl)-tetrahydro-pyran-3-yl]-carbamic acid tert-butyl ester:

To a solution of intermediate l.iv (28.4 g) in EtOH (240 ml) and THF (240 ml) were added ammonium molybdate heptahydrate (17.73 g) and 50% aq. hydrogen peroxide (82.5 ml). The mixture was heated at 60°C overnight. After cooling to rt, the reaction mixture was diluted with water (250 ml) and the volatiles were removed under reduced pressure. EA (150 ml) was added to the aq. residue and the phases were separated. The aq. layer was extracted three times with EA (150 ml). The combined org. layers were dried over MgSO₄, filtered and evaporated under reduced pressure. The residue was purified by column chromatography (DCM-MeOH 19-1 to 9-1) to afford first the desired sulfone as a colourless thick oil (4.9 g), then [*(2R, 3R, 6S*)-2-((2*R*)-2,3-dihydroxy-propyl)-6-(1-phenyl-1H-tetrazole-5-sulfonylmethyl)-tetrahydro-pyran-3-yl]-carbamic acid *tert*-butyl ester as a white foam (9.4 g). The latter was taken up in THF (90 ml). PTSA (0.187 g) and 2,2-dimethoxypropane (6.95 ml) were added to the stirring solution. The reaction mixture was stirred at rt for 2 h 30. Water (8 ml) and aq. NaHCO₃ (10 ml) were added before concentration to dryness. The residue was partitioned between water (50 ml) and EA (100 ml) and the phases were separated. The org. layer was washed with brine, dried over MgSO₄, filtered and evaporated under reduced pressure. The residue was further dried under HV to afford more of the title sulfone (9.94 g).
MS (ESI, m/z): 538.0 [M+H⁺].

### l.vi. {(2R, 3R, 6S)-2-((4RS)-2,2-dimethyl-[1,3]dioxolan-4-ylmethyl)-6-[(E)-2-(3-methoxy-quinolin-5-yl)-vinyl]-tetrahydro-pyran-3-yl}-carbamic acid tert-butyl ester:

To a solution of intermediate l.v. (1.99 g, 3.70 mmol) and 3-methoxy-quinoline-5-carbaldehyde (see Preparation A; 0.67 g, 3.58 mmol) in 1,2-DME (25 ml) cooled to -60°C was added drop wise over 15 min. a solution of KHMDS (11.1 ml, 0.5*M* in toluene). The reaction mixture was stirred at -60°C for 30 min., then allowed to warm up to 0°C over 1 h. Water (30 ml) and EA (40 ml) were added. The phases were separated. The aq. layer was extracted three times with EA. The combined org. layers were washed with brine, dried over MgSO₄, filtered and evaporated under reduced pressure. The residue was purified by column chromatography (EA-Hex 1-1) to afford the title compound (1.07 g, 2.14 mmol) as a white foam. The compound was obtained as a 2-1 mixture of epimers. MS (ESI, m/z): 499.2 [M+H⁺]

### 1.vii. {(2R,3R,6R)-2-((4RS)-2,2-dimethyl-[1,3]dioxolan-4-ylmethyl)-6-[2-(3-methoxy-quinolin-5-yl)-ethyl]-tetrahydro-pyran-3-yl}-carbamic acid tert-butvl ester:

To a solution of intermediate 1.vi (1.07 g, 2.14 mmol) in EA (35 ml) was added 10% Pd/C (0.23 g). The resulting suspension was stirred under hydrogen for 1 h. The catalyst was removed by filtration. The filtrate was evaporated under reduced pressure and further dried under HV to afford the title compound as a white foam (1.03 g, 2.05 mmol).
MS (ESI, m/z): 501.1 [M+H⁺].

### 1.viii. 3-(2RS)-{(2R,3R,6R)-3-Amino-6-[2-(3-methoxy-quinolin-5yl)-ethyl]-tetrahydro-pyran-2yl}-propane-1,2-diol:

A solution of intermediate 1.vii (1.03 g) in TFA (21 ml) was stirred for 10 min. Water (7 ml) was added. The resulting mixture was stirred for 1 h and concentrated to dryness. The residue was basified with 1M aq. NaOH. DCM-MeOH 9-1 was added and the phases were separated. The aq. layer was extracted six times with DCM-MeOH 9-1 and the combined extracts were dried over MgSO₄, filtered and evaporated under reduced pressure. The title compound (white foam; 0.69 g; 1.91 mmol) was obtained as a 2-1 mixture of epimers.
¹H NMR (CDCl₃) main signals δ: 8.65 (d, J = 2.8 Hz, 1H); 7.89 (d, J = 8.4 Hz, 1H); 7.56 (m, 1H); 7.45 (m, 1H); 7.31 (m, 1H); 4.03 (m, 1H); 3.94 (s, 3H); 3.79 (m, 1H); 3.63 (m, 2H); 3.47 (m, 1H); 3.14 (m, 2H); 2.95 (m, 1H); 2.25-2.05 (m, 5H); 2.03-1.87 (m, 3H); 1.85-1.67 (m, 3H); 1.59 (m, 1H); 1.23 (m, 1H).
MS (ESI, m/z): 361.1 [M+H⁺].

### 1.ix. (E)-3-(2,5-difluoro-phenyl)-N-{(2R,3R,6R)-2-((2RS)-2,3-dihydroxy-propyl)-6-[2-(3-methoxy-quinolin-5-yl)-ethyl]-tetrahydro-pyran-3-yl}-acrylamide:

To a solution of intermediate 1.viii (0.360 g, 1 mmol) and 3-(2,5-difluoro-phenyl)-acrylic acid (0.203 g, 1.1 mmol) in DMF (15 ml) were added DIPEA (0.514 ml, 3 mmol) and HATU (0.456 g, 1.2 mmol). The reaction mixture was stirred at rt for 90 min. and concentrated to dryness. The residue was dissolved in DCM-MeOH 9-1. The solution was washed with saturated NaHCO₃. The aq. layer was extracted twice with DCM-MeOH 9-1 and the combined org. layers were dried over MgSO₄, filtered and evaporated under reduced pressure. The residue was purified by column chromatography (DCM-MeOH 19-1 1% aq. NH₄OH to 9-11% aq., NH₄OH) to afford a brown-orange solid (0.331 g, 62% yield. The compound was obtained as a 2-1 mixture of epimers.
¹H NMR (d6-DMSO) main signals δ: 8.69 (d, J = 2.7 Hz, 1H); 8.30 (d, J = 8.6 Hz, 1H); 7.87-7.79 (m, 2H); 7.56-7.50 (m, 4H); 7.40-7.27 (m, 2H); 7.02 (dd, J = 5.1, 15.9 Hz, 1H); 4.58 (m, 2H); 4.23 (m, 1H); 4.06 (overlapped m, 2H); 4.02 (overlapped s, 3H); 3.86-3.76 (m, 2H); 3.45-3.24 (overlapped m, 2H); 3.09-2.99 (m, 1H); 2.16 (m, 1H); 2.00 (m, 3H); 1.67 (m, 2H); 1.37 (m, 1H); 1.21 (m, 1H).
MS (ESI, m/z): 527.0 [M+H⁺]

### Example 2 : 3-oxo-3,4-dihydro-2H-pyrido[3,2-b][1,4]thiazine-6-carboxylic acid {(2R,3R,6R)-2-((2RS)-2,3-dihydroxy-propyl)-6-[2-(3-methoxy-quinolin-5-yl)-ethyl]-tetrahydro-pyran-3-yl}-amide:

Starting from intermediate 1.viii (0.270 g, 0.75 mmol) and 3-oxo-3,4-dihydro-2*H*-pyrido[3,2-*b*][1,4]thiazine-6-carboxylic acid (0.174 g, 1.1 eq), the title compound (0.187 g, 0.32 mmol) was obtained as a pale brown-orange foam according to the procedure described in Example 1, step 1.ix. The compound was obtained as a 2-1 mixture of epimers.
¹H NMR (DMSO) main signals δ: 11.29 (s, 1H); 8.69 (dd, J = 1.7, 2.7 Hz, 1H); 8.36 (d, J = 9.3 Hz, 1H); 8.01 (d, J = 7.9 Hz, 1H); 7.88-7.79 (m, 2H); 7.66 (dd, J = 1.3, 7.9 Hz, 1H); 7.51 (m, 1H); 4.64 (d, J = 5.2 Hz, 2H); 4.58 (m, 1H); 4.25 (m, 1H); 4.08 (overlapped m, 1H); 4.03 (overlapped s, 3H); 3.95 (m, 1H); 3.77 (m, 1H); 3.69 (d, J = 5.1 Hz, 2H); 3.39-3.25 (overlapped m, 4H); 3.07 (m, 1H); 2.28 (m, 1H); 1.98 (m, 2H); 1.83-1.58 (m, 3H); 1.27 (m, 1H); 1.20 (m, 1H).
MS (ESI, m/z): 553.0 [M+H⁺].

### Example 3: 6-({(2R,3R,6R)-2-((2RS)-2,3-dihydroxy-propyl)-6-[2-(3-methoxy-quinolin-5-yl)-ethyl]-tetrahydro-pyran-3-ylamino}-methyl)-4H-pyrido[3,2-b] [1,4]thiazin-3-one:

To a solution of intermediate 1.viii (0.18 g, 0.5 mmol) in MeOH (2 ml) and 1,2-DCE (7 ml) were added 3Å molecular sieves (2.9 g) and 3-oxo-3,4-dihydro-2*H*-pyrido[3,2-*b*][1,4]thiazine-6-carbaldehyde (0.107 g, 1.1 eq.). The mixture was stirred at 50°C overnight. NaBH₄ (0.18 g, 4.76 mmol) was added and the reaction mixture was stirred for 2 h. The reaction mixture was filtered over hydromatrix^{®} (treated with sat. NaHCO₃) and the filtrate was evaporated under reduced pressure. The residue was purified by column chromatography (DCM-MeOH 19-1 1% aq. NH₄OH then 9-1 1% aq. NH₄OH) to afford the title compound (0.176 g, 65% yield) as a pale yellow solid. The compound was obtained as a 2-1 mixture of epimers.
¹H NMR (DMSO) main signals δ: 10.92 (s, 1H); 8.68 (d, J = 2.8 Hz, 1H); 7.90 (d, J = 8.2Hz, 1H); 7.78 (m, 2H); 7.53-7.43 (m, 2H); 7.13 (d, J = 7.9 Hz, 1H); 4.69 (br.s, 1H); 4.53 (m, 2H); 4.23 (m, 1H); 3.99 (s, 3H); 3.75 (overlapped s, 2H); 3.66 (overlapped m, 2H); 3.56 (s, 2H); 3.45-3.29 (overlapped m, 2H); 3.02 (m, 1H); 2.75 (m, 1H); 2.05-1.97 (m, 2H); 1.79-1.74 (m, 5H); 1.57-1.50 (m, 1H); 1.40-1.32 (m, 1H).
MS (ESI, m/z): 539.1 [M+H⁺].

### Example 4: 6-({(2R,3R,6R)-2-((2RS)-2,3-dihydroxy-propyl)-6-[2-(6-methoxy-[1,5]naphthyridin-4-yl)-ethyl]-tetrahydro-pyran-3-ylamino}-methyl)-4H-pyrido[3,2-b][1,4]thiazin-3-one:

### 4.i. {(2R,3R,6S)-2-((4RS)-2,2-dimethyl-[1,3]dioxolan-4-ylmethyl)-6-trans-[2-(6-methoxy-[1,5]naphthyridin-4-yl)-vinyl]-tetrahydro-pyran-3-yl}-carbamic acid tert-butyl ester:

Starting from 6-methoxy-[1,5]naphthyridine-4-carbaldehyde (see Preparation B; 1.7 g, 9.03 mmol) and intermediate 1.v (4 g, 7.45 mmol), the title alkene (2.8 g, 62% yield) was obtained as a colourless foam using the procedure described in Example 1, step 1.v. The compound was purified by chromatography using Hex-EA 1-1 then 1-4 as eluents.
MS (ESI, m/z): 500.4 [M+H⁺].

### 4.ii. (2RS)-3-{(2R,3R,6R)-3-amino-6-[2-(6-methoxy-[1,5]naphthyridin-4-yl)-ethyl]-tetrahydro-pyran-2-yl}-ppropane-1,2-diol:

This compound (1.30 g, 94% yield) was obtained as a white solid starting from intermediate 4.i (1.98 g, 3.98 mmol) using the procedures described in Example 1, steps 1.vii and 1.viii.
MS (ESI, m/z): 362.1 [M+H⁺].

### 4.iii. 6-({(2R,3R,6R)-2-((2RS)-2,3-diydroxy-propyl)-6-[2-(6-methoxy-[1,5]naphthyridin-4-yl)-ethyl]-tetrahydro-pyran-3-ylamino}-methyl)-4H-pyrido[3,2-b][1,4]thiazin-3-one:

Starting from intermediate 4.ii (0.644 g, 1.78 mmol) and 3-oxo-3,4-dihydro-2H-pyrido[3,2-b][1,4]thiazine-6-carbaldehyde (0.380g, 1.1 eq.), the title compound (0.583 g, 60% yield) was obtained as a white foam using the procedure of Example 3. ¹H NMR (DMSO) δ: 10.93 (s, 1H); 8.69 (dd, J = 1.1, 4.4 Hz, 1H); 8.27 (d, J = 9.0 Hz, 1H); 7.78 (dd, J = 1.3, 7.9 Hz, 1H); 7.56 (t, J = 4.4 Hz, 1H); 7.28 (d, J = 9.0 Hz, 1H); 7.12 (d, J = 7.9 Hz, 1H); 4.65-4.46 (m, 2H); 4.20-4.05 (overlapped m, 1H), 4.05 (overlapped s, 3H); 3.70 (overlapped s, 2H); 3.70-3.56 (overlapped m, 2H); 3.56 (overlapped s, 2H); 3.41-3.19 (overlapped m, 3H); 3.17-3.07 (m, 1H); 2.74 (m, 1H); 2.09 (m, 1H); 1.92-1.70 (m, 5H); 1.51 (m, 1H); 1.37 (m, 1H); 1.25 (m, 1H).
MS (ESI, m/z): 540.0 [M+H⁺].

### Example 5: (2RS)-3-{(2R,3R,6R)-3-[(E)-3-(2,5-difluoro-phenyl)-allylamino]-6-[2-(6-methoxy-[1,5]naphthyridin-4-yl)-ethyl]-tetrahydro-pyran-2-yl}-propane-1,2-diol:

Starting from intermediate 4.ii (0.470 g, 1.3 mmol) and (*E*)-3-(2,5-difluoro-phenyl)-propenal (see Preparation C; 0.240 g; 1.1 eq.), the title compound (0.450 g, 67% yield) was obtained as a white foam using the procedure of Example 3.
¹H NMR (DMSO) δ: 8.69 (dd, J = 1.1, 4.4 Hz, 1H); 8.27 (d, J = 9.0 Hz, 1H); 7.58-7.48 (m, . 2H); 7.31-7.22 (m, 2H); 7.18-7.10 (m, 1H); 6.62 (t, J = 16.1 Hz, 1H); 6.52 (m, 1H); 4.73 (br. s, 1H); 4.55-4.44 (m, 2H); 4.17 (m, 1H); 4.05 (s, 3H); 3.69-3.58 (m, 2H); 3.38 (overlapped m, 2H); 3.28 (overlapped m, 2H); 3.12 (m, 1H); 2.77 (m, 1H); 1.99-1.72 (m, 6H); 1.54-1.23 (m, 3H).
MS (ESI, m/z): 514.0 [M+H⁺].

### Example 6: 2-{(2R,3R,6R)-3-[(E)-3-(2,5-difluoro-phenyl)allylamino]-6-[2-(6-methoxy-[1,5] naphthyridin-4-yl)-ethyl)-tetrahydro-pyran-2-yl}-N-hydroxy-acetamide dihydrochloride:

### 6.i. [(E)-3-(2,5-difluoro-phenyl)-allyl]-{(2R,3R,6R)-2-((2RS)-2,3-dihydroxy-propyl)-6-[2--(6-methoxy-[1,5]naphthyridin-4-yl)-ethyl]-tetrahydro-pyran-3-yl}-carbamic acid tert-butyl ester:

To a solution of the compound of Example 5 (0.73 g, 1.42 mmol) in dioxane (6.5 ml) and water (0.9 ml) was added 1*M* NaOH (1.5 ml). The solution was cooled to 0°C and Boc₂O (0.465 g, 2.13 mmol) was added. After stirring overnight, 1*M* NaOH (0.5 ml) and Boc₂O (0.465 g, 2.13 mmol) were added. The reaction mixture was stirred for 8 h. EA was added and the phases were separated. The aq. layer was extracted twice with EA and the combined org. layers were washed with brine, dried over MgSO_{4,} filtered and evaporated under reduced pressure. The residue was purified by column chromatography (DCM-MeOH 19-1 to 9-1) to afford the title compound (0.65 g, 74% yield) as a white foam. The compound was obtained as a 2-1 mixture of epimers.
¹H NMR (CDCl₃) main signals δ: 8.66 (d, J = 3.7 Hz, 1H); 8.30 (dd, J = 6.3, 9.0 Hz, 1H); 7.42 (m, 1H); 7.14 (overlapped d, J = 9.1 Hz, 1H); 7.09 (overlapped m, 1H); 7.01-6.93 (m, 1H); 6.90-6.84 (m, 1H); 6.52 (d, J = 16.0 Hz, 1H); 6.24 (m, 1H); 4.35-4.20 (m, 3H); 4.06 (s, 3H); 3.96-3.89 (m, 1H); 3.83 (dd, J = 5.2, 16.9 Hz, 1H); 3.66 (m, 2H); 3.50 (m, 1H); 3.26-3.14 (m, 3H); 2.27-2.20 (br. m, 2H); 2.10-1.84 (m, 6H); 1.47 (overlapped s, 9H); 1.46 (overlapped m, 1H).
MS (ESI, m/z): 614.1 [M+H⁺].

### 6.ii. [(E)-3-(2,5-difluoro-phenyl)-allyl]-[(2R,3R,6R)-6-[2-(6-methoxy-[1,5]naphthyridin-4-yl)-ethyl]-2-(2-oxo-ethyl)-tetrahydro-pyran-3-yl]-carbamic acid tert-butyl ester:

To a solution of intermediate 6.i (0.645 g, 1.05 mmol) in acetone (5.7 ml) was added at rt a solution of NalO₄ (0.562 g, 2.63 mmol) in water (1.6 ml). The reaction mixture was stirred for 1 h and concentrated to dryness. The residue was purified by column chromatography (DCM-MeOH 97-3 to 19-1) to afford the desired aldehyde (0.557 g, 91% yield) as a white foam.
¹H NMR (CDCl₃) δ: 9.75 (m, 1H); 8.65 (d, J = 4.6 Hz, 1H); 8.29 (d, J = 9.1 Hz, 1H); 7.43 (d, J = 4.6 Hz, 1H); 7.14 (overlapped d, J = 9.1 Hz, 1H); 7.08 (overlapped m, 1H); 7.02-6.95 (m, 1H); 6.92-6.85 (m, 1H); 6.52 (d, J = 16.1 Hz, 1H); 6.22 (td, J = 5.2, 15.8 Hz, 1H); 4.79 (m, 1H); 4.24 (m, 1H); 4.08 (s, 3H); 3.76 (dd, J = 4.5, 16.8 Hz, 1H); 3.66-3.58 (m, 1H); 3.31-3.21 (m, 1H); 3.14-3.04 (m, 1H); 2.89-2.80 (m, 1H); 2.54 (dd, J = 4.7, 15.3 Hz, 1H); 1.93-1.89 (m, 4H); 1.86-1.79 (m, 1H); 1.48 (overlapped s, 9H); 1.47 (overlapped m, 2H).
MS (ESI, m/z): 582.0 [M+H⁺].

### 6.iii. {(2R,3R,6R)-3-{tert-butoxycarbonyl-[(E)-3-(2,5-difluoro-phenyl)-allyl]-amino}-6-[2-(6-methoxy-[1,5]naphthyridin-4-yl)-ethyl]-tetrahydro-pyran-2-yl}-acetic acids:

To a solution of intermediate 6.ii (0.557 g, 0.96 mmol) in 2-methyl-2-propanol (20 ml) and 2-methyl-2-butene (5 ml) was added drop wise a solution of sodium chlorite (0.805 g, 8.90 mmol) and sodium dihydrogen phosphate (0.807 g, 5.17 mmol) in water (8 ml). The resulting solution was stirred at rt overnight and the volatiles were removed under vacuum. The residue was diluted with water and pH was adjusted to 3. The aq. mixture was extracted three times with DCM-MeOH (9-1, 3 x 50 ml). The combined extracts were dried over MgSO₄, filtered and evaporated under reduced pressure. The residue was further dried under HV to give the title acid (0.58 g, 99% yield) as a colourless foam. The compound was purified by trituration in ether.
¹H NMR (CDCl₃) δ: 8.52 (d, J = 4.6 Hz, 1H); 8.36 (d, J = 9.1 Hz, 1H); 7.27 (overlapped m, 1H); 7.12-6.86 (m, 4H); 6.53 (d, J = 15.8 Hz, 1H); 6.27 (m, 1H); 4.60 (m; 1H); 4.30-4.07 (m, 2H); 3.99 (s, 3H); 3.85-3.70 (m, 2H); 3.02 (m, 2H); 2.79 (m, 1H); 2.49 (dd, J = 3.7, 14.2 Hz, 1H); 1.94-1.90 (m, 3H); 1.84-1.77 (m, 3H); 1.52-1.40 (overlapped m, 3H); 1.27 (s, 9H).
MS (ESI, m/z): 598.1 [M+H⁺].

### 6.iv. [(E)-3-(2,5-difluoro-phenyl)-allyl]-{(2R,3R,6R)-2-hydroxycarbamoylmethyl-6-[2-(6-methoxy-[1,5]naphthyridin-4-yl)-ethyl]-tetrahydro-pyran-3-yl}-carbamic acid tert-butyl ester and {(2R,3R,6R)-2-carbamoylmethyl-6-[2-(6-methoxy-[1,5]naphthyridin-4-yl)-ethyl]-tetrahydro-pyran-3-yl}-[(E)-3-(2,5-difluoro-phenyl)-allyl]-carbamic acid tert-butyl ester:

To a solution of intermediate 6.iii (0.11 g, 0.19 mmol) in DMF (2.3 ml) were added DIPEA (0.16 ml, 0.93 mmol) and HATU (0.11 g, 0.28 mmol). The resulting solution was stirred at rt for 30 min. Hydroxylamine hydrochloride (0.019 g, 0.28 mmol) was added. After stirring at rt overnight, hydroxylamine hydrochloride (0.019 g, 0.28 mmol) was added and the reaction stirred at rt four 24 h. The reaction mixture was concentrated to dryness. The residue was partitioned between water and DCM-MeOH 9-1 and the phases were separated. The aq. layer was extracted five times with DCM-MeOH. The combined org. layers were dried over Na₂SO₄, filtered and the filtrate was evaporated under reduced pressure. The residue was purified by column chromatography (DCM-MeOH 9-1 containing 1% aq. NH₄OH to afford the title amide as a yellow oil (0.032 g, 29% yield) and then the title hydroxamic acid (0.045 g, 40% yield).
Amide : MS (ESI, m/z): 597.0 [M+H⁺].
Hydroxamic acid: MS (ESI, m/z): 613.0 [M+H⁺].

### 6.v. 2-{(2R,3R,6R)-3-[(E)-3-(2,5-difluoro-phenyl)-allylamino]-6-[2-(6-methoxy-[1,5]naphthyridin-4-yl)-ethyl]-tetrahydro-pyran-2yl}-N-hydroxy-acetamide dihydrochloride:

To a suspension of [(*E*)-3-(2,5-difluoro-phenyl)-allyl]-{(2*R*,3*R*,6*R*)-2-hydroxycarbamoylmethyl-6-[2-(6-methoxy-[1,5]naphthyridin-4-yl)-ethyl]-tetrahydro-pyran-3-yl}-carbamic acid *tert*-butyl ester (0.045 g, 0.072 mmol) in dioxane (2 ml) was added anhydrous HCl in dioxane (5N, 1 ml). The resulting solution was stirred at rt for 3 h. The mixture was concentrated to dryness. The residual solid was diluted with ether and evaporated under reduced pressure. The solid thus obtained was triturated in ether and filtered. After drying under HV, the trihydrochloride salt (0.033 g, 77% yield) was collected as a yellow solid.
¹H NMR (D₂O) δ: 8.78 (br. s, 1H); 8.36 (d, J = 8.6 Hz, 1H); 8.00 (d, J = 5.1 Hz, 1H); 7.50 (d, J= 9.3 Hz, 1H); 7.32 (m, 1H); 7.18-7.08 (overlapped m, 3H); 7.01 (overlapped t, J = 16.1 Hz, 1H); 6.37 (m, 1H); 4.64 (m, 1H); 4.14 (s, 3H); 4.10-3.86 (m, 3H); 3.62 (m, 1H); 3.40 (t, J = 7.1 Hz, 2H); 2.78 (m, 1H); 2.42 (m, 1H); 2.20 (m, 1H); 2.13-1.89 (m, 5H); 1.53 (m, 1H).
MS (ESI, m/z): 513.0 [M+H⁺].

### Example 7: (E)-3-(2,5-difluoro-phenyl)-N-{(2R,3R,6R)-2-((2RS)-2,3-dihydroxy-propyl)-6-[2-(6-methoxy-[1,5]naphthyridin-4-yl)-ethyl]-tetrahydro-pyran-3-yl}-acrylamide:

Starting from intermediate 4.ii (0.506 g, 1.4 mmol) and (*E*)-3-(2,5-difluoro-phenyl)-acrylic acid (0.284 g, 1.1 eq.), the title compound (0.640 g, 86% yield) was obtained as a white solid using the procedure of Example 1, step 1.ix.
¹H NMR (DMSO) δ: 8.71 (dd, J = 1.1, 4.4 Hz, 1H); 8.29 (d, J = 5.6Hz, 2H); 7.61 (d, J = 4.5 Hz, 1H); 7.51 (m, 1H); 7.43-7.28 (m, 3H); 7.02 (t, J= 11.3Hz, 1H); 4.56 (t, J = 5.7 Hz, 1H); 4.53 (m, 1H); 4.17 (m, 1H); 4.08 (overlapped s, 3H); 4.05 (overlapped m, 1H); 3.87-3.61 (m, 2H); 3.29 (m, 3H); 3.16 (m, 1H); 2.16 (m, 1H); 1.89 (m, 1H); 1.58 (m, 1H); 1.39 (m, 1H); 1.30-1.14 (m, 3H).
Rf = 0.31 (DCM-MeOH 9-1 containing 1% aq. NH₄OH).
MS (ESI, m/z): 528.0 [M+H⁺].

### Example 8: (2RS)-3-{(2R,3R,6S)-3-[(E)-3-(2,5-difluoro-phenyl)-allylamino]-6-[(S)-1-hydroxy-2-(6-methoxy-[1,5]naphthyridin-4-yl)-ethyl]-tetrahydro-pyran-2-yl}-propane-1,2-diol:

### 8.i. [(2R,3R,6S)-6-[(1R,2R)-1,2-dihydroxy-2-(6-methoxy-[1,5]naphthyridin-4-yl)-ethyl]-2-((4RS)-2,2-dimethyl-[1,3]dioxolan-4-ylmethyl)-tetrahydro-pyran-3-yl]-carbamic acid tert-butyl ester:

To a suspension of intermediate 4.i (1.274 g, 2.55 mmol) and methane sulfonamide (0.291 g, 3.06 mmol) in 2-methyl-2-propanol (13 ml), water (13 ml) and EA (4 ml) was added AD-mix β (4.5 g). The reaction mixture was stirred at rt for 24 h. Sodium metabisulfite (10 g) and EA (50 ml) were added. The two layers were decanted and the aq. layer was extracted twice with EA. The combined org. layers were dried over Na₂SO₄ filtered and concentrated *in vacuo.* The residue was chromatographed (DCM-MeOH 9-1) to afford the desired diol (1.2 g) as a white foam. The compound was obtained as a 2-1 mixture of epimers.
MS (ESI, m/z): 534.0 [M+H⁺].

### 8.ii. {(2R,3R,6S)-2-((4RS)-2,2-dimethyl-[1,3]dioxolan-4-ylmethyl)-6-[(4R,5R)-5-(6-methoxy-[1,5]naphthyridin-4-yl)-2-oxo-[1,3]dioxolan-4-yl]-tetrahydro-pyran-3-yl}-carbamic acid tert-butyl ester:

To an ice-chilled solution of intermediate 8.i (1.22 g, 2.30 mmol) in DCM (12 ml) were added pyridine (1.12 ml, 13.80 mmol) and triphosgene (0.341 g, 1.15 mmol). The reaction was stirred 30 min. at 0°C and then 1 h at rt. The reaction mixture was diluted with saturated NaHCO₃ and the two layers were decanted. The org. layer was dried over Na₂SO₄, filtered, and concentrated to dryness. The residue was chromatographed (DCM-MeOH 19-1) to afford the title cyclic carbonate (1.18 g, 92% yield) as a white foam. The compound was obtained as a 2-1 mixture of epimers.
MS (ESI, m/z): 560.0 [M+H⁺].

### 8.iii. {(2R,3R,6S)-2-((4RS)-2,2-dimethyl-[1,3]dioxolan-4-ylmethyl)-6-[(1S)-1-hydroxy-2-(6-methoxy-[1,5]naphthyridin-4-yl)-ethyl]-tetrahydro-pyran-3-yl}-carbamic acid tert-butyl ester:

To a solution of intermediate 8.ii (1.178g, 2.11 mmol) in EA (80 ml) was added 20% Pd(OH)₂ on carbon (moisturized, 0.443 g) and the suspension was stirred under hydrogen atmosphere for 2 h. The catalyst was filtered off and the filtrate concentrated to dryness. The residue was purified by column chromatography (DCM-MeOH 9-1) to afford the title compound (0.973 g, 89% yield) as a white foam.
MS (ESI, m/z): 518.0 [M+H⁺].

### 8.iv. 3-{3-amino-6-[1-hydroxy-2-(6-methoxy-[1,5]naphthyridin-4-yl)-ethyl]-tetrahydro-pyran-2-yl}-propane-1,2-diol:

Starting from intermediate 8.iii (0.97 g, 1.87 mmol), and using the procedure of Example 1, step 1.viii the title amine (brown foam; 0.57 g, 80% yield) was obtained as a 2-1 mixture of epimers.
¹H NMR (DMSO) main signals δ: 8.69 (d, J = 4.4 Hz, 1H); 8.27 (d, J = 9.0 Hz, 1H); 7.59 (d, J = 4.5 Hz, 1H); 7.27 (d, J = 9.0 Hz, 1H); 4.57-4.42 (m, 2H); 4.06 (overlapped s, 3H); 4.05-3.89 (overlapped m, 2H); 3.68 (m, 1H); 3.59-3.45 (m, 2H); 3.28 (m, 3H); 2.93-2.82 (m, 2H); 1.87-1.69 (m, 1H); 1.62-1.57 (m, 3H); 1.54-1.27 (m, 4H).
MS (ESI, m/z): 378.2 [M+H⁺].

### 8.v. (2RS)-3-{(2R,3R,6S)3-[(E)-3-(2,5-difluoro-phenyl)-allylamino]-6-[(S)-1-hydroxy-2-(6-methoxy-[1,5]naphthyridin-4-yl)-ethyl]-tetrahydro-pyran-2-yl}-propane-1,2-dliol:

Starting from intermediate 8.iv (0.570 g, 1.51 mmol) and (*E*)-3-(2,5-difluoro-phenyl)-propenal (0.279 g, 1.1 eq), the title compound (0.420 g, 52% yield) was obtained as a white solid according to the procedure described in Example 3.
¹H NMR (DMSO) main signals δ: 8.69 (d, J = 4.4 Hz, 1H); 8.27 (d, J = 9.0 Hz, 1H); 7.59 (d, J = 4.5 Hz, 1H); 7.52 (m, 2H); 7.32-7.23 (m, 2H); 7.22-7.10 (m, 1H); 6.62 (overlapped t, J = 16.3 Hz, 1H); 6.53 (overlapped m, 1H); 4.58 (m, 1H); 4.48 (d, J = 6.5 Hz, 1H); 4.26-4.09 (m, 1H); 4.04 (s, 3H); 3.91 (m, 1H); 3.72 (m, 1H); 3.53 (m, 2H); 3.40-3.29 (m, 4H); 2.92 (m, 1H); 2.76 (m, 1H); 1.96 (m, 1H); 1-.82-1.68 (m, 4H); 1.62-1.37 (m, 3H).
Rf= 0.38 (DCM-MeOH 9-1 1% aq. NH₄OH).
MS (ESI, m/z): 529.8.

### Example 9: (2RS)-3-{(2R,3R,6R)-3-[(E)-3-(2,5-difluoro-phenyl)-allylamino]-6-[2-(6-methoxy-quinolin-4-yl)-ethyl]-tetrahydro-pyran-2-yl}-propane-1,2-diol:

Starting from 6-methoxy-quinoline-4-carbaldehyde (prepared as described in WO 2004/035569; 1.8 g, 9.6 mmol) and intermediate 1.v (5.68 g, 10.57 mmol), the title compound (1.89 g, 3.68 mmol) was obtained as a colourless foam using the procedure of Example 1, steps 1.vi, 1.vii and 1.viii followed by the procedure of Example 3.
MS (ESI, m/z): 513.0 [M+H⁺].

### Example 10: {(2R,3R,6R)-3-[(E)-3-(2,5-difluoro-phenyl)-allylamino]-6-[2-(6-methoxy-quinolin-4-yl)-ethyl]-tetrahydro-pyran-2-yl}-acetonitrile ditrifluoroacetate:

### 10.i. {(2R,3R,6R)-3-{tert-butoxycarbonyl-[(E)-3-(2,5-difluoro-phenyl)-allyl]-amino}-6-[2-(6-methoxy-quinolin-4-yl)-ethyl]-tetrahydro-pyran-2yl}-acetic acid:

Starting from the compound of Example 9 (1.89 g, 3.68 mmol), the title compound (1.48 g, 2.41 mmol) was obtained as a colourless foam using successively the procedures of steps 6.i, 6.ii and 6.iii of Example 6.
MS (ESI, m/z): 597.0 [M+H ⁺].

### 10.ii. {(2R,3R,6R)-2-carbamoylmethyl-6-[2-(6-methoxy-quinolin-4-yl)-ethyl]-tetrahydro-pyran-3-yl}-(E)-3-(2,5-difluoro-phenyl)-allyl]-carbamic acid tert-butyl ester:

To a solution of intermediate 10.i (1.16 g, 1.94 mmol) in THF (13 ml) were added at 0°C, TEA (0.35 ml, 2.52 mmol) and isobutylchloroformate (0.301 ml, 2.32 mmol). The reaction was stirred 1 h at this temperature and aq. NH₄OH (4 ml) was added. The reaction was vigorously stirred for 45 min. EA (50 ml) was added. The two layers were decanted and the aq. layer was extracted once with EA (10 ml). The combined org. layers were washed with brine, dried over Na₂SO₄, filtered and concentrated to dryness. The residue was purified by column chromatography (DCM-MeOH 19-1 containing 0.5% aq. NH₄OH to give the title amide (0.507 g) as an off-white foam.
MS (ESI, m/z): 596.0 [M+H ⁺].

### l0.iii. {(2R,3R,6R)-2-cyanomethyl-6-[2-(6-methoxy-quinolin-4-yl)-ethyl]-tetrahydro-pyran-3-yl}-[(E)-3-(2,5-difluoro-phenyl)-allyl]-carbamic acid tert-butyl ester:

To a solution of DMSO (0.058 ml, 0.82 mmol) and intermediate 10.ii (0.245 g, 0.41 mmol) in DCM (1.5 ml) cooled at -78°C, were successively added oxalyl chloride (0.049 ml, 0.58 mmol) and TEA (0.172 ml, 1.24 mmol) at intervals of 10 min. The reaction mixture was stirred for 1 h at -78°C. Saturated NaHCO₃ (10 ml) and DCM-MeOH 9-1 (20 ml) were added. The phases were separated and the aq. layer was extracted twice with DCM-MeOH 9-1. The combined org. layers were washed with brine, dried over Na₂SO₄, filtered and evaporated under reduced pressure. The residue was purified by column chromatography (DCM-MeOH 97-3 containing 0.3% aq. NH₄OH) to give the nitrile (0.096 g, 40% yield) as a yellow gum.
MS (ESI, m/z): 577.9 [M⁺].

### 10.iv. {(2R,3R,6R)-3-[(E)-3-(2,5-difluoro-phenyl)-allylamino]-6-[2-(6-methoxy-quinolin-4-yl)-ethyl]-tetrahydro-pyran-2-yl}-acetonitrile ditrifluoroacetate:

A solution of intermediate 10.iii (0.005 g, 0.009 mmol) in TFA (0.2 ml) was stirred at rt for 5 min. The solvent was removed *in vacuo* and the residue was triturated with ether. After removal of the ethereal layer the residue was dried under HV to afford the title compound (0.006 g, 93% yield) as a yellowish solid.
MS (ESI, m/z): 478.0 [M+H ⁺].

### Example 11: (E)-3-(2,5-difluoro-phenyl)-N-{(2R,3R,6S)-2-((2RS)-2,3-dihydroxy-propyl)-6-[(E)-2-(3-fluoro-6-methoxy-[1,5]naphthyridin-4-yl)-vinyl]-tetrahydro-pyran-3-yl} acrylamide:

### 11.i. {(2R,3R,6S)-2-((4RS)-2,2-dimethyl-[1,3]dioxolan-4-ylmethyl)-6-[(E)-2-(3-fluoro-6-methoxy-[1,5]naphthyridin-4-yl)-vinyl]-tetrahydro-pyran-3-yl}-carbamic acid tert-buyl ester:

Starting from 3-fluoro-6-methoxy-[1,5]naphthyridine-4-carbaldehyde (see Preparation D; 3.17 g, 15.37 mmol) and intermediate 1.v (8.98 g, 16.71 mmol), the title alkene (4.02 g, 50% yield) was obtained as a pale yellow foam using the procedure described in Example 1, step 1.v. The compound was purified by chromatography using Hept-EA 1-2 as an eluent. The compound was obtained as a 2-1 mixture of epimers.
MS (ESI, m/z): 500.4 [M+H⁺].

### 11.ii. (2RS)-3-{(2R,3R,6R)-3-amino-6-[(E)-2-(3-fluoro-6-methoxy-[1,5]naphthyridin-4-yl)-vinyl]-tetrahydro-pyran-2-yl}-propane-1,2-diol:

This compound (0.125 g, 99% yield) was obtained as an off-white solid starting from intermediate 11.i (0.170 g, 3.98 mmol) and using procedures of Example 1, step 1.viii The compound was obtained as a 2-1 mixture of epimers.
MS (ESI, m/z): 378.1 [M+H⁺].

### 11.iii. (E)-3-(2,5-difluoro-phenyl)-N-{(2R3R,6S)-2-((2RS)-2,3-dihydroxy-propyl)-6-[(E)-2-(3-fluoro-6-methoxy-[1,5]naphthyridin-4-yl)-vinyl]-tetrahydro-pyran-3-yl} acrylamide:

Starting from intermediate 11.ii (0.05 g, 1.4 mmol) and (E)-3-(2,5-difluoro-phenyl)-acrylic acid (0.027 g, 1.1 eq.), the title compound (0.041 g, 58% yield) was obtained as a white solid according to the procedure of Example 1, step 1.ix. The compound was purified by column chromatography (DCM-MeOH 19-1 containing 1% aq. NH₄OH then 9-1 containing 1% aq. NH₄OH) The compound was obtained as a 2-1 mixture of epimers.
MS (ESI, m/z): 544.0 [M+H⁺].

### Example 12: (2RS)-3-{(2R,3R,6S)-3-[(E)-3-(2,5-difluoro-phenyl)-allylamino]-6-[(E)-2-(3-fluoro-6-methoxy-[1,5]naphthyridin-4-yl)-vinyl]-tetrahydro-pyran-2-yl}-propane-1,2-diol:

Starting from intermediate 11.ii (0.075 g, 0.2 mmol) and (*E*)-3-(2,5-difluoro-phenyl)-propenal (0.037 g, 1.1 eq), the title compound (0.065 g, 62% yield) was obtained as a white solid according to the procedure of Example 3. The compound was purified by chromatography using DCM-MeOH 19-1 containing 0.5% aq. NH₄OH as an eluent. The compound was obtained as a 2-1 mixture of epimers.
MS (ESI, m/z): 530.1 [M+H⁺].

### Example 13: (E)-3-(2,5-difluoro-phenyl)-N-[(2R,3R,6S)-2-((2RS)-2,3-dihydroxy-propyl)-6-(6-methoxy-quinolin-4-yloxymethyl)-tetrahydro-pyran-3-yl]-acrylamide:

### 13.i. [(2R,3R,6S)-2-((4RS)-2,2-dimethyl-[1,3]dioxolan-4-ylmethyl)-6-(6-methoxy-quinolin-4-yloxymethyl)-tetrahydro-pyran-3-yl]-carbamic acid tert-buyl ester:

To an ice-chilled solution of 6-methoxy-quinolin-4-ol (1.9081 g, 10.9 mmol) in THF (50 ml) were added intermediate 1.iii (3.0 g, 8.7 mmol), PPh₃ (3.1433 g, 11.9 mmol). DIAD (2.3 ml, 11.6 mmol) was added drop wise over 40 min. The reaction mixture was stirred at rt for 2 days. The solvent was removed under reduced pressure and the residue was chromatographed over SiO₂ (Hept - EA : 1 - 1, then EA - MeOH : 9 - 1) to afford the title compound (3.04 g, 6.05 mmol) as a yellowish solid. The compound was obtained as a 2-1 mixture of epimers.
MS (ESI, m/z): 503.1 [M+H⁺].

### 13.ii. (2RS)-3-[(2R, 3R, 6S)-3-amino-6-(6-methoxy-quinolin-4-yloxymethyl)-tetrahydro-pyran-2-yl]-propane-1,2-diol:

Starting from the intermediate 13.i (1.3 g, 2.58 mmol), the title amine (0.827 g, 88% yield) was obtained as a 2-1 mixture of epimers using the procedure of Example 1, step 1.viii.
MS (ESI, m/z): 362.9 [M⁺]

### 13.iii. (E)-3-(2,5-difluoro-phenyl)-N-(2R,3R,6S)-2-((2RS)-2,3-dihydroxy-propyl)-6-(6-methoxy-quinolin-4-yloxymethyl)-tetrahydro-pyran-3-yl]-acrylamide:

Starting from intermediate 13.ii (0.163 g, 0.45 mmol) and (E)-3-(2,5-difluoro-phenyl)-acrylic acid (0.091 g, 1.1 eq.), the title compound (0.062 g, 26% yield) was prepared as a white foam according to the procedure of Example 1, step 1.ix. The compound was purified by column chromatography (DCM-MeOH 19-1 containing 1% aq. NH₄OH then 9-1 containing 1% aq. NH₄OH). The compound was obtained as a 2-1 mixture of epimers.
MS (ESI, m/z): 528.0 [M+H⁺].

### Example 14: (2S,5R,6R)-5-[(E)-3-(2,5-difluoro-phenyl)-allylamino]-6-[(2RS)-2,3-dihydroxy-propyl]-tetrahydro-pyran-2-carboxylic acid (6-methoxy-[1,5] naphthyridin-4-yl)-amide:

### 14.i. (2S,5R,6R)-5-tert-butoxycarbonylamino-6-[(4RS)2,2-dimethyl-[1,3]dioxolan-4-ylmethyl]-tetrahydro-pyran-2-carboxylic acid:

To an ice-chilled solution of intermediate 1.ii (5 g, 14.48 mmol) in DCM (32 ml), MeCN (32 ml) and water (46 ml) was added NaIO₄ (14.6 g, 68.17 mmol) and RuCl₃ (0.030 g, 0.15 mmol). The reaction mixture was stirred at the same temperature for 5 h. The reaction mixture was diluted with EA (150 ml) and the solids were removed by filtration. MeOH (30 ml) was added and the resulting suspension was filtered. The filtrate was treated with aq. 10% NaHSO₃ (60 ml). The phases were separated and the aq. layer was extracted three times with EA. The combined org. layers were washed with brine, dried over Na₂SO₄, filtered and evaporated under reduced pressure to give a brown foam (quant.). The compound was obtained as a 2-1 mixture of epimers.
MS (ESI, m/z): 360.1 [M+H⁺].

### 14.ii. {(2S,5R,6R)-6-carbamoy-2-[(4RS)-2,2-dimethyl-[1,3]dioxolan-4-ylmethyl]-tetrahydro-pyran-3-yl}-carbamic acid tert-butyl ester:

Starting from intermediate 14.i (5.27 g, 14.6 mmol), the title amide (3.16 g, 60% yield) was obtained as a colourless foam using the procedure of Example 10, step 10.ii. The compound was purified by chromatography using EA-cyclohexane 4-1 as an eluent. The compound was obtained as a 2-1 mixture of epimers.
MS (ESI, m/z): 359.1 [M+H⁺].

### 14.iii. {(2S,5R,6R)-1-[(4RS)-2,2-dimethyl-[1,3]dioxolan-4-ylmethyl)-6-(6-methoxy-[1,5]naphthyridin-4-ylcarbamoyl)-tetrahydro-pyran-3-yl]-carbamic acid tert-butyl ester:

To a mixture of intermediate 14.ii (2.3 g, 6.42 mmol), rac-BINAP (0.288 g, 0.46 mmol), (dba)₃Pd₂.CHCl₃ (0.120 g, 0.12 mmol) and cesium carbonate (2.56 g, 37.86 mmol) was added dioxane (82 ml) . The mixture was sonicated for 10 min, whereupon trifluoromethanesulfonic acid 6-methoxy-[1,5]naphthyridin-4-yl ester (prepared as described in WO 03/064431, 2.10 g, 6.81 mmol) was added in one portion. The resulting mixture was heated at 100°C for 4 h. The reaction mixture was filtrated through a pad of celite and the filtrate was concentrated *in vacuo.* The residue was purified over SiO₂ (DCM-MeOH 19-1) to afford the title amide (3.27 g, 6.32 mmol) as a reddish foam.. The compound was obtained as a 2-1 mixture of epimers.
MS (ESI, m/z): 477.0 [M+H⁺].

### 14.iv. (2S,5R,6S)-5-amino-6-[(2RS)-2,3-dihydroxy-propyl]-tetrahydro-pyran-2-carboxylic acid (6-methoxy-[1,5]naphthyridin-4-yl)-amide:

Starting from intermediate 14.iii (2.72 g, 2.06 mmol), the title amine (1.27 g, 64% yield) was obtained as a reddish solid using the procedure of Example 1, step 1.viii. The compound was purified by chromatography using DCM-MeOH 6-1 containing 1% aq. NH₄OH as an eluent. The compound was obtained as a 2-1 mixture of epimers.
¹H NMR (d6-DMSO) δ: 10.59 (s, 0.66H); 10.51 (s, 0.34H); 8.70 (d, J = 5.0 Hz, 1H); 8.45 (d, J = 5.0Hz, 0.34H); 8.39 (d, J = 5.0 Hz, 0.66H); 8.28 (d, J = 9.0 Hz, 1H); 7.33 (d, J = 9.0 Hz, 1H); 4.50 (br s, 1H); 4.47 (dd, J = 4.3, 7.0 Hz, 0.34H); 4.31 (dd, J = 3.5, 9.0 Hz, 0.66H); 4.11 (m, 0.66H); 4.09 (s, 3 x 0.34H); 4.08 (s, 3 x 0.66H); 3.96 (m, 0.34H); 3.63 (m, 1H); 3.37-3.21 (m, 5H), 2.97 (m, 0.66H); 2.91 (m, 0.34H); 2.08 (m, 1H); 1.96 (m, 0.66H); 1.82-1.42 (m, 4.34H).
MS (ESI, m/z): 377.0 [M+H⁺].

### 14.v. (2S,5R,6R)-5-[(E)-3-(2,5-difluoro-phenyl)-allylamino]-6-[(2RS)-1,3-dihydroxypropyl]-tetrahydro-pyran-2-carboxylic acid (6-methoxy-[1,5]naphthyridin-4-yl)-amide:

Starting from intermediate 14.iv (0.58 g, 1.55 mmol) and (*E*)-3-(2,5-difluoro-phenyl)-propenal (0.286 g, 1.1 eq.), the title compound (0.393 g, 48% yield) was obtained as a white solid according to the procedure of Example 3. The compound was purified by chromatography using DCM-MeOH 9-1 containing 1% aq. NH₄OH as an eluent. The compound was obtained as a 2-1 mixture of epimers.
¹H NMR (d6-DMSO) δ: 10.64 (s, 0.66H); 10.56 (s, 0.34H); 8.69 (d. J = 5.0 Hz, 1H); 8.42 (d, J = 5.0 Hz, 0.34H); 8.37 (d, J = 5.0 Hz, 0.66H); 8.28 (d, J = 9.0 Hz, 1H); 7.49 (m, 1H); 7.32 (d, J = 9.0 Hz, 1H); 7.27 (m, 1H); 7.12 (m, 1H); 6.63 (d, J = 16.2 Hz, 1H); 6.49 (m, 1H); 4.52-4.42 (m, 3H); 4.26 (m, 1H); 4.06 (s, 3 x 0.34H); 4.04 (s, 3 x 0.66H); 3.65 (m, 1H); 3.44-3.24 (m, 4H); 2.81 (m, 1H); 2.12-1.41 (m, 7H).
MS (ESI, m/z): 529.0 [M+H⁺].

### Example 15: (2S,5R,6R)-6-[(2RS)-2,3-dihydroxy-propyl]-5-[(3-oxo-3,4-dihydro-2H-pyrido[3,2-b][1,4]thiazin-6-ylmethyl)-amino]-tetrahydro-pyran-2-carboxylic acid (6-methoxy-[1,5] naphthyridin-4-yl)-amide:

Starting from intermediate 14.iv (0.539 g, 1.43 mmol) and 3-oxo-3,4-dihydro-2*H*-pyrido[3,2-*b*][1,4]thiazine-6-carbaldehyde (0.306 g, 1.1 eq.), the title compound (0.223 g, 28% yield) was obtained as a yellowish foam using the procedure of Example 3. The compound was purified by chromatography using DCM-MeOH 9-1 containing 1% aq. NH₄OH as an eluent. The compound was obtained as a 2-1 mixture of epimers of 70% purity.
MS (ESI, m/z): 544.9 [M⁺].

### Example 16: (2S,5R,6R)-5-[(E)-3-(2,5-difluoro-phenyl)-acryloylamino]-6-[(2RS)-2,3-dihydroxy-propyl]-tetrahydro-pyran-2-carboxylic acid (6-methoxy-[1,5] naphthyridin-4-yl)-amide:

Starting from intermediate 14.iv (0.187 g, 0.5 mmol) and (*E*)-3-(2,5-difluoro-phenyl)-acrylic acid (0.101 g, 1.1 eq.), the title compound (0.043 g, 16% yield) was obtained as a white solid using the procedure of Example 1, step 1.ix. The compound was purified by chromatography using DCM-MeOH 9-1 containing 1% aq. NH₄OH as an eluent. The compound was obtained as a 1-1 mixture of epimers.
¹H NMR (d6-DMSO) δ: 10.57 (s, 0.5H); 10.49 (s, 0.5H); 8.72 (d, J = 5.0 Hz, 0.5H); 8.71 (d, J = 5.0 Hz, 0.5H); 8.49 (d, J = 5.0 Hz, 0.5H); 8.41 (d, J = 5.0 Hz, 0.5H); 8.38 (d, J = 8.3 Hz, 0.5H); 8.33 (d, J = 8.3 Hz, 0.5H); 8.30 (d, J = 2.2 Hz, 0.5H); 8.27 (d, J = 2.2 Hz, 0.5H); 7.53-7.25 (m, 5H); 6.97 (d, J = 16.0 Hz, 0.5H); 6.90 (d, J = 16.0 Hz, 0.5H); 4.68 (t, J = 4.7 Hz, 0.5H); 4.53-4.43 (m, 2H); 4.30 (m, 1H); 4.11 (s, 3 x 0.5H); 4.09 (s, 3 x 0.5H); 4.10 (m, 1.5H); 3.69 (m, 0.5H); 3.56 (m, 0.5H); 3.36-3.17 (m, 2H); 2.16-1.60 (m, 5.5H); 1.30-1.20 (m, 1.5H).
MS (ESI, m/z): 542.6 [M+H⁺].

### Example 17: 3-oxo-3,4-dihydro-2H-pyrido[3,2-b][1,4]thiazine-6-carboxylic acid {(2R,3R,6S)-2-((2R)-2,3-dihydroxy-propyl)-6-[(E)-2-(6-methoxy-[1,5]naphthyridin-4-yl)-vinyl]-tetrahydro-pyran-3-yl}-amide and 3-oxo-3,4-dihydro-2H-pyrido[3,2-b][1,4]thiazine-6-carboxylic acid {(2R,3R,6S)-2-((2S)-2,3-dihydroxy-propyl)-6-[(E)-2-(6-methoxy-[1,5]naphthyridin-4-yl)-vinyl]-tetrahydro-pyran-3-yl}-amide:

### 17.i. (2RS)-3-{(2R,3R,6S)-3-amino-6-[(E)-2-(6-methoxy-[1,5]naphthyridin-4-yl)-vinyl}-tetrahydro-pyran-2-yl}-propane-1,2-diol:

A solution of intermediate 4.i (2.19 g, 4.38 mmol) in TFA (20 ml) was stirred at rt for 20 min. Water (20 ml) was added. The reaction proceeded 1 h. After concentration to dryness, the residue was taken up in 2M NaOH (20 ml). The aq. layer was extracted with DCM-MeOH (9-1, 10 x 50 ml). The combined org. layers were washed with brine (20 ml), dried over Na₂SO₄, filtered and concentrated to dryness. The residue was triturated in ether, filtered and dried under HV to afford the title amine (1.35 g, 85% yield) as an off-white solid.
MS (ESI, m/z): 360.3 [M+H⁺].

### 17.ii. 3-oxo-3,4-dihydro-2H-pyrido[3,2-b][1,4]thiazine-6-carboxylic acid {(2R, 3R, 6S)-2-((2R)-2,3-dihydroxy-propyl)-6-[(E)-2-(6-methoxy-[1,5]naphthyridin-4-yl)-vinyl]-tetrahydro-pyran-3-yl}-amide and 3-oxo-3,4-dihydro-2H-pyrido[3,2-b][1,4]thiazine-6-carboxylic acid {(2R,3R,6S)-2-((2S)-2,3-dihydroxypropyl)-6-[(E)-2-(6-methoxy-[1,5]naphthyridin-4-yl)-vinyl]-tetrahydro-pyran-3-yl}-amide:

Starting from intermediate 17.i (0.2 g, 0.55 mmol) and 3-oxo-3,4-dihydro-2H-pyrido[3,2-*b*][1,4]thiazine-6-carboxylic acid (0.116 g, 1.1 eq.) and using the procedure of Example 1, step 1.x., the title compounds were obtained as white solids. The isomerss were separated by chromatography over SiO₂ (DCM-MeOH93-7 containing 0.7% NH₄OH) to afford a first eluting isomer (0.019 g, 6% yield), a fraction containing both isomers (0.12 g, 39% yield) and the second eluting isomer (0.055 g, 18% yield).

### First eluting isomer:

MS (ESI, m/z): 551.9 [M+H⁺].

### Second eluting isomer:

¹H NMR (d6-DMSO) δ: 11.29 (s, 1H); 8.76 (d, J = 4.6 Hz, 1H); 8.37 (d, J = 9.0 Hz, 1H); 8.27 (d, J = 9.0 Hz, 1H); 7.98 (d, J = 7.8 Hz, 1H); 7.89 (d, J = 4.6 Hz, 1H); 7.63 (d, J = 7.8 Hz, 1H); 7.60 (dd, J = 1.6, 16.6 Hz, 1H); 7.29 (d, J = 9.0 Hz, 1H); 7.14 (dd, J = 4.5, 16.6 Hz, 1H); 4.71 (br s, 1H); 4.53-4.48 (m, 2H); 4.23 (m, 1H); 4.07 (s, 3H); 4.06 (m, 1H); 3.66 (m, 2H); 3.33-3.20 (, 2H); 2.13-1.97 (m, 2H); 1.81-1.73 (m, 3H); 1.23 (m, 1H).
MS (ESI, m/z): 551.9 [M+H⁺].

### Example 18: (2RS)-3-{(2R,3R,6S)-3-[(E)-3-(2,5-difluoro-phenyl)-allylamino]-6-[(E)-2-(6-methoxy-[1,5]naphthyridin-4-yl)-vinyl]-tetrahydro-pyran-2-yl}-propane-1,2-diol:

Starting from intermediate 17.i (0.1 g, 0.28 mmol) and (*E*)-3-(2,5-difluoro-phenyl)-propenal (0.047 g, 1eq.), the title compound (0.104 g, 73% yield) was obtained as a yellowish foam using the procedure of Example 3. The compound was purified by chromatography using DCM-MeOH 93-7 containing 0.7% aq. NH₄OH as an eluent. The compound was obtained as a 2-1 mixture of epimers.
¹H NMR (d6-DMSO) δ: 8.71 (d, J = 4.6 Hz, 1H); 8.24 (d, J = 9.0 Hz, 1H); 7.84 (d, J = 4.5Hz, 0.33H); 7.82 (d, J = 4.5 Hz, 0.67H); 7.58-7.45 (m, 2H); 7.27 (d, J = 9.0 Hz, 1H); 7.25 (m, 1H); 7.12 (m, 1H); 7.01-6.92 (m, 1H), 6.63 (d, J= 16.1 Hz, 1H); 6.51 (td, J = 5.1, 16.1 Hz, 1H); 4.68 (br s, 0.67H); 4.51-4.35 (m, 2.33H); 4.21 (m, 0.67H); 4.11 (m, 0.33H); 4.04 (s, 3 x 0.33H); 4.02 (s, 3 x 0.67H); 3.62 (m, 1H); 3.39-3.25 (m, 4H); 2.75 (m, 1H); 2.05-1.37 (m, 7H).
MS (ESI, m/z): 512.0 [M⁺].

### Example 19: (2RS)-3-{2R,3R,6R)-3-[(E)-3-(2,5-difluoro-phenyl)-allylamino]-6-[2-(6-fluoro-quinolin-4-yl)-ethyl]-tetrahydro-pyran-2-yl}-propane-1,2-diol:

### 19.i. {(2R,3R,6S)-1-[(4RS)-2,2-dimethyl-[1,3]dioxolan-4-ylmethyl]-6-[(E)-1-(6-fluoro-quinolin-4-yl)-vinyl]-tetrahydro-pyran-3-yl}-carbamic acid tert-butyl ester:

Starting from 3-fluoro-6-methoxy-quinoline-5-carbaldehyde (1.65 g, 9.43 mmol) and intermediate 1.v (5.14 g, 9.56 mmol), the title alkene (3.21 g, 69% yield) was obtained as a colourless foam using the procedure of Example 1, step 1.v. The compound was purified by chromatography using Hex-EA 1-1 then 1-4 as eluents. The compound was obtained as a 3-1 mixture of epimers.

### 19.ii. {2-(2,2-dimethyl-[1,3]dioxolan-4-ylmethyl)-6-[2-(6-fluoro-quinolin-4-yl)-ethyl]-tetrahydro-pyran-3-yl}-carbamic acid tert-butyl ester:

Starting from intermediate 19.i (0.370 g, 0.76 mmol), the title compound (0.36 g, 97% yield) was obtained as a colourless foam using the procedure of Example 1, step 1.vii. The compound was obtained as a 3-1 mixture of epimers.
¹H NMR (CDCl₃) δ: 8.79 (d, J = 4.5 Hz, 1H); 8.17 (dd, J = 5.1, 9.3 Hz, 1H); 7.75 (dd, J = 2.7, 9.3 Hz, 0.75H); 7.67 (dd, J = 2.7, 10.1 Hz, 0.25H); 7.34 (m, 1H); 5.11 (d, J = 9.1 Hz, 0.75H); 4.93 (m, 0.25H); 4.40 (m, 0.75H; 4.37 (m, 0.25H); 4.15-4.08 (m, 2H); 3.95 (m, 1H); 3.81 (m, 0.25H); 3.76 (m, 0.75H); 3.60 (t, J = 7.6 Hz, 0.25H); 3.56 (t, J = 7.7 Hz, 0.75H); 3.32 (m, 0.75H); 3.22 (m, 0.25H); 3.01 (m, 1H); 2.43 (m, 0.75H); 2.37 (m, 0.25H); 2.02-1.35 (m, 8H); 1.47 (s, 9H); 1.45 (s, 3 x 0.75H), 1.32 (s, 3 x 0.25H); 1.27 (3 x 0.75H); 1.25 (s, 3 x 0.25H).
MS (ESI, m/z): 489.0 [M+H⁺].

### 19.iii. (2RS)-3-{(2R,3R,6R)-3-amino-6-[2-(6-fluoro-quinolin-4-yl)-ethyl]-tetrahydro-pyran-2-yl}-propane-1,2-diol:

Starting from intermediate 19.ii (0.36 g, 0.73 mmol) and using the procedure of Example 1, step 1.viii, the title amine (0.25 g, 97% yield) was obtained as a beige foam. The compound was obtained as a 3-1 mixture of epimers.
MS (ESI, m/z): 349.1 [M+H⁺].

### 19.iv. (2RS)-3-{2R,3R,6R)-3-[(E)-3-(2,5-difluoro-phenyl)-allylamino]-6-[2-(6-fluoro-quinolin-4-yl)-ethyl]-tetrahydro-pyran-2-yl}-propane-1,2-diol:

Starting from intermediate 19.iii (0.25 g, 0.72 mmol) and (E)-3-(2,5-difluoro-phenyl)-propenal (0.120 g, 1 eq.), the title compound (0.220 g, 61% yield) was obtained as a white foam using the procedure of Example 3. The compound was purified by chromatography using DCM-MeOH 93-7 containing 0.7% aq. NH₄OH as an eluent. The compound was obtained as a 3-1 mixture of epimers.
MS (ESI, m/z): 501.2 [M+H⁺].

### Example 20: 2-{(2R,3R,6S)-3-[(E)-3-(2,5-difluoro-phenyl)-allylamino]-6-[(E)-2-(6-methoxy-[1,5] naphthyridin-4-yl)-vinyl]-tetrahydro-pyran-2-yl}-N-(2-hydroxy-ethyl)-acetamide:

### 20.i. {(2R,3R,6S)-2-[(2RS)-2,3-dihydroxy-propyl]-6-[(E)-2-(6-methoxy-[1,5]naphthyridin-4-yl)-vinyl]-tetrahydro-pyran-3-yl}-carbamic acid tert-butyl ester:

A. solution of intermediate 4.i (5.5 g, 11 mmol) in AcOH (90 ml), THF (30 ml) and water (30 ml) was heated at 60°C for 12 h. The volatiles were removed *in vacuo.* The residue was taken up in 1M NaOH (until pH 8 was reached). The residue was taken up in DCM-MeOH (9-1, 200 ml). The aq. layer was extracted once more. The combined org. layers were dried over Na₂SO₄, filtered and concentrated to dryness. The residue was chromatographed (DCM-MeOH 19-1 then 9-1) to afford the title compound (4.5 g) as a colourless foam. The compound was obtained as a 2-1 mixture of epimers.
¹H NMR (d6-DMSO) δ: 8.73 (d, J = 4.6 Hz, 0.33H); 8.72 (d, J = 4.6 Hz; 0.67H); 8.25 (d, J = 9.0 Hz, 1H); 7.86 (d, J = 4.6 Hz, 0.33H); 7.84 (d, J = 4.6.Hz, 0.67H); 7.56 (d, J = 16.5 Hz, 0.33H); 7.54 (d, J = 16.5 Hz, 0.67H); 7.27 (d, J = 9.0 Hz, 1H); 7.02 (dd, J = 4.2, 16.5 Hz, 0.33H); 6.98 (dd, J = 5.0, 16.5 Hz, 0.67H); 6.80 (d, J = 8.5 Hz, 0.67H); 6.77 (d, J = 8.4 Hz, 0.33H); 4.51-4.37 (m, 3H); 4.10 (br s, 1H); 4.06 (s, 3 x 0.33H); 4.05 (s,3 x 0.67H); 3.61 (m, 2H); 3.28 (m, 2H); 1.99-1.52 (m, 5H); 1.39 (s, 9H); 1.19 (m, 1H).
MS (ESI, m/z): 459.9 [M+H⁺].

### 20.ii. {(2R,3R,6S)-3-tert-butoxycarbonylamino-6-[(E)-2-(6-methoxy-[1,5]naphthyridin-4-yl)-vinyl]-tetrahydro-pyran-2-yl]-acetic acids:

The title acid (2.91 g, 6.56 mmol) was obtained as a white foam, starting from intermediate 20.i (3.92 g, 8.55 mmol) and using successively the procedures of Example 6, step 6.ii (99% yield) and step 6.iii (76% yield).
MS (ESI, m/z): 443.9 [M+H⁺].

### 20.iii. {(2R,3R,6S)-2-{[2-(tert-butyl-dimethyl-silanyloxy)-ethylcarbamoyl]-methyl}-6-[(E)-2-(6-methoxy-[1,5]naphthyridin-4-yl)-vinyl]-tetrahydro-pyran-3-yl)-carbamic acid tert-butyl ester:

The title compound (0.188 g, 39% yield) was obtained as a yellow solid starting from intermediate 20.ii (0.35 g, 0.79 mmol) and 2-(*tert*-butyl-dimethyl-silanyloxy)-ethylamine (prepared as described in *Bioorg. Med. Chem.* (2005), **13**(11), 3821-3839; 0.152 g, 1.1 eq), and following the procedure of Example 1, step 1.ix The compound was purified by chromatography over SiO₂ using DCM-MeOH 9-1 containing 1% aq. NH₄OH as eluent.
MS (ESI, m/z): 601.1 [M+H⁺].

### 20.iv. 2-{(2R,3R,6S)-3-amino-6-[(E)-2-(6-methoxy-[1,5]naphthyridin-4-yl)-vinyl]-tetrahydro-pyran-2-yl}-N-(2-hydroxy-ethyl)-acetamide:

The title amine (0.036 g, 31% yield) was prepared as a yellow oil, starting from intermediate 20.iii (0.183 g, 0.3 mmol) and using the procedure of Example 1, step 1.viii. The compound was purified by chromatography over SiO₂ using DCM-MeOH 6-1 containing 1% aq. NH₄OH) as eluent.
MS (ESI, m/z): 387.0 [M+H⁺].

### 20.v. 2-{(2R,3R,6S)-3-{(E)-3-(2,5-difluoro-phenyl)-allylamino)-6-{(E)-2-(6-methoxy-[1,5]naphthyridin-4-yl)-vinyl]-tetrahydro-pyran-2yl}-N-(2-hydroxy-ethyl)-acetamide:

Starting from intermediate 20.iv (0.035 g, 0.09 mmol) and (E)-3-(2,5-difluoro-phenyl)-propenal (0.017g, 1.1 eq.), the title compound (0.026 g, 53% yield) was prepared as a off-white foam using the procedure of Example 3. The compound was purified by chromatography using DCM-MeOH 9-1 containing 1% aq. NH₄OH as an eluent.
¹H NMR (d6-DMSO) δ: 8.72 (d, J = 4.6 Hz, 1H); 8.24 (d, J = 9.0 Hz, 1H); 7.89 (t, J = 5.5 Hz, 1H); 7.81 (d, J = 4.6 Hz, 1H); 7.53-7.45 (m, 2H); 7.27 (d, J = 9.0 Hz, 1H); 7.23 (m, 1H); 7.12 (m, 1H); 6.91 (dd, J = 5.2, 16.3 Hz, 1H); 6.62 (d, J = 15.8 Hz, 1H); 6.51 (td, J = 5.3, 15.8 Hz, 1H); 4.60 (t, J = 5.4 Hz, 1H); 4.49-4.40 (m, 2H); 4.05 (s, 3H); 4.01 (m, 1H); 3.40-3.36 (m, 4H); 3.15-3.11 (m, 2H); 2.78 (m, 1H); 2.62 (dd, J = 9.5, 14.4 Hz, 1H); 2.39 (dd, J=4.5, 14.4 Hz, 1H); 1.91 (m, 1H); 1.80 (m, 1H); 1.56-1.46 (m, 2H).
MS (ESI, m/z): 501.2 [M+H⁺].

### Example 21: 2-{(2R,3R,6S)-3-[(E)-3-(2,5-difluoro-phenyl)-allylamino]-6-[(1R,2R)-1,2-dihydroxy-2-(6-methoxy-[1,5]naphthyridin-4-yl)-ethyl]-tetrahydro-pyran-2-yl)-N-(2-hydroxy-ethyl)-acetamide:

### 21.i. {(2R,3R,6S)-2-{[2-(tert-tutyl-dimethyl-silanyloxy)-ethylcarbamoyl]-methyl}-6-[(1R,2R)-1,2-dihydroxy-2-(6-methoxy-[1,5]naphthyridin-4-yl)-ethyl]-tetrahydro-pyran-3-yl}-carbamic acid tert-butyl ester:

Starting from intermediate 20.iii (0.317 g, 0.53 mmol), the title diol (0.122 g, 36% yield) was obtained as a white solid, using the procedure of Preparation B, step B.ii. The compound was purified by chromatography using DCM-MeOH 19-1 containing 0.5% aq. NH₄OH as eluent.
MS (ESI, m/z): 635.0 [M+H⁺].

### 21.ii. 2-{(2R,3R,6S)-3-amino-6-[(1R,2R)-1,2-dihydroxy-2-(6-methoxy-[1,5]naphthyridin-4-yl)-ethyl]-tetrahydro-pyran-2-yl]-N-(2-hydroxy-ethyl)-acetamide:

Starting from intermediate 21.i (0.118 g, 0.17 mmol), the title amine (0.065 g, 91% yield) was obtained as a beige solid, using the procedure of Example 1, step 1.viii.
MS (ESI, m/z): 421.0 [M+H⁺].

### 21.iii. 2-{(2R,3R,6S)-3-[(E)-3-(2,5-difluoro-phenyl)-allylamino]-6-[(1R,2R)-1,2-dihydroxy-2-(6-methoxy-[1,5]naphthyridin-4-yl)-ethyl]-tetrahydro-pyran-2-yl}-N-(2-hydroxy-ethyl)-acetamide:

Starting from intermediate 21.ii (0.064 g, 0.15mmol) and (*E*)-3-(2,5-difluoro-phenyl)-propenal (0.028g, 1.1eq.), the title compound (0.013 g, 15% yield) was obtained as a white solid using the procedure of Example 3. The compound was purified by chromatography using DCM-MeOH 9-1 containing 1% aq. NH₄OH as an eluent.
¹H NMR (d6-DMSO) δ: 8.77 (d, J = 4.5 Hz, 1H); 8.26 (d, J = 9.0 Hz, 1H); 7.90 (t, J = 5.4 Hz, 1H); 7.77 (d, J = 4.5 Hz, 1H); 7.49 (m, 1H); 7.31-7.21 (m, 1H); 7.25 (d, J = 9.0 Hz, 1H); 7.15 (m, 1H); 6.63 (d, J = 16.2 Hz, 1H); 6.52 (td, J = 6.0, 16.2 Hz, 1H); 5.66 (d, J = 6.7 Hz, 1H); 5.21. (d, J = 6.6 Hz, 1H); 4.61 (t, J = 5.6 Hz, 1H); 4.32 (m, 1H); 4.24 (m, 1H); 3.99 (s, 3H); 3.93 (m, 2H); 3.41-3.31 (m, 3H); 3.13-3.06 (m, 2H); 2.73 (m, 1H); 2.61 (dd, J = 9.9, 15.3 Hz, 1H); 2.33 (dd, J = 3.5, 15.3 Hz, 1H); 1.99 (m, 1H); 1.82 (m, 1H); 1.72-1.61 (m, 2H); 1.2 (br s, 2H).
**MS (ESI, m/z): 572.9 [M+H⁺].**

### Example 22: 2-{(2R,3R,6S)-3-[(E)-3-(2,5-difluoro-phenyl)-allylamino]-6-[(1S)-1-hydroxy-2-(6-methoxy-[1,5]naphthyridin-4-yl)-ethyl]-tetrahydro-pyran-2-yl}-N-(2-hydroxy-ethyl)-acetamide:

### 22.i. {(2R,3R,6S}-2-{[tert-butyl-dimethyl-silanyloxy)-ethylcarbamoyl]-methyl}-6-[(1S)-1-hydroxy-2-(6-methoxy-[1,5]naphthyridin-4-yl)-ethyl]-tetrahydro-pyran-3-yl}-[(E)-3-(-2,5-difluoro-phenyl)-allyl]-carbamic acid tert-butyl ester:

To a solution of intermediate 6.iii (0.05 g, 0.08 mmol) in DCM (1 ml) were added 2-(*tert*-butyl-dimethyl-silanyloxy)-ethylamine (0.014 g, 1.1 eq.), DIPEA (0.042 ml), 3 eq.) and 1-propylphosphonic acid cyclic anhydride (T3P^{®}, 0.058 ml, 50% in EA, 1.2 eq.). The mixture was stirred at rt for 2 h. The reaction mixture was directly subjected to chromatography (DCM-MeOH 19-1 containing 0.5% aq. NH₄OH to afford the title compound (0.055 g, 88% yield) as a colourless oil.
MS (ESI, m/z): 770.9 [M+H⁺].

### 22.ii. 2-{(2R,3R,6S)-3-[(E)-3-(2,5-Difluoro-phenyl)-allylamino]-6-[(1S)-1-hydroxy-2-(6-methoxy-[1,5]naphthyridin-4-yl)-ethyl]-tetrahydro-pyran-2-yl)-N-(2-hydroxy-ethyl)-acetamide:

To a solution of intermediate 22.i. (0.054 g, 0.071 mmol) in dioxane (0.5 ml) was added 5N HCl in dioxane (0.5 ml). The mixture was stirred overnight at rt and concentrated to dryness. The residue was partitioned between saturated NaHCO₃ (5 ml) and DCM-MeOH (9-1, 20 ml). The org. layer was dried over Na₂SO₄ filtered and concentrated to dryness and the residue was chromatographed (DCM-MeOH 9-1 containing 1% aq. NH₄OH) to afford the title compound (0.025 g, 63% yield) as a colourless foam.
MS (ESI, m/z): 556.9 [M+H⁺].

### Example 23: 2-{(2R,3R,6S)-3-[(E)-3-(2,5-difluoro-phenyl)-allylamino]-6-[(1S)-1-hydroxy-2-(6-methoxy-[1,5]naphthyridin-4-yl)-ethyl-tetrahydro-pyran-2-yl}-1-morpholin-4-yl-ethanone:

Starting from intermediate 6.iii (0.05 g, 0.08 mmol) and morpholine (0.007 ml, 1 eq), the title compound (0.027 g) was obtained as a beige solid, using the procedures of Example 22, step 22.i. (amide coupling, 90% yield) and step 22.ii (Boc deprotection, 63% yield) After each step, the product was purified by chromatography using DCM-MeOH 9-1 containing 1% aq. NH₄OH as eluent.
¹H NMR (CDCl₃) 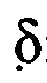 : 8.70 (d, J = 4.5 Hz, 1H); 8.21 (d, J = 9.0 Hz, 1H); 7.66 (d, J = 4.5 Hz, 1H); 7.15 (m, 1H); 7.12 (d, J = 9.0 Hz, 1H); 7.00 (m, 1H); 6.90 (m, 1H); 6.71 (d, J = 15.4 Hz, 1H); 6.35 (td, J = 5.8, 15.4 Hz, 1H); 4.95 (br s, 1H); 4.46 (br t, J = 8.6 Hz, 1H); 4.37 (m, 1H); 4.08 (s, 3H); 3.72-3.33 (m, 12H); 2.98-2.84 (m, 2H); 2.74 (br s, 1H); 2.38 (dd, J = 2.1, 16.8 Hz, 1H); 2.00-1.71 (m, 5H).
MS (ESI, m/z): 583.0 [M+H⁺].

### Example 24: N-cyclopropyl-2-{(2R,3R,6S)-3-[(E)-3-(2,5-difluoro-phenyl)-allylamino]-6-[(1S)-1-hydroxy-2-(6-methoxy-[1,5]naphthyridin-4-yl)-ethyl]-tetrahydro-pyran-2-yl}-acetamide:

Starting from intermediate 6.iii. (0.05 g, 0.08 mmol) and cyclopropylamine (0.006 ml, 1 eq), the title compound (0.044 g) was obtained as a beige foam, using the procedures of Example 22, step 22.i. (amide coupling, 92% yield) and step 22.ii (Boc deprotection, 95% yield). The product was purified by chromatography using DCM-MeOH 9-1 containing 1% aq. NH₄OH after the first step, and by trituration in ether after the second step.
¹H NMR (CDCl₃) 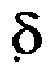 : 8.70 (d, J = 4.5 Hz, 1H); 8.24 (d, J = 9.0 Hz, 1H); 7.52 (d, J = 4.5 Hz, 1H); 7.15 (d, J = 9.0 Hz, 1H); 7.13 (m, 1H); 6.99 (m, 1H); 6.88 (m, 1H); 6.68 (overlapped m, 1H); 6.68 (d, J = 16.0 Hz, 1H); 6.33 (td, J = 6.2, 16.0 Hz, 1H); 4.43 (m, 1H); 4.15 (m, 1H); 4.08 (s, 3H); 3.59-3.36 (m, 4H); 3.16 (dd, J = 8.9, 13.6 Hz, 1H); 2.94 (m, 1H); 2.74-2.49 (m, 3H); 2.06 (br s, 1H); 2.04-1.90 (m, 2H); 1.78-1.68 (m, 2H); 0.87 (m, 1H); 0.75-0.69 (m, 2H); 0.49-0.43 (m, 2H).
MS (ESI, m/z): 553.0 [M+H⁺].

### Example 25: 2-{(2R,3R,6S)-3-[(E)-3-(2,5-difluoro-phenyl)-allylamino]-6-[(1S)-1-hydroxy-2-(6-methoxy-[1,5]naphthyridin-4-yl)-ethyl]-tetrahydro-pyran-2-yl}-N,N-dimethyl-acetamide:

Starting from intermediate 6.iii (0.075 g, 0.12 mmol) and dimethylamine hydrochloride (0.011 g, 1.05 eq), the title compound (0.053 g) was obtained as a beige foam, using the procedures of Example 22, step 22.i. (amide coupling, 80% yield) and step 22.ii (Boc deprotection, 95% yield). The product was purified by chromatography using DCM-MeOH 9-1 containing 1% aq. NH₄OH after the first step, and by trituration in ether after the second step. ¹H NMR (CDCl₃) 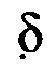 : 8.70 (d, J = 4.5 Hz, 1H); 8.22 (d, J = 9.0 Hz, 1H); 7.66 (d, J = 4.5 Hz, 1H); 7.13 (m, 1H); 7.11 (d, J = 9.0 Hz, 1 H); 6.99 (m, 1H); 6.91 (m, 1H); 6.73 (d, J = 16.5 Hz, 1H); 6.36 (td, J = 5.9, 16.5 Hz, 1H); 4.98 (br, s, 1H); 4.48-4.38 (m, 2H); 4.08 (s, 3H); 3.68-3.58 (m, 3H); 3.38 (m, 1H); 3.03 (s, 3H); 2.96 (s, 3H); 2.95-2.79 (m, 2H); 2.78 (m, 1H); 2.43 (m, 1H); 2.00-1.57 (m, 5H).
**MS (ESI, m/z): 540.6 [M+H⁺].**

### Example 26: 2-{(2R,3R,6S)-3-[(E)-3-(2,5-difluoro-phenyl)-allylamino]-6-[(1S)-1-hydroxy-2-(6-methoxy-[1,5]naphthyridin-4-yl)-ethyl]-tetrahydro-pyran-2-yl}-1-(4-hydroxy-piperidin-1-yl)-ethanone:

Starting from intermediate 6.iii (0.075 g, 0.12 mmol) and 4-hydroxypiperidine (0.012 g, 1.0 eq), the title compound (0.064 g) was obtained as a yellowish oil, using the procedures of Example 22, step 22.i. (amide coupling, 88% yield) and step 22.ii (Boc deprotection, 95% yield) The product was purified by chromatography using DCM-MeOH 9-1 containing 1% aq. NH₄OH after the first step, and by trituration in ether after the second step.
MS (ESI, m/z): 597.0 [M+H⁺].

### Example 27: 2-{(2R,3R,6S)-3-[(E)-3-[2,5-difluoro-phenyl)-allylamino]-6-[(1S)-1-hydroxy-2-(6-methoxy-[1,5)naphthyridin-4-yl)-ethyl]-tetrahydro-pyran-2-yl}-N-prop-2-ynyloxy-acetamide:

Starting from intermediate 6.iii (0.2 g, 0.32 mmol) and *O*-prop-2-ynyl-hydroxylamine hydrochloride (0.039 g, 1.1eq), the title compound (0.080 g) was obtained as a yellowish oil, using the procedures of Example 22, step 22.i. (amide coupling, 82% yield) and step 22.ii (Boc deprotection, 52% yield) The product was purified by chromatography using DCM-MeOH 9-1 containing 1% aq. NH₄OH after the first step, and by trituration in ether after the second step.
MS (ESI, m/z): 567.7 [M+H⁺].

### Example 28: (2RS)-3-{(2R,3R,6S)-3-[(E)-3-(2,5-difluoro-phenyl)-allylamino]-6-[(E)-2-(3-fluoro-6-methoxy-quinolin-4-yl)-vinyl]-tetrahydro-pyran-2-yl}-propane-1,2-diol:

### 28.i. 3-fluoro-6-methoxy-quinoline-4-carbaldehyde:

To a solution of diisopropylamine (3.5 ml) in THF (100 ml), cooled to -78°C, was added n-BuLi (2.5*N* in hexanes, 10 ml). The reaction mixture was stirred 5 min at this temperature before warming to 0°C. The reaction mixture was stirred 15 min before cooling to -78°C. 3-fluoro-6-methoxy-quinoline (4.38 g, 24.7 mmol) in THF (20 ml + 5 ml rinse) was added and the mixture was stirred 3 h at -78°C. DMF (3 ml) was added dropwise. After 15 min, the reaction mixture was warmed up to rt. 10% aq. NaHSO₄ (10 ml) was added. The volatiles were removed *in vacuo* and the residue was made basic with saturated sodium bicarbonate. The aq. layer was extracted twice with EA (2 x 200 ml). The combined org. layers were washed with brine, dried over Na₂SO₄, filtered and concentrated to dryness. The residue was chromatographed (Hex-EA 1-1) to afford the title aldehyde (3.05 g, 60% yield) as a yellowish solid. The compound was contaminated by 20% of the remaining starting material.
¹H NMR (CDCl₃) δ : 10.86 (s, 1H); 8.81 (d, J = 1.8 Hz, 1H); 8.50 (d, J = 2.8 Hz, 1H); 8.03 (d, J = 9.2 Hz, 1H); 7.40 (dd, J = 2.8, 9.2 Hz, 1H); 4.01 (s, 3H).

### 28.ii. {(2R,3R,6S)-2-[(4RS)-2,2-dimethyl-[1,3]dioxolan-4-ylmethyl]-6-[(E)-2-(3-fluoro-6-methoxy-quinolin-4-yl)-vinyl]-tetrahydro-pyran-3-yl}-carbamic acid tert-butyl ester:

Starting from intermediate 28.i (3.05 g, 11.9 mmol) and intermediate 1.v (6.95 g, 12.9 mmol), the title alkene (5.38 g, 80% yield) was prepared as a pale yellow foam using the procedure of Example 1, step 1.v. The compound was purified by chromatography using Hept-EA 1-2 as an eluent. The compound was obtained as a 2-1 mixture of epimers and a 3-1 E-Z mixture.
MS (ESI, m/z): 517.1 [M+H⁺].

### 28.iii. (2RS)-3-{(2R,3R,6S)-3-[(E)-3-(2,5-difluoro-Phenyl)-allylamino]-6-[(E)-2-(3-fluoro-6-methoxy-quinolin-4yl)-vinyl]-tetrahydro-pyran-2-yl}-propane-1,2-diol:

Starting from intermediate 28.ii (2.63 g, 5.1 mmol), the title compound (1.62 g) was obtained as a yellowish foam, using the procedures of Example 1, step 1.viii (deprotection, 67% yield) and of Example 3 (reductive amination, 91% yield). After each step, the crude reaction mixture was purified by chromatography over SiO₂ using respectively DCM-MeOH 6-1 containing 1% aq. NH₄OH and DCM-MeOH 9-1 containing 1% aq. NH₄OH as eluents. The compound was obtained as a 2-1 mixture of epimers and a 3-1 E-Z mixture.
MS (ESI, m/z): 567.7 [M+H⁺].

### Example 29: 2-{(2R,3R,6R)-3-[(E)-3-(2,5-difluoro-phenyl)-allylamino]-6-[2-(3-fluoro-6-methoxy-quinolin-4-yl)-ethyl]-tetrahydro-pyran-2-yl)-N-(2-hydroxy-ethyl)-acetamide:

### 29.i. {(2R,3R,6S)-2-{[2-(tert-butyl-dimethyl-silanyloxy)-ethylcarbamoyl]-methyl}-6-[(E)-2-(3-fluoro-6-methoxy-quinolin-4-yl)-vinyl]-tetrahydro-pyran-3-y}-carbamic acid tert-butyl ester:

Starting from intermediate 28.ii (2.65 g, 5.12 mmol), the title amide was obtained as a yellowish foam, using respectively the procedures of Example 20, step 20.i (acetonide deprotection, 86% yield), step 20.ii (acid formation, 56% yield) and step 20.iii (amide formation, 53% yield). All intermediates were purified by chromatography over SiO₂. The title amide was obtained as a 3-1 E-Z mixture.
MS (ESI, m/z): 618.0 [M+H⁺].

### 29.ii. {(2R,3R,6R)-2-{[2-(tert-butyl-dimethyl-silanyloxy)-ethylcarbamoy]-methyl}-6-[(E)-2-(3-fluoro-6-methoxy-quinolin-4-yl)-ethyl]-tetrahydro-pyran-3-yl}-carbamic acid tert-butyl ester:

Starting from intermediate 29.i (0.388 g, 0.62 mmol), the title alkane (0.365 g, 93% yield) was obtained as a white foam, using the procedure of Example 1, step 1.vii. The compound was purified by chromatography over SiO₂ using DCM-MeOH 19-1 containing 0.5% aq. NH₄OH) as eluent.
MS (ESI, m/z): 620.7 [M+H⁺].

### 29.iii. (2R,3R,6R)-2-{3-amino-6-[2-(3-fluoro-6-methoxy-quinolin-4-yl)-ethyl]-tetrahydro-pyran-2-yl}-N-(2-hydroxy-ethyl)-acetamide:

The title amine (0.191 g, 81% yield) was obtained as a yellowish gum, starting from intermediate 29.ii (0.361 g, 0.8 mmol) and using the procedure of Example 1, step 1.viii. The compound was triturated in ether.
MS (ESI, m/z): 406.0 [M+H⁺].

### 29.iv. 2-{(2R,3R,6R)-3-(E)-3-(2,5-difluoro-phenyl)-allylamino]-6-[2-(3-fluoro-6-methoxy-quinolin-4-yl)-ethyl]-tetrahydro-pyran-2-yl}-N-(2-hydroxy-ethyl)-acetamide:

Starting from intermediate 29.iii (0.191 g, 0.47 mmol) and (*E*)-3-(2,5-difluoro-phenyl)-propenal (0.087 g, 1.1eq.), the title compound (0.144 g, 55% yield) was prepared as a white foam using the procedure of Example 3. The compound was purified by chromatography using DCM-MeOH 93-7 containing 1% aq. NH₄OH as an eluent.
¹H NMR (d6-DMSO) δ: 8.66 (d, J = 1.0 Hz, 1H); 7.93 (d, J = 9.2 Hz, 1H); 7.93 (overlapped m, 1H); 7.49 (m, 1H); 7.42-7.35 (m, 2H); 7.24 (m, 1H); 7.11 (m 1H); 6.62 (d, J = 16.0 Hz, 1H); 6.50 m(td, J = 5.4, 16.0 Hz, 1H); 4.59 (t, J = 5.4 Hz, 1H); 4.41 (m, 1H); 3.92 (s, 3H); 3.80 (m, 1H); 3.43-3.32 (m, 3H); 3.15-3.05 (m, 3H); 2.95 (m, 1H); 2.75 (m, 1H); 2.65 (dd, J = 10.3, 14.1 Hz, 1H); 2.34 (dd, J = 3.8, 14.1 Hz, 1H); 1.84 (brs, 1H); 1.76-1.60 (m, 3H); 1.43 (m, 1H); 1.29-1.15 (m, 3H).
MS (ESI, m/z): 557.9 [M+H⁺].

### Example 30: 2-{(2R,3R,6S)-3-[(E)-3-(2,5-difluoro-phenyl)-allylamino]-6-[(E)-2-(3-fluoro-6-methoxy-quinolin-4-yl)-vinyl]-tetrahydro-pyran-2-yl}-N,N-dimethyl-acetamide:

Starting from the compound of Example 28 (1.58 g, 3 mmol), the title compound (0.037 g) was prepared as an off-white solid using the procedures of Example 6, step 6.i (Boc formation, 74% yield), step 6.ii (aldehyde formation, 82% yield), step 6.iii (acid formation, 98% yield), Example 22, step 22.i (amide formation, 80% yield) and step 22.ii (deprotection, 53% yield). The compound was purified after the last step by chromatography over SiO₂ using as eluent DCM-MeOH 19-1 containing 0.5% aq. NH₄OH,
¹H NMR (d6-DMSO) δ: 8.72 (d, J = 1.2 Hz, 1H); 7.95 (d, J = 9.2 Hz, 1H); 7.51-7.45 (m, 2H); 7.40 (dd, J = 2.6, 9.2 Hz, 1H); 7.23 (m, 1H); 7.15-7.06 (m, 2H); 6.63 (d, J = 16.3 Hz, 1H); 6.55-6.46 (m, 2H); 4.55-4.50 (m, 2H); 3.94 (s, 3H); 4.44 (dd, J = 5.7, 15.7 Hz, 1H); 3.29 (dd, J = 5.7, 15.7 Hz, 1H); 3.06 (s, 3H); 2.83 (s, 3H); 2.77 (m, 2H); 2.64 (dd, J = 4.2, 15.8 Hz, 1H); 2.05 (m, 1H); 1.74-1.66 (m, 2H); 1.55 (m, 1H); 1.26 (br s, 1H).
MS (ESI, m/z): 540.0 [M+H⁺].

### Example 31: (2R,3R,6S)-6-({2-[(2RS)-2,3-dihydroxy-propyl]-6-[(E)-(6-methoxy-[1,5]naphthyridin-4-yl)-vinyl]-tetrahydro-pyran-3-ylamino}-methyl)-4H-pyrido[3,2-b][1,4]thiazin-3-one:

To a solution of intermediate 17.i (1.35 g, 3.75 mmol) in 1,2-DCE (30 ml) and MeOH (10 ml) were added molecular sieves 3Å (10 g) and 3-oxo-3,4-dihydro-2*H-*pyrido[3,2-*b*][1,4]thiazine-6-carbaldehyde (0.81 g, 1.1 eq.). The mixture was heated at 50°C for 13 h. The reaction mixture was cooled to 0°C and NaBH₄ (1 g) was added. The reaction proceeded for 1 h at the same temperature. The reaction mixture was filtered and the solid was washed with DCM-MeOH (9-1, 200ml). The org. layer was washed with saturated NaHCO₃ (200 ml) and the org. layer was dried over Na₂SO₄, filtered and concentrated to dryness. The residue was chromatographed (DCM-MeOH 93-7 containing 0.7% aq. NH₄OH) to afford the title compound (1.65 g, 81% yield) as a colourless foam. The compound was obtained as a 2-1 mixture of epimers.
MS (ESI, m/z): 538.3 [M+H⁺].

### Example 32: (2R,3R,6S)-6-[(2-hydroxy-ethylcarbamoyl)-methyl]-5-[(3-oxo-3,4-dihydro-2H-pyrido[3,2-b][1,4]thiazin-6-ylmethyl)-amino]-tetrahydro-pyran-2-carboxylic acid (6-methoxy-[1,5]naphthyridin-4-yl)-amide:

Starting from Example 15 (1.20 g, 2.16 mmol), the title compound (0.037 g) was obtained as an off-white solid using the procedures of Example 6, step 6.i (Boc formation, 67% yield), step 6.ii (aldehyde formation, 99% yield), step 6.iii (acid formation, 61% yield), Example 20, step 20.iii (amide formation, 87% yield) and Example 22, step 22.ii (deprotection, 83% yield). The title compound was purified by trituration in ether after the last step.
MS (ESI, m/z): 582.2 [M+H⁺].

### Pharmacological properties of the invention compounds

### In vitro assay

### Experimental method:

These assays have been performed following the description given in "Methods for dilution Antimicrobial Susceptibility Tests for Bacteria that Grow Aerobically, 4th ed.; Approved standard: NCCLS Document M7-A4; National Committee for Clinical Laboratory Standards: Villanova, PA, USA, 1997". Minimal inhibitory concentrations (MICs; mg/l) were determined in cation-adjusted Mueller-Hinton Broth (BBL) by a microdilution method following NCCLS guidelines (National Committee for Clinical Laboratory Standards. Methods for Dilution Antimicrobial Susceptibility). The pH of the test medium was 7.2-7.3.

### Results:

All Example compounds were tested against several Gram positive and Gram negative bacteria.

Typical antibacterial test results are given in the table hereafter (MIC in mg/l).

| **Example No.** | ***S. aureus* A798** | ***S. Pneumoniae* 49619** | ***M. catarrhalis* A894** |
|---|---|---|---|
| 1 | <=.031 | 1 | 0.25 |
| 2 | 0.25 | 1 | 0.25 |
| 4 | 0.25 | 0.063 | <=.031 |

## Claims

1. A compound of formula I 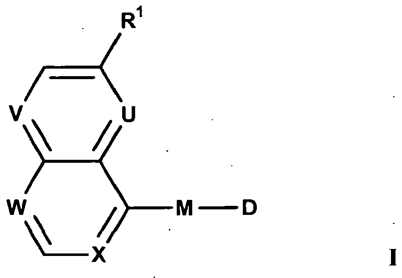 wherein
R¹ represents alkyl, alkoxy, haloalkoxy, halogen or cyano;
one or two of U, V, W and X represent(s) N and the remaining each represent CH or, in the case of X, may also represent CR^{a};
R^{a} represents halogen;
M is 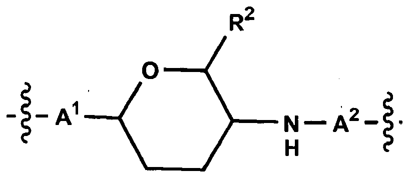 wherein
A¹ represents NHCO, CH₂CH₂, CH=CH, CH(OH)CH₂, CH₂CH(OH), CH(OH)CH(OH) or OCH₂;
A² represents CH₂, CO, CH₂CH=CH or COCH=CH;
R² represents carboxy, carbamoyl, hydroxycarbamoyl, cyano or -CH₂R³,
R³ representing hydroxycarbamoyl, (C₂-C₄)alkynylmethoxycarbamoyl, (C₁-C₃)dialkylcarbamoyl, cycloalkylcarbamoyl, [(C₂-C₅)hydroxyalkyl]carbamoyl, (arylalkyl)carbamoyl, cyano, (C₁-C₅)alkylaminomethyl, (C₁-C₅)dialkylaminomethyl, (C₁-C₃)alkoxyimino, hydroxyimino, 5-membered heteroaryl, or heteroarylalkyl wherein the heteroaryl is a 5-membered heteroaryl and the alkyl is a (C₁-C₄)alkyl,
or R³ representing a radical =NR⁴R⁵ wherein R⁴ and R⁵, taken together with the nitrogen atom that carries them, form a 5- or 6-membered heterocyclic ring, the members being used to complete said heterocyclic ring being chosen independently from -CH₂-, -O- and -S-, it being understood that only one member selected from -O- and -S- may be present in said heterocyclic ring,
or also R³ representing a radical -CH(OH)R⁶,
R⁶ representing hydroxymethyl, (C₁-C₃)alkylaminomethyl, (C₁-C₃)dialkylaminomethyl,
phenylaminomethyl wherein the phenyl group may be substituted once by a member of the group consisting of halogen, alkoxy and carboxy, heteroarylaminomethyl wherein the heteroaryl is a 5-membered heteroaryl, benzylaminomethyl wherein the phenyl of the benzyl group may be substituted once by a member of the group consisting of halogen, alkoxy and carboxy, or a radical -CH₂-NR⁷R⁸ wherein R⁷ and R⁸, taken together with the nitrogen atom that carries them, form a 5- or 6-membered heterocyclic ring, the members being used to complete said heterocyclic ring being chosen independently from -CH₂-, -O- and -S-, it being understood that only one member selected from -O- and -S- may be present in said heterocyclic ring,
or furthermore R³ representing a radical -CO-NR^{x}R^{y} wherein the group -NR^{x}R^{y} is selected from pyrrolidin-1-yl, piperidin-1-yl, morpholin-4-yl, thiomorpholin-4-yl, (3*R*)-3-hydroxypyrrolidin-1-yl, (3*S*)-3-hydroxy-pyrrolidin-1-yl and 4-hydroxy-piperidin-1-yl;
D represents aryl or heteroaryl; wherein any aryl group may be substituted with one, two or more substituents independently selected from halogen, alkyl, alkoxy, trifluoromethyl and trifluoromethoxy : wherein any heteroaryl group may be substituted on its aromatic ring (s) with one two or more of halogen, alkyl and alkoxy;
or a salt thereof.

2. A compound of formula I according to claim 1, wherein R¹ represents methoxy or fluorine;
or a salt thereof.

3. A compound of formula I according to claim 1, wherein one or two of U, V, W and X represent(s) N, and the remaining represent each CH, or, in the case of X, may also represent CR^{a}, R^{a} representing fluorine;
or a salt thereof.

4. A compound of formula I according to claim 1, which is also a compound of formula I_{Ar} 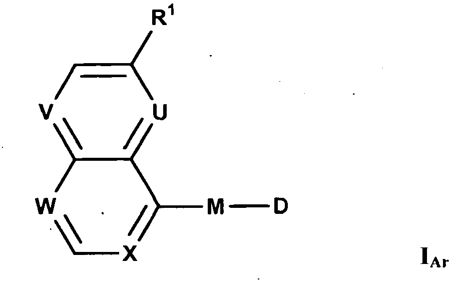 wherein:
R¹, U, V, W, X and M are as defined in formula I of claim 1, and
D represents aryl;
or a salt thereof.

5. A compound of formula I according to claim 1, which is also a compound of formula I_{Het} 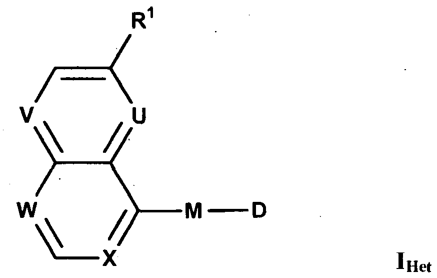 wherein:
R¹, U, V, W, X and M are as defined in formula I of claim 1, and
D represents heteroaryl;
or a salt thereof.

6. A compound of formula I_{Het} according to claim 5, wherein D is a bicyclic heteroaryl of the general formula 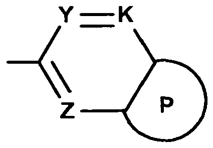 wherein:
K, Y and Z are each independently N or CR^{α};
R^{α} is hydrogen or halogen;
P is a ring selected from the group consisting of the following rings: 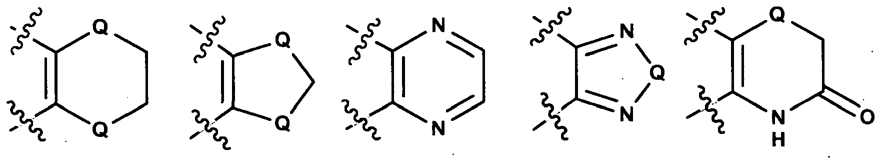
in which Q is O or S;
or a salt thereof.

7. A compound of formula I according to claim 1, wherein:
R² represents -CH₂R³,
R³ being selected from the group consisting of hydroxycarbamoyl, (C₂-C₄)alkynylmethoxycarbamoyl, cycloalkylcarbamoyl, [(C₂-C₅)hydroxyalkyl]carbamoyl, cyano, -CH(OH)R⁶ and -CO-NR^{x}R^{y}, and
R⁶ being hydroxymethyl, and
the group NR^{x}R^{y} is selected from pyrrolidin-1-yl, piperidin-1-yl, morpholin-4-yl, thiomorpholin-4-yl, (3*R*)-3-hydroxy-pyrrolidin-1-yl, (3*S*)-3-hydroxy-pyrrolidin-1-yl and 4-hydroxy-piperidin-1-yl;
or a salt thereof.

8. A compound of formula I according to claim 1, wherein A¹ represents NHCO, CH₂CH₂, CH=CH, CH(OH)CH₂, CH₂CH(OH) or OCH₂, or also, provided U is N, CH(OH)CH(OH); or a salt thereof.

9. A compound of formula I according to claim 1, wherein A² represents CH₂ or CO, or also, provided D is a non-annelated aryl or heteroaryl group, CH₂CH=CH or COCH=CH; or a salt thereof.

10. A compound of formula I according to claim 1, selected from the following compounds:
- (*E*)-3-(2,5-difluoro-phenyl)-*N*-{(2*R*,3*R*,6*R*)-2-((2*R*)-2,3-dihydroxy-propyl)-6-[2-(3-methoxy-quinolin-5-yl)-ethyl]-tetrahydro-pyran-3-yl}-acrylamide;
- (*E*)-3-(2,5-difluoro-phenyl)-*N*-{(2*R*,3*R*,6*R*)-2-((2*S*)-2,3-dihydroxy-propyl)-6-[2-(3-methoxy-quinolin-5-yl)-ethyl]-tetrahydro-pyran-3-yl}-acrylamide;
- 3-oxo-3,4-dihydro-2*H*-pyrido[3,2-*b*][1,4]thiazine-6-carboxylic acid {(2*R*,3*R*,6*R*)-2-((2*R*)-2,3-dihydroxy-propyl)-6-[2-(3-methoxy-quinolin-5-yl)-ethyl]-tetrahydro-pyran-3-yl}-amide;
- 3-oxo-3,4-dihydro-2*H*-pyrido[3,2-*b*][1,4]thiazine-6-carboxylic acid (2*R*,3*R*,6*R*)-2-((2S)-2,3-dihydroxy-propyl)-6-[2-(3-methoxy-quinolin-5-yl)-ethyl]-tetrahydro-pyran-3-yl}-amide;
- 6-({(2*R*,3*R*,6*R*)-2-((2*R*)-2,3-dihydroxy-propyl)-6-[2-(3-methoxy-quinolin-5-yl)-ethyl]-tetrahydro-pyran-3-ylamino}-methyl)-4*H*-pyrido[3,2-*b*][1,4]thiazin-3-one;
- 6-({(2*R*,3*R*,6*R*)-2-((2*S*)-2,3-dihydroxy-propyl)-6-[2-(3-methoxy-quinolin-5-yl)-ethyl]-tetrahydro-pyran-3-ylamino}-methyl)-4*H*-pyrido[3,2-*b*][1,4]thiazin-3-one;
- 6-({(2*R*,3*R*,6*R*)-2-((2*R*)-2,3-dihydroxy-propyl)-6-[2-(6-methoxy-[1,5]naphthyridin-4-yl)-ethyl]-tetrahydro-pyran-3-ylamino}-methyl)-4*H*-pyrido[3,2-*b*][1,4]thiazin-3-one;
- 6-({(2*R*,3*R*,6*R*)-2-((2*S*)-2,3-dihydroxy-propyl)-6-[2-(6-methoxy-[1,5]naphthyridin-4-yl)-ethyl]-tetrahydro-pyran-3-ylamino}-methyl)-4*H-*pyrido[3,2-*b*][1,4]thiazin-3-one;
- (2*R*)-3- {(2*R*,3*R*,6*R*)-3-[(*E*)-3-(2,5-difluoro-phenyl)-allylamino]-6-[2-(6-methoxy-[1,5]naphthyridin-4-yl)-ethyl]-tetrahydro-pyran-2-yl}-propane-1,2-diol;
- (2*S*)-3-{(2*R*,3*R*,6*R*)-3-[(*E*)-3-(2,5-difluoro-phenyl)-allylamino]-6-[2-(6-methoxy-[1,5]naphthyridin-4-yl)-ethyl]-tetrahydro-pyran-2-yl-propane-1,2-diol;
- 2-{(2*R*,3*R*,6*R*)-3-[(*E*)-3-(2,5-difluoro-phenyl)-allylamino]-6-[2-(6-methoxy-[1,5]naphthyridin-4-yl)-ethyl]-tetrahydro-pyran-2-yl}-*N*-hydroxy-acetamide;
- (*E*)-3-(2,5-difluoro-phenyl)-*N*{(2*R*,3*R*,6*R*)-2-((2*R*)-2,3-dihydroxy-propyl)-6-[2-(6-methoxy-[1,5]naphthyridin-4-yl)-ethyl]-tetrahydro-pyran-3-yl}-acrylamide;
- (*E*)-3-(2,5-difluoro-phenyl)-*N*-{(2*R*,3*R*,6*R*)-2-((2*S*)-2,3-dihydroxy-propyl)-6-[2-(6-methoxy-[1,5]naphthyridin-4-yl)-ethyl]-tetrahydro-pyran-3-yl-acrylamide;
- (2*R*)*-*3*-*{(2*R*,3*R*,6*S*)-3-[(*E*)-3-(2,5-difluoro-phenyl)-allylamino]-6-[(*S*)-1-hydroxy-2-(6-methoxy-[1,5]naphthyridin-4-yl)-ethyl]-tetrahydro-pyran-2-yl}-propane-1,2-diol;
- (2*S*)-3-{(2*R*,3*R*,6*S*)-3-[(*E*)-3-(2,5-difluoro-phenyl)-allylamino]-6-[(*S*)-1-hydroxy-2-(6-methoxy-[1,5]naphthyridin-4-yl)-ethyl]-tetrahydro-pyran-2-yl}-propane-1,2-diol;
- (2*R*)-3-{(2*R*,3*R*,6*R*)-3-[(*E*)-3-(2,5-difluoro-phenyl)-allylamino]-6-[2-(6-methoxy-quinolin-4-yl)-ethyl]-tetrahydro-pyran-2-yl}-propane-1,2-diol;
- (2*S*)-3-{(2*R,*3*R,*6*R*)-3-[(*E*)-3-(2,5-difluoro-phenyl)-allylamino]-6-[2-(6-methoxy-quinolin-4-yl)-ethyl]-tetrahydro-pyran-2-yl}-propane-1,2-diol;
- {(2*R*,3*R*,6*R*)-3-[(*E*)-3-(2,5-difluoro-phenyl)-allylamino]-6-[2-(6-methoxy-quinolin-4-yl)-ethyl]-tetrahydro-pyran-2-yl}-acetonitrile;
- (*E*)-3-(2,5-difluoro-phenyl)-*N-*{(2*R,*3*R,*6*S*)-2*-*((2*R*)-2,3-dihydroxy-propyl)-6-[(*E*)-2-(3-fluoro-6-methoxy-[1,5]naphthyridin-4-yl)-vinyl]-tetrahydro-pyran-3-yl} acrylamide;
- (*E*)-3-(2,5-difluoro-phenyl)-*N-*{(2*R*,3*R*,6*S*)-2-((2*S*)-2,3-dihydroxy-propyl)-6-[(*E*)-2-(3-fluoro-6-methoxy-[1,5]naphthyridin-4-yl)-vinyl]-tetrahydro-pyran-3-yl} acrylamide;
- (2*R*)-3-((2*R*,3*R*,6*S*)-3-[(*E*)-3-(2,5-difluoro-phenyl)-allylamino]-6-[(*E*)-2-(3-fluoro-6-methoxy-[1,5]naphthyridin-4-yl)-vinyl]-tetrahydro-pyran-2-yl)-propane-1,2-diol;
- (2*S*)-3-{(2*R*,3*R*,6*S*)-3-[(*E*)-3-(2,5-difluoro-phenyl)-allylamino]-6-[(*E*)-2-(3-fluoro-6-methoxy-[1,5]naphthyridin-4-yl)-vinyl]-tetrahydro-pyran-2-yl} -propane-1,2-diol;
- (*E*)-3-(2,5-difluoro-phenyl)-*N-*[(2*R*,3*R*,6*S*)-2-((2*R*)-2,3-dihydroxy-propyl)-6-(6-methoxy-quinolin-4-yloxymethyl)-tetrahydro-pyran-3-yl]-acrylamide;
- (*E*)-3-(2,5-difluoro-phenyl)-*N*-[(2*R*,3*R*,6*S*)-2-((2*S*)-2,3-dihydroxy-propyl)-6-(6-methoxy-quinolin-4-yloxymethyl)-tetrahydro-pyran-3-yl]-acrylamide;
- (2*S*,5*R*,6*R*)-5-[(*E*)-3-(2,5-difluoro-phenyl)-allylamino]-6-[(2*R*)-2,3-dihydroxy-propyl]-tetrahydro-pyran-2-carboxylic acid (6-methoxy-[1,5]naphthyridin-4-yl)-amide;
- (2*S*,5*R*,6*R*)-5-[(*E*)-3-(2,5-difluoro-phenyl)-allylamino]-6-[(2*S*)-2,3-dihydroxy-propyl]-tetrahydro-pyran-2-carboxylic acid (6-methoxy-[1,5]naphthyridin-4-yl)-amide;
- (2*S*,5*R*,6*R*)-6-[(2*R*)-2,3-dihydroxy-propyl]-5-[(3-oxo-3,4-dihydro-2*H*-pyrido[3,2-*b*][1,4]thiazin-6-ylmethyl)-amino]-tetrahydro-pyran-2-carboxylic acid (6-methoxy-[1,5]naphthyridin-4-yl)-amide;
- (2*S*,5*R*,6*R*)-6-[(2*S*)-2,3-dihydroxy-propyl]-5-[(3-oxo-3,4-dihydro-2*H*-pyrido[3,2-*b*][1,4]thiazin-6-ylmethyl)-amino]-tetrahydro-pyran-2-carboxylic acid (6-methoxy-[1,5]naphthyridin-4-yl)-amide;
- (2*S*,5*R*,6*R*)-5-[(*E*)-3-(2,5-difluoro-phenyl)-acryloylamino]-6-[(2*R*)-2,3-dihydroxypropyl]-tetrahydro-pyran-2-carboxylic acid (6-methoxy-[1,5]naphthyridin-4-yl)-amide;
- (2*S*,5*R*,6*R*)-5-[(*E*)-3-(2,5-difluoro-phenyl)-acryloylamino]-6-[(2*S*)-2,3-dihydroxy-propyl]-tetrahydro-pyran-2-carboxylic acid (6-methoxy-[1,5]naphthyridin-4-yl)-amide;
- 3-oxo-3,4-dihydro-2*H*-pyrido[3,2-*b*][1,4]thiazine-6-carboxylic acid {(2*R*,3*R*,6*S*)-2-((2*R*)-2,3-dihydroxy-propyl)-6-[(*E*)-2-(6-methoxy-[1,5]naphthyridin-4-yl)-vinyl]-tetrahydro-pyran-3-yl)-amide;
- 3-oxo-3,4-dihydro-2*H*-pyrido[3,2-*b*][1,4]thiazine-6-carboxylic acid {(2*R*,3*R*,6*S*)-2-((2*S*)-2,3-dihydroxy-propyl)-6-[(*E*)-2-(6-methoxy-[1,5]naphthyridin-4-yl)-vinyl]-tetrahydro-pyran-3-yl}-amide;
- (2*R*)-3-((2*R*,3*R*,6*S*)-3-[(*E*)-3-(2,5-difluoro-phenyl)-allylamino]-6-[(*E*)-2-(6-methoxy-1,5]naphthyridin-4-yl)-vinyl]-tetrahydro-pyran-2-yl}-propane-1,2-diol;
- (2*S*)-3-{(2*R,*3*R,*6*S*)-3-[(*E*)-3-(2,5-difluoro-phenyl)-allylamino]-6-[(*E*)*-*2-(6-methoxy-1,5]naphthyridin-4-yl)-vinyl]-tetrahydro-pyran-2-yl}-propane-1,2-diol;
- (2*R*)-3-{2*R*,3*R*,6*R*)-3-[(*E*)-3-(2,5-difluoro-phenyl)-allylamino]-6-[2-(6-fluoro-quinolin-4-yl)-ethyl]-tetrahydro-pyran-2-yl}-propane-1,2-diol;
- (2*S*)-3-{2*R*,3*R*,6*R*)-3-[(*E*)-3-(2,5-difluoro-phenyl)-allylamino]-6-[2-(6-fluoro-quinolin-4-yl)-ethyl]-tetrahydro-pyran-2-yl}-propane-1,2-diol;
- 2-{(2*R*,3*R*,6*S*)-3-[(*E*)-3-(2,5-difluoro-phenyl)-allylamino]-6-[(*E*)-2-(6-methoxy-[1,5]naphthyridin-4-yl)-vinyl]-tetrahydro-pyran-2-yl}-*N*-(2-hydroxy-ethyl)-acetamide;
- 2-{(2*R*,3*R*,6*S*)-3-[(*E*)-3-(2,5-difluoro-phenyl)-allylamino]-6-[(1*R*,2*R*)-1,2-dihydroxy-2-(6-methoxy-[1,5]naphthyridin-4-yl)-ethyl]-tetrahydro-pyran-2-yl}-*N*-(2-hydroxy-ethyl)-acetamide;
- 2-{(2*R*,3*R*,6*S*)-3-[(*E*)-3-(2,5-difluoro-phenyl)-allylamino]-6-[(1*S*)-1-hydroxy-2-(6-methoxy-[1,5]naphthyridin-4-yl)-ethyl]-tetrahydro-pyran-2-yl}-*N*-(2-hydroxy-ethyl)-acetamide;
- 2-{(2*R*,3*R*,6*S*)-3-[(*E*)-3-(2,5-difluoro-phenyl)-allylamino]-6-[(1*S*)-1-hydroxy-2-(6-methoxy-[1,5]naphthyridin-4-yl)-ethyl]-tetrahydro-pyran-2-yl}-1-morpholin-4-yl-ethanone;
- *N*-cyclopropyl-2-{(2*R*,3*R*,6*S*)-3-[(*E*)-3-(2,5-difluoro-phenyl)-allylamino]-6-[(1*S*)-1-hydroxy-2-(6-methoxy-[1,5]naphthyridin-4-yl)-ethyl]-tetrahydro-pyran-2-yl}-acetamide;
- 2-{(2*R*,3*R*,6S)-3-[(*E*)-3-(2,5-difluoro-phenyl)-allylamino]-6-[(*1*S)-1-hydroxy-2-(6-methoxy-[1,5]naphthyridin-4-yl)-ethyl]-tetrahydro-pyran-2-yl}-*N,N*-dimethylacetamide;
- 2-{(2*R*,3*R*,6*S*)-3-[(*E*)-3-(2,5-difluoro-phenyl)-allylamino]-6-[(1*S*)-1-hydroxy-2-(6-methoxy-[1,5]naphthyridin-4-yl)-ethyl]-tetrahydro-pyran-2-yl}-1-(4-hydroxy-piperidin-1-yl)-ethanone;
- 2-{(2*R,*3*R,*6*S)-*3*-*[(*E*)-3-(2,5-difluoro-phenyl)-allylamino]-6-[(1*S*)-1-hydroxy-2-(6-methoxy-[1,5]naphthyridin-4-yl)-ethyl]-tetrahydro-pyran-2-yl}-*N*-prop-2-ynyloxy-acetamide;
- 2-{(2*R*,3*R*,6*R*)-3-[(*E*)-3-(2,5-difluoro-phenyl)-allylamino]-6-[2-(3-fluoro-6-methoxy-quinolin-4-yl)-ethyl]-tetrahydro-pyran-2-yl}-*N*-(2-hydroxy-ethyl)-acetamide;
- 2-{2*R*,3*R*,6*S*)-3-[(*E*)-3-(2,5-difluoro-phenyl)-allylamino]-6-[(*E*)-2-(3-fluoro-6-methoxy-quinolin-4-yl)-vinyl]-tetrahydro-pyran-2-yl}-*N,N*-dimethyl-acetamide;
- (2*R*,3*R*,6*S*)-6-({2-[(2*R*)-2,3-dihydroxy-propyl]-6-[(*E*)-(6-methoxy-[1,5]naphthyridin-4-yl)-vinyl]-tetrahydro-pyran-3-ylamino}-methyl)-4*H*-pyrido[3,2-*b*][1,4]thiazin-3-one;
- (2*R*,3*R*,6*S*)-6-({2-[(2*S*)-2,3-dihydroxy-propyl]-6-[(*E*)-(6-methoxy-[1,5]naphthyridin-4-yl)-vinyl]-tetrahydro-pyran-3-ylamino}-methyl)-4*H*-pyrido[3,2-*b*][1,4]thiazin-3-one;
- (2*R*,3*R*,6*S*)-6-[(2-hydroxy-ethylcarbamoyl)-methyl]-5-[(3-oxo-3,4-dihydro-2*H*-pyrido[3,2-*b*][1,4]thiazin-6-ylmethyl)-amino]-tetrahydro-pyran-2-carboxylic acid (6-methoxy-[1,5]naphthyridin-4-yl)-amide;
or a salt of one of these compounds.

11. A compound according to claim 10, which is selected from the following compounds:
- (*E*)-3-(2,5-difluoro-phenyl)-*N*-{(2*R*,3*R*,6*R*)-2-((2*R*)-2,3-dihydroxy-propyl)-6-[2-(3-methoxy-quinolin-5-yl)-ethyl]-tetrahydro-pyran-3-yl-acrylamide;
- (*E*)-3-(2,5-difluoro-phenyl)-*N*-{(2*R*,3*R*,6*R*)-2-((2*S*)-2,3-dihydroxy-propyl)-6-[2-(3-methoxy-quinolin-5-yl)-ethyl]-tetrahydro-pyran-3-yl}-acrylamide;
- 3-oxo-3,4-dihydro-2*H*-pyrido[3,2-*b*][1,4]thiazine-6-carboxylicacid {(2*R*,3*R*,6*R*)-2-((2*R*)-2,3-dihydroxy-propyl)-6-[2-(3-methoxy-quinolin-5-yl)-ethyl]-tetrahydro-pyran-3-yl-amide;
- 3-oxo-3,4-dihydro-2*H*-pyrido[3,2-*b*][1,4]thiazine-6-carboxylic acid {(2*R*,3*R*,6*R*)-2-((2,*S*)-2,3-dihydroxy-propyl)-6-[2-(3-methoxy-quinolin-5-yl)-ethyl]-tetrahydro-pyran-3-yl -amide;
- 6-({(2*R*,3*R*,6*R*)-2-((2*R*)-2,3-dihydroxy-propyl)-6-[2-(3-methoxy-quinolin-5-yl)-ethyl]-tetrahydro-pyran-3-ylamino}-methyl)-4*H*-pyrido[3,2-*b*][1,4]thiazin-3-one;
- 6-({(2*R*,3*R*,6*R*)-2-((2*S*)-2,3-dihydroxy-propyl)-6-[2-(3-methoxy-quinolin-5-yl)-ethyl]-tetrahydro-pyran-3-ylamino}-methyl)-4*H*-pyrido[3,2-*b*][1,4]thiazin-3-one;
- 6-({(2*R*,3*R*,6*R*)-2-((2*R*)-2,3-dihydroxy-propyl)-6-[2-(6-methoxy-[1,5]naphthyridin-4-yl)-ethyl]-tetrahydro-pyran-3-ylamino}-methyl)-4*H*-pyrido[3,2-*b*][1,4]thiazin-3-one;
- 6-({(2*R*,3*R*,6*R*)-2-((2*S*)-2,3-dihydroxy-propyl)-6-[2-(6-methoxy-[1,5]naphthyridin-4-yl)-ethyl]-tetrahydro-pyran-3-ylamino}-methyl)-4*H*-pyrido[3,2-*b*][1,4]thiazin-3-one;
- (2*R*)-3-{(2*R*,3*R*,6*R*)-3-[(*E*)-3-(2,5-difluoro-phenyl)-allylamino]-6-[2-(6-methoxy-[1,5]naphthyridin-4-yl)-ethyl]-tetrahydro-pyran-2-yl}-propane-1,2-diol;
- (2*S*)-3-{(2*R*,3*R*,6*R*)-3-[(*E*)-3-(2,5-difluoro-phenyl)-allylamino]-6-[2-(6-methoxy-[1,5]naphthyridin-4-yl)-ethyl]-tetrahydro-pyran-2-yl}-propane-1,2-diol;
- 2-{(2*R*,3*R*,6*R*)-3-[(*E*)-3-(2,5-difluoro-phenyl)-allylamino]-6-[2-(6-methoxy-[1,5]naphthyridin-4-yl)-ethyl]-tetrahydro-pyran-2-yl}-*N*-hydroxy-acetamide;
- (*E*)-3-(2,5-difluoro-phenyl)-*N*-{(2*R*,3*R*,6*R*)-2-((2*R*)-2,3-dihydroxy-propyl)-6-[2-(6-methoxy-[1,5]naphthyridin-4-yl)-ethyl]-tetrahydro-pyran-3-yl}-acrylamide;
- (*E*)-3-(2,5-difluoro-phenyl)-*N*-{(2*R*,3*R*,6*R*)-2-((2*S*)-2,3-dihydroxy-propyl)-6-[2-(6-methoxy-[1,5]naphthyridin-4-yl)-ethyl]-tetrahydro-pyran-3-yl}-acrylamide;
- (2*R*)-3-{(2*R*,3*R*,6*S*)-3-[(*E*)-3-(2,5-difluoro-phenyl)-allylamino]-6-[(*S*)-1-hydroxy-2-(6-methoxy-[1,5]naphthyridin-4-yl)-ethyl]-tetrahydro-pyran-2-yl}-propane-1,2-diol;
- (2*S*)-3-{(2*R*,3*R*,6*S*)-3-[(*E*)-3-(2,5-difluoro-phenyl)-allylamino]-6-[(*S*)-1-hydroxy-2-(6-methoxy-[1,5]naphthyridin-4-yl)-ethyl]-tetrahydro-pyran-2-yl}-propane-1,2-diol;
- (2*R*)-3-{(2*R*,3*R*,6*R*)-3-[(*E*)-3-(2,5-difluoro-phenyl)-allylamino]-6-[2-(6-methoxy-quinolin-4-yl)-ethyl]-tetrahydro-pyran-2-yl}-propane-1,2-diol;
- (2*S*)-3-{(2*R*,3*R*,6*R*)-3-[(*E*)-3-(2,5-difluoro-phenyl)-allylamino]-6-[2-(6-methoxy-quinolin-4-yl)-ethyl]-tetrahydro-pyran-2-yl}-propane-1,2-diol;
- {(2*R*,3*R*,6*R*)-3-[(*E*)-3-(2,5-difluoro-phenyl)-allylamino]-6-[2-(6-methoxy-quinolin-4-yl)-ethyl]-tetrahydro-pyran-2-yl}-acetonitrile;
- (*E*)-3-(2,5-difluoro-phenyl)-*N*-{(2*R*,3*R*,6*S*)-2-((2*R*)-2,3-dihydroxy-propyl)-6-[(*E*)-2-(3-fluoro-6-methoxy-[1,5]naphthyridin-4-yl)-vinyl]-tetrahydro-pyran-3-yl} acrylamide;
- (*E*)-3-(2,5-difluoro-phenyl)-*N*-{(2*R*,3*R*,6*S*)-2-((2*S*)-2,3-dihydroxy-propyl)-6-[(*E*)-2-(3-fluoro-6-methoxy-[1,5]naphthyridin-4-yl)-vinyl]-tetrahydro-pyran-3-yl} acrylamide;
- (2*R*)-3-{(2*R*,3*R*,6*S*)-3-[(*E*)-3-(2,5-difluoro-phenyl)-allylamino]-6-[(*E*)-2-(3-fluoro-6-methoxy-[1,5]naphthyridin-4-yl)-vinyl]-tetrahydro-pyran-2-yl}-propane-1,2-diol;
- (2*S*)-3-{(2*R*,3*R*,6*S*)-3-[(*E*)-3-(2,5-difluoro-phenyl)-allylamino]-6-[(*E*)-2-(3-fluoro-6-methoxy-[1,5]naphthyridin-4-yl)-vinyl]-tetrahydro-pyran-2-yl}-propane-1,2-diol;
- (*E*)-3-(2,5-difluoro-phenyl)-*N*-[(2*R*,3*R*,6*S*)-2-((2*R*)-2,3-dihydroxy-propyl)-6-(6-methoxy-quinolin-4-yloxymethyl)-tetrahydro-pyran-3-yl]-acrylamide;
- (*E*)-3-(2,5-difluoro-phenyl)-*N*-[(2*R*,3*R*,6*S*)-2-((2*S*)-2,3-dihydroxy-propyl)-6-(6-methoxy-quinolin-4-yloxymethyl)-tetrahydro-pyran-3-yl]-acrylamide;
- (2*S*,5*R*,6*R*)-5-[(*E*)-3-(2,5-difluoro-phenyl)-allylamino]-6-[(2*R*)-2,3-dihydroxy-propyl]-tetrahydro-pyran-2-carboxylic acid (6-methoxy-[1,5]naphthyridin-4-yl)-amide;
- (2*S*,5*R*,6*R*)-5-[(*E*)-3-(2,5-difluoro-phenyl)-allylamino]-6-[(2*S*)-2,3-dihydroxy-propyl]-tetrahydro-pyran-2-carboxylic acid (6-methoxy-[1,5]naphthyridin-4-yl)-amide;
- (*2S*,*5R*,*6R*)-6-[(*2R*)-2,3-dihydroxy-propyl]-5-[(3-oxo-3,4-dihydro-2*H*-pyrido[3,2-*b*][1,4]thiazin-6-ylmethyl)-amino]-tetrahydro-pyran-2-carboxylic acid (6-methoxy-[1,5]naphthyridin-4-yl)-amide;
- (*2S*,*5R*,*6R*)-6-[(*2S*)-2,3-dihydroxy-propyl]-5-[(3-oxo-3,4-dihydro-2*H*-pyrido[3,2-*b*][1,4]thiazin-6-ylmethyl)-amino]-tetrahydro-pyran-2-carboxylic acid (6-methoxy-[1,5]naphthyridin-4-yl)-amide;
or a salt of one of these compounds.

12. A compound according to claim 11, which is selected from the following compounds:
- (*E*)-3-(2,5-difluoro-phenyl)-*N*-{(2*R*,3*R*,6*R*)-2-((2*R*)-2,3-dihydroxy-propyl)-6-[2-(3-methoxy-quinolin-5-yl)-ethyl]-tetrahydro-pyran-3-yl}-acrylamide;
- (*E*)-3-(2,5-difluoro-phenyl)-*N*-{(2*R*,3*R*,6*R*)-2-((2*S*)-2,3-dihydroxy-propyl)-6-[2-(3-methoxy-quinolin-5-yl)-ethyl]-tetrahydro-pyran-3-yl}-acrylamide;
- 3-oxo-3,4-dihydro-2*H*-pyrido[3,2-*b*][1,4]thiazine-6-carboxylic acid {(2*R*,3*R*,6*R*)-2-((2*R*)-2,3-dihydroxy-propyl)-6-[2-(3-methoxy-quinolin-5-yl)-ethyl]-tetrahydro-pyran-3-yl}-amide;
- 3-oxo-3,4-dihydro-2*H*-pyrido[3,2-*b*][1,4]thiazine-6-carboxylic acid {(2*R*,3*R*,6*R*)-2-((2,*S*)-2,3-dihydroxy-propyl)-6-[2-(3-methoxy-quinolin-5-yl)-ethyl]-tetrahydro-pyran-3-yl}-amide;
- 6-({(2*R*,3*R*,6*R*)-2-((2*R*)-2,3-dihydroxy-propyl)-6-[2-(3-methoxy-quinolin-5-yl)-ethyl]-tetrahydro-pyran-3-ylamino}-methyl)-4*H*-pyrido[3,2-*b*][1,4]thiazin-3-one;
- 6-({(2*R*,3*R*,6*R*)-2-((2*S*)-2,3-dihydroxy-propyl)-6-[2-(3-methoxy-quinolin-5-yl)-ethyl]-tetrahydro-pyran-3-ylamino}-methyl)-4*H*-pyrido[3,2-*b*][1,4]thiazin-3-one;
- 6-({(2*R*,3*R*,6*R*)-2-((2*R*)-2,3-dihydroxy-propyl)-6-[2-(6-methoxy-[1,5]naphthyridin-4-yl)-ethyl]-tetrahydro-pyran-3-ylamino}-methyl)-4*H*-pyrido[3,2-*b*][1,4]thiazin-3-one;
- 6-({(2*R*,3*R*,6*R*)-2-((2*S*)-2,3-dihydroxy-propyl)-6-[2-(6-methoxy-[1,5]naphthyridin-4-yl)-ethyl]-tetrahydro-pyran-3-ylamino}-methyl)-4*H*-pyrido[3,2-*b*][1,4]thiazin-3-one;
- (2*R*)-3-{(2*R*,3*R*,6*R*)-3-[(*E*)-3-(2,5-difluoro-phenyl)-allylamino]-6-[2-(6-methoxy-[1,5]naphthyridin-4-yl)-ethyl]-tetrahydro-pyran-2-yl}-propane-1,2-diol;
- (2*S*)-3-{(2*R*,3*R*,6*R*)-3-[(*E*)-3-(2,5-difluoro-phenyl)-allylamino]-6-[2-(6-methoxy-[1,5]naphthyridin-4-yl)-ethyl]-tetrahydro-pyran-2-yl}-propane-1,2-diol;
- 2-{(2*R*,3*R*,6*R*)-3-[(*E*)-3-(2,5-difluoro-phenyl)-allylamino]-6-[2-(6-methoxy-[1,5]naphthyridin-4-yl)-ethyl]-tetrahydro-pyran-2-yl}-*N*-hydroxy-acetamide;
- (*E*)-3-(2,5-difluoro-phenyl)-*N*-{(2*R*,3*R*,6*R*)-2-((2*R*)-2,3-dihydroxy-propyl)-6-[2-(6-methoxy-[1,5]naphthyridin-4-yl)-ethyl]-tetrahydro-pyran-3-yl}-acrylamide;
- (*E*)-3-(2,5-difluoro-phenyl)-*N*-{(2*R*,3*R*,6*R*)-2-((2*S*)-2,3-dihydroxy-propyl)-6-[2-(6-methoxy-[1,5]naphthyridin-4-yl)-ethyl]-tetrahydro-pyran-3-yl}-acrylamide;
- (2*R*)-3-{(2*R*,3*R*,6*S*)-3-[(*E*)-3-(2,5-difluoro-phenyl)-allylamino]-6-[(*S*)-1-hydroxy-2-(6-methoxy-[1,5]naphthyridin-4-yl)-ethyl]-tetrahydro-pyran-2-yl}-propane-1,2-diol;
- (2*S*)-3-{(2*R*,3*R*,6*S*)-3-[(*E*)-3-(2,5-difluoro-phenyl)-allylamino]-6-[(*S*)-1-hydroxy-2-(6-methoxy-[1,5]naphthyridin-4-yl)-ethyl]-tetrahydro-pyran-2-yl}-propane-1,2-diol;
- (2*R*)-3-{(2*R*,3*R*,6*R*)-3-[(*E*)-3-(2,5-difluoro-phenyl)-allylamino]-6-[2-(6-methoxy-quinolin-4-yl)-ethyl]-tetrahydro-pyran-2-yl}-propane-1,2-diol;
- (2*S*)-3-{(2*R*,3*R*,6*R*)-3-[(*E*)-3-(2,5-difluoro-phenyl)-allylamino]-6-[2-(6-methoxy-quinolin-4-yl)-ethyl]-tetrahydro-pyran-2-yl}-propane-1,2-diol;
- {(2*R*,3*R*,6*R*)-3-[(*E*)-3-(2,5-difluoro-phenyl)-allylamino]-6-[2-(6-methoxy-quinolin-4-yl)-ethyl]-tetrahydro-pyran-2-yl}-acetonitrile;
- (*E*)-3-(2,5-difluoro-phenyl)-*N*-{(2*R*,3*R*,6*S*)-2-((2*R*)-2,3-dihydroxy-propyl)-6-[(*E*)-2-(3-fluoro-6-methoxy-[1,5]naphthyridin-4-yl)-vinyl]-tetrahydro-pyran-3-yl} acrylamide;
- (*E*)-3-(2,5-difluoro-phenyl)-*N*-{(2*R*,3*R*,6*S*)-2-((2*S*)-2,3-dihydroxy-propyl)-6-[(*E*)-2-(3-fluoro-6-methoxy-[1,5]naphthyridin-4-yl)-vinyl]-tetrahydro-pyran-3-yl} acrylamide;
- (2*R*)-3-{(2*R*,3*R*,6*S*)-3-[(*E*)-3-(2,5-difluoro-phenyl)-allylamino]-6-[(*E*)-2-(3-fluoro-6-methoxy-[1,5]naphthyridin-4-yl)-vinyl]-tetrahydro-pyran-2-yl}-propane-1,2-diol;
- (2*S*)-3-{(2*R*,3*R*,6*S*)-3-[(*E*)-3-(2,5-difluoro-phenyl)-allylamino]-6-[(*E*)-2-(3-fluoro-6-methoxy-[1,5]naphthyridin-4-yl)-vinyl]-tetrahydro-pyran-2-yl}-propane-1,2-diol;
- (*E*)-3-(2,5-difluoro-phenyl)-*N*-[(2*R*,3*R*,6*S*)-2-((2*R*)-2,3-dihydroxy-propyl)-6-(6-methoxy-quinolin-4-yloxymethyl)-tetrahydro-pyran-3-yl]-acrylamide;
- (*E*)-3-(2,5-difluoro-phenyl)-*N*-[(2*R*,3*R*,6*S*)-2-((2*S*)-2,3-dihydroxy-propyl)-6-(6-methoxy-quinolin-4-yloxymethyl)-tetrahydro-pyran-3-yl]-acrylamide;
or a salt of one of these compounds.

13. As a medicament, a compound of formula I as defined in claim 1 or a pharmaceutically acceptable salt thereof.

14. A pharmaceutical composition containing, as active principle, a compound of formula I as defined in claim 1 or a pharmaceutically acceptable salt thereof, and at least one therapeutically inert excipient.

15. Use of a compound of formula I as defined in claim 1, or of a pharmaceutically acceptable salt thereof, for the manufacture of a medicament for the prevention or treatment of a bacterial infection.

## Patentansprüche

1. Verbindung der Formel I 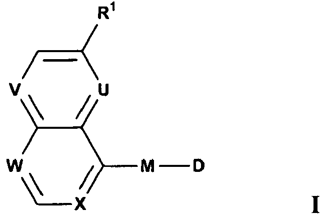 wobei
R¹ Alkyl, Alkoxy, Haloalkoxy, Halogen oder Cyano repräsentiert;
eines oder zwei von U, V, W und X N repräsentiert/repräsentieren und die Übrigen jeweils CH repräsentieren oder, im Falle von X, auch CR^{a} repräsentieren können;
R^{a} Halogen repräsentiert;
M Folgendes ist 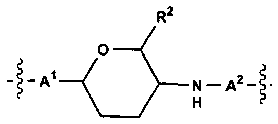 wobei
A¹ NHCO, CH₂CH₂, CH=CH, CH(OH)CH₂, CH₂CH(OH), CH(OH)CH(OH) oder OCH₂ repräsentiert;
A² CHO, CO, CH₂CH=CH oder COCH=CH repräsentiert;
R² Carboxy, Carbamoyl, Hydroxycarbamoyl, Cyano oder -CH₂R³ repräsentiert,
wobei R³ Hydroxycarbamoyl, (C₂-C₄)Alkynylmethoxycarbamoyl, (C₁-C₃)Dialkylcarbamoyl, Cycloalkylcarbamoyl, [(C₂-C₅)Hydroxyalkyl]carbamoyl, (Arylalkyl)carbamoyl, Cyano, (C₁-C₅)Alkylaminomethyl, (C₁-C₅)Dialkylaminomethyl, (C₁-C₃)Alkoxyimino, Hydroxyimino, 5-gliedriges Heteroaryl oder Heteroarylalkyl repräsentiert, wobei das Heteroaryl ein 5-gliedriges Heteroaryl ist und das Alkyl ein (C₁-C₄)Alkyl ist,
oder wobei R³ ein Radikal -NR⁴R⁵ repräsentiert, wobei R⁴ und R⁵ zusammen mit dem Stickstoffatom, das sie trägt, einen 5- oder 6-gliedrigen heterocyclischen Ring bilden, wobei die Glieder, die zur Vervollständigung des genannten heterocyclischen Rings verwendet werden, unabhängig ausgewählt sind aus -CH₂-, -O- und -S-, wobei zu verstehen ist, dass nur ein Glied, ausgewählt aus -O- und -S-, in dem genannten heterocyclischen Ring vorhanden sein kann,
oder auch wobei R³ ein Radikal -CH(OH)R⁶ repräsentiert,
wobei R⁶ Folgendes repräsentiert: Hydroxymethyl, (C₁-C₃)Alkylaminomethyl, (C₁-C₃)Dialkylaminomethyl, Phenylaminomethyl wobei die Phenylgruppe einmal substituiert sein kann durch ein Glied der Gruppe bestehend aus Halogen, Alkoxy und Carboxy, Heteroarylaminomethyl wobei das Heteroaryl ein 5-gliedriges Heteroaryl ist, Benzylaminomethyl wobei das Phenyl der Benzylgruppe einmal substituiert sein kann durch ein Glied der Gruppe bestehend aus Halogen, Alkoxy und Carboxy, oder ein Radikal -CH₂-NR⁷R⁸, wobei R⁷ und R⁸ zusammen mit dem Stickstoffatom, das sie trägt, einen 5-oder 6-gliedrigen heterocyclischen Ring bilden, wobei die Glieder, die zur Vervollständigung des genannten heterocyclischen Rings verwendet werden, unabhängig ausgewählt sind aus -CH₂-, -O- und -S-, wobei zu verstehen ist, dass nur ein Glied, ausgewählt aus -O- und -S-, in dem genannten heterocyclischen Ring vorhanden sein kann,
oder ferner wobei R³ ein Radikal -CO-NR^{x}R^{y} repräsentiert, wobei die Gruppe -NR^{x}R^{y} ausgewählt ist aus Pyrrolidin-1-yl, Piperidin-1-yl, Morpholin-4-yl, Thiomorpholin-4-yl, (3*R*)-3-Hydroxy-pyrrolidin-1-yl, (3*S*)-3-Hydroxy-pyrrolidin-1-yl und 4-Hydroxy-piperidin-1-yl;
D Aryl oder Heteroaryl repräsentiert; wobei jede beliebige Arylgruppe substituiert sein kann durch einen, zwei oder mehrere Substituenten, die unabhängig ausgewählt sind aus Halogen, Alkyl, Alkoxy, Trifluormethyl und Trifluormethoxy; wobei jede beliebige Heteroarylgruppe auf ihrem/ihren aromatischen Ring(en) durch eines, zwei oder mehrere von Halogen, Alkyl und Alkoxy substituiert sein kann;
oder ein Salz davon.

2. Verbindung der Formel I nach Anspruch 1, wobei R¹ Methoxy oder Fluor repräsentiert; oder ein Salz davon.

3. Verbindung der Formel I nach Anspruch 1, wobei eines oder zwei von U, V, W und X N repräsentiert/repräsentieren und die Übrigen jeweils CH repräsentieren oder, im Falle von X, auch CR^{a} repräsentieren können, wobei R^{a} Fluor repräsentiert;
oder ein Salz davon.

4. Verbindung der Formel I nach Anspruch 1, die auch eine Verbindung der Formel I_{Ar} ist 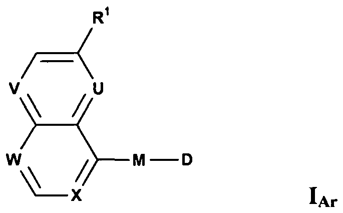 wobei:
R¹, U, V, W, X und M der Definition in Formel I aus Anspruch 1 entsprechen, und
D Aryl repräsentiert;
oder ein Salz davon.

5. Verbindung der Formel I nach Anspruch 1, die auch eine Verbindung der Formel I_{Het} ist 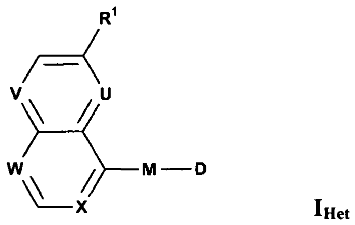 wobei:
R¹, U, V, W, X und M der Definition in Formel I aus Anspruch 1 entsprechen, und
D Heteroaryl repräsentiert;
oder ein Salz davon.

6. Verbindung der Formel I_{Het} nach Anspruch 5, wobei D ein bicyclisches Heteroaryl der folgenden allgemeinen Formel ist 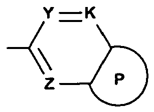 wobei:
K, Y und Z jeweils unabhängig N oder CR^{α} sind;
R^{α} Wasserstoff oder Halogen ist;
P ein Ring ist, der ausgewählt ist aus der Gruppe bestehend aus den folgenden Ringen: 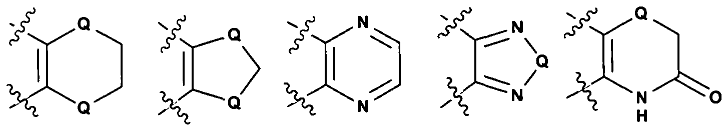
wobei Q O oder S ist;
oder ein Salz davon.

7. Verbindung der Formel I nach Anspruch 1, wobei:
R² -CH₂R³ repräsentiert,
wobei R³ ausgewählt ist aus der Gruppe bestehend aus Hydroxycarbamoyl, (C₂-C₄)Alkynylmethoxycarbamoyl, Cycloalkylcarbamoyl, [(C₂-C₅)Hydroxyalkyl]carbamoyl, Cyano, -CH(OH)R⁶ und -CO-NR^{x}R^{y}, und
wobei R⁶ Hydroxymethyl ist, und
die Gruppe -NR^{x}R^{y} ausgewählt ist aus Pyrrolidin-1-yl, Piperidin-1-yl, Morpholin-4-yl, Thiomorpholin-4-yl, (3*R*)-3-Hydroxy-pyrrolidin-1-yl, (3*S*)-3-Hydroxy-pyrrolidin-1-yl und 4-Hydroxy-piperidin-1-yl;
oder ein Salz davon.

8. Verbindung der Formel I nach Anspruch 1, wobei A¹ NHCO, CH₂CH₂, CH=CH, CH(OH)CH₂, CH₂CH(OH) oder OCH₂ repräsentiert oder auch, vorausgesetzt U ist N, CH(OH)CH(OH);
oder ein Salz davon.

9. Verbindung der Formel I nach Anspruch 1, wobei A² CH₂ oder CO repräsentiert oder auch, vorausgesetzt D ist eine nicht annelierte Aryl- oder Heteroarylgruppe, CH₂CH=CH oder COCH=CH;
oder ein Salz davon.

10. Verbindung der Formel I nach Anspruch 1, die ausgewählt ist aus den folgenden Verbindungen:
- (*E*)-3-(2,5-Difluor-phenyl)-*N*-{(2R,3*R*,6*R*)-2-((2*R*)-2,3-dihydroxy-propyl)-6-[2-(3-methoxy-chinolin-5-yl)-ethyl]-tetrahydro-pyran-3-yl}-acrylamid;
- (*E*)-3-(2,5-Difluor-phenyl)-*N*{(2*R*,3*R*,6*R*)-2-((2*S*)-2,3-dihydroxy-propyl)-6-[2-(3-methoxy-chinolin-5-yl)-ethyl]-tetrahydro-pyran-3-yl}-acrylamid;
- 3-Oxo-3,4-dihydro-2*H*-pyrido[3,2-*b*][1,4]thiazin-6-carbonsäure{(2*R*,3*R*,6*R*)-2-((2*R*)-2,3-dihydroxy-propyl)-6-[2-(3-methoxy-chinolin-5-yl)-ethyl]-tetrahydro-pyran-3-yl}-amid;
- 3-Oxo-3,4-dihydro-2*H*-pyrido[3,2-*b*][1,4]thiazin-6-carbonsäure {(2*R*,3*R*,6*R*)-2-((2*S*)-2,3-dihydroxy-propyl)-6-[2-(3-methoxy-chinolin-5-yl)-ethyl]-tetrahydro-pyran-3-yl}-amid;
- 6-({(2*R*,3*R*,6*R*)-2-((2*R*)-2,3-Dihydroxy-propyl)-6-[2-(3-methoxy-chinolin-5-yl)-ethyl]-tetrahydro-pyran-3-ylamino}-methyl)-4*H*-pyrido[3,2-*b*][1,4]thiazin-3-on;
- 6-({(2*R*,3*R*,6*R*)-2-((2*S*)-2,3-Dihydroxy-propyl)-6-[2-(3-methoxy-chinolin-5-yl)-ethyl]-tetrahydro-pyran-3-ylamino}-methyl)-4*H*-pyrido[3,2-*b*][1,4]thiazin-3-on;
- 6-({(2*R*,3*R*,6*R*)-2-((2*R*)-2,3-Dihydroxy-propyl)-6-[2-(6-methoxy-[1,5]naphthyridin-4-yl)-ethyl]-tetrahydro-pyran-3-ylamino}-methyl)-4*H*-pyrido[3,2-*b*][1,4]thiazin-3-on;
- 6-({(2*R*,3*R*,6*R*)-2-((2*S*)-2,3-Dihydroxy-propyl)-6-[2-(6-methoxy-[1,5]naphthyridin-4-yl)-ethyl]-tetrahydro-pyran-3-ylamino}-methyl)-4*H*-pyrido[3,2-*b*][1,4]thiazin-3-on;
- (2*R*)-3-{(2*R*,3*R*,6*R*)-3-[(*E*)-3-(2,5-Difluor-phenyl)-allylamino]-6-[2-(6-methoxy-[1,5]naphthyridin-4-yl)-ethyl]-tetrahydro-pyran-2-yl}-propan-1,2-diol;
- (2*S*)-3-{(2*R*,3*R*,6*R*)-3-[(*E*)-3-(2,5-Difluor-phenyl)-allylamino]-6-[2-(6-methoxy-[1,5]naphthyridin-4-yl)-ethyl]-tetrahydro-pyran-2-yl}-propan-1,2-diol;
- 2-{(2*R*,3*R*,6*R*)-3-[(*E*)-3-(2,5-Difluor-phenyl)-allylamino]-6-[2-(6-methoxy-[1,5]naphthyridin-4-yl)-ethyl]-tetrahydro-pyran-2-yl}-*N*-hydroxy-acetamid;
- (*E*)-3-(2,5-Difluor-phenyl)-*N*-{(2*R*,3*R*,6*R*)-2-((2*R*)-2,3-dihydroxy-propyl)-6-[2-(6-methoxy-[1,5]naphthyridin-4-yl)-ethyl]-tetrahydro-pyran-3-yl}-acrylamid;
- (*E*)-3-(2,5-Difluor-phenyl)-*N*{(2*R*,3*R*,6*R*)-2-((2*S*)-2,3-dihydroxy-propyl)-6-[2-(6-methoxy-[1,5]naphthyridin-4-yl)-ethyl]-tetrahydro-pyran-3-yl}-acrylamid;
- (2*R*)-3-{(2*R*,3*R*,6*S*)-3-[(*E*)-3-(2,5-Difluor-phenyl)-allylamino]-6-[(*S*)-1-hydroxy-2-(6-methoxy-[1,5]naphthyridin-4-yl)-ethyl]-tetrahydro-pyran-2-yl}-propan-1,2-diol;
- (2*S*)-3-{(2*R*,3*R*,6*S*)-3-[(*E*)-3-(2,5-Difluor-phenyl)-allylamino]-6-[(*S*)-1-hydroxy-2-(6-methoxy-[1,5]naphthyridin-4-yl)-ethyl]-tetrahydro-pyran-2-yl}-propan-1,2-diol;
- (2*R*)-3-{(2*R*,3*R*,6*R*)-3-[(*E*)-3-(2,5-Difluor-phenyl)-allylamino]-6-[2-(6-methoxy-chinolin-4-yl)-ethyl]-tetrahydro-pyran-2-yl}-propan-1,2-diol;
- (2*S*)-3-{(2*R*,3*R*,6*R*)-3-[(*E*)-3-(2,5-Difluor-phenyl)-allylamino]-6-[2-(6-methoxy-chinolin-4-yl)-ethyl]-tetrahydro-pyran-2-yl}-propan-1,2-diol;
- {(2*R*,3*R*,6*R*)-3-[(*E*)-3-(2,5-Difluor-phenyl)-allylamino]-6-[2-(6-methoxy-chinolin-4-yl)-ethyl]-tetrahydro-pyran-2-yl}-acetonitril;
- (*E*)-3-(2,5-Difluor-phenyl)-*N*-{(2*R*,3*R*,6*S*)-2-((2*R*)-2,3-dihydroxy-propyl)-6-[(*E*)-2-(3-fluor-6-methoxy-[1,5]naphthyridin-4-yl)-vinyl]-tetrahydro-pyran-3-yl} acrylamid;
- (*E*)-3-(2,5-Difluor-phenyl)-*N*-{(2*R*,3*R*,6*S*)-2-((2*S*)-2,3-dihydroxy-propyl)-6-[(*E*)-2-(3-fluor-6-methoxy-[1,5]naphthyridin-4-yl)-vinyl]-tetrahydro-pyran-3-yl} acrylamid;
- (2*R*)-3-{(2*R*,3*R*,6*S*)-3-[(*E*)-3-(2,5-Difluor-phenyl)-allylamino]-6-[(*E*)-2-(3-fluor-6-methoxy-[1,5]naphthyridin-4-yl)-vinyl]-tetrahydro-pyran-2-yl}-propan-1,2-diol;
- (2*S*)-3-{(2*R*,3*R*,6*S*)-3-[(*E*)-3-(2,5-Difluor-phenyl)-allylamino]-6-[(*E*)-2-(3-fluor-6-methoxy-[1,5]naphthyridin-4-yl)-vinyl]-tetrahydro-pyran-2-yl}-propan-1,2-diol;
- (*E*)-3-(2,5-Difluor-phenyl)-*N*-[(2*R*,3*R*,6*S*)-2-((2*R*)-2,3-dihydroxy-propyl)-6-(6-methoxy-chinolin-4-yloxymethyl)-tetrahydro-pyran-3-yl]-acrylamid;
- (*E*)-3-(2,5-Difluor-phenyl)-*N*-[(2*R*,3*R*,6*S*)-2-((2*S*)-2,3-dihydroxy-propyl)-6-(6-methoxy-chinolin-4-yloxymethyl)-tetrahydro-pyran-3-yl]-acrylamid;
- (2*S*,5*R*,6*R*)-5-[(*E*)-3-(2,5-Difluor-phenyl)-allylamino]-6-[(2*R*)-2,3-dihydroxy-propyl]-tetrahydro-pyran-2-carbonsäure(6-methoxy-[1,5]naphthyridin-4-yl)-amid;
- (2*S*,5*R*,6*R*)-5-[(*E*)-3-(2,5-Difluor-phenyl)-allylamino]-6-[(2*S*)-2,3-dihydroxy-propyl]-tetrahydro-pyran-2-carbonsäure(6-methoxy-[1,5]naphthyridin-4-yl)-amid;
- (*2S*,*5R*,*6R*)-6-[(*2R*)-2,3-Dihydroxy-propyl]-5-[(3-oxo-3,4-dihydro-2*H*-pyrido[3,2-*b*][1,4]thiazin-6-ylmethyl)-amino]-tetrahydro-pyran-2-carbonsäure (6-methoxy-[1,5]naphthyridin-4-yl)-amid;
- (2*S*,5*R*,6*R*)-6-[(2*S*)-2,3-Dihydroxy-propyl]-5-[(3-oxo-3,4-dihydro-2*H*-pyrido[3,2-*b*][1,4]thiazin-6-ylmethyl)-amino]-tetrahydro-pyran-2-carbonsäure (6-methoxy-[1,5]naphthyridin-4-yl)-amid;
- (*2S*,*5R*,*6R*)-5-[(*E*)-3-(2,5-Difluor-phenyl)-acryloylamino]-6-[(2*R*)-2,3-dihydroxy-propyl]-tetrahydro-pyran-2-carbonsäure(6-methoxy-[1,5]naphthyridin-4-yl)-amid;
- (*2S*,*5R*,*6R*)-5-[(*E*)-3-(2,5-Difluor-phenyl)-acryloylamino]-6-[(2*S*)-2,3-dihydroxy-propyl]-tetrahydro-pyran-2-carbonsäure(6-methoxy-[1,5]naphthyridin-4-yl)-amid;
- 3-Oxo-3,4-dihydro-2*H*-pyrido[3,2-*b*][1,4]thiazin-6-carbonsäure{(*2R*,3R,6S)-2-((2*R*)-2,3-dihydroxy-propyl)-6-[(*E*)-2-(6-methoxy-[1,5]naphthyridin-4-yl)-vinyl]-tetrahydro-pyran-3-yl}-amid;
- 3-Oxo-3,4-dihydro-2*H*-pyrido[3,2-*b*][1,4]thiazin-6-carbonsäure{(*2R*,*3R*,*6S*)-2-((2*S*)-2,3-dihydroxy-propyl)-6-[(*E*)-2-(6-methoxy-[1,5]naphthyridin-4-yl)-vinyl]-tetrahydro-pyran-3-yl}-amid;
- (2*R*)-3-{(*2R*,*3R*,*6S*)-3-[(*E*)-3-(2,5-Difluor-phenyl)-allylamino]-6-[(*E*)-2-(6-methoxy-[1,5]naphthyridin-4-yl)-vinyl]-tetrahydro-pyran-2-yl}-propan-1,2-diol;
- (2*S*)-3-{(*2R*,*3R*,*6S*)-3-[(*E*)-3-(2,5-Difluor-phenyl)-allylamino]-6-[(*E*)-2-(6-methoxy-[1,5]naphthyridin-4-yl)-vinyl]-tetrahydro-pyran-2-yl}-propan-1,2-diol;
- (2*R*)-3-{*2R*,*3R*,*6R*)-3-[(*E*)-3-(2,5-Difluor-phenyl)-allylamino]-6-[2-(6-fluor-chinolin-4-yl)-ethyl]-tetrahydro-pyran-2-yl}-propan-1,2-diol;
- (2*S*)-3-{*2R*,*3R*,*6R*)-3-[(*E*)-3-(2,5-Difluor-phenyl)-allylamino]-6-[2-(6-fluor-chinolin-4-yl)-ethyl]-tetrahydro-pyran-2-yl}-propan-1,2-diol;
- 2-{(2*R*,3*R*,6*S*)-3-[(*E*)-3-(2,5-Difluor-phenyl)-allylamino]-6-[(*E*)-2-(6-methoxy-[1,5]naphthyridin-4-yl)-vinyl]-tetrahydro-pyran-2-yl}-*N*-(2-hydroxy-ethyl)-acetamid;
- 2-{(*2R*,*3R*,*6S*)-3-[(*E*)-3-(2,5-Difluor-phenyl)-allylamino]-6-[(*1R*,*2R*)-1,2-dihydroxy-2-(6-methoxy-[1,5]naphthyridin-4-yl)-ethyl]-tetrahydro-pyran-2-yl}-*N*-(2-hydroxy-ethyl)-acetamid;
- 2-{(*2R*,*3*R,*6S*)-3-[(*E*)-3-(2,5-Difluor-phenyl)-allylamino]-6-[(1*S*)-1-hydroxy-2-(6-methoxy-[1,5]naphthyridin-4-yl)-ethyl]-tetrahydro-pyran-2-yl}-*N*-(2-hydroxy-ethyl)-acetamid;
- 2-{(*2R*,*3R*,*6S*)-3-[(*E*)-3-(2,5-Difluor-phenyl)-allylamino]-6-[(*1S*)-1-hydroxy-2-(6-methoxy-[1,5]naphthyridin-4-yl)-ethyl]-tetrahydro-pyran-2-yl}-1-morpholin-4-yl-ethanon;
- *N*-Cyclopropyl-2-{(*2R*,*3R*,*6S*)-3-[(*E*)-3-(2,5-difluor-phenyl)-allylamino]-6-[(*1S*)-1-hydroxy-2-(6-methoxy-[1,5]naphthyridin-4-yl)-ethyl]-tetrahydro-pyran-2-yl}-acetamid;
- 2-{(*2R*,*3R*,*6S*)-3-[(*E*)-3-(2,5-Difluor-phenyl)-allylamino]-6-[(*1S*)-1-hydroxy-2-(6-methoxy-[1,5]naphthyridin-4-yl)-ethyl]-tetrahydro-pyran-2-yl}-*N*,*N*-dimethylacetamid;
- 2-{(2*R*,3*R*,6*S*)-3-[(*E*)-3-(2,5-Difluor-phenyl)-allylamino]-6-[(*1S*)-1-hydroxy-2-(6-methoxy-[1,5]naphthyridin-4-yl)-ethyl]-tetrahydro-pyran-2-yl}-1-(4-hydroxypiperidin-1-yl)-ethanon;
- 2-{(*2R*,*3R*,*6S*)-3-[(*E*)-3-(2,5-Difluor-phenyl)-allylamino]-6-[(*1S*)-1-hydroxy-2-(6-methoxy-[1,5]naphthyridin-4-yl)-ethyl]-tetrahydro-pyran-2-yl}-*N*-prop-2-ynyloxy-acetamid;
- 2-{(2*R*,3*R*,6*R*)-3-[(*E*)-3-(2,5-Difluor-phenyl)-allylamino]-6-[2-(3-fluor-6-methoxy-chinolin-4-yl)-ethyl]-tetrahydro-pyran-2-yl}-*N*-(2-hydroxy-ethyl)-acetamid;
- 2-{(2*R*,3*R*,6*S*)-3-[(*E*)-3-(2,5-Difluor-phenyl)-allylamino]-6-[(*E*)-2-(3-fluor-6-methoxy-chinolin-4-yl)-vinyl]-tetrahydro-pyran-2-yl}-*N*,*N*-dimethyl-acetamid;
- (2*R*,3*R*,6*S*)-6-({2-[(2*R*)-2,3-Dihydroxy-propyl]-6-[(*E*)-(6-methoxy-[1,5]naphthyridin-4-yl)-vinyl]-tetrahydro-pyran-3-ylamino}-methyl)-4*H*-pyrido[3,2-*b*][1,4]thiazin-3-on;
- (2*R*,3*R*,6*S*)-6-({2-[(2*S*)-2,3-Dihydroxy-propyl]-6-[(*E*)-(6-methoxy-[1,5]naphthyridin-4-yl)-vinyl]-tetrahydro-pyran-3-ylamino}-methyl)-4*H*-pyrido[3,2-*b*][1,4]thiazin-3-on;
- (*2R*,*3R*,*6S*)-6-[(2-Hydroxy-ethylcarbamoyl)-methyl]-5-[(3-oxo-3,4-dihydro-2*H*-pyrido[3,2-*b*][1,4]thiazin-6-ylmethyl)-amino]-tetrahydro-pyran-2-carbonsäure (6-methoxy-[1,5]naphthyridin-4-yl)-amid;
oder ein Salz einer dieser Verbindungen.

11. Verbindung nach Anspruch 10, die ausgewählt ist aus den folgenden Verbindungen:
- (*E*)-3-(2,5-Difluor-phenyl)-*N*-{(2*R*,3*R*,6*R*)-2-((2*R*)-2,3-dihydroxy-propyl)-6-[2-(3-methoxy-chinolin-5-yl)-ethyl]-tetrahydro-pyran-3-yl}-acrylamid;
- (*E*)-3-(2,5-Difluor-phenyl)-*N*-{(2*R*,3*R*,6*R*)-2-((2*S*)-2,3-dihydroxy-propyl)-6-[2-(3-methoxy-chinolin-5-yl)-ethyl]-tetrahydro-pyran-3-yl}-acrylamid;
- 3-Oxo-3,4-dihydro-2*H*-pyrido[3,2-*b*][1,4]thiazin-6-carbonsäure{(2*R*,3*R*,6*R*)-2-((2*R*)-2,3-dihydroxy-propyl)-6-[2-(3-methoxy-chinolin-5-yl)-ethyl]-tetrahydro-pyran-3-yl}-amid;
- 3-Oxo-3,4-dihydro-2*H*-pyrido[3,2-*b*][1,4]thiazin-6-carbonsäure{(2*R*,3*R*,6*R*)-2-((2*S*)-2,3-dihydroxy-propyl)-6-[2-(3-methoxy-chinolin-5-yl)-ethyl]-tetrahydro-pyran-3-yl}-amid;
- 6-({(2*R*,3*R*,6*R*)-2-((2*R*)-2,3-Dihydroxy-propyl)-6-[2-(3-methoxy-chinolin-5-yl)-ethyl]-tetrahydro-pyran-3-ylamino}-methyl)-4*H*-pyrido[3,2-*b*][1,4]thiazin-3-on;
- 6-({(2*R*,3*R*,6*R*)-2-((2*S*)-2,3-Dihydroxy-propyl)-6-[2-(3-methoxy-chinolin-5-yl)-ethyl]-tetrahydro-pyran-3-ylamino}-methyl)-4*H*-pyrido[3,2-*b*][1,4]thiazin-3-on;
- 6-({(2*R*,3*R*,6*R*)-2-((2*R*)-2,3-Dihydroxy-propyl)-6-[2-(6-methoxy-[1,5]naphthyridin-4-yl)-ethyl]-tetrahydro-pyran-3-ylamino}-methyl)-4*H*-pyrido[3,2-*b*][1,4]thiazin-3-on;
- 6-({(2*R*,3*R*,6*R*)-2-((2*S*)-2,3-Dihydroxy-propyl)-6-[2-(6-methoxy-[1,5]naphthyridin-4-yl)-ethyl]-tetrahydro-pyran-3-ylamino}-methyl)-4*H*-pyrido[3,2-*b*][1,4]thiazin-3-on;
- (2*R*)-3-{(2*R*,3*R*,6*R*)-3-[(*E*)-3-(2,5-Difluor-phenyl)-allylamino]-6-[2-(6-methoxy-[1,5]naphthyridin-4-yl)-ethyl]-tetrahydro-pyran-2-yl}-propan-1,2-diol;
- (2*S*)-3-{(2*R*,3*R*,6*R*)-3-[(*E*)-3-(2,5-Difluor-phenyl)-allylamino]-6-[2-(6-methoxy-[1,5]naphthyridin-4-yl)-ethyl]-tetrahydro-pyran-2-yl}-propan-1,2-diol;
- 2-{(2*R*,3*R*,6*R*)-3-[(*E*)-3-(2,5-Difluor-phenyl)-allylamino]-6-[2-(6-methoxy-[1,5]naphthyridin-4-yl)-ethyl]-tetrahydro-pyran-2-yl}-*N*-hydroxy-acetamid;
- (*E*)-3-(2,5-Difluor-phenyl)-*N*-{(2*R*,3*R*,6*R*)-2-((2*R*)-2,3-dihydroxy-propyl)-6-[2-(6-methoxy-[1,5]naphthyridin-4-yl)-ethyl]-tetrahydro-pyran-3-yl}-acrylamid;
- (*E*)-3-(2,5-Difluor-phenyl)-*N*-{(2*R*,3*R*,6*R*)-2-((2*S*)-2,3-dihydroxy-propyl)-6-[2-(6-methoxy-[1,5]naphthyridin-4-yl)-ethyl]-tetrahydro-pyran-3-yl}-acrylamid;
- (2*R*)-3-{(2*R*,3*R*,6*S*)-3-[(*E*)-3-(2,5-Difluor-phenyl)-allylamino]-6-[(*S*)-1-hydroxy-2-(6-methoxy-[1,5]naphthyridin-4-yl)-ethyl]-tetrahydro-pyran-2-yl}-propan-1,2-diol;
- (2*S*)-3-{(2*R*,3*R*,6*S*)-3-[(*E*)-3-(2,5-Difluor-phenyl)-allylamino]-6-[(*S*)-1-hydroxy-2-(6-methoxy-[1,5]naphthyridin-4-yl)-ethyl]-tetrahydro-pyran-2-yl}-propan-1,2-diol;
- (2*R*)-3-{(2*R*,3*R*,6*R*)-3-[(*E*)-3-(2,5-Difluor-phenyl)-allylamino]-6-[2-(6-methoxy-chinolin-4-yl)-ethyl]-tetrahydro-pyran-2-yl}-propan-1,2-diol;
- (2*S*)-3-{(2*R*,3*R*,6*R*)-3-[(*E*)-3-(2,5-Difluor-phenyl)-allylamino]-6-[2-(6-methoxy-chinolin-4-yl)-ethyl]-tetrahydro-pyran-2-yl}-propan-1,2-diol;
- {(2*R*,3*R*,6*R*)-3-[(*E*)-3-(2,5-Difluor-phenyl)-allylamino]-6-[2-(6-methoxy-chinolin-4-yl)-ethyl]-tetrahydro-pyran-2-yl}-acetonitril;
- (*E*)-3-(2,5-Difluor-phenyl)-*N*-{(2*R*,3*R*,6*S*)-2-((2*R*)-2,3-dihydroxy-propyl)-6-[(*E*)-2-(3-fluor-6-methoxy-[1,5]naphthyridin-4-yl)-vinyl]-tetrahydro-pyran-3-yl} acrylamid;
- (*E*)-3-(2,5-Difluor-phenyl)-*N*-{(2*R*,3*R*,6*S*)-2-((2*S*)-2,3-dihydroxy-propyl)-6-[(*E*)-2-(3-fluor-6-methoxy-[1,5]naphthyridin-4-yl)-vinyl]-tetrahydro-pyran-3-yl} acrylamid;
- (2*R*)-3-{(2*R*,3*R*,6*S*)-3-[(*E*)-3-(2,5-Difluor-phenyl)-allylamino]-6-[(*E*)-2-(3-fluor-6-methoxy-[1,5]naphthyridin-4-yl)-vinyl]-tetrahydro-pyran-2-yl}-propan-1,2-diol;
- (2*S*)-3-{(2*R*,3*R*,6*S*)-3-[(*E*)-3-(2,5-Difluor-phenyl)-allylamino]-6-[(*E*)-2-(3-fluor-6-methoxy-[1,5]naphthyridin-4-yl)-vinyl]-tetrahydro-pyran-2-yl}-propan-1,2-diol;
- (*E*)-3-(2,5-Difluor-phenyl)-*N*-[(2*R*,3*R*,6*S*)-2-((2*R*)-2,3-dihydroxy-propyl)-6-(6-methoxy-chinolin-4-yloxymethyl)-tetrahydro-pyran-3-yl]-acrylamid;
- (*E*)-3-(2,5-Difluor-phenyl)-*N*-[(2*R*,3*R*,6*S*)-2-((2*S*)-2,3-dihydroxy-propyl)-6-(6-methoxy-chinolin-4-yloxymethyl)-tetrahydro-pyran-3-yl]-acrylamid;
- (2*S*,5*R*,6*R*)-5-[(*E*)-3-(2,5-Difluor-phenyl)-allylamino]-6-[(2*R*)-2,3-dihydroxy-propyl]-tetrahydro-pyran-2-carbonsäure(6-methoxy-[1,5]naphthyridin-4-yl)-amid;
- (2*S*,5*R*,6*R*)-5-[(*E*)-3-(2,5-Difluor-phenyl)-allylamino]-6-[(2*S*)-2,3-dihydroxy-propyl]-tetrahydro-pyran-2-carbonsäure(6-methoxy-[1,5]naphthyridin-4-yl)-amid;
- (*2S*,*5R*,*6R*)-6-[(*2R*)-2,3-Dihydroxy-propyl]-5-[(3-oxo-3,4-dihydro-2*H*-pyrido[3,2-*b*][1,4]thiazin-6-ylmethyl)-amino]-tetrahydro-pyran-2-carbonsäure (6-methoxy-[1,5]naphthyridin-4-yl)-amid;
- (*2S*,*5R*,*6R*)-6-[(*2S*)-2,3-Dihydroxy-propyl]-5-[(3-oxo-3,4-dihydro-2*H*-pyrido[3,2-*b*][1,4]thiazin-6-ylmethyl)-amino]-tetrahydro-pyran-2-carbonsäure (6-methoxy-[1,5]naphthyridin-4-yl)-amid;
oder ein Salz einer dieser Verbindungen.

12. Verbindung nach Anspruch 11, die ausgewählt ist aus den folgenden Verbindungen:
- (*E*)-3-(2,5-Difluor-phenyl)-*N*-{(2*R*,3*R*,6*R*)-2-((2*R*)-2,3-dihydroxy-propyl)-6-[2-(3-methoxy-chinolin-5-yl)-ethyl]-tetrahydro-pyran-3-yl}-acrylamid;
- (*E*)-3-(2,5-Difluor-phenyl)-*N*-{(2*R*,3*R*,6*R*)-2-((2*S*)-2,3-dihydroxy-propyl)-6-[2-(3-methoxy-chinolin-5-yl)-ethyl]-tetrahydro-pyran-3-yl}-acrylamid;
- 3-Oxo-3,4-dihydro-2*H*-pyrido[3,2-*b*][1,4]thiazin-6-carbonsäure{(2*R*,3*R*,6*R*)-2-((2*R*)-2,3-dihydroxy-propyl)-6-[2-(3-methoxy-chinolin-5-yl)-ethyl]-tetrahydro-pyran-3-yl}-amid;
- 3-Oxo-3,4-dihydro-2*H*-pyrido[3,2-*b*][1,4]thiazin-6-carbonsäure{(2*R*,3*R*,6*R*)-2-((2*S*)-2,3-dihydroxy-propyl)-6-[2-(3-methoxy-chinolin-5-yl)-ethyl]-tetrahydro-pyran-3-yl}-amid;
- 6-({(2*R*,3*R*,6*R*)-2-((2*R*)-2,3-Dihydroxy-propyl)-6-[2-(3-methoxy-chinolin-5-yl)-ethyl]-tetrahydro-pyran-3-ylamino}-methyl)-4*H*-pyrido[3,2-*b*][1,4]thiazin-3-on;
- 6-({(2*R*,3*R*,6*R*)-2-((2*S*)-2,3-Dihydroxy-propyl)-6-[2-(3-methoxy-chinolin-5-yl)-ethyl]-tetrahydro-pyran-3-ylamino}-methyl)-4*H*-pyrido[3,2-*b*][1,4]thiazin-3-on;
- 6-({(2*R*,3*R*,6*R*)-2-((2*R*)-2,3-Dihydroxy-propyl)-6-[2-(6-methoxy-[1,5]naphthyridin-4-yl)-ethyl]-tetrahydro-pyran-3-ylamino}-methyl)-4*H*-pyrido[3,2-*b*][1,4]thiazin-3-on;
- 6-({(2*R*,3*R*,6*R*)-2-((2*S*)-2,3-Dihydroxy-propyl)-6-[2-(6-methoxy-[1,5]naphthyridin-4-yl)-ethyl]-tetrahydro-pyran-3-ylamino}-methyl)-4*H*-pyrido[3,2-*b*][1,4]thiazin-3-on;
- (2*R*)-3-{(2*R*,3*R*,6*R*)-3-[(*E*)-3-(2,5-Difluor-phenyl)-allylamino]-6-[2-(6-methoxy-[1,5]naphthyridin-4-yl)-ethyl]-tetrahydro-pyran-2-yl}-propan-1,2-diol;
- (2*S*)-3-{(2*R*,3*R*,6*R*)-3-[(*E*)-3-(2,5-Difluor-phenyl)-allylamino]-6-[2-(6-methoxy-[1,5]naphthyridin-4-yl)-ethyl]-tetrahydro-pyran-2-yl}-propan-1,2-diol;
- 2-{(2*R*,3*R*,6*R*)-3-[(*E*)-3-(2,5-Difluor-phenyl)-allylamino]-6-[2-(6-methoxy-[1,5]naphthyridin-4-yl)-ethyl]-tetrahydro-pyran-2-yl}-*N*-hydroxy-acetamid;
- (*E*)-3-(2,5-Difluor-phenyl)-*N*-{(2*R*,3*R*,6*R*)-2-((2*R*)-2,3-dihydroxy-propyl)-6-[2-(6-methoxy-[1,5]naphthyridin-4-yl)-ethyl]-tetrahydro-pyran-3-yl}-acrylamid;
- (*E*)-3-(2,5-Difluor-phenyl)-*N*-{(2*R*,3*R*,6*R*)-2-((2*S*)-2,3-dihydroxy-propyl)-6-[2-(6-methoxy-[1,5]naphthyridin-4-yl)-ethyl]-tetrahydro-pyran-3-yl}-acrylamid;
- (2*R*)-3-{(2*R*,3*R*,6*S*)-3-[(*E*)-3-(2,5-Difluor-phenyl)-allylamino]-6-[(*S*)-1-hydroxy-2-(6-methoxy-[1,5]naphthyridin-4-yl)-ethyl]-tetrahydro-pyran-2-yl}-propan-1,2-diol;
- (2*S*)-3-{(2*R*,3*R*,6*S*)-3-[(*E*)-3-(2,5-Difluor-phenyl)-allylamino]-6-[(*S*)-1-hydroxy-2-(6-methoxy-[1,5]naphthyridin-4-yl)-ethyl]-tetrahydro-pyran-2-yl}-propan-1,2-diol;
- (2*R*)-3-{(2*R*,3*R*,6*R*)-3-[(*E*)-3-(2,5-Difluor-phenyl)-allylamino]-6-[2-(6-methoxy-chinolin-4-yl)-ethyl]-tetrahydro-pyran-2-yl}-propan-1,2-diol;
- (2*S*)-3-{(2*R*,3*R*,6*R*)-3-[(*E*)-3-(2,5-Difluor-phenyl)-allylamino]-6-[2-(6-methoxy-chinolin-4-yl)-ethyl]-tetrahydro-pyran-2-yl}-propan-1,2-diol;
- {(2*R*,3*R*,6*R*)-3-[(*E*)-3-(2,5-Difluor-phenyl)-allylamino]-6-[2-(6-methoxy-chinolin-4-yl)-ethyl]-tetrahydro-pyran-2-yl}-acetonitril;
- (*E*)-3-(2,5-Difluor-phenyl)-*N*-{(2*R*,3*R*,6*S*)-2-((2*R*)-2,3-dihydroxy-propyl)-6-[(*E*)-2-(3-fluor-6-methoxy-[1,5]naphthyridin-4-yl)-vinyl]-tetrahydro-pyran-3-yl} acrylamid;
- (*E*)-3-(2,5-Difluor-phenyl)-*N*-{(2*R*,3*R*,6*S*)-2-((2*S*)-2,3-dihydroxy-propyl)-6-[(*E*)-2-(3-fluor-6-methoxy-[1,5]naphthyridin-4-yl)-vinyl]-tetrahydro-pyran-3-yl} acrylamid;
- (2*R*)-3-{(2*R*,3*R*,6*S*)-3-[(*E*)-3-(2,5-Difluor-phenyl)-allylamino]-6-[(*E*)-2-(3-fluor-6-methoxy-[1,5]naphthyridin-4-yl)-vinyl]-tetrahydro-pyran-2-yl}-propan-1,2-diol;
- (2*S*)-3-{(2*R*,3*R*,6*S*)-3-[(*E*)-3-(2,5-Difluor-phenyl)-allylamino]-6-[(*E*)-2-(3-fluor-6-methoxy-[1,5]naphthyridin-4-yl)-vinyl]-tetrahydro-pyran-2-yl}-propan-1,2-diol;
- (*E*)-3-(2,5-Difluor-phenyl)-*N*-[(2*R*,3*R*,6*S*)-2-((2*R*)-2,3-dihydroxy-propyl)-6-(6-methoxy-chinolin-4-yloxymethyl)-tetrahydro-pyran-3-yl]-acrylamid;
- (*E*)-3-(2,5-Difluor-phenyl)-*N*-[(2*R*,3*R*,6*S*)-2-((2*S*)-2,3-dihydroxy-propyl)-6-(6-methoxy-chinolin-4-yloxymethyl)-tetrahydro-pyran-3-yl]-acrylamid;
oder ein Salz einer dieser Verbindungen.

13. Als Medikament, eine Verbindung der Formel I nach Anspruch 1 oder ein pharmazeutisch akzeptables Salz davon.

14. Pharmazeutische Zusammensetzung, die als Wirkstoff eine Verbindung der Formel I nach Anspruch 1 oder ein pharmazeutisch akzeptables Salz davon und wenigstens einen therapeutisch inerten Exzipienten enthält.

15. Verwendung einer Verbindung der Formel I nach Anspruch 1 oder eines pharmazeutisch akzeptablen Salzes davon zur Herstellung eines Medikaments zur Verhütung oder Behandlung einer bakteriellen Infektion.

## Revendications

1. Composé de formule 1 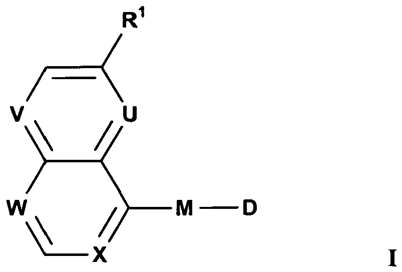 où
R¹ représente un radical alkyle, alkoxy, haloalkoxy, halogène ou cyano ;
un ou deux des radicaux U, V, W et X représente(nt) N et chacun des radicaux restants représente CH ou, dans le cas de X, peut également représenter CR^{a} ;
R^{a} représente un halogène ;
M représente 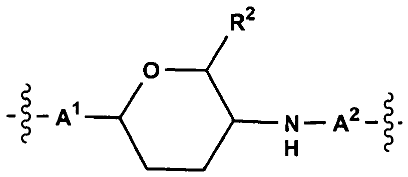 où
A¹ représente NHCO, CH₂CH₂, CH=CH, CH(OH)CH₂, CH₂CH(OH), CH(OH)CH(OH) ou OCH₂ ;
A² représente CH₂, CO, CH₂CH=CH ou COCH=CH ;
R² représente un radical carboxy, carbamoyle, hydroxycarbamoyle, cyano ou -CH₂R³,
R³ représentant un radical hydroxycarbamoyle, [(C₂-C₄)alkynyl]méthoxycarbamoyle, [(C₁-C₃)dialkyl]carbamoyle, cycloalkylcarbamoyle, [(C₂-C₅)hydroxyalkyl]carbamoyle, (arylalkyl)carbamoyle, cyano, [(C₁-C₅)alkyl]aminométhyle, [di-(C₁-C₅)alkyl]aminométhyle, [(C₁-C₃)alkoxy]imino, hydroxyimino ou hétéroarylalkyle,
où l'hétéroaryle est un hétéroaryle à 5 chaînons et où l'alkyle est un (C₁-C₄)alkyle,
ou R³ représentant un radical -NR⁴R⁵ où R⁴ et R⁵ forment, avec l'atome d'azote sur lequel ils sont portés, un noyau hétérocyclique à 5 ou 6 chaînons, les motifs utilisés pour fermer ledit noyau hétérocyclique étant indépendamment sélectionnés parmi -CH₂-, -O- et -S-, il étant sous-entendu qu'un seul des motifs sélectionnés entre -O- et -S- peut être présent dans ledit noyau hétérocyclique,
ou encore R³ représentant un radical -CH(OH)R⁶,
R⁶ représentant un radical hydroxyméthyle, [(C₁-C₃)alkyl]aminométhyle, [(C₁-C₃)dialkyl]aminométhyle, phénylaminométhyle où le groupement phényle peut être substitué une fois par un substituant du groupe consistant en un halogène, un alkoxy et un carboxy, hétéroarylaminométhyle où l'hétéroaryle est un hétéroaryle à 5 chaînons, benzylaminométhyle où le phényle du groupement benzyle peut être substitué une fois par un substituant du groupe consistant en un halogène, un alkoxy et un carboxy, ou un radical -CH₂-NR⁷R⁸ où R⁷ et R⁸ forment, avec l'atome d'azote qui les porte, un noyau hétérocyclique à 5 ou 6 chaînons, les motifs utilisés pour fermer ledit noyau hétérocyclique étant indépendamment sélectionnés parmi -CH₂-, -O- et -S-, étant sous-entendu qu'un seul des motifs sélectionnés entre -O- et -S- peut être présent dans ledit noyau hétérocyclique, ou encore R³ représentant un radical -CO-NR^{x}R^{y}, où le groupement -NR^{x}R^{y} est sélectionné parmi les suivants : pyrrolidin-1-yle, pipéridin-1-yle, morpholin-4-yle, thiomorpholin-4-yle, (3*R*)-3-hydroxy-pyrrolidin-1-yle, (3*S*)-3-hydroxy-pyrrolidin-1-yle et 4-hydroxy-pipéridin-1-yle ;
D représente un aryle ou un hétéroaryle ; où tout groupement aryle peut être substitué par un, deux ou plusieurs substituants indépendamment sélectionnés parmi un halogène, alkyle, alkoxy, trifluorométhyle et trifluorométhoxy ; et où tout groupement hétéroaryle peut être substitué sur le(s) noyau(x) aromatique(s) par un, deux ou plusieurs halogènes, alkyles et alkoxy ;
ou sel de celui-ci.

2. Composé de formule I selon la revendication 1, où R¹ représente un méthoxy ou un fluor ;
ou sel de celui-ci.

3. Composé de formule I selon la revendication 1, où un ou deux des radicaux U, V, W et X représente(nt) N et chacun des radicaux restants représente CH ou, dans le cas de X, peut également représenter CR^{a}, R^{a} représentant un fluor ;
ou sel de celui-ci.

4. Composé de formule I selon la revendication 1, qui est également un composé de formule I_{Ar} 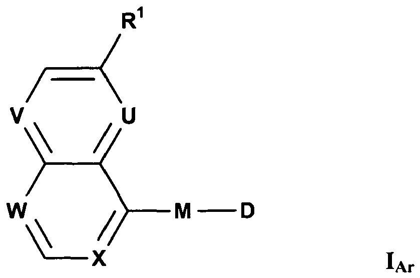 où :
R¹, U, V, W, X et M sont tels que définis à la formule I de la revendication 1 et
D représente un aryle ;
ou sel de celui-ci.

5. Composé de formule I selon la revendication 1, qui est également un composé de formule I_{Het} 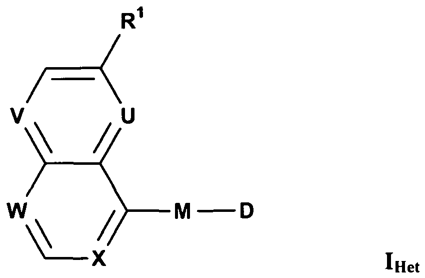 où :
R¹, U, V, W, X et M sont tels que définis à la formule I de la revendication 1 et
D représente un hétéroaryle ;
ou sel de celui-ci.

6. Composé de formule I_{Het} selon la revendication 5, où D est un hétéroaryle bicyclique répondant à la formule générale 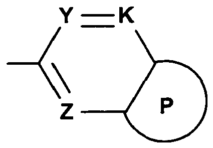 où :
K, Y et Z représentent chacun indépendamment N ou CR^{α} ;
R^{α} représente un hydrogène ou un halogène ;
P est un noyau sélectionné dans le groupe consistant en les suivants : 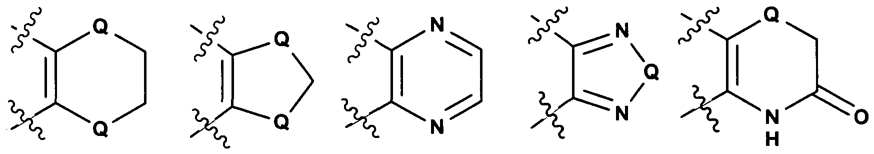
où Q représente O ou S ;
ou sel de celui-ci.

7. Composé de formule I selon la revendication 1, où :
R² représente -CH₂R³,
R³ étant sélectionné dans le groupe consistant en un hydroxycarbamoyle, [(C₂-C₄)alkynyl]méthoxycarbamoyle, cycloalkylcarbamoyle, [(C₂-C₅)hydroxyalkyl]carbamoyle, cyano, -CH(OH)R⁶ et -CO-NR^{x}R^{y} et
R⁶ représentant un hydroxyméthyle et
le groupement -NR^{x}R^{y} est sélectionné parmi les suivants : pyrrolidin-1-yle, pipéridin-1-yle, morpholin-4-yle, thiomorpholin-4-yle, (3*R*)-3-hydroxy-pyrrolidin-1-yle, (3*S*)-3-hydroxypyrrolidin-1-yle et 4-hydroxy-pipéridin-1-yle ;
ou sel de celui-ci.

8. Composé de formule I selon la revendication 1, où A¹ représente un radical NHCO, CH₂CH₂, CH=CH, CH(OH)CH₂, CH₂CH(OH) ou OCH₂ ou également, à condition que U représente N, un radical CH(OH)CH(OH) ;
ou sel de celui-ci.

9. Composé de formule I selon la revendication 1, où A² représente un radical CH₂ ou CO ou également, à condition que D représente un groupement aryle ou hétéroaryle non annelé, un radical CH₂CH=CH ou COCH=CH ;
ou sel de celui-ci.

10. Composé de formule I selon la revendication 1, qui est sélectionné parmi les suivants :
- (*E*)-3-(2,5-difluoro-phényl)-*N*-{(2*R*,3*R*,6*R*)-2-((2*R*)-2,3-dihydroxy-propyl)-6-[2-(3-méthoxy-quinoléin-5-yl)-éthyl]-tétrahydro-pyran-3-yl}-acrylamide ;
- (*E*)-3-(2,5-difluoro-phényl)-*N*-{(2*R*,3*R*,6*R*)-2-((2*S*)-2,3-dihydroxy-propyl)-6-[2-(3-méthoxy-quinoléin-5-yl)-éthyl]-tétrahydro-pyran-3-yl}-acrylamide ;
- {(2*R*,3*R*,6*R*)-2-((2*R*)-2,3-dihydroxy-propyl)-6-[2-(3-méthoxy-quinoléin-5-yl)-éthyl]-tétrahydro-pyran-3-yl}-amide de l'acide 3-oxo-3,4-dihydro-2*H*-pyrido[3,2-*b*][1,4]thiazine-6-carboxylique ;
- {(2*R*,3*R*,6*R*)-2-((2*S*)-2,3-dihydroxy-propyl)-6-[2-(3-méthoxy-quinoléin-5-yl)-éthyl]-tétrahydro-pyran-3-yl}-amide de l'acide 3-oxo-3,4-dihydro-2*H*-pyrido[3,2-*b*][1,4]thiazine-6-carboxylique ;
- 6-({(2*R*,3*R*,6*R*)-2-((2*R*)-2,3-dihydroxy-propyl)-6-[2-(3-méthoxy-quinoléin-5-yl)-éthyl]-tétrahydro-pyran-3-ylamino}-méthyl)-4*H*-pyrido[3,2-*b*][1,4]thiazin-3-one ;
- 6-({(2*R*,3*R*,6*R*)-2-((2*S*)-2,3-dihydroxy-propyl)-6-[2-(3-méthoxy-quinoléin-5-yl)-éthyl]-tétrahydro-pyran-3-ylamino}-méthyl)-4*H*-pyrido[3,2-*b*][1,4]thiazin-3-one ;
- 6-({(2*R*,3*R*,6*R*)-2-((2*R*)-2,3-dihydroxy-propyl)-6-[2-(6-méthoxy-[1,5]naphtyridin-4-yl)-éthyl]-tétrahydro-pyran-3-ylamino}-méthyl)-4*H*-pyrido[3,2-*b*][1,4]thiazin-3-one ;
- 6-({(2*R*,3*R*,6*R*)-2-((2*S*)-2,3-dihydroxy-propyl)-6-[2-(6-méthoxy-[1,5]naphtyridin-4-yl)-éthyl]-tétrahydro-pyran-3-ylamino}-méthyl)-4*H*-pyrido[3,2-*b*][1,4]thiazin-3-one ;
- (2*R*)-3-{(2*R*,3*R*,6*R*)-3-[(*E*)-3-(2,5-difluoro-phényl)-allylamino]-6-[2-(6-méthoxy-[1,5]naphtyridin-4-yl)-éthyl]-tétrahydro-pyran-2-yl}-propane-1,2-diol ;
- (2*S*)-3-{(2*R*,3*R*,6*R*)-3-[(*E*)-3-(2,5-difluoro-phényl)-allylamino]-6-[2-(6-méthoxy-[1,5]naphtyridin-4-yl)-éthyl]-tétrahydro-pyran-2-yl}-propane-1,2-diol ;
- 2-{(2*R*,3*R*,6*R*)-3-[(*E*)-3-(2,5-difluoro-phényl)-allylamino]-6-[2-(6-méthoxy-[1,5]naphtyridin-4-yl)-éthyl]-tétrahydro-pyran-2-yl}-*N*-hydroxy-acétamide ;
- (*E*)-3-(2,5-difluoro-phényl)-*N*-{(2*R*,3*R*,6*R*)-2-((2*R*)-2,3-dihydroxy-propyl)-6-[2-(6-méthoxy-[1,5]naphtyridin-4-yl)-éthyl]-tétrahydro-pyran-3-yl}-acrylamide ;
- (*E*)-3-(2,5-difluoro-phényl)-*N*-{(2*R*,3*R*,6*R*)-2-((2*S*)-2,3-dihydroxy-propyl)-6-[2-(6-méthoxy-[1,5]naphtyridin-4-yl)-éthyl]-tétrahydro-pyran-3-yl}-acrylamide ;
- (2*R*)-3-{(2*R*,3*R*,6*S*)-3-[(*E*)-3-(2,5-difluoro-phényl)-allylamino]-6-[(*S*)-1-hydroxy-2-(6-méthoxy-[1,5]naphtyridin-4-yl)-éthyl]-tétrahydro-pyran-2-yl}-propane-1,2-diol ;
- (2*S*)-3-{(2*R*,3*R*,6*S*)-3-[(*E*)-3-(2,5-difluoro-phényl)-allylamino]-6-[(*S*)-1-hydroxy-2-(6-méthoxy-[1,5]naphtyridin-4-yl)-éthyl]-tétrahydro-pyran-2-yl}-propane-1,2-diol ;
- (2*R*)-3-{(2*R*,3*R*,6*R*)-3-[(*E*)-3-(2,5-difluoro-phényl)-allylamino]-6-[2-(6-méthoxy-quinoléin-4-yl)-éthyl]-tétrahydro-pyran-2-yl}-propane-1,2-diol ;
- (2*S*)-3-{(2*R*,3*R*,6*R*)-3-[(*E*)-3-(2,5-difluoro-phényl)-allylamino]-6-[2-(6-méthoxy-quinoléin-4-yl)-éthyl]-tétrahydro-pyran-2-yl}-propane-1,2-diol ;
- {(2*R*,3*R*,6*R*)-3-[(*E*)-3-(2,5-difluoro-phényl)-allylamino]-6-[2-(6-méthoxy-quinoléin-4-yl)-éthyl]-tétrahydro-pyran-2-yl}-acétonitrile ;
- (*E*)-3-(2,5-difluoro-phényl)-*N*-{(2*R*,3*R*,6*S*)-2-((2*R*)-2,3-dihydroxy-propyl)-6-[(*E*)-2-(3-fluoro-6-méthoxy-[1,5]naphtyridin-4-yl)-vinyl]-tétrahydro-pyran-3-yl}-acrylamide ;
- (*E*)-3-(2,5-difluoro-phényl)-*N*-{(2*R*,3*R*,6*S*)-2-((2*S*)-2,3-dihydroxy-propyl)-6-[(*E*)-2-(3-fluoro-6-méthoxy-[1,5]naphtyridin-4-yl)-vinyl]-tétrahydro-pyran-3-yl}-acrylamide ;
- (2*R*)-3-{(2*R*,3*R*,6*S*)-3-[(*E*)-3-(2,5-difluoro-phényl)-allylamino]-6-[(*E*)-2-(3-fluoro-6-méthoxy-[1,5]naphtyridin-4-yl)-vinyl]-tétrahydro-pyran-2-yl}-propane-1,2-diol ;
- (2*S*)-3-{(2*R*,3*R*,6*S*)-3-[(*E*)-3-(2,5-difluoro-phényl)-allylamino]-6-[(*E*)-2-(3-fluoro-6-méthoxy-[1,5]naphtyridin-4-yl)-vinyl]-tétrahydro-pyran-2-yl}-propane-1,2-diol ;
- (*E*)-3-(2,5-difluoro-phényl)-*N*-[(2*R*,3*R*,6*S*)-2-((2*R*)-2,3-dihydroxy-propyl)-6-(6-méthoxyquinoléin-4-yloxyméthyl)-tétrahydro-pyran-3-yl]-acrylamide ;
- (*E*)-3-(2,5-difluoro-phényl)-*N*-[(2*R*,3*R*,6*S*)-2-((2*S*)-2,3-dihydroxy-propyl)-6-(6-méthoxyquinoléin-4-yloxyméthyl)-tétrahydro-pyran-3-yl]-acrylamide ;
- (6-méthoxy-[1,5]naphtyridin-4-yl)-amide de l'acide (2*S*,5*R*,6*R*)-5-[(*E*)-3-(2,5-difluorophényl)-allylamino]-6-[(2*R*)-2,3-dihydroxy-propyl]-tétrahydro-pyran-2-carboxylique ;
- (6-méthoxy-[1,5]naphtyridin-4-yl)-amide de l'acide (2*S*,5*R*,6*R*)-5-[(*E*)-3-(2,5-difluorophényl)-allylamino]-6-[(2*S*)-2,3-dihydroxy-propyl]-tétrahydro-pyran-2-carboxylique ;
- (6-méthoxy-[1,5]naphtyridin-4-yl)-amide de l'acide (*2S*,*5R*,*6R*)-6-[(*2R*)-2,3-dihydroxypropyl]-5-[(3-oxo-3,4-dihydro-2*H*-pyrido[3,2-*b*][1,4]thiazin-6-ylméthyl)-amino]-tétrahydro-pyran-2-carboxylique ;
- (6-méthoxy-[1,5]naphtyridin-4-yl)-amide de l'acide (*2S*,*5R*,*6R*)-6-[(*2S*)-2,3-dihydroxypropyl]-5-[(3-oxo-3,4-dihydro-2*H*-pyrido[3,2-*b*][1,4]thiazin-6-ylméthyl)-amino]-tétrahydro-pyran-2-carboxylique ;
- (6-méthoxy-[1,5]naphtyridin-4-yl)-amide de l'acide (*2S*,*5R*,*6R*)-5-[(*E*)-3-(2,5-difluorophényl)-acryloylamino]-6-[(2*R*)-2,3-dihydroxy-propyl]-tétrahydro-pyran-2-carboxylique ;
- (6-méthoxy-[1,5]naphtyridin-4-yl)-amide de l'acide (*2S*,*5R*,*6R*)-5-[(*E*)-3-(2,5-difluorophényl)-acryloylamino]-6-[(2*S*)-2,3-dihydroxy-propyl]-tétrahydro-pyran-2-carboxylique ;
- {(*2R*,*3R*,*6S*)-2-((2*R*)-2,3-dihydroxy-propyl)-6-[(*E*)-2-(6-méthoxy-[1,5]naphtyridin-4-yl)-vinyl]-tétrahydro-pyran-3-yl}-amide de l'acide 3-oxo-3,4-dihydro-2*H*-pyrido[3,2-*b*][1,4]thiazine-6-carboxylique ;
- {(*2R*,*3R*,*6S*)-2-((2*S*)-2,3-dihydroxy-propyl)-6-[(*E*)-2-(6-méthoxy-[1,5]naphtyridin-4-yl)-vinyl]-tétrahydro-pyran-3-yl}-amide de l'acide 3-oxo-3,4-dihydro-2*H*-pyrido[3,2-*b*][1,4]thiazine-6-carboxylique ;
- (2*R*)-3-{(*2R*,*3R*,*6S*)-3-[(*E*)-3-(2,5-difluoro-phényl)-allylamino]-6-[(*E*)-2-(6-méthoxy-[1,5]naphtyridin-4-yl)-vinyl]-tétrahydro-pyran-2-yl}-propane-1,2-diol ;
- (2*S*)-3-{(*2R*,*3R*,*6S*)-3-[(*E*)-3-(2,5-difluoro-phényl)-allylamino]-6-[(*E*)-2-(6-méthoxy-[1,5]naphtyridin-4-yl)-vinyl]-tétrahydro-pyran-2-yl}-propane-1,2-diol ;
- (*2R*)-3-{*2R*,*3*R,*6R*)-3-[(*E*)-3-(2,5-difluoro-phényl)-allylamino]-6-[2-(6-fluoro-quinoléin-4-yl)-éthyl]-tétrahydro-pyran-2-yl}-propane-1,2-diol ;
- (*2S*)-3-{*2R*,*3R*,*6R*)-3-[(*E*)-3-(2,5-difluoro-phényl)-allylamino]-6-[2-(6-fluoro-quinoléin-4-yl)-éthyl]-tétrahydro-pyran-2-yl}-propane-1,2-diol ;
- 2-{(2*R*,3*R*,6*S*)-3-[(*E*)-3-(2,5-difluoro-phényl)-allylamino]-6-[(*E*)-2-(6-méthoxy-[1,5]naphtyridin-4-yl)-vinyl]-tétrahydro-pyran-2-yl}-*N*-(2-hydroxy-éthyl)-acétamide ;
- 2-{(*2R*,*3R*,*6S*)-3-[(*E*)-3-(2,5-difluoro-phényl)-allylamino]-6-[(*1R*,*2R*)-1,2-dihydroxy-2-(6-méthoxy-[1,5]naphtyridin-4-yl)-éthyl]-tétrahydro-pyran-2-yl}-*N*-(2-hydroxyéthyl)-acétamide ;
- 2-{(*2R*,*3R*,*6S*)-3-[(*E*)-3-(2,5-difluoro-phényl)-allylamino]-6-[(1*S*)-1-hydroxy-2-(6-méthoxy-[1,5]naphtyridin-4-yl)-éthyl]-tétrahydro-pyran-2-yl}-*N*-(2-hydroxy-éthyl)-acétamide ;
- 2-{(*2R*,*3R*,*6S*)-3-[(*E*)-3-(2,5-difluoro-phényl)-allylamino]-6-[(*1S*)-1-hydroxy-2-(6-méthoxy-[1,5]naphtyridin-4-yl)-éthyl]-tétrahydro-pyran-2-yl}-1-morpholin-4-yl-éthanone ;
- *N*-cyclopropyl-2-{(*2R*,*3R*,*6S*)-3-[(*E*)-3-(2,5-difluoro-phényl)-allylamino]-6-[(*1S*)-1-hydroxy-2-(6-méthoxy-[1,5]naphtyridin-4-yl)-éthyl]-tétrahydro-pyran-2-yl}-acétamide ;
- 2-{(*2R*,*3R*,*6S*)-3-[(*E*)-3-(2,5-difluoro-phényl)-allylamino]-6-[(*1S*)-1-hydroxy-2-(6-méthoxy-[1,5]naphtyridin-4-yl)-éthyl]-tétrahydro-pyran-2-yl}-*N*,*N*-diméthylacétamide ;
- 2-{(*2R*,*3R*,*6S*)-3-[(*E*)-3-(2,5-difluoro-phényl)-allylamino]-6-[(*1S*)-1-hydroxy-2-(6-méthoxy-[1,5]naphtyridin-4-yl)-éthyl]-tétrahydro-pyran-2-yl}-1-(4-hydroxypipéridin-1-yl)-éthanone ;
- 2-{(*2R*,*3R*,*6S*)-3-[(*E*)-3-(2,5-difluoro-phényl)-allylamino]-6-[(*1S*)-1-hydroxy-2-(6-méthoxy-[1,5]naphtyridin-4-yl)-éthyl]-tétrahydro-pyran-2-yl}-*N*-prop-2-ynyloxyacétamide ;
- 2-{(2*R*,3*R*,6*R*)-3-[(*E*)-3-(2,5-difluoro-phényl)-allylamino]-6-[2-(3-fluoro-6-méthoxyquinoléin-4-yl)-éthyl]-tétrahydro-pyran-2-yl}-*N*-(2-hydroxy-éthyl)-acétamide ;
- 2-{(2*R*,3*R*,6*S*)-3-[(*E*)-3-(2,5-difluoro-phényl)-allylamino]-6-[(*E*)-2-(3-fluoro-6-méthoxyquinoléin-4-yl)-vinyl]-tétrahydro-pyran-2-yl}-*N*,*N*-diméthyl-acétamide ;
- (2*R*,3*R*,6*S*)-6-({2-[(2*R*)-2,3-dihydroxy-propyl]-6-[(*E*)-(6-méthoxy-[1,5]naphtyridin-4-yl)-vinyl]-tétrahydro-pyran-3-ylamino}-méthyl)-4*H*-pyrido[3,2-*b*][1,4]thiazin-3-one;
- (2*R*,3*R*,6*S*)-6-({2-[(2*S*)-2,3-dihydroxy-propyl]-6-[(*E*)-(6-méthoxy-[1,5]naphtyridin-4-yl)-vinyl]-tétrahydro-pyran-3-ylamino}-méthyl)-4*H*-pyrido[3,2-*b*][1,4]thiazin-3-one ;
- (6-méthoxy-[1,5]naphtyridin-4-yl)-amide de l'acide (*2R*,*3R*,*6S*)-6-[(2-hydroxyéthylcarbamoyl)-méthyl]-5-[(3-oxo-3,4-dihydro-2*H*-pyrido[3,2-*b*][1,4]thiazin-6-ylméthyl)-amino]-tétrahydro-pyran-2-carboxylique ;
ou sel de l'un de ces composés.

11. Composé selon la revendication 10, qui est sélectionné parmi les suivants :
- (*E*)-3-(2,5-difluoro-phényl)-*N*-{(2*R*,3*R*,6*R*)-2-((2*R*)-2,3-dihydroxy-propyl)-6-[2-(3-méthoxy-quinoléin-5-yl)-éthyl]-tétrahydro-pyran-3-yl}-acrylamide ;
- (*E*)-3-(2,5-difluoro-phényl)-*N*-{(2*R*,3*R*,6*R*)-2-((2*S*)-2,3-dihydroxy-propyl)-6-[2-(3-méthoxy-quinoléin-5-yl)-éthyl]-tétrahydro-pyran-3-yl}-acrylamide ;
- {(2*R*,3*R*,6*R*)-2-((2*R*)-2,3-dihydroxy-propyl)-6-[2-(3-méthoxy-quinoléin-5-yl)-éthyl]-tétrahydro-pyran-3-yl}-amide de l'acide 3-oxo-3,4-dihydro-2*H*-pyrido[3,2-*b*][1,4]thiazine-6-carboxylique ;
- {(2*R*,3*R*,6*R*)-2-((2*S*)-2,3-dihydroxy-propyl)-6-[2-(3-méthoxy-quinoléin-5-yl)-éthyl]-tétrahydro-pyran-3-yl}-amide de l'acide 3-oxo-3,4-dihydro-2*H*-pyrido[3,2-*b*][1,4]thiazine-6-carboxylique ;
- 6-({(2*R*,3*R*,6*R*)-2-((2*R*)-2,3-dihydroxy-propyl)-6-[2-(3-méthoxy-quinoléin-5-yl)-éthyl]-tétrahydro-pyran-3-ylamino}-méthyl)-4*H*-pyrido[3,2-*b*][1,4]thiazin-3-one ;
- 6-({(2*R*,3*R*,6*R*)-2-((2*S*)-2,3-dihydroxy-propyl)-6-[2-(3-méthoxy-quinoléin-5-yl)-éthyl]-tétrahydro-pyran-3-ylamino}-méthyl)-4*H*-pyrido[3,2-*b*][1,4]thiazin-3-one ;
- 6-({(2*R*,3*R*,6*R*)-2-((2*R*)-2,3-dihydroxy-propyl)-6-[2-(6-méthoxy-[1,5]naphtyridin-4-yl)-éthyl]-tétrahydro-pyran-3-ylamino}-méthyl)-4*H*-pyrido[3,2-*b*][1,4]thiazin-3-one ;
- 6-({(2*R*,3*R*,6*R*)-2-((2*S*)-2,3-dihydroxy-propyl)-6-[2-(6-méthoxy-[1,5]naphtyridin-4-yl)-éthyl]-tétrahydro-pyran-3-ylamino}-méthyl)-4*H*-pyrido[3,2-*b*][1,4]thiazin-3-one ;
- (2*R*)-3-{(2*R*,3*R*,6*R*)-3-[(*E*)-3-(2,5-difluoro-phényl)-allylamino]-6-[2-(6-méthoxy-[1,5]naphtyridin-4-yl)-éthyl]-tétrahydro-pyran-2-yl}-propane-1,2-diol ;
- (2*S*)-3-{(2*R*,3*R*,6*R*)-3-[(*E*)-3-(2,5-difluoro-phényl)-allylamino]-6-[2-(6-méthoxy-[1,5]naphtyridin-4-yl)-éthyl]-tétrahydro-pyran-2-yl}-propane-1,2-diol ;
- 2-{(2*R*,3*R*,6*R*)-3-[(*E*)-3-(2,5-difluoro-phényl)-allylamino]-6-[2-(6-méthoxy-[1,5]naphtyridin-4-yl)-éthyl]-tétrahydro-pyran-2-yl}-*N*-hydroxy-acétamide ;
- (*E*)-3-(2,5-difluoro-phényl)-*N*-{(2*R*,3*R*,6*R*)-2-((2*R*)-2,3-dihydroxy-propyl)-6-[2-(6-méthoxy-[1,5]naphtyridin-4-yl)-éthyl]-tétrahydro-pyran-3-yl}-acrylamide ;
- (*E*)-3-(2,5-difluoro-phényl)-*N*-{(2*R*,3*R*,6*R*)-2-((2*S*)-2,3-dihydroxy-propyl)-6-[2-(6-méthoxy-[1,5]naphtyridin-4-yl)-éthyl]-tétrahydro-pyran-3-yl}-acrylamide ;
- (2*R*)-3-{(2*R*,3*R*,6*S*)-3-[(*E*)-3-(2,5-difluoro-phényl)-allylamino]-6-[(*S*)-1-hydroxy-2-(6-méthoxy-[1,5]naphtyridin-4-yl)-éthyl]-tétrahydro-pyran-2-yl}-propane-1,2-diol ;
- (2*S*)-3-{(2*R*,3*R*,6*S*)-3-[(*E*)-3-(2,5-difluoro-phényl)-allylamino]-6-[(*S*)-1-hydroxy-2-(6-méthoxy-[1,5]naphtyridin-4-yl)-éthyl]-tétrahydro-pyran-2-yl}-propane-1,2-diol ;
- (2*R*)-3-{(2*R*,3*R*,6*R*)-3-[(*E*)-3-(2,5-difluoro-phényl)-allylamino]-6-[2-(6-méthoxyquinoléin-4-yl)-éthyl]-tétrahydro-pyran-2-yl}-propane-1,2-diol ;
- (2*S*)-3-{(2*R*,3*R*,6*R*)-3-[(*E*)-3-(2,5-difluoro-phényl)-allylamino]-6-[2-(6-méthoxyquinoléin-4-yl)-éthyl]-tétrahydro-pyran-2-yl}-propane-1,2-diol ;
- {(2*R*,3*R*,6*R*)-3-[(*E*)-3-(2,5-difluoro-phényl)-allylamino]-6-[2-(6-méthoxy-quinoléin-4-yl)-éthyl]-tétrahydro-pyran-2-yl}-acétonitrile ;
- (*E*)-3-(2,5-difluoro-phényl)-*N*-{(2*R*,3*R*,6*S*)-2-((2*R*)-2,3-dihydroxy-propyl)-6-[(*E*)-2-(3-fluoro-6-méthoxy-[1,5]naphtyridin-4-yl)-vinyl]-tétrahydro-pyran-3-yl}-acrylamide ;
- (*E*)-3-(2,5-difluoro-phényl)-*N*-{(2*R*,3*R*,6*S*)-2-((2*S*)-2,3-dihydroxy-propyl)-6-[(*E*)-2-(3-fluoro-6-méthoxy-[1,5]naphtyridin-4-yl)-vinyl]-tétrahydro-pyran-3-yl}-acrylamide ;
- (2*R*)-3-{(2*R*,3*R*,6*S*)-3-[(*E*)-3-(2,5-difluoro-phényl)-allylamino]-6-[(*E*)-2-(3-fluoro-6-méthoxy-[1,5]naphtyridin-4-yl)-vinyl]-tétrahydro-pyran-2-yl}-propane-1,2-diol ;
- (2*S*)-3-{(2*R*,3*R*,6*S*)-3-[(*E*)-3-(2,5-difluoro-phényl)-allylamino]-6-[(*E*)-2-(3-fluoro-6-méthoxy-[1,5]naphtyridin-4-yl)-vinyl]-tétrahydro-pyran-2-yl}-propane-1,2-diol ;
- (*E*)-3-(2,5-difluoro-phényl)-*N*-[(2*R*,3*R*,6*S*)-2-((2*R*)-2,3-dihydroxy-propyl)-6-(6-méthoxyquinoléin-4-yloxyméthyl)-tétrahydro-pyran-3-yl]-acrylamide ;
- (*E*)-3-(2,5-difluoro-phényl)-*N*-[(2*R*,3*R*,6*S*)-2-((2*S*)-2,3-dihydroxy-propyl)-6-(6-méthoxyquinoléin-4-yloxyméthyl)-tétrahydro-pyran-3-yl]-acrylamide ;
- (6-méthoxy-[1,5]naphtyridin-4-yl)-amide de l'acide (2*S*,5*R*,6*R*)-5-[(*E*)-3-(2,5-difluorophényl)-allylamino]-6-[(2*R*)-2,3-dihydroxy-propyl]-tétrahydro-pyran-2-carboxylique ;
- (6-méthoxy-[1,5]naphtyridin-4-yl)-amide de l'acide (2*S*,5*R*,6*R*)-5-[(*E*)-3-(2,5-difluorophényl)-allylamino]-6-[(2*S*)-2,3-dihydroxy-propyl]-tétrahydro-pyran-2-carboxylique ;
- (6-méthoxy-[1,5]naphtyridin-4-yl)-amide de l'acide (*2S*,*5R*,*6R*)-6-[(*2R*)-2,3-dihydroxypropyl]-5-[(3-oxo-3,4-dihydro-2*H*-pyrido[3,2-*b*][1,4]thiazin-6-ylméthyl)-amino]-tétrahydro-pyran-2-carboxylique ;
- (6-méthoxy-[1,5]naphtyridin-4-yl)-amide de l'acide (*2S*,*5R*,*6R*)-6-[(*2S*)-2,3-dihydroxypropyl]-5-[(3-oxo-3,4-dihydro-2*H*-pyrido[3,2-*b*][1,4]thiazin-6-ylméthyl)-amino]-tétrahydro-pyran-2-carboxylique ;
ou sel de l'un de ces composés.

12. Composé selon la revendication 11, qui est sélectionné parmi les suivants :
- (*E*)-3-(2,5-difluoro-phényl)-*N*-{(2*R*,3*R*,6*R*)-2-((2*R*)-2,3-dihydroxy-propyl)-6-[2-(3-méthoxy-quinoléin-5-yl)-éthyl]-tétrahydro-pyran-3-yl}-acrylamide ;
- (*E*)-3-(2,5-difluoro-phényl)-*N*-{(2*R*,3*R*,6*R*)-2-((2*S*)-2,3-dihydroxy-propyl)-6-[2-(3-méthoxy-quinoléin-5-yl)-éthyl]-tétrahydro-pyran-3-yl}-acrylamide ;
- {(2*R*,3*R*,6*R*)-2-((2*R*)-2,3-dihydroxy-propyl)-6-[2-(3-méthoxy-quinoléin-5-yl)-éthyl]-tétrahydro-pyran-3-yl}-amide de l'acide 3-oxo-3,4-dihydro-2*H*-pyrido[3,2-*b*][1,4]thiazine-6-carboxylique ;
- {(2*R*,3*R*,6*R*)-2-((2*S*)-2,3-dihydroxy-propyl)-6-[2-(3-méthoxy-quinoléin-5-yl)-éthyl]-tétrahydro-pyran-3-yl}-amide de l'acide 3-oxo-3,4-dihydro-2*H*-pyrido[3,2-*b*][1,4]thiazine-6-carboxylique ;
- 6-({(2*R*,3*R*,6*R*)-2-((2*R*)-2,3-dihydroxy-propyl)-6-[2-(3-méthoxy-quinoléin-5-yl)-éthyl]-tétrahydro-pyran-3-ylamino}-méthyl)-4*H*-pyrido[3,2-*b*][1,4]thiazin-3-one ;
- 6-({(2*R*,3*R*,6*R*)-2-((2*S*)-2,3-dihydroxy-propyl)-6-[2-(3-méthoxy-quinoléin-5-yl)-éthyl]-tétrahydro-pyran-3-ylamino}-méthyl)-4*H*-pyrido[3,2-*b*][1,4]thiazin-3-one ;
- 6-({(2*R*,3*R*,6*R*)-2-((2*R*)-2,3-dihydroxy-propyl)-6-[2-(6-méthoxy-[1,5]naphtyridin-4-yl)-éthyl]-tétrahydro-pyran-3-ylamino}-méthyl)-4*H*-pyrido[3,2-*b*][1,4]thiazin-3-one ;
- 6-({(2*R*,3*R*,6*R*)-2-((2*S*)-2,3-dihydroxy-propyl)-6-[2-(6-méthoxy-[1,5]naphtyridin-4-yl)-éthyl]-tétrahydro-pyran-3-ylamino}-méthyl)-4*H*-pyrido[3,2-*b*][1,4]thiazin-3-one ;
- (2*R*)-3-{(2*R*,3*R*,6*R*)-3-[(*E*)-3-(2,5-difluoro-phényl)-allylamino]-6-[2-(6-méthoxy-[1,5]naphtyridin-4-yl)-éthyl]-tétrahydro-pyran-2-yl}-propane-1,2-diol ;
- (2*S*)-3-{(2*R*,3*R*,6*R*)-3-[(*E*)-3-(2,5-difluoro-phényl)-allylamino]-6-[2-(6-méthoxy-[1,5]naphtyridin-4-yl)-éthyl]-tétrahydro-pyran-2-yl}-propane-1,2-diol ;
- 2-{(2*R*,3*R*,6*R*)-3-[(*E*)-3-(2,5-difluoro-phényl)-allylamino]-6-[2-(6-méthoxy-[1,5]naphtyridin-4-yl)-éthyl]-tétrahydro-pyran-2-yl}-*N*-hydroxy-acétamide ;
- (*E*)-3-(2,5-difluoro-phényl)-*N*-{(2*R*,3*R*,6*R*)-2-((2*R*)-2,3-dihydroxy-propyl)-6-[2-(6-méthoxy-[1,5]naphtyridin-4-yl)-éthyl]-tétrahydro-pyran-3-yl}-acrylamide ;
- (*E*)-3-(2,5-difluoro-phényl)-*N*-{(2*R*,3*R*,6*R*)-2-((2*S*)-2,3-dihydroxy-propyl)-6-[2-(6-méthoxy-[1,5]naphtyridin-4-yl)-éthyl]-tétrahydro-pyran-3-yl}-acrylamide ;
- (2*R*)-3-{(2*R*,3*R*,6*S*)-3-[(*E*)-3-(2,5-difluoro-phényl)-allylamino]-6-[(*S*)-1-hydroxy-2-(6-méthoxy-[1,5]naphtyridin-4-yl)-éthyl]-tétrahydro-pyran-2-yl}-propane-1,2-diol ;
- (2*S*)-3-{(2*R*,3*R*,6*S*)-3-[(*E*)-3-(2,5-difluoro-phényl)-allylamino]-6-[(*S*)-1-hydroxy-2-(6-méthoxy-[1,5]naphtyridin-4-yl)-éthyl]-tétrahydro-pyran-2-yl}-propane-1,2-diol ;
- (2*R*)-3-{(2*R*,3*R*,6*R*)-3-[(*E*)-3-(2,5-difluoro-phényl)-allylamino]-6-[2-(6-méthoxyquinoléin-4-yl)-éthyl]-tétrahydro-pyran-2-yl}-propane-1,2-diol ;
- (2*S*)-3-{(2*R*,3*R*,6*R*)-3-[(*E*)-3-(2,5-difluoro-phényl)-allylamino]-6-[2-(6-méthoxyquinoléin-4-yl)-éthyl]-tétrahydro-pyran-2-yl}-propane-1,2-diol ;
- {(2*R*,3*R*,6*R*)-3-[(*E*)-3-(2,5-difluoro-phényl)-allylamino]-6-[2-(6-méthoxy-quinoléin-4-yl)-éthyl]-tétrahydro-pyran-2-yl}-acétonitrile ;
- (*E*)-3-(2,5-difluoro-phényl)-*N*-{(2*R*,3*R*,6*S*)-2-((2R)-2,3-dihydroxy-propyl)-6-[(*E*)-2-(3-fluoro-6-méthoxy-[1,5]naphtyridin-4-yl)-vinyl]-tétrahydro-pyran-3-yl}-acrylamide ;
- (*E*)-3-(2,5-difluoro-phényl)-*N*-{(2*R*,3*R*,6*S*)-2-((2*S*)-2,3-dihydroxy-propyl)-6-[(*E*)-2-(3-fluoro-6-méthoxy-[1,5]naphtyridin-4-yl)-vinyl]-tétrahydro-pyran-3-yl}-acrylamide ;
- (2*R*)-3-{(2*R*,3*R*,6*S*)-3-[(*E*)-3-(2,5-difluoro-phényl)-allylamino]-6-[(*E*)-2-(3-fluoro-6-méthoxy-[1,5]naphtyridin-4-yl)-vinyl]-tétrahydro-pyran-2-yl}-propane-1,2-diol ;
- (2*S*)-3-{(2*R*,3*R*,6*S*)-3-[(*E*)-3-(2,5-difluoro-phényl)-allylamino]-6-[(*E*)-2-(3-fluoro-6-méthoxy-[1,5]naphtyridin-4-yl)-vinyl]-tétrahydro-pyran-2-yl}-propane-1,2-diol ;
- (*E*)-3-(2,5-difluoro-phényl)-*N*-[(2*R*,3*R*,6*S*)-2-((2*R*)-2,3-dihydroxy-propyl)-6-(6-méthoxyquinoléin-4-yloxyméthyl)-tétrahydro-pyran-3-yl]-acrylamide ;
- (*E*)-3-(2,5-difluoro-phényl)-*N*-[(2*R*,3*R*,6*S*)-2-((2*S*)-2,3-dihydroxy-propyl)-6-(6-méthoxyquinoléin-4-yloxyméthyl)-tétrahydro-pyran-3-yl]-acrylamide ;
ou sel de l'un de ces composés.

13. *E*n tant que médicament, composé de formule I tel que défini à la revendication 1 ou sel pharmaceutiquement acceptable de celui-ci.

14. Composition pharmaceutique contenant, comme principe actif, un composé de formule I tel que défini à la revendication 1 ou un sel pharmaceutiquement acceptable de celui-ci et au moins un excipient thérapeutiquement inerte.

15. Utilisation d'un composé de formule I tel que défini à la revendication 1 ou d'un sel pharmaceutiquement acceptable de celui-ci dans la fabrication d'un médicament indiqué dans la prévention ou le traitement d'une infection bactérienne.
